# EUROPEAN PATENT SPECIFICATION

(11) **EP 4 540 241 B1**
(45) Date of publication and mention of the grant of the patent: **25.11.2026**
(21) Application number: 23748629.5
(22) Date of filing: 16.06.2023
(51) Int. Cl.: C07D 401/14, C07D 405/14, A61P 35/00, A61K 31/454

(54) **SUBSTITUTED PIPERIDINES AS CK1A DEGRADERS**
SUBSTITUIERTE PIPERIDINE ALS CK1A DEGRADERS
PIPÉRIDINES SUBSTITUÉES COMME DÉGRADEURS DE CK1A

(30) Priority: 16.06.2022 US 202263352860 P
(43) Date of publication of application: 23.04.2025
(73) Proprietor: Monte Rosa Therapeutics AG, 4057 Basel (CH)
(72) Inventor: MCALLISTER, Laura Ann, 4057 Basel (CH); WIEDMER, Lars, 4057 Basel (CH); DONCKELE, Ètienne, 4057 Basel (CH); FASCHING, Bernhard, 4057 Basel (CH); ILIC-WIDLUND, Nina, 4057 Basel (CH)
(74) Representative: Carpmaels & Ransford LLP
(86) International application number: PCT/US2023/025591
(87) International publication number: WO 2023/244817

(56) References cited:
- WO-A1-2019/241274
- WO-A1-2020/102195
- WO-A1-2022/081925

## Description

This application claims priority to U.S. Provisional Patent Application No. 63/352,860, filed on June 16, 2022.

### BACKGROUND

The ubiquitin proteasome system can be manipulated with different small molecules to trigger targeted degradation of specific proteins of interest. Promoting the targeted degradation of pathogenic proteins using small molecule degraders is emerging as a new modality in the treatment of diseases. One such modality relies on redirecting the activity of E3 ligases such as cereblon (a phenomenon known as E3 reprogramming) using low molecular weight compounds, which have been termed molecular glues to promote the poly-ubiquitination and ultimately proteasomal degradation of new protein substrates involved in the development of diseases. The molecular glues bind to both the E3 ligase and the target protein, thereby mediating an alteration of the ligase surface and enabling an interaction with the target protein.

WO 2019/241274 A1 relates to *"Compounds with amino amide linkers and pharmaceutical compositions and medicaments comprising such compounds".* WO 2020/102195 A1 relates to *"Substituted isoindolinones of Formula (I), methods of making such compounds, pharmaceutical compositions and medicaments comprising such compounds, and methods of using such compounds to treat or ameliorate diseases, disorders, or conditions associated with protein malfunction, such as cancer".* WO 2022/081925 A1 relates to *"Tricyclic compounds that degrade IKZF2 and*/*or IKZF4".*

There exists a need for therapeutics that effectively mediate the degradation of certain proteins for the treatment of diseases.

### SUMMARY

Described herein, in part, are compounds contemplated as modulators of cereblon to mediate the degradation of a protein, and are therefore are useful in the treatment of disorders, such as cancer. For example, it has been found that compounds of the present disclosure mediate the targeted degradation of the protein casein kinase 1α (CK1α). Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

In one aspect, described herein is a compound of Formula (I): 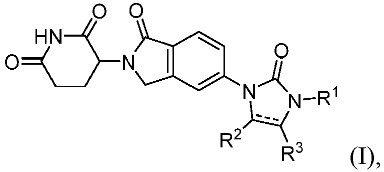 or a pharmaceutically acceptable salt thereof, wherein ------ denotes a single bond or a double bond; and R¹ is selected from the group consisting of 5-6 membered monocyclic aryl, 5-6 membered heteroaryl, and 8-10 bicyclic heteroaryl, wherein the aryl or heteroaryl is optionally substituted by one, two or three substituents each independently selected from halogen, cyano, hydroxyl, C₁₋₆ alkoxy and C₁₋₆ alkyl; and each of R² and R³ is independently selected from the group consisting of H, -CN, C₁₋₆ alkoxy, C₁₋₆ alkyl, and C₃₋₁₀ cycloalkyl, wherein the alkoxy, alkyl, or cycloalkyl is optionally substituted by one or more halogen; or R² and R³ together with the atoms to which they are attached to form a C₃₋₁₀ cycloalkyl, wherein the cycloalkyl is optionally substituted by one or more halogen.

### BRIEF DESCRIPTION OF THE DRAWINGS

**FIG. 1** depicts effects of compound 101 on CK1α levels in an HEK293 cell line in comparison to effects of compound 101 on CK1α levels in a cereblon (CRBN) knock-out HEK293 cell line. The data show that compound 101 decreases CK1α levels in the HEK293 cell line while in the presence of cereblon, whereas compound 101 demonstrates no effect on CK1α levels in the absence of cereblon in the cereblon knock-out HEK293 cell line.
**FIG. 2** depicts time-dependent plasma concentrations for Mouse oral PK at 10 mg/kg for compound 107, and compound 125.

### DETAILED DESCRIPTION

The features and other details of the disclosure will now be more particularly described. Certain terms employed in the specification, examples and appended claims are collected here. These definitions should be read in light of the remainder of the disclosure and as understood by a person of skill in the art. Unless defined otherwise, all technical and scientific terms used herein have the same meaning as commonly understood by a person of ordinary skill in the art.

### Compounds

In one aspect, described herein is a compound of Formula (I): 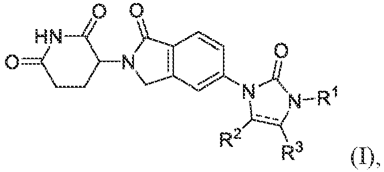 or a pharmaceutically acceptable salt thereof, wherein - - - - - - denotes a single bond or a double bond; and R¹ is selected from the group consisting of 5-6 membered monocyclic aryl, 5-6 membered heteroaryl, and 8-10 bicyclic heteroaryl, wherein the aryl or heteroaryl is optionally substituted by one, two or three substituents each independently selected from halogen, cyano, hydroxyl, C₁₋₆ alkoxy and C₁₋₆ alkyl; and each of R² and R³ is independently selected from the group consisting of H, -CN, C₁₋₆ alkoxy, C₁₋₆ alkyl, and C₃₋₁₀ cycloalkyl, wherein the alkoxy, alkyl, or cycloalkyl is optionally substituted by one or more halogen; or R² and R³ together with the atoms to which they are attached to form a C₃₋₁₀ cycloalkyl, wherein the cycloalkyl is optionally substituted by one or more halogen.

In one aspect, described herein is a compound of Formula (1): 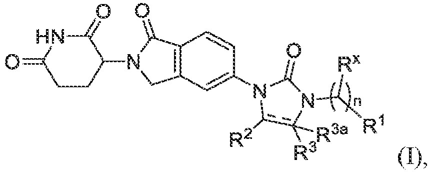 or a pharmaceutically acceptable salt thereof, wherein - - - - - - denotes a single bond or a double bond; and R¹ is selected from the group consisting of 5-6 membered monocyclic aryl, 5-6 membered heteroaryl, 8-10 bicyclic heteroaryl, 8-10 membered bicyclic heterocyclyl, 7-8 membered spirocyclic bicyclic heterocyclyl, C₃₋₁₀ cycloalkyl, 4-6 membered heterocyclyl, 6-8 membered bicyclic cycloalkyl, and 7-8 membered spirocyclic cycloalkyl, wherein the aryl, heteroaryl, heterocyclyl, and cycloalkyl is optionally substituted by one, two or three substituents each independently selected from halogen, cyano, hydroxyl, C₁₋₆ alkoxy, C₁₋₆ alkyl, C₁₋₆ haloalkyl, C₃₋₁₀ cycloalkyl, 4-6 membered heterocyclyl, and -C(O)-(C₁₋₆ alkyl), wherein the alkyl is optionally substituted by one or more C₁₋₆ alkoxy; and the cycloalkyl or heterocyclyl is optionally substituted by one, two or three substituents each independently selected from C₁₋₆ alkyl, C₁₋₆ haloalkyl, phenyl, and pyridinyl; and each of R² and R³ is independently selected from the group consisting of H, -CN, C₁₋₆ alkoxy, C₁₋₆ alkyl, and C₃₋₁₀ cycloalkyl, wherein the alkoxy, or cycloalkyl is optionally substituted by one or more halogen, and alkyl is optionally substituted by one or more halogen or C₁₋₆ alkoxy; or R² and R³ together with the atoms to which they are attached to form a C₃₋₁₀ cycloalkyl, wherein the cycloalkyl is optionally substituted by one or more halogen; R^{3a} is hydrogen, or R³ and R^{3a}, together with the atoms to which they are attached to, form a C₃₋₁₀ cycloalkyl; and R^{3a} is absent when - - - - - - is a double bond; R^{x} is hydrogen, C₁₋₆ alkyl, or C₁₋₆ haloalkyl; and n is 0 or 1.

In some embodiments, - - - - - - is a single bond. In some embodiments,is a double bond.

In some embodiments, R², R³, and R^{3a} are hydrogen. In some embodiments, R² is hydrogen. In some embodiments, R³ is hydrogen. In some embodiments, R^{3a} is hydrogen.

In some embodiments, R² and R^{3a} are hydrogen, and R³ is C₁₋₆ alkyl or C₁₋₆ haloalkyl, wherein the alkyl is optionally substituted with C₁₋₆ alkoxy. In some embodiments, R3 is C₁₋₆ alkyl. In some embodiments, R³ is C₁₋₆ haloalkyl. In some embodiments, R³ is - CH₂O(CH₃), -CH₃, or -CF₃. . In some embodiments, R³ is hydrogen.

In some embodiments, R² is C₁₋₆ alkyl, and R³ and R^{3a} are hydrogen. In some embodiments, R² is C₁₋₆ alkyl. In some embodiments, R² is -CH₃.

In some embodiments, the compound of Formula I is represented by Formula I-A:

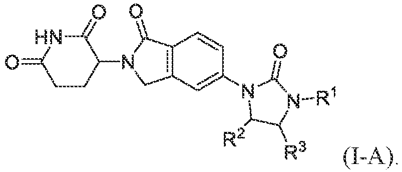

In some embodiments, the compound of Formula I is represented by Formula I-B:

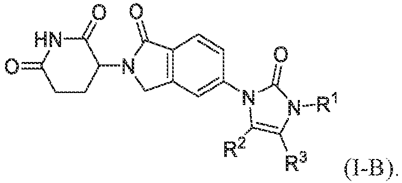

In some embodiments, R² is H and R³ is H. In some embodiments, R² is H and R³ is C₁₋₆ alkyl or C₁₋₆ haloalkyl, wherein the alkyl is optionally substituted with C₁₋₆ alkoxy. In some embodiments, R² is C₁₋₆ alkyl and R³ C₁₋₆ alkyl.

In some embodiments, the compound of Formula I is represented by Formula I-AB:

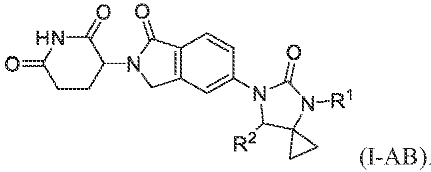

In some embodiments, R² is H.

In some embodiments, R¹ is unsubstituted or substituted 5-6 membered monocyclic aryl. In some embodiments, R¹ is an unsubstituted 5-6 membered monocyclic aryl. In some embodiments, R¹ is a substituted 5-6 membered monocyclic aryl.

In some embodiments, R¹ is 5-6 membered monocyclic aryl, wherein the aryl is optionally substituted by one, two or three substituents each independently selected from halogen, cyano, hydroxyl, C₁₋₆ alkoxy, C₁₋₆ alkyl, C₁₋₆ haloalkyl, C₃₋₁₀ cycloalkyl, 4-6 membered heterocyclyl, and -C(O)-(C₁₋₆ alkyl), wherein the alkyl is optionally substituted by one or more (e.g., one to three) C₁₋₆ alkoxy; and the cycloalkyl or heterocyclyl is optionally substituted by one, two or three substituents each independently selected from C₁₋₆ alkyl, C₁₋₆ haloalkyl, phenyl, and pyridinyl.

In some embodiments, R¹ is an unsubstituted phenyl. In some embodiments, R¹ is a substituted phenyl. In some embodiments, R¹ is phenyl optionally substituted by one, two or three substituents each independently selected from -OCH₃, -CH₃, -CH(CH₃)₂, -CF₃, - Cl, -CN, -CHF₂, -CH₂OCH₃, -CH(CH₃)OCH₃, -C(O)CH(CH₃)₂,

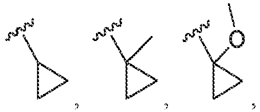

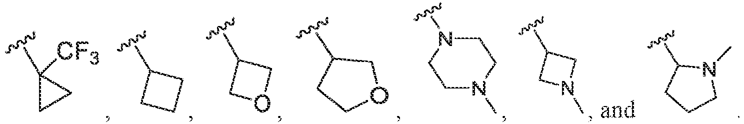

In some embodiments, R¹ is unsubstituted or substituted 5-6 membered heteroaryl. In some embodiments, R¹ is an unsubstituted 5-6 membered heteroaryl. In some embodiments, R¹ is a substituted 5-6 membered heteroaryl.

In some embodiments, R¹ is a 5-6 membered heteroaryl, wherein the heteroaryl is optionally substituted by one, two or three substituents each independently selected from halogen, cyano, hydroxyl, C₁₋₆ alkoxy, C₁₋₆ alkyl, C₁₋₆ haloalkyl, C₃₋₁₀ cycloalkyl, 4-6 membered heterocyclyl, and -C(O)-(C₁₋₆ alkyl), wherein the alkyl is optionally substituted by one or more (e.g., one to three) C₁₋₆ alkoxy, and the cycloalkyl or heterocyclyl is optionally substituted by one, two or three substituents each independently selected from C₁₋₆ alkyl, C₁₋₆ haloalkyl, phenyl, and pyridinyl.

In some embodiments, R¹ is optionally susbistuted pyridinyl or pyrimidinyl. In some embodiments, R¹ is pyridinyl. In some embodiments, R¹ is pyrimidinyl. In some embodiments, R¹ is pyridinyl optionally substituted by one, two or three substituents each independently selected from F, Cl, Br, -CN, -CH₃, -CH₂CH₃, -CF₃, -OCH₂CH₃,

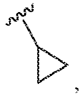

In some embodiments, R¹ is substituted by one, two or three substituents each independently selected from F, Cl, Br, -CN, -CH₃, -CH₂CH₃, -CF₃, -OCH₂CH₃, and 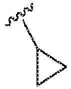 In some embodiments, R¹ is substituted by one, two or three substituents each independently selected from -OCH₃, -CH₃, -CH(CH₃)₂, -CF₃, -Cl, -CN, -CHF₂, -CH₂OCH₃, - CH(CH₃)OCH₃, -C(O)CH(CH₃)₂,

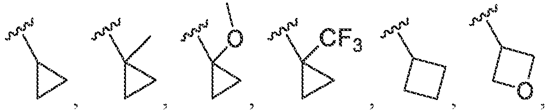

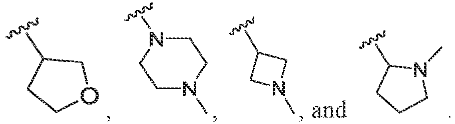

In some embodiments, R¹ is unsubstituted or substituted 8-10 bicyclic heterocyclyl or 7-8 membered spirocyclic bicyclic heterocyclyl. In some embodiments, R¹ is an unsubstituted 8-10 bicyclic heterocyclyl. In some embodiments, R¹ is a substituted 8-10 bicyclic heterocyclyl. In some embodiments, R¹ is an unsubstituted 7-8 membered spirocyclic bicyclic heterocyclyl. In some embodiments, R¹ is a substituted 7-8 membered spirocyclic bicyclic heterocyclyl. In some embodiments, R¹ is dihydrobenzofuranyl, isoindolinyl, benzoimidazolyl, or 2-oxaspiroheptanyl.

In some embodiments, R¹ is unsubstituted or substituted 8-10 bicyclic heteroaryl. In some embodiments, R¹ is an unsubstituted 8-10 bicyclic heteroaryl. In some embodiments, R¹ is a substituted 8-10 bicyclic heteroaryl.

In some embodiments, R¹ is unsubstituted or substituted 4-6 membered heterocyclyl. In some embodiments, R¹ is an unsubstituted 4-6 membered heterocyclyl. In some embodiments, R¹ is a substituted 4-6 membered heterocyclyl. In some embodiments, R¹ is oxetanyl, tetrahydrofuranyl, or tetrahydropyranyl.

In some embodiments, R¹ is unsubstituted or substituted 6-8 membered bicyclic cycloalkyl or 7-8 membered spirocyclic cycloalkyl. In some embodiments, R¹ is

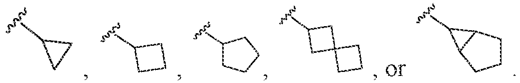

In some embodiments, n is 1 and R^{x} is C₁₋₆ alkyl or C₁₋₆ haloalkyl, wherein the alkyl is optionally substituted with one or more (e.g., one to three) halogen. In some embodiments, n is 1 and R^{x} is CF₃.

In some embodiments, n is 1. In some embodiments, n is 0.

Provided herein, in an aspect, is a compound described in Table 1 below. Table 1 also includes the compound number of each compound in accordance with the contents of the present specification.

In some embodiments, the compound is a compound identified in Table 1 below or a pharmaceutically acceptable salt thereof.

**Table 1. Exemplary compounds.**

| **Cpmd No.** | **Structure** |
|---|---|
| 101 | 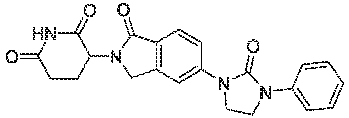 |
| 102 | 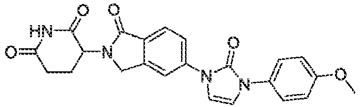 |
| 103 | 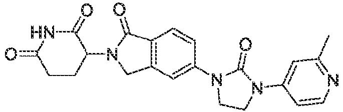 |
| 104 | 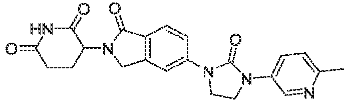 |
| 105 | 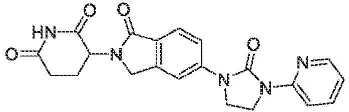 |
| 106 | 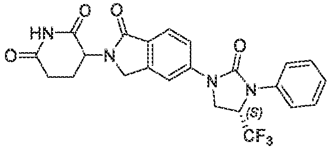 |
| 107 | 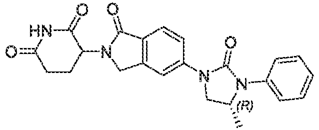 |
| 108 | 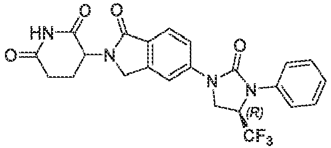 |
| 109 | 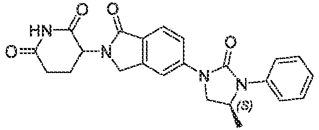 |
| 110 | 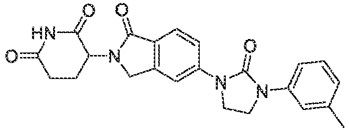 |
| 111 | 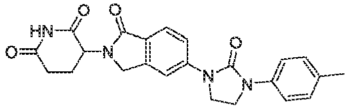 |
| 112 | 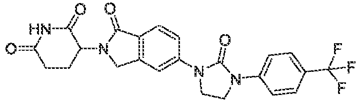 |
| 113 | 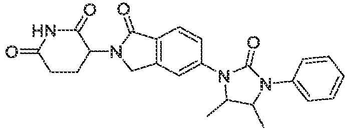 |
| 114 | 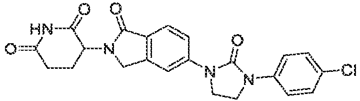 |
| 115 | 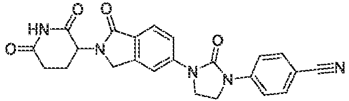 |
| 116 | 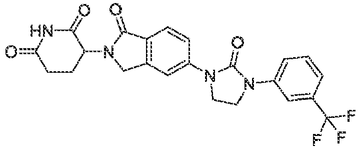 |
| 117 | 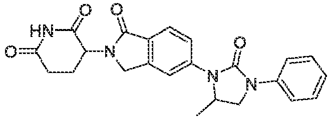 |
| 118 | 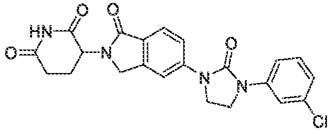 |
| 119 | 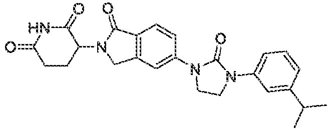 |
| 120 | 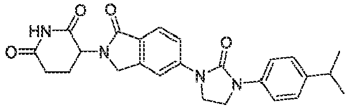 |
| 121 | 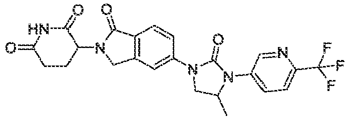 |
| 122 | 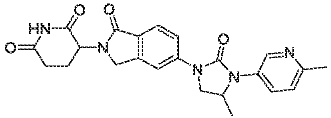 |
| 123 | 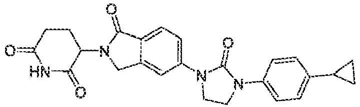 |
| 124 | 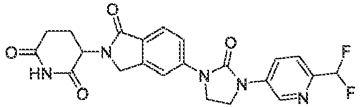 |
| 125 | 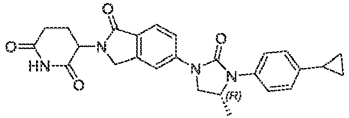 |
| 126 | 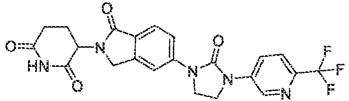 |
| 127 | 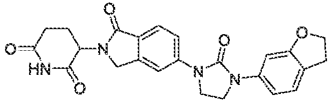 |
| 128 | 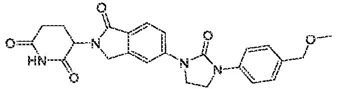 |
| 129 | 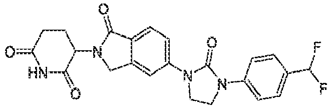 |
| 130 | 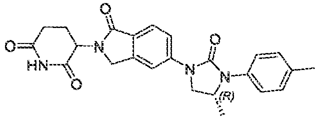 |
| 131 | 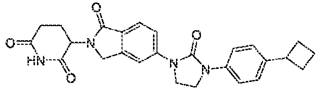 |
| 132 | 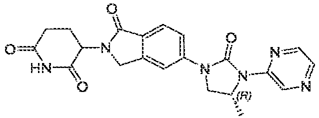 |
| 133 | 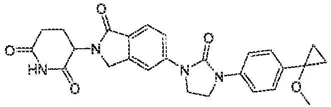 |
| 134 | 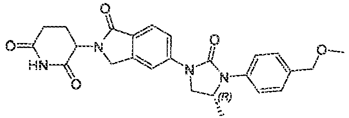 |
| 135 | 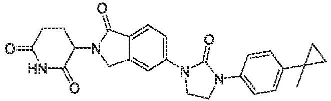 |
| 136 | 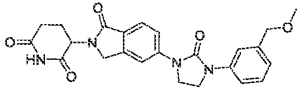 |
| 137 | 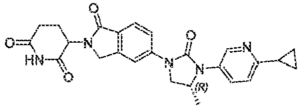 |
| 138 | 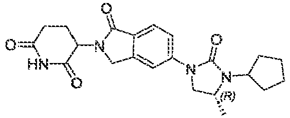 |
| 139 | 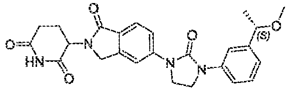 |
| 140 | 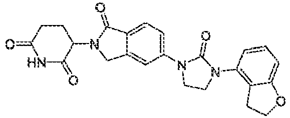 |
| 141 | 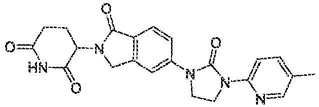 |
| 142 | 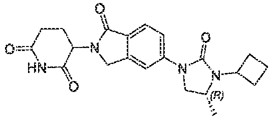 |
| 143 | 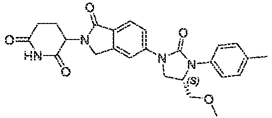 |
| 144 | 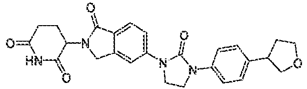 |
| 145 | 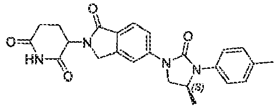 |
| 146 | 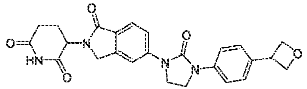 |
| 147 | 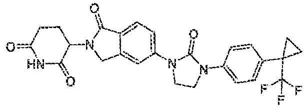 |
| 148 | 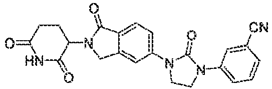 |
| 149 | 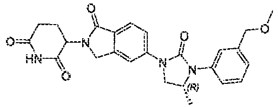 |
| 150 | 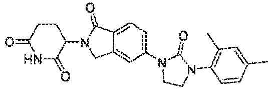 |
| 151 | 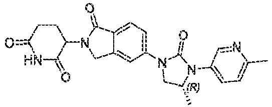 |
| 152 | 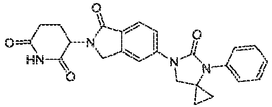 |
| 153 | 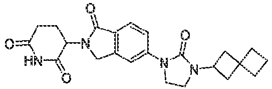 |
| 154 | 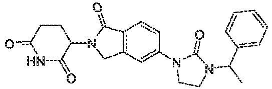 |
| 155 | 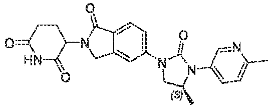 |
| 156 | 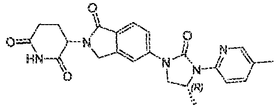 |
| 157 | 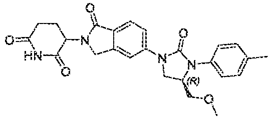 |
| 158 | 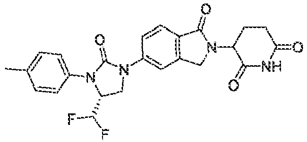 |
| 159 | 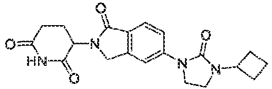 |
| 160 | 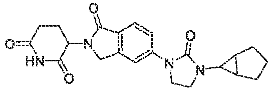 |
| 161 | 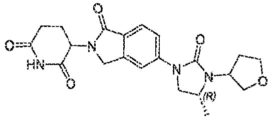 |
| 162 | 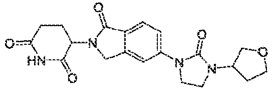 |
| 163 | 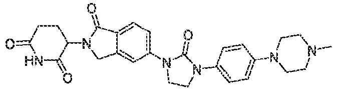 |
| 164 | 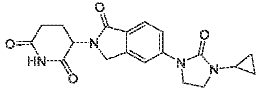 |
| 165 | 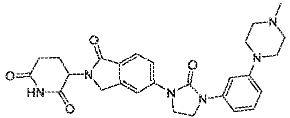 |
| 166 | 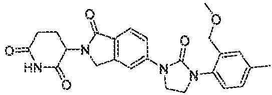 |
| 167 | 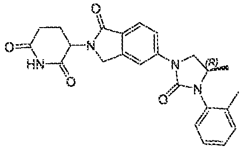 |
| 168 | 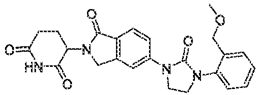 |
| 169 | 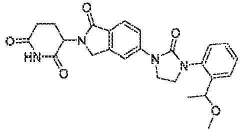 |
| 170 | 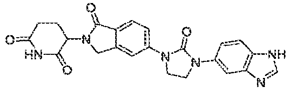 |
| 171 | 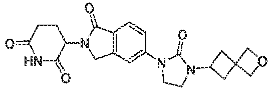 |
| 172 | 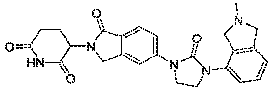 |
| 173 | 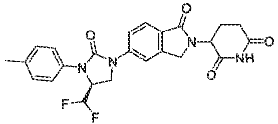 |
| 174 | 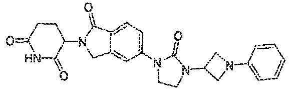 |
| 175 | 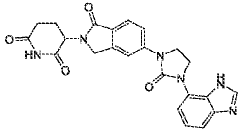 |
| 176 | 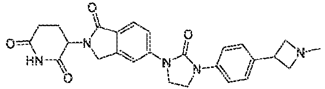 |
| 177 | 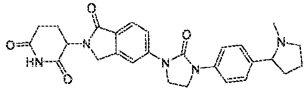 |
| 178 | 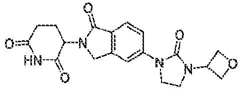 |
| 179 | 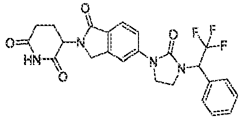 |
| 180 | 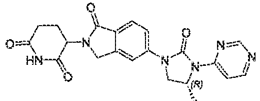 |
| 181 | 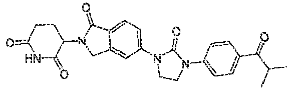 |
| 182 | 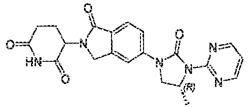 |
| 183 | 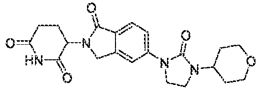 |
| 184 | 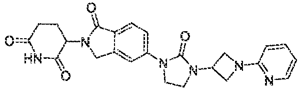 |

### Pharmaceutical Compositions

In another embodiment, the present disclosure provides a pharmaceutical composition comprising a compound described herein, or pharmaceutically acceptable salt thereof, and a pharmaceutically acceptable excipient. In certain embodiments, the pharmaceutical composition comprises an effective amount of the compound. In certain embodiments, the pharmaceutical composition comprises a therapeutically effective amount of the compound.

The pharmaceutical compositions provided herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration.

Compositions for oral administration can take the form of bulk liquid solutions or suspensions, or bulk powders. In some embodiments, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component with the remainder being various vehicles or excipients and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope of the disclosure provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

### Methods of Treatment and Uses

Furthermore, the compounds and pharmaceutical compositions described herein are contemplated as useful in the treatment or prevention of disorders in subjects in need thereof. Compounds described herein, in one embodiment, are used to degrade casein kinase 1α (CK1α) for the treatment of prevention of a disorder.

Casein kinase I (CK1) is a monomeric serine-threonine protein kinase with 7 isoforms: alpha, beta, gamma1, gamma2, gamma3, delta and epsilon. CK1 is involved in many cellular processes including DNA repair, cell division, nuclear localization and membrane transport. Isoforms are also integral to development. CK1α (casein kinase 1 alpha 1) is a protein coding gene that enables protein serine/threonine kinase activity involving in several processes, including negative regulation of canonical Wnt signaling pathway; peptidyl-serine phosphorylation; and positive regulation of proteasomal ubiquitin-dependent protein catabolic process. Through phosphorylation of different substrate proteins, CK1α is able to activate, stabilize, inactivate, or destabilize the functions of these substrate proteins, thus regulating their functions.

In one embodiment of the disclosure, a compound, or pharmaceutically acceptable salt thereof, or pharmaceutical composition described herein is administered to a subject to degrade CK1α in the subject.

In one aspect of the disclosure, described herein is a method of treating or preventing a disorder in a subject in need thereof, comprising administering to the subject an effective amount of a compound, or pharmaceutically acceptable salt thereof, or pharmaceutical composition described herein.

In another aspect, described herein is a method of degrading CK1α in a subject suffering from a disorder, comprising administering to the subject a therapeutically effective amount of a compound described herein, or pharmaceutically acceptable salt thereof, or pharmaceutical composition described herein. In some embodiments, the compound binds to cereblon and a CK1α protein to induce ubiquitination and subsequent proteasomal degradation of the CK1α.

In certain embodiments, the compounds provided herein are degraders of a casein kinase 1. In certain embodiments, the compounds provided herein are degraders of casein kinase 1α (CK1α). In certain embodiments, the compounds provided herein are selective degraders of casein kinase 1α (CK1α). In certain embodiments, the compounds provided herein are degraders of human casein kinase 1α (CK1α). In certain embodiments, the compounds provided herein are selective degraders of human casein kinase 1α (CK1α).

Exemplary disorders that can be treated or prevented by the methods of the present disclosure include but are not limited to, cancer of the bladder, bone, brain, breast, cervix, chest, colon, endrometrium, esophagus, eye, head, kidney, liver, lymph nodes, lung, upper aerodigestive tract (including nasal cavity and paranasal sinuses, nasopharynx or cavum, oral cavity, oropharynx, larynx, hypopharynx and salivary glands, neck, ovaries, pancreas, prostate, rectum, skin, stomach, testis, throat, or uterus. Other exemplary disorders include, but are not limited to, amyloidosis, neuroblastoma, meningioma, hemangiopericytoma, multiple brain metastase, glioblastoma multiforms, glioblastoma, brain stem glioma, poor prognosis malignant brain tumor, malignant glioma, recurrent malignant glioma, anaplastic astrocytoma, anaplastic oligodendroglioma, neuroendocrine tumor, e.g., neuroendocrine prostate cancer such as castration-resistant neuroendocrine prostate cancer (NEPC) and lung neuroendocrine tumors (Lu-NETs), rectal adenocarcinoma, colorectal cancer, including stage 3 and stage 4 colorectal cancer, unresectable colorectal carcinoma, metastatic hepatocellular carcinoma, Kaposi's sarcoma, malignant melanoma, malignant mesothelioma, malignant pleural effusion mesothelioma syndrome, peritoneal carcinoma, papillary serous carcinoma, gynecologic sarcoma, soft tissue sarcoma, scleroderma, cutaneous vasculitis, Langerhans cell histiocytosis, non-Langerhans cell histiocytosis, leiomyosarcoma, fibrodysplasia ossificans progressive, hormone refractory prostate cancer, resected high-risk soft tissue sarcoma, unrescectable hepatocellular carcinoma, fallopian tube cancer, androgen independent prostate cancer, androgen dependent stage IV non-metastatic prostate cancer, hormone-insensitive prostate cancer, chemotherapy -insensitive prostate cancer, papillary thyroid carcinoma, follicular thyroid carcinoma, medullary thyroid carcinoma, and leiomyoma; and blood bourne (liquid) or hematological cancers, including but not limited to leukemias, lymphomas, and myelomas, such as diffuse large B-cell lymphoma (DLBCL), B-cell immunoblastic lymphoma, small non-cleaved cell lymphoma, human lymphotropic virus-type 1 (HTLV-1) leukemia/lymphoma, adult T-cell lymphoma, peripheral T-cell lymphoma (PTCL), cutaneous T-cell lymphoma (CTCL), mantle cell lymphoma (MCL), Hodgkin's lymphoma (HL), non-Hodgkin's lymphoma (NHL), AIDS-related lymphoma, follicular lymphoma, small lymphocytic lymphoma, T-cell/histiocyte rich large B-cell lymphoma, transformed lymphoma, primary mediastitial (thymic) large B-cell lymphoma, splenic marginal zone lymphoma, Richter's transformation, nodal marginal zone lymphoma, ALK-positive large B-cell lymphoma, indolent lymphoma (for example, DLBCL, follicular lymphoma, or marginal zone lymphoma), acute myelogenous leukemia (AML), acute lymphocytic leukemia (ALL), adult T-cell leukemia, chronic lymphocytic leukemia (CLL), small lymphocytic lymphoma (SLL), hairy cell leukemia, myelodysplasia, myeloproliferative disorders, chronic myelogenous leukemia (CML), acute monocytic leukemia (AMoL), myelodysplastic syndrome (MDS), human lymphotropic virus- type 1 (HTLV-1) leukemia, mastocytosis, B-cell acute lymphoblastic leukemia, Non-Hodgkin's Lymphoma, Hodgkin's Lymphoma, and multiple myeloma (MM).

In another aspect of the disclosure, described herein is a method of treating cancer (e.g., a cancer described herein) in a subject in need thereof, comprising administering to the subject a therapeutically effective amount of a compound described herein, or pharmaceutically acceptable salt thereof, or pharmaceutical composition described herein.

In another aspect, described herein is a method of degrading CK1a in a subject suffering from cancer (e.g., a cancer described herein), comprising administering to the subject a therapeutically effective amount of a compound described herein, or pharmaceutically acceptable salt thereof, or pharmaceutical composition described herein.

In another aspect, described herein is a method of treating a solid tumor (e.g., a solid tumor described herein) in a subject in need thereof, comprising administering to the subject a therapeutically effective amount of a compound described herein, or pharmaceutically acceptable salt thereof, or pharmaceutical composition described herein.

### Definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

When a range of values is listed, it is intended to encompass each value and sub-range within the range. For example "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The term "alkyl" as used herein refers to a radical of a straight-chain or branched saturated hydrocarbon group. In some embodiments, an alkyl group has 1 to 12 carbon atoms ("C₁₋₁₂ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl", also referred to herein as "lower alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Common alkyl abbreviations include Me (-CH₃), Et (-CH₂CH₃), iPr (-CH(CH₃)₂), nPr (-CH₂CH₂CH₃), n-Bu (-CH₂CH₂CH₂CH₃), or i-Bu (-CH₂CH(CH₃)₂).

The term "alkenyl" as used herein refers to a radical of a straight-chain or branched hydrocarbon group having , one or more carbon-carbon double bonds. In some embodiments, an alkenyl group has 2 to 10 carbon atoms ("C₂₋₁₀ alkenyl"). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms (''C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like.

The term "alkynyl" as used herein refers to a radical of a straight-chain or branched hydrocarbon group having one or more carbon-carbon triple bonds (*e.g*., *1,* 2, 3, or 4 carbon-carbon triple bonds). In some embodiments, an alkynyl group has 2 to 10 carbon atoms ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like.

The term "cycloalkyl" as used herein refers to a radical of a saturated or partially unsaturated cyclic hydrocarbon group having from 3 to 12 ring carbon atoms ("C₃₋₁₂ cycloalkyl") and zero heteroatoms in the ring system. In some embodiments, a cycloalkyl group has 3 to 10 ring carbon atoms ("C₃₋₁₀ cycloalkyl"). In some embodiments, a cycloalkyl group has 3 to 8 ring carbon atoms ("C₃₋₈ cycloalkyl"). In some embodiments, a cycloalkyl group has 3 to 6 ring carbon atoms ("C₃₋₆ cycloalkyl"). In some embodiments, a cycloalkyl group has 3 to 6 ring carbon atoms ("C₃₋₆ cycloalkyl"). In some embodiments, a cycloalkyl group has 5 to 10 ring carbon atoms ("C₅₋₁₀ cycloalkyl"). Exemplary C₃₋₆ cycloalkyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ cycloalkyl groups include, without limitation, the aforementioned C₃₋₆ cycloalkyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ cycloalkyl groups include, without limitation, the aforementioned C₃₋₈ cycloalkyl groups as well as cyclononyl (C₉), cyclononenyl (C₉), cyclodecyl (C₁₀), cyclodecenyl (C₁₀), octahydro-1*H*-indenyl (C₉), decahydronaphthalenyl (C₁₀), spiro[4.5]decanyl (C₁₀), and the like. As the foregoing examples illustrate, in certain embodiments, the cycloalkyl group is either monocyclic ("monocyclic cycloalkyl") or contain a fused, bridged or spiro ring system such as a bicyclic system ("bicyclic cycloalkyl"). "Cycloalkyl" also includes ring systems wherein the cycloalkyl ring, as defined above, is fused with one or more aryl or heteroaryl groups wherein the point of attachment is on the cycloalkyl ring or the one or more aryl or heteroaryl groups, and in such instances, the number of carbons continue to designate the number of carbons in the cycloalkyl ring system.

The term "heterocyclyl" as used herein refers to a radical of a saturated or partially unsaturated 3 to 10-membered ring system having ring carbon atoms and 1 to 4 ring heteroatoms, wherein each heteroatom is independently selected from nitrogen, oxygen, sulfur, boron, phosphorus, and silicon ("3 to 10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"). Heterocyclyl bicyclic ring systems can include one or more heteroatoms in one or both rings. "Heterocyclyl" also includes ring systems wherein the heterocyclyl ring, as defined above, is fused with one or more cycloalkyl groups wherein the point of attachment is either on the cycloalkyl or heterocyclyl ring, or ring systems wherein the heterocyclyl ring, as defined above, is fused with one or more aryl or heteroaryl groups, wherein the point of attachment is on the heterocyclyl ring or the one or more aryl or heteroaryl groups, and in such instances, the number of ring members continue to designate the number of ring members in the heterocyclyl ring system.

In some embodiments, a heterocyclyl group is a 5 to 10 membered saturated or partially unsaturated ring system having ring carbon atoms and 1 to 4 ring heteroatoms, wherein each heteroatom is independently selected from nitrogen, oxygen, sulfur, boron, phosphorus, and silicon ("5 to 10 membered heterocyclyl"). In some embodiments, a heterocyclyl group is a 5 to 8 membered saturated or partially unsaturated ring system having ring carbon atoms and 1 to 4 ring heteroatoms, wherein each heteroatom is independently selected from nitrogen, oxygen, and sulfur ("5 to 8 membered heterocyclyl"). In some embodiments, a heterocyclyl group is a 5 to 6 membered saturated or partially unsaturated ring system having ring carbon atoms and 1 to 4 ring heteroatoms, wherein each heteroatom is independently selected from nitrogen, oxygen, and sulfur ("5 to 6 membered heterocyclyl"). In some embodiments, the 5 to 6 membered heterocyclyl has 1 to 3 ring heteroatoms selected from nitrogen, oxygen, and sulfur. In some embodiments, the 5 to 6 membered heterocyclyl has 1 to 2 ring heteroatoms selected from nitrogen, oxygen, and sulfur. In some embodiments, the 5 to 6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

The term "Spiro heterocyclyl," "spiro heterocycle," or "spirocyclic heterocycle," refers to a polycyclic heterocyclyl with rings connected through one common atom (called a spiro atom), wherein the rings have one or more heteroatoms selected from the group consisting of N, O, and S(O)m (wherein m is an integer of 0 to 2) as ring atoms.

The term "bridged-heterocycle" as used herein refers to a 4, 5, 6, 7, 8, 9, 10, 11, or 12-membered heterocycle as defined herein connected at two non-adjacent atoms of the 4, 5, 6, 7 or 8-membered heterocycle with one or more (e.g., 1 or 2) 3, 4, 5 or 6-membered heterocycles or (C₃-C₇)carbocycles as defined herein. Such bridged-heterocycles include bicyclic and tricyclic ring systems (e.g., 6-azabicyclo[3.1.1]heptane).

As used herein, the term "haloalkyl" refers to alkyl group (as defined above) is substituted with one or more halogens. A monohaloalkyl radical, for example, may have a chlorine, bromine, iodine or fluorine atom. Dihalo and polyhaloalkyl radicals may have two or more of the same or different halogen atoms. Examples of haloalkyl include, but are not limited to, chloromethyl, dichloromethyl, trichloromethyl, dichloroethyl, dichloropropyl, fluoromethyl, difluoromethyl, trifluoromethyl, pentafluoroethyl, heptafluoropropyl, difₗuorochloromethyl, dichlorofluoromethyl, difluoroethyl, difluoropropyl and the like.

As used herein, the term "haloalkoxy" refers to radicals wherein one or more of the hydrogen atoms of the alkoxy group are substituted with one or more halogens. Representative examples of "haloalkoxy" groups include, but not limited to, difluoromethoxy (-OCHF₂), trifluoromethoxy (-OCF₃) or trifluoroethoxy (-OCH₂CF₃).

The term "aryl" as used herein refers to a radical of a monocyclic or polycyclic (*e.g.,* bicyclic or tricyclic) 4n+2 aromatic ring system (*e.g.,* having 6, 10, or 14 π electrons shared in a cyclic array) having 6 to 14 ring carbon atoms and zero heteroatoms provided in the aromatic ring system ("C₆₋₁₄ aryl"). In some embodiments, an aryl group has six ring carbon atoms ("C₆ aryl" ; *e.g.,* phenyl). In some embodiments, an aryl group has ten ring carbon atoms ("C₁₀ aryl"; *e.g*., naphthyl such as 1-naphthyl and 2-naphthyl). In some embodiments, an aryl group has fourteen ring carbon atoms ("C₁₄ aryl"; *e.g.,* anthracyl). Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl.

The term "heteroaryl" as used herein refers to a radical of a 5 to 10 membered monocyclic or bicyclic 4n+2 aromatic ring system (*e.g.,* having 6 or 10 π electrons shared in a cyclic array) having ring carbon atoms and 1 to 4 ring heteroatoms provided in the aromatic ring system, wherein each heteroatom is independently selected from nitrogen, oxygen and sulfur ("5 to 10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system Bicyclic heteroaryl groups wherein one ring does not contain a heteroatom (*e.g.,* indolyl, quinolinyl, carbazolyl, and the like) the point of attachment can be on either ring, *i.e.,* either the ring bearing a heteroatom (*e.g*., 2-indolyl) or the ring that does not contain a heteroatom (*e.g.,* 5---indolyl).

In some embodiments, a heteroaryl group is a 5 to 10 membered aromatic ring system having ring carbon atoms and 1 to 4 ring heteroatoms provided in the aromatic ring system, wherein each heteroatom is independently selected from nitrogen, oxygen, and sulfur ("5 to 10 membered heteroaryl"). In some embodiments, a heteroaryl group is a 5 to 8 membered aromatic ring system having ring carbon atoms and 1 to 4 ring heteroatoms provided in the aromatic ring system, wherein each heteroatom is independently selected from nitrogen, oxygen, and sulfur ("5 to 8 membered heteroaryl"). In some embodiments, a heteroaryl group is a monocyclic 5 to 6 membered aromatic ring system having ring carbon atoms and 1 to 4 ring heteroatoms provided in the aromatic ring system, wherein each heteroatom is independently selected from nitrogen, oxygen, and sulfur ("5 to 6 membered heteroaryl"). In some embodiments, the 5 to 6 membered heteroaryl has 1 to 3 ring heteroatoms selected from nitrogen, oxygen, and sulfur. In some embodiments, the 5 to 6 membered heteroaryl has 1 to 2 ring heteroatoms selected from nitrogen, oxygen, and sulfur. In some embodiments, the 5 to 6 membered heteroaryl has 1 ring heteroatom selected from nitrogen, oxygen, and sulfur. In some embodiments, a heteroaryl group is a monocyclic 5 membered aromatic ring system having ring carbon atoms and 1 to 4 ring heteroatoms provided in the aromatic ring system, wherein each heteroatom is independently selected from nitrogen, oxygen, and sulfur ("5-membered heteroaryl"). In some embodiments, a heteroaryl group is a monocyclic 6 membered aromatic ring system having ring carbon atoms and 1 to 4 ring heteroatoms provided in the aromatic ring system, wherein each heteroatom is independently selected from nitrogen, oxygen, and sulfur ("6-membered heteroaryl").

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

The term "alkoxy" as used herein refers to the group -OR¹⁰⁰ where R¹⁰⁰ is alkyl, alkenyl, alkynyl, cycloalkyl, heterocyclyl, aryl, or heteroaryl. Exemplary alkoxy groups include methoxy, ethoxy, n-propoxy, isopropoxy, n-butoxy, tert-butoxy, sec-butoxy, n-pentoxy, n-hexoxy, and 1,2-dimethylbutoxy. Other exemplary alkoxy groups are lower alkoxy, i.e. with between 1 and 6 carbon atoms. In other examples, alkoxy groups have between 1 and 4 carbon atoms.

The term "cyano" as used herein refers to the radical -CN.

The term "halogen" as used herein refers to F, Cl, Br, or I.

The term "oxo" as used herein refers to =O.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describes pharmaceutically acceptable salts in detail in J. Pharmaceutical Sciences (1977) 66:1-19, Pharmaceutically acceptable salts of the compounds of the present disclosure include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and N(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

A "subject" to which administration is contemplated includes, but is not limited to, humans (*i.e.,* a male or female of any age group, *e.g.,* a pediatric subject (*e.g,* infant, child, adolescent) or adult subject (*e.g.,* young adult, middle-aged adult or senior adult)) and/or a non-human animal, *e.g.,* a mammal such as primates (*e.g.,* cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human," "patient," and "subject" are used interchangeably herein.

The terms "disease," "disorder," and "condition" are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition. In an alternative embodiment, the present disclosure contemplates administration of the compounds described herein as a prophylactic before a subject begins to suffer from the specified disease, disorder or condition.

In general, the "effective amount" of a compound as used herein refers to an amount sufficient to elicit the desired biological response. As will be appreciated by those of ordinary skill in this art, the effective amount of a compound of the present disclosure may vary depending on such factors as the desired biological endpoint, the pharmacokinetics of the compound, the disease being treated, the mode of administration, and the age, health, and condition of the subject.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

It is also to be understood that compounds that have the same molecular formula but differ in the nature or sequence of bonding of their atoms or the arrangement of their atoms in space are termed "isomers." Isomers that differ in the arrangement of their atoms in space are termed "stereoisomers." Stereoisomers that are not mirror images of one another are termed "diastereomers" and those that are non-superimposable mirror images of each other are termed "enantiomers." When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (*i.e*., as (+) or (-)-isomers respectively). A chiral compound can exist as either individual enantiomer or as a mixture thereof. A mixture containing equal proportions of the enantiomers is called a "racemic mixture".

Isomers, e.g., stereoisomers, can be isolated from mixtures by methods known to those skilled in the art, including chiral high pressure liquid chromatography (HPLC) and the formation of chiral salts; or preferred isomers can be prepared by asymmetric syntheses. See, for example, Jacques et al., Enantiomers, Racemates and Resolutions (Wiley Interscience, New York, 1981), Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The present disclosure additionally encompasses compounds described herein as individual isomers substantially free of other isomers, and alternatively, as mixtures of various isomers.

The compounds provided herein can be administered as the sole active agent, or they can be administered in combination with other active agents. In some embodiments, the present invention provides a combination of a compound of the present invention and another pharmacologically active agent. Administration in combination can proceed by any technique apparent to those of skill in the art including, for example, separate, sequential, concurrent, and alternating administration.

The present disclosure, in an alternative embodiment, also embraces isotopically labeled compounds which are identical to those recited herein, except that one or more atoms are replaced by an atom having an atomic mass or mass number different from the atomic mass or mass number usually found in nature. Examples of isotopes that can be incorporated into compounds described herein include isotopes of hydrogen, carbon, nitrogen, oxygen, phosphorus, sulfur, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. For example, a compound of the disclosure may have one or more H atom replaced with deuterium.

### EXAMPLES

The compounds provided herein can be prepared from readily available starting materials using the following general methods and procedures. It will be appreciated that where typical or preferred process conditions (*i.e.,* reaction temperatures, times, mole ratios of reactants, solvents, pressures, *etc*.) are given, other process conditions can also be used unless otherwise stated. Optimum reaction conditions may vary with the particular reactants or solvent used, but such conditions can be determined by one skilled in the art by routine optimization.

*Abbreviations:* ACN: acetonitrile; DMEDA: 1,2-dimethylethylenediamine; DMF: N,N-dimethylformamide; eq: equivalents; DMSO: dimethyl sulfoxide; EI: electron ionization; ESI: electrospray ionization; h: hours; HPLC: high-performance liquid chromatography; LCMS: liquid chromatography mass spectrometry; MeCN: acetonitrile; MS: mass spectrometry; MeI: methyl iodide; NMR: nuclear magnetic resonance; Py: pyridine; TEA: triethylamine; THF: tetrahydrofuran; Tol.: toluene; and *t*-BuONa: sodium tert-butoxide.

### Example 1. Synthesis of Compound 101

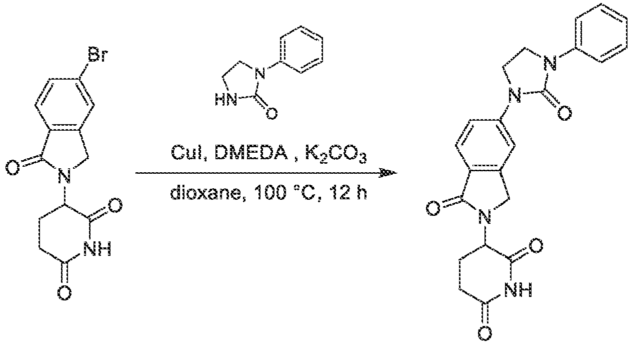

To a mixture of 3-(5-bromo-1-oxoisoindolin-2-yl)piperidine-2,6-dione (50.0 mg, 155 µmol, 1.00 *eq*), 1-phenylimidazolidin-2-one (37.6 mg, 232 µmol, 1.50 *eq*), potassium carbonate (64.2 mg, 464 µmol, 3.00 *eq*) in dioxane (2.00 mL) was added copper iodide (2.95 mg, 15.5 µmol, 0.100 *eq*) and *N,N*'-dimethylethylenediamine (2.73 mg, 30.9 µmol, 3.33 µL, 0.200 *eq*) under nitrogen. The mixture was stirred at 100 °C for 12 h. The 20 batches were combined for workup. The mixture was added dimethylsulfoxide (10 mL) and filtered. The filtrate was concentered in vacuum. The residue was adjusted PH<7 with formic acid (2 mL) and filtered. The filtrate was purified by reverse phase chromatography (column: spherical C18, 20-45 um, 100A, SW 120, mobile phase: [water(0.1%Formic Acid)-ACN). The desired fraction was collected and concentrated in vacuum. Then the solution was filtered, the filter cake was washed with acetonitrile (2 mL) and concentrated in vacuum to give 3-(1-oxo-5-(2-oxo-3-phenylimidazolidin-1-yl)isoindolin-2-yl)piperidine-2,6-dione (91.8 mg, 220 µmol, 14% yield) as a green solid.

¹H NMR (400 MHz, DMSO-*d*₆) δ = 10.98 (s, 1H), 7.92 (s, 1H), 7.81 - 7.76 (m, 11-1), 7.75 - 7.70 (m, 1H), 7.65 (d, *J* = 8.4 Hz, 2H), 7.39 (t, *J* = 7.8 Hz, 2H), 7.10 (t, *J =* 7.3 Hz, 1H), 5.10 (dd, *J* = 5.1, 13.2 Hz, 1H), 4.53 - 4.41 (m, 1H), 4.39 - 4.28 (m, 1H), 4.09 - 3.97 (m, 4H), 2.98 - 2.85 (m, 1H), 2.60 (br d, *J =* 16.8 Hz, 1H), 2.40 (dt, *J =* 8.8, 13.2 Hz, 1H), 2.05 - 1.96 (m, 1H).

### Example 2. Synthesis of Compound 102

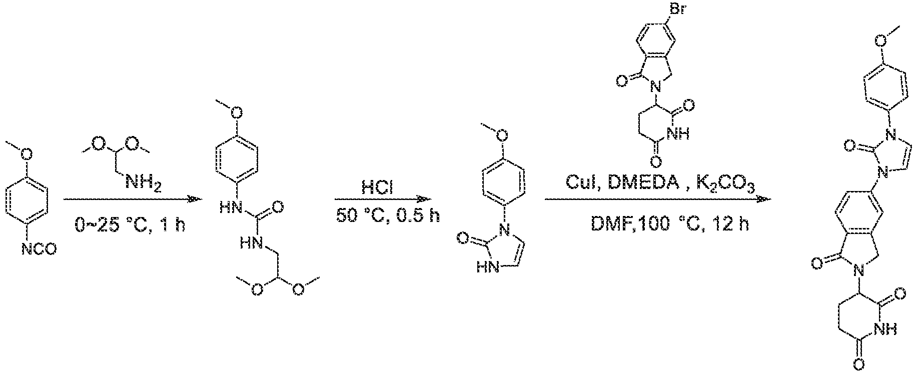

Step 1. A mixture of 1-isocyanato-4-methoxy-benzene (200 mg, 1.34 mmol, 172 µL, 1.00 *eq*) in 2,2-dimethoxyethanamine (140 mg, 1.34 mmol, 146 µL, 1.00 *eq*) was stirred at 20 °C for 1 h under nitrogen. The crude product was triturated with petroleum ether (10.0 mL) and filtered. The filter cake was washed with petroleum ether (2.00 mL) and dried in vacuum to give 1-(1,2-dimethoxyethyl)- 3-(4-methoxyphenyl)urea (300 mg, 1.18 mmol, 87% yield) as a white solid.

¹H NMR (400 MHz, CDCl₃) *δ* = 7.22 - 7.17 (m, 2H), 6.90 - 6.84 (m, 2H), 6.42 (br s, 1H), 5.21 - 4 (m, 1H), 4.41 (t, *J =* 5.2 Hz, 1H), 3.80 (s, 3H), 3.40 (s, 6H), 3.39 (d, *J* = 5.4 Hz, 2H).

Step 2. To a solution of 3-(4-methoxyphenyl)-1*H*-imidazol-2-one (160 mg, 841 µmol, 1.00 *eq*) in dimethyl formamide (1.00 mL) was added 3-(5-bromo-1-oxo-isoindolin-2-yl)piperidine-2,6-dione (271 mg, 841 µmol, 1.00 *eq*), potassium carbonate (348 mg, 2.52 mmol, 3.00 *eq*), *N*¹,*N*²-dimethylethane-1,2-diamine (37.0 mg, 420 µmol, 45.2 µL, 0.500 *eq*) and cuprous iodide (32.0 mg, 168 µmol, 0.200 *eq*) under nitrogen. The mixture was stirred at 100 °C for 12 h. The reaction mixture was purified by reversed-phase HPLC (column: spherical C18, 20-45 um, 100A, SW 80, mobile phase: [water(0.1%Formic Acid)-ACN]) and lyophilized to give 3-(5-(3-(4-methoxyphenyl)-2-oxo-2,3-dihydro-1*H*-imidazol-1-yl)-1-oxoisoindolin-2-yl) piperidine-2,6-dione (73.0 mg, 169 µmol, 20% yield) as an off-white solid.

¹H NMR. (400 MHz, DMSO-*d*₆) *δ* = 11.00 (s, 1H), 8.08 (s, 1H), 7.93 (d, *J* = 8.5 Hz, 1H), 7.82 (d, *J* = 8.3 Hz, 1H), 7.61 (d, *J* = 8.8 Hz, 2H), 7.37 (d, *J* = 3.3 Hz, 1H), 7.25 (d, *J =* 3.1 Hz, 1H), 7.05 (d, *J =* 8.9 Hz, 2H), 5.13 (dd, *J* = 5.1, 13.3 Hz, 1H), 4.55 - 4.47 (m, 1H), 4.41 - 4.33 (m, 1H), 3.79 (s, 3H), 2.98 - 2.87 (m, 1H), 2.61 (br d, *J =* 17.1 Hz, 1H), 2.46 - 2.35 (m, 1H), 2.06 - 1.98 (m, 1H).

### Example 3. Synthesis of Compound 105

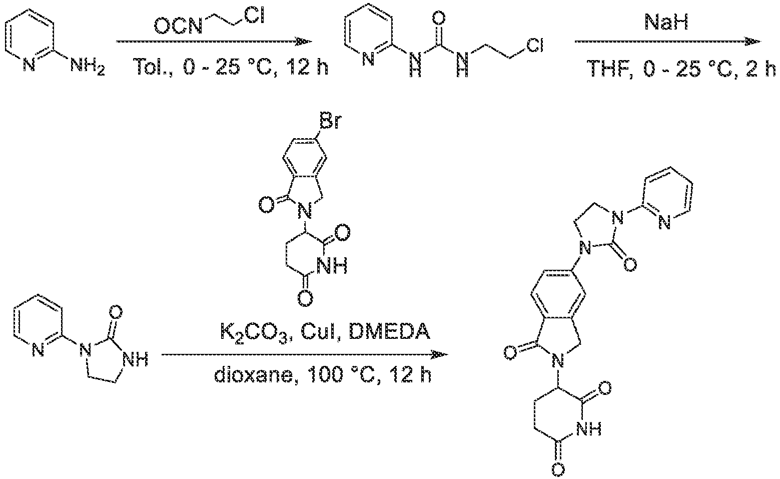

Step 1. To a solution of pyridin-2-amine (2.00 g, 21.2 mmol, 1.00 *eq)* in toluene (30.0 mL) was added 1-chloro-2-isocyanatoethane (3.36 g, 31.8 mmol, 2.71 mL, 1.50 *eq)* at 0 °C. The mixture was stirred at 25 °C for 12 h. The mixture was filtered to give a filter cake, then it was dried under reduced pressure to give 1-(2-chloroethyl)-3-(pyridin-2-yl)urea (1.80 g, 9.02 mmol, 42% yield) as a white solid.

¹H NMR (400 MHz, DMSO-*d*₆) *δ* = 9.34 (s, 1H), 8.53 (br s, 1H), 8.19 - 8.15 (m, 1H), 7.70 - 7.63 (m, 1H), 7.32 (d, *J* = 8.4 Hz, 1H), 6.92 (dd, *J* = 5.6, 6.7 Hz, 1H), 3.72 - 3.67 (m, 2H), 3.51 (q, *J* = 6.0 Hz, 2H),

Step 2. To a solution of 1-(2-chloroethyl)-3-(pyridin-2-yl)urea (500 mg, 2.50 mmol, 1.00 *eq*) in tetrahydrofuran (5.00 mL) was added sodium hydride (150 mg, 3.76 mmol, 60% purity, 1.50 *eq*) at 0 °C under nitrogen. The mixture was stirred at 25 °C for 2 h. The reaction mixture was quenched with methanol (10.0 mL). The mixture was concentrated under reduced pressure to give 1-(pyridin-2-yl)imidazolidin-2-one (400 mg, 2.45 mmol, 97% yield) as a white solid.

¹H NMR (400 MHz, DMSO-d₆) δ = 8.23 (d, *J* = 8.6 Hz, 1H), 8.18 (dd, *J* = 1.0, 4.8 Hz, 1H), 7.55 (dt, *J =* 1.9, 7.8 Hz, 1H), 6.80 (dd, *J =* 5.4, 6.3 Hz, 1H), 4.92 (br s, 1H), 3.90 - 3.84 (m, 2H), 3.38 - 3.33 (m, 2H).

Step 3. To a solution of 3-(5-bromo-1-oxoisoindolin-2-yl)piperidine-2,6-dione (118 mg, 367 µmol, 1.20 *eq),* 1-(pyridin-2-yl)imidazolidin-2-one (50.0 mg, 306 µmol, 1.00 *eq)* and potassium carbonate (169 mg, 1.23 mmol, 4.00 *eq)* in 1,4-dioxane (2.00 mL) was added copper(I) iodide (11.6 mg, 61.2 µmol, 0.200 *eq)* and *N,N'*-dimethylethylenediamine (5.40 mg, 61.2 µmol, 6.60 µL, 0.200 *eq)* in portions under nitrogen. The mixture was stirred at 100 °C for 12 h. The mixture was filtered to give a filter liquor, then it was concentrated under reduced pressure. The residue was purified by *Prep*-HPLC (column: Phenomenex luna C18 150*25mm* 10um; mobile phase: [water (formic acid) - acetonitrile]; B%: 8%-38%,10 min) to give 3-(1-oxo-5-(2-oxo-3-(pyridin-2-yl)imidazolidin-1-yl)isoindolin-2-yl)piperidine-2,6-dione (31.9 mg, 78.8 µmol, 8% yield) as a white solid.

¹H NMR (400 MHz, DMSO-*d*₆) *δ* = 10.96 (br s, 1H), 8.37 (d, *J* = 4.1 Hz, 1H), 8.24 (d, *J =* 8.5 Hz, 1H), 7.93 (s, 1H), 7.84 - 7.77 (m, 2H), 7.76 - 7.72 (m, 1H), 7.09 (dd, *J =* 5.4, 6.8 Hz, 1H), 5.10 (dd, *J* = 5.1, 13.2 Hz, 1H), 4.52 - 4.42 (m, 1H), 4.38 - 4.30 (m, 1H), 4.18 - 4.10 (m, 2H), 4.08 - 4.02 (m, 2H), 2.97 - 2.86 (m, 1H), 2.65 - 2.56 (m, 1H), 2.42 (br d, *J* = 4.4 Hz, 1H), 2.05 - 1.98 (m, 1H).

### Example 4. Synthesis of Compound 103

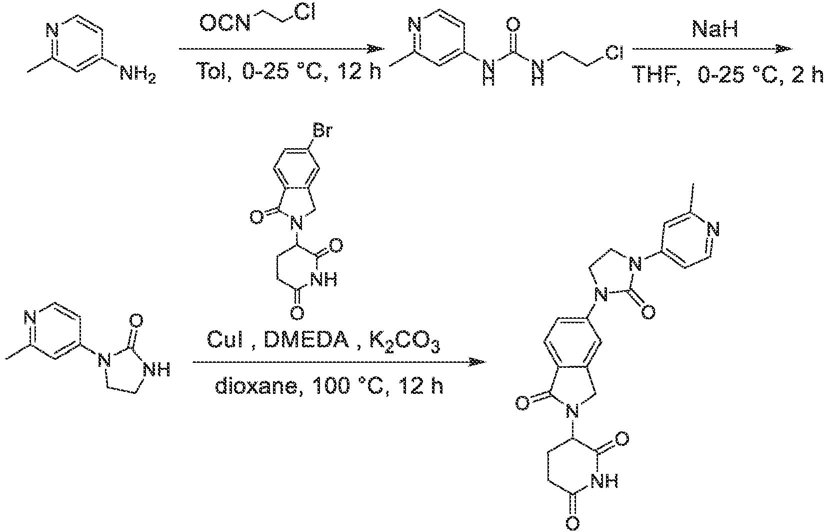

Step 1. To a solution of 2-methylpyridin-4-amine (2.00 g, 18.4 mmol, 1.00 *eq)* in toluene (20.0 mL) was added 1-chloro-2-isocyanato-ethane (2.93 g, 27.7 mmol, 1.50 *eq)* at 0 °C. Then the solution was stirred at 25 °C for 12 h. The mixture was filtered. The filter cake was dried in vacuum to give 1-(2-chloroethyl)-3-(2-methyl-4-pyridyl)urea (2.00 g, crude) as yellow oil.

¹H NMR (400 MHz, DMSO-*d*ₑ) *δ* = 9.15 (s, 1H), 8.16 (d, *J =* 5.6 Hz, 1H), 7.29 - 7.23 (m, 1H), 7.20 (dd, *J* = 2.0, 5.6 Hz, 1H), 6.74 - 6.65 (m, 1H), 3.69 - 3.65 (m, 2H), 3.46 - 3.41 (m, 2H), 2.36 (s, 3H).

Step 2. To a solution of 1-(2-chloroethyl)-3-(2-methyl-4-pyridyl)urea (1.50 g, 7.02 mmol, 1.00 *eq)* in tetrahydrofuran (15.0 mL) was added sodium hydride (421 mg, 10.5 mmol, 60% purity, 1.50 *eq*) at 0 °C. Then the mixture was stirred at 25 °C for 2 h. The reaction mixture was quenched by addition saturated ammonium chloride aqueous solution (100 mL), extracted with dichloromethane (3 ×**100** mL). The combined organic layers were washed with brine (2 × 20 mL), dried over sodium sulfate, filtered and concentrated under reduced pressure to give a residue. The residue was purified by reversed phase column chromatography (C18, 80 g; condition: water/acetonitrile = 1/0 to 0/1, 0.1% formic acid) and further purified by reversed phase column chromatography (C18, 80 g; condition: water/acetonitrile = 1/0 to 0/1, 0.1% ammonium hydroxide) to afford 1-(2-methylpyridin-4-yl)imidazolidin-2-one (160 mg, 866 µmol, 12% yield) as a yellow solid.

¹H NMR (400 MHz, DMSO-*d*₆) *δ* = 8.24 (d, *J =* 5.8 Hz, 1H), 7.43 (dd, *J* = 1.8, 5.8 Hz, 1H), 7.38 (s, 1H), 7.33 (br s, 1H), 3.88 - 3.82 (m, 2H), 3.45 (br d, *J =* 8.5 Hz, 2H), 2.41 (s, 3H).

Step 3. To a solution of 1-(2-methyl-4-pyridyl)imidazolidin-2-one (30.0 mg, 169 µmol, 1.00 *eq)* and 3-(5-bromo-1-oxo-isoindolin-2-yl)piperidine-2,6-dione (65.6 mg, 203 µmol, 1.20 *eq)* in dioxane (2.00 mL) was added *N,N'*-dimethylethane-1,2-diamine (4.48 mg, 50.7 µmol, 5.47 µL, 0.300 *eq*), copper iodide (6.45 mg, 33.8 µmol, 0.200 eq), potassium carbonate (93.5 mg, 677 µmol, 4.00 *eq)* under nitrogen atmosphere. Then the mixture was stirred at 100 °C for 12 h. The mixture was concentrated in vacuum. The residue was added *N,N*-dimethyl formamide (3 mL). The mixture was adjust pH to 5-6 by formic acid (0.1 mL) and filtered. The filtrate was purified by *prep*-HPLC (column: Phenomenex luna C 18 150*25mm* 10um;mobile phase: [water(FA)-ACN];B%: 0%-23%), 1min) and lyophilized to afford 3-(5-(3-(2-methylpyridin-4-yl)-2-oxoimidazolidin-1-yl)-1-oxoisoindolin-2-yl)piperidine-2,6-dione (90.2 mg, 210 µmol, 24% yield) as a yellow solid.

¹H NMR (400 MHz, DMSO-*d*₆) *δ* = 10.99 (s, 1H), 7.94 (s, 1H), 7.88 - 7.69 (m, 4H), 5.11 (dd, *J* = 5.1, 13.2 Hz, 1H), 4.52 - 4.43 (m, 1H), 4.38 - 4.30 (m, 1H), 4.15 - 4.07 (m, 2H), 4.06 - 3.99 (m, 2H), 3.33 (br s, 3H), 2.96 - 2.88 (m, 1H), 2.63 (br s, 1H), 2.41 (br dd, *J* = 4.4, 13.1 Hz, 1H), 2.06 - 1.97 (m, 1H).

### Example 5. Synthesis of Compound 104

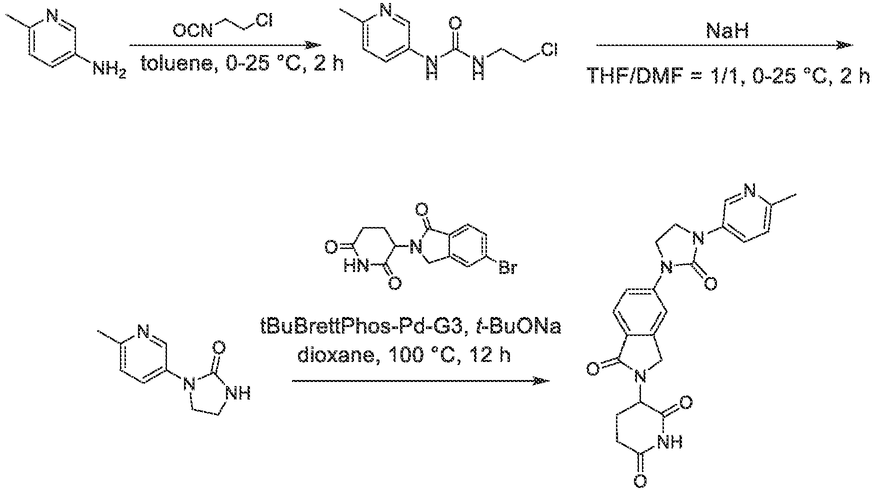

Step 1. To a mixtrue of 6-methylpyridin-3-amine (2.00 g, 18.4 mmol, 1.00 *eq)* in toluene (20.0 mL) was added 1-chloro-2-isocyanatoethane (2.93 g, 27.7 mmol, 2.36 mL, 1.50 *eq*) dropwise at 0 °C. The mixture was stirred at 25 °C for 2 h. The mixture was filtered and washed with toluene (30 mL). The filter cake was dried in vacuum to give 1-(2-chloroethyl)-3-(6-methylpyridin-3-yl)urea (3.60 g, 16.8 mmol, 91% yield) as a white solid.

¹H NMR (400 MHz, DMSO-*d*₆) *δ* = 8.71 (s, 1H), 8.40 (d, *J* = 2.6 Hz, 1H), 7.76 (dd, *J* = 2.6, 8.4 Hz, 1H), 7.10 (d, *J* = 8.4 Hz, 1H), 6.48 (t, *J* = 5.8 Hz, 1H), 3.65 (t, *J* = 6.1 Hz, 2H), 3.44 - 3.39 (m, 2H), 2.37 (s, 3H).

Step 2. To a mixture of 1-(2-chloroethyl)-3-(6-methylpyridin-3-yl)urea (3.00 g, 14.0 mmol, 1.00 *eq)* in dimethyformamide (15.0 mL) and tetrahydrofuran (15.0 mL) was added sodium hydride (842 mg, 21.0 mmol, 60.0% purity, 1.50 *eq)* at 0 °C. The mixture was stirred at 25 °C for 2 h. The reaction mixture was quenched with methol (100 mL) and concentrated in vacuum to give 1-(6-methylpyridin-3-yl)imidazolidin-2-one (1.20 g, 6.77 mmol, 48% yield) as a yellow solid.

¹H NMR (400 MHz, DMSO-*d*₆) *δ* = 8.57 (d, *J* = 2.6 Hz, 1H), 7.89 (dd, *J* = 2.8, 8.6 Hz, 1H), 7.16 (d, *J* = 8.6 Hz, 1H), 7.03 (br s, 1H), 3.84 (dd, *J* = 6.9, 8.9 Hz, 2H), 3.42 (t, *J* = 8.0 Hz, 2H), 2.39 (s, 3H).

Step 3. To a mixture of 1-(6-methylpyridin-3-yl)imidazolidin-2-one (27.4 mg, 154 µmol, 1.00 *eq)* and 3-(5-bromo-1-oxoisoindolin-2-yl)piperidine-2,6-dione (50.0 mg, 154 µmol, 1.00 *eq)* in dioxane (1.00 mL) was added potassium carbonate (42.8 mg, 309 µmol, *2.00 eq).* The mixture was degassed and purged with nitrogen for 3 times, and then was added copper iodide (2.95 mg, 15.5 µmol, 0.100 eq) and *N,N'*-dimethylethylenediamine (2.73 mg, 30.9 µmol, 3.33 µL, 0.200 *eq*) at 25 °C. The mixture was stirred at 100 °C for 12 h under nitrogen atmosphere. The reaction mixture was diluted with water (20 mL) and extracted with ethyl acetate (20 mL) (3 × 20 mL). The combined organic layers were washed with brine (20 mL), dried over sodium sulfate, filtered and concentrated under reduced pressure to give a residue. The residue was purified by *prep*-HPLC(column: Phenomenex luna C18 150*25mm* 10um;mobile phase: [water(FA)-ACN];B%: 0%-30%, 10min} and lyophilized to give 3-(5-(3-(6-methylpyridin-3-yl)-2-oxoimidazolidin-1-yl)-1-oxoisoindolin-2-yl)piperidine-2,6-dione (45.0 mg, 107 µmol, 69% yield) as a brown solid.

¹H NMR (400 MHz, DMSO-*d*₆ *δ* = 10.98 (s, 1H), 7.98 (br d, *J* = 8.0 Hz, 1H), 7.91 (s, 1H), 7.82 - 7.76 (m, 1H), 7.75 - 7.70 (m, 1H), 7.70 - 7.28 (m, 1H), 5.10 (dd, *J* = 5.1, 13.1 Hz, 1H), 4.50 - 4.41 (m, 1H), 4.36 - 4.28 (m, 1H), 4.11 - 3.99 (m, 4H), 2.96 - 2.87 (m, 1H), 2.60 (br d, *J* = 17.4 Hz, 1H), 2.48 - 2.30 (m, 4H), 2.05 - 1.96 (m, 1H).

### Example 6. Synthesis of Compound 109

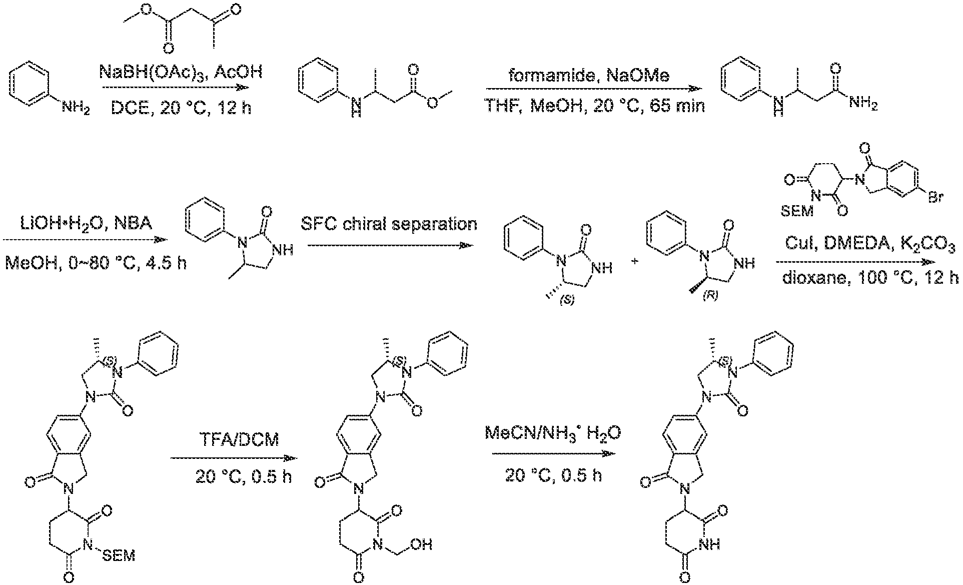

Step 1.To a solution of methyl 3-oxobutanoate (7.48 g, 64.4 mmol, 6.93 mL, 1.20 *eq*) in 1, 2-dichloroethane (200 mL) was added acetic acid (322 mg, 5.37 mmol, 307 µL, *0.100 eq).* The mixture was stirred at 20 °C for 10 min. Then aniline (5.00 g, 53.6 mmol, 4.90 mL, 1.00 eq) was added into the mixture and the mixture was stirred at 20 °C for 5 min. At last, sodium triacetoxyborohydride (34.1 g, 161 mmol, 3.00 *eq)* was added into the mixture and the mixture was stirred at 20 °C for 12 h. The reaction mixture was added water (200 mL) and extracted with ethyl acetate (2 × 200 mL). The organic phase was separated, washed with brine (100 mL), dried over sodium sulfate, filtered and concentrated under reduced pressure to give a residue. The crude product was purified by column chromatography (SiO₂, petroleum ether/ethyl acetate = 8/1 to 0/1) to give methyl 3-(phenylamino)butanoate (15.0 g, 77.6 mmol, 72% yield) as yellow oil.

¹H NMR (400 MHz, CDCl₃) *δ* = 7.22 - 7.15 (m, 2H), 6.72 (t, *J* = 7.3 Hz, 1H), 6.67 - 6.60 (m, 2H), 3.87 - 3.76 (m, 1H), 3.69 (s, 3H), 2.66 (dd, J= 5.2, 15.1 Hz, 1H), 2.45 (dd, *J* = 6.9, 15.1 Hz, 1H), 1.31 - 1.28 (m, 3H), 1.27 - 1.25 (m, 1H).

Step 2. To a solution of formamide (13.0 g, 289 mmol, 11.5 mL, 8.00 *eq)* in tetrahydrofuran (45.0 mL) was added a solution of sodiummethoxide (7.83 g, 144 mmol, 4.00 *eq)* in methanol (21.0 mL). The mixture was stirred at 20 °C for 5 min. Then methyl 3-(phenylamino)butanoate (7.00 g, 36.2 mmol, 1.00 *eq)* in tetrahydrofuran (15.0 mL) was added into the reaction mixture. The reaction mixture was stirred at 20 °C for 1 h. The reaction mixture was concentrated under reduced pressure to give a residue which was purified by reversed-phase HPLC (column: spherical C18, 20-45 um, 100Å, SW 330, mobile phase: [water(0.1%Formic Acid)-ACN]) to give 3-(phenylamino)butanamide (4.50 g, 25.2 mmol, 69% yield) as yellow oil.

¹H NMR (400 MHz, CDCl₃) *δ* = 7.18 (t, *J* = 7.6 Hz, 2H), 6.73 (t, *J =* 7.3 Hz, 1H), 6.65 (br d, *J =* 8.4 Hz, 2H), 6.40 (br d, *J* = 12.4 Hz, 2H), 3.93 - 3.82 (m, 2H), 2.47 - 2.38 (m, 1H), 2.29 (dd, *J =* 5.8, 14.6 Hz, 1H), 1.23 (d, *J =* 6.5 Hz, 3H).

Step 3. To a solution of 3-(phenylamino)butanamide (2.00 g, 11.2 mmol, 1.00 *eq)* in methanol (50.0 mL) was added lithium hydroxide hydrate (2.83 g, 67.3 mmol, 6.00 *eq)* with the flask protected from light and cooled to 0 °C with stirring. Then N-bromoacetamide (3.10 g, 22.4 mmol, 2.00 *eq)* was added into the mixture and the mixture was stirred at 0 °C for 0.5 h. At last, the mixture was stirred at 80 °C for 4 h. The reaction mixture was added into 10% aqueous sodium thiosulfate solution (100 mL) and concentrated on a rotary evaporator (30 °C, 30 min). The liquid residue was diluted with 5% aqueous sodium hydroxide (50 mL) and extracted with dichloromethane (2 × 100 mL). The crude product was purified by reversed-phase HPLC (column: spherical C18, 20-45 um, 100Å, SW 120, mobile phase: [water(0.1%Formic Acid)-[ACN]) to give 5-methyl-1-phenyl-imidazolidin-2-one (1.00 g, 2.84 mmol, 25% yield) as a white solid.

Step 4. 5-methyl-1-phenyl-imidazolidin-2-one (1.00 g, 2.84 mmol, 25% yield, 50% purity) was purified by chiral SFC (column: DAICEL CHIRALPAK IC(250mm*30mm,10um);mobile phase: [0.1%NH3H₂O MEOH];B%: 20%-20%,A3.2;66min) to give (*S*)-5-methyl-1-phenylimidazolidin-2-one (460 mg, 2.58 mmol, 45% yield) as a white solid and (*R*)-5-methyl-1-phenylimidazolidin-2-one (440 mg, 2.47 mmol, 44% yield) as a white solid.

¹H NMR (400 MHz, DMSO-*d*₆) *δ* = 7.46 (dd, *J =* 0.9, 8.7 Hz, 2H), 7.34 - 7.28 (m, 2H), 7.04 - 6.99 (m, 1H), 6.83 (br s, 1H), 4.46 (quind, *J =* 5.9, 8.6 Hz, 1H), 3.56 (dt, *J* = 0.9, 8.7 Hz, 1H), 2.98 (ddd, *J* = 0.9, 5.5, 8.7 Hz, 1H), 1.18 (d, *J =* 6.1 Hz, 3H). MS (ESI) m/z 177.1 [M+H]⁺

¹H NMR (400 MHz, DMSO-*d*₆) *δ* = 7.49 - 7.42 (m, 2H), 7.34 - 7.27 (m, 2H), 7.02 (t, *J* = 7.3 Hz, 1H), 6.83 (br s, 1H), 4.46 (quind, *J =* 5.9, 8.6 Hz, 1H), 3.56 (t, *J =* 8.6 Hz, 1H), 2.98 (dd, *J =* 5.4, 8.8 Hz, 1H), 1.18 (d, *J =* 6.0 Hz, 3H). MS (ESI) m/z 177.1 [M+H]⁺

Step 5. To a solution of (*S*)-5-methyl-1-phenylimidazolidin-2-one (50.0 mg, 283 µmol, 1.00 *eq)* in dioxane (1.50 mL) was added 3-(5-bromo-1-oxoisoindolin-2-yl)-1-((2-(trimethylsilyl)ethoxy)methyl)piperidine-2,6-dione (141 mg, 312 µmol, 1.10 *eq*), potassium carbonate (117 mg, 851 µmol, 3.00 *eq*), *N,N*-dimethylethylenediamine (12.5 mg, 141 µmol, 15.2 µL, 0.500 *eq)* and copper iodide (10.8 mg, 56.7 µmol, 0.200 *eq)* under nitrogen. The mixture was stirred at 100 °C for 12 h. The reaction mixture was adjusted pH to 5-6 with formic acid. The solution was diluted with water (30 mL) and extracted with ethyl acetate (2 × 30 mL). The organic phase was separated, washed with brine (10 mL), dried over sodium sulfate, filtered and concentrated under reduced pressure to give a residue. The crude product was purified by reversed-phase HPLC (column: spherical C18, 20-45 um, 100Å, SW 80, mobile phase: [water(0.1%Formic Acid)-ACN]) to give 3-(5-((*S*)-4-methyl-2-oxo-3-phenylimidazolidin-1-yl)-1-oxoisoindolin-2-yl)-1-((2-(trimethylsilyl)ethoxy) methyl)piperidine-2,6-dione (300 mg, 546 µmol, 48% yield) as a yellow solid.

¹H NMR (400 MHz, DMSO-*d*₆) *δ* = 7.93 - 7.88 (m, 1H), 7.82 - 7 66 (m, 2H), 7.58 - 7.48 (m, 2H), 7.41 (t, *J* = 7.9 Hz, 2H), 7.22 - 7.11 (m, 1H), 5.21 (dd, *J* = 5.1, 13.4 Hz, 1H), 5.08 - 4.99 (m, 2H), 4.64 (td, *J* = 5.9, 8.9 Hz, 1H), 4.50 - 4.44 (m, 1H), 4.31 - 4.17 (m, 2H), 3.65 (ddd, *J* = 2.8, 5.9, 8.9 Hz, 1H), 3.55 - 3.51 (m, 2H), 3.09 - 2.99 (m, 1H), 2.83 - 2.76 (m, 1H), 2.42 - 2.32 (m, 1H), 2.08 - 2.02 (m, 1H), 1.30 - 1.23 (m, 3H), 0.87 - 0.81 (m, 2H), - 0.02 (s, 9H).

Step 6. A solution of 3-(5-((*S*)-4-methyl-2-oxo-3-phenylimidazolidin-1-yl)-1-oxoisoindolin-2-yl)-1-((2- (trimethylsilyl)ethoxy)methyl)piperidine-2,6-dione (300 mg, 546 µmol, 1.00 *eq)* in trifluoroacetic acid (1.54 g, 13.5 mmol, 1.00 mL, 24.7 *eq)* and dichloromethane (10.0 mL) was stirred at 20 °C for 0.5 h. The reaction mixture was concentrated under reduced pressure to give 1-(hydroxymethyl)- 3-(5-((*S*)-4-methyl-2-oxo-3-phenylimidazolidin-1-yl)-1-oxoisoindolin-2-yl)piperidine-2,6-dione (240 mg, 535 µmol, 97% yield) as a yellow solid.

Step 7. A solution of 1-(hydroxymethyl)-3-(5-((S)-4-methyl-2-oxo-3-phenylimidazolidin-1-yl)-1-oxoisoindolin-2- yl)piperidine-2,6-dione (240 mg, 53.5 µmol, *1.00 eq)* in ammonium hydroxide (227 mg, 1.82 mmol, 0.250 mL, 28% purity, 3.40 *eq)* and acetonitrile (5.00 mL) was stirred at 20 °C for 30 min. The reaction mixture was concentrated under reduced pressure to give a residue. The crude product was purified by reversed-phase HPLC (column: spherical C18, 20-45 um, 100Å, SW 40, mobile phase: [water(0.1%Formic Acid)-ACN]) to give 3-(5-((5)-4-methyl-2-oxo-3-phenylimidazolidin-1-yl)-1-oxoisoindolin-2-yl) piperidine-2,6-dione (39.3 mg, 84.7 µmol, 15% yield) as a white solid.

¹H NMR (400 MHz, DMSO-*d*₆) *δ* = 10.97 (br s, 1H), 8.47 (s, 0.1H), 7.91 - 7.85 (m, 1H), 7.79 - 7.74 (m, 1H), 7.73 - 7.69 (m, 1H), 7.57 - 7.50 (m, 2H), 7.44 - 7.37 (m, 2H), 7.19 - 7.13 (m, 1H), 5.10 (dd, *J* = 5.1, 13.3 Hz, 1H), 4.69 - 4.58 (m, 1H), 4.48 - 4.41 (m, 1H), 4.36 - 4.28 (m, 1H), 4.24 (dt, *J =* 2.0, 9.0 Hz, 1H), 3.64 (ddd, *J =* 2.8, 5.9, 9.0 Hz, 1H), 2.97 - 2.84 (m, 1H), 2.60 (td, *J =* 1.9, 15.3 Hz, 1H), 2.43 - 2.34 (m, 1H), 2.05 - 1.95 (m, 1H), 1.27 (d, *J =* 6.0 Hz, 3H).

### Example 7. Synthesis of Compound 108

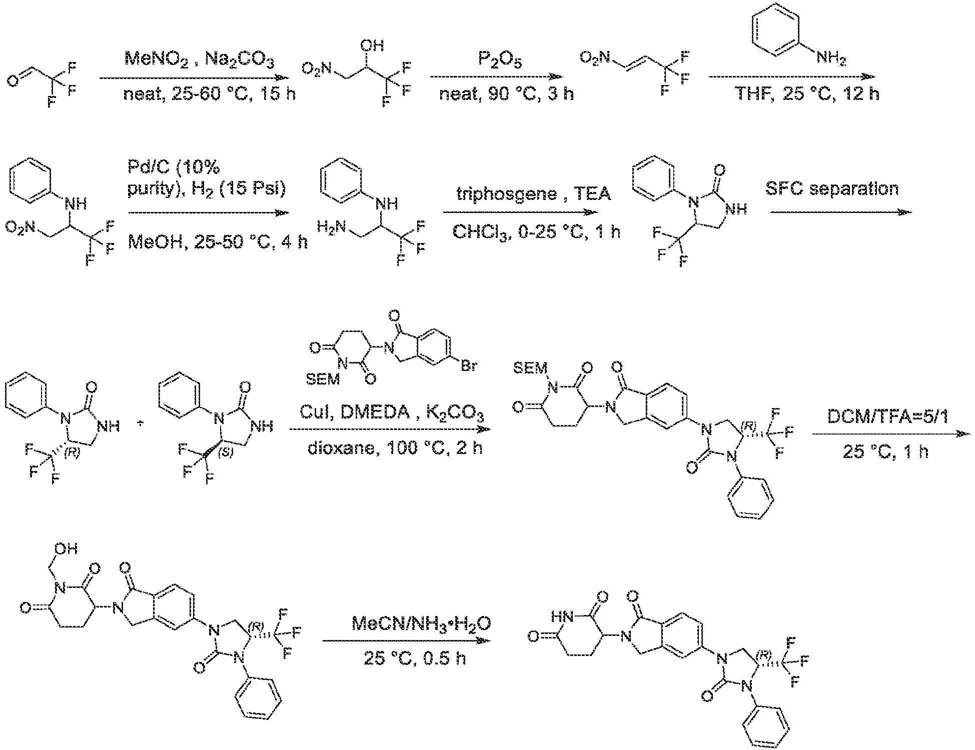

Step 1. To a mixture of 2,2,2-trifluoroacetaldehyde (48.0 g, 367 mmol, 75% aqueous solution, 1.00 *eq)* in nitromethane (67.3 g, 1.10 mol, 59.5 mL, 3.00 *eq)* was added sodium carbonate (3.89 g, 36.7 mmol, 0.100 *eq*) at 25 °C. The mixture was stirred at 60 °C for 3 h. Then the mixture was stirred at 25 °C for 12 h. The mixture was diluted with water (200 mL) and extracted with *tert*-butyl methyl ether (3 × 100 mL). The combined organic layer was washed with brine (60 mL), dried over sodium sulfate, filtered and concentrated in vacuum at 25 °C to give 1,1,1-trifluoro-3-nitropropan-2-ol (58.0 g, crude) as yellow oil.

Step 2. A mixture of 1,1,1-trifluoro-3-nitropropan-2-ol (38.0 g, 239 mmol, 1.00 *eq)* and phosphorus pentoxide (33.91 g, 238.90 mmol, 14.74 mL, 1 *eq*) was stirred at 90 °C for 3 h. The crude product was distilled in vacuum (90 °C, 0.06 MPa) to give (*E*)-3,3,3-trifluoro-1-nitro-prop-1-ene (6.40 g, crude) as yellow oil.

¹H NMR (400 MHz, CDCl₃) *δ =* 7.46 - 7.36 (m, 1H), 7.04 (qd, *J* = 6.5, 13.3 Hz, 1H). (The ¹H NMR comes from the pilot run)

Step 3. A mixture of (*E*)-3,3,3-trifluoro-1-nitroprop-1-ene (6.40 g, 45.4 mmol, 1.00 *eq)* and aniline (5.07 g, 54.5 mmol, 4.97 mL, 1.20 *eq)* in tetrahydrofuran (30.0 mL) was stirred at 25 °C for 12 h. The mixture was concentrated in vacuum. The residue was purified by silica gel chromatography (petroleum ether/ethyl acetate = 10/1) to give *N*-(1,1,1-trifluoro-3-nitropropan-2-yl)aniline (12.40 g, crude) as yellow oil.

¹H NMR (400 MHz, CDCl₃) = 7.19 - 7.12 (m, 2H), 6.84 - 6.77 (m, 1H), 6.67 (d, *J* = 7.6 Hz, 2H), 4.81 (br s, 1H), 4.71 - 4.63 (m, 1H), 4.54 - 4.46 (m, 1H), 4.05 - 3.88 (m, 1H).

Step 4. To a mixture of *N*-(1,1,1-trifluoro-3-nitropropan-2-yl)aniline (12.4 g, 52.9 mmol, 1.00 *eq)* in methanol (100 mL) was added palladium on carbon (1.00 g, 10% purity) in one portion. The mixture was stirred at 25 °C for 2 h under hydrogen (15 Psi). The mixture was added palladium on carbon (1.00 g, 10% purity) in one portion. The mixture was stirred at 50 °C for 2 h under hydrogen (15 Psi). The mixture was filtered and the filtrate was concentrated in vacuum. The residue was purified by silica gel chromatography (petroleum ether/ethyl acetate = 0/1) to give 3,3,3-trifluoro-*N*²-phenylpropane-1,2-diamine (6.30 g, 30.9 mmol, 58% yield) as yellow oil.

¹H NMR (400 MHz, DMSO-*d*₆) *δ* = 7.18 - 7.05 (m, 2H), 6.84 - 6.71 (m, 2H), 6.67 - 6.56 (m, 1H), 5.89 (br dd, *J =* 2.6, 8.3 Hz, 1H), 4.15 - 3.99 (m, 1H), 2.94 (td, *J =* 4.0, 13.2 Hz, 1H), 2.86 - 2.72 (m, 1H).

Step 5. To a mixture of 3,3,3-trifluoro-*N*²-phenylpropane-1,2-diamine (5.80 g, 28.4 mmol, 1.00 *eq),* and triethylamine (5.75 g, 56.8 mmol, 7.91 mL, 2.00 *eq*) in trichloromethane (60.0 mL) was added a solution of triphosgene (8.43 g, 28.4 mmol, 1.00 *eq)* in trichloromethane (20.0 mL) dropwise at 0 °C. The mixture was stirred at 25 °C for 1 h. The mixture was concentrated in vacuum. The residue was purified by reverse phase chromatography (column: spherical C18, 20-45 µm, 100Å, SW 330, mobile phase: [water(0. 1%Formic Acid)-ACN) and concentrated in vacuum to give 1-phenyl-5-(trifluoromethyl)imidazolidin-2-one (4.00 g, 17.4 mmol, 61% yield) as a white solid.

¹H NMR (400 MHz, DMSO-*d*₆) *δ* = 7.50 (d, *J* = 7.8 Hz, 2H), 7.35 (t, *J* = 7.9 Hz, 2H), 7.29 (s, 1H), 7.16 - 7.09 (m, 1H), 5.44 (dqd, *J* = 3.1, 6.6, 9.9 Hz, 1H), 3.76 (t, *J =* 10.1 Hz, 1H), 3.44 (dd, *J* = 2.9, 10.3 Hz, 1H).

Step 6. 1-phenyl-5-(trifluoromethyl)imidazolidin-2-one (1.00 g, 4.34 mmol, 1.00 *eq)* was purified by SFC separation (column: DAICEL CHIRALPAK AS(250mm*30mm,10um); mobile phase: [Neu-MeOH];B%: 30%-30%,C7;60min) and concentrated in vacuum to give (*R*)-1-phenyl-5-(trifluoromethyl)imidazolidin-2- one (490 mg, 2.13 mmol, 49% yield) as a white solid and (S)-1-phenyl-5-(trifluoromethyl)imidazolidin- 2-one (480 mg, 2.09 mmol, 48% yield) as a white solid.

¹H NMR (400 MHz, DMSO-*d*₆) 6 = 7.50 (d, *J =* 7.6 Hz, 2H), 7.39 - 7.32 (m, 2H), 7.29 (s, 1H), 7.17 - 7.10 (m, 1H), 5.52 - 5.35 (m, 1H), 3.75 (t, *J =* 10.1 Hz, 1H), 3.44 (dd, *J =* 2.8, 10.4 Hz, 1H).

¹H NMR (400 MHz, DMSO-*d*₆) *δ* = 7.50 (d, *J* = 7.8 Hz, 2H), 7.38 - 7.32 (m, 2H), 7.29 (s, 1H), 7.17 - 7.10 (m, 1H), 5.44 (dqd, *J =* 3.1, 6.7, 9.9 Hz, 1H), 3.75 (t, *J =* 10.1 Hz, 1H), 3.44 (dd, *J* = 2.6, 10.3 Hz, 1H).

Step 7. To a mixture of (*R*)-1-phenyl-5-(trifluoromethyl)imidazolidin-2-one (50.8 mg, 221 µmol, *1.00 eq),* 3-(5-bromo-1-oxoisoindolin-2-yl)-1-((2-(trimethylsilyl)ethoxy)methyl)piperidine-2,6-dione (100 mg, 221 µmol, 1.00 *eq),* and potassium carbonate (91.5 mg, 662 µmol, 3.00 *eq)* in dioxane (3.00 mL) was added copper iodide (4.20 mg, 22.06 µmol, 0.100 *eq*) and *N,N'*-dimethylethylenediamine (3.89 mg, 44.1 µmol, 4.75 µL, 0.200 *eq*) in portions under nitrogen. The mixture was stirred at 100 °C for 2 h. The mixture was diluted with water (30 mL) and extracted with ethyl acetate (3 × 20 mL). The combined organic layer was washed with brine (10 mL), dried over sodium sulfate, filtered and concentrated in vacuum. The residue was purified by silica gel chromatography (petroleum ether/ethyl acetate = 5/1 to 0/1) to give 3-(1-oxo-5-((*R*)-2-oxo-3-phenyl-4-(trifluoromethyl)imidazolidin-1-yl)isoindolin-2-yl)-1-((2-(trimethylsilyl)ethoxy)methyl)piperidine-2,6-dione (120 mg, 199 µmol, 45% yield) as yellow oil.

¹H NMR (400 MHz, DMSO-*d*₆) *δ* = 7.93 (s, 1H), 7.86 - 7.79 (m, 1H), 7.78 - 7.72 (m, 1H), 7.59 (d, *J* = 7.9 Hz, 2H), 7.45 (t, *J =* 7.9 Hz, 2H), 7.31 - 7.21 (m, 1H), 5.70 - 5.56 (m, 1H), 5.28 - 5.16 (m, 1H), 5.05 (q, *J* = 9.8 Hz, 2H), 4.55 - 4.39 (m, 2H), 4.35 - 4.23 (m, 1H), 4.14 (td, *J* = 3.0, 10.7 Hz, 1H), 3.60 - 3.46 (m, 2H), 3.13 - 3.00 (m, 1H), 2.80 (br d, *J* = 17.1 Hz, 1H), 2.40 (br dd, *J* = 4.3, 13.1 Hz, 1H), 2. 14 - 2.02 (m, 1H), 0.84 (br t, *J* = 8.0 Hz, 2H), 0.02 (s, 9H).

Step 8. A mixture of 3-(1-oxo-5-((*R*)-2-oxo-3-phenyl-4-(trifluoromethyl)imidazolidin-1-yl)isoindolin- 2-yl)-1-((2-(trimethylsilyl)ethoxy)methyl)piperidine-2,6-dione (120 mg, 199 µmol, 1.00 *eq)* in dichloromethane (5.00 mL) and trifluoroacetic acid (1.00 mL) was stirred at 25 °C for 1 h. The mixture was concentrated in vacuum to give 1-(hydroxymethyl)-3-(1-oxo-5-((*R*)-2-oxo-3-phenyl-4-(trifluoromethyl) imidazolidin-1-yl)isoindolin-2-yl)piperidine-2,6-dione (100 mg, crude) as yellow oil.

Step 9. A mixture of 1-(hydroxymethyl)-3-(1-oxo-5-((*R*)-2-oxo-3-phenyl-4-(trifluoromethyl)imidazolidin-1-yl) isoindolin-2-yl)piperidine-2,6-dione (100 mg, 199 µmol, 1.00 *eq*) in acetonitrile (5.00 mL) and ammonium hydroxide (0.100 mL) was stirred at 25 °C for 0.5 h. The mixture was adjusted pH<7 with formic acid (0.1 mL) and concentrated in vacuum. The residue was purified by reverse phase chromatography (column: spherical C18, 20-45 um, 100Å, SW 40, mobile phase: [water(0.1%Formic Acid)-ACN) and lyophilized to give 3-(1-oxo-5-((*R*)-2-oxo-3-phenyl-4-(trifluoromethyl)imidazolidin-1-yl)isoindolin-2-yl)piperidine-2,6- dione (34.3 mg, 71.9 µmol, 36% yield) as an off-white solid.

¹H NMR (400 MHz, DMSO-d₆) δ = 10.98 (br s, 1H), 7.92 (br d, *J* = 7.3 Hz, 1H), 7.87 - 7.79 (m, 1H), 7.77 - 7.70 (m, 1H), 7.59 (d, *J* = 7.8 Hz, 2H), 7.44 (t, *J* = 7.9 Hz, 2H), 7.31 - 7.21 (m, 1H), 5.70 - 5.57 (m, 1H), 5.10 (dd, *J* = 5.1, 13.3 Hz, 1H), 4.53 - 4.39 (m, 2H), 4.37 - 4.27 (m, 1H), 4.13 (td, J= 3.1, 10.7 Hz, 1H), 2.99 - 2.86 (m, 1H), 2.60 (br d, *J* = 17.5 Hz, 1H), 2.45 - 2.35 (m, 1H), 2.05 - 1.93 (m, 1H),

### Example 8. Synthesis of Compound 107

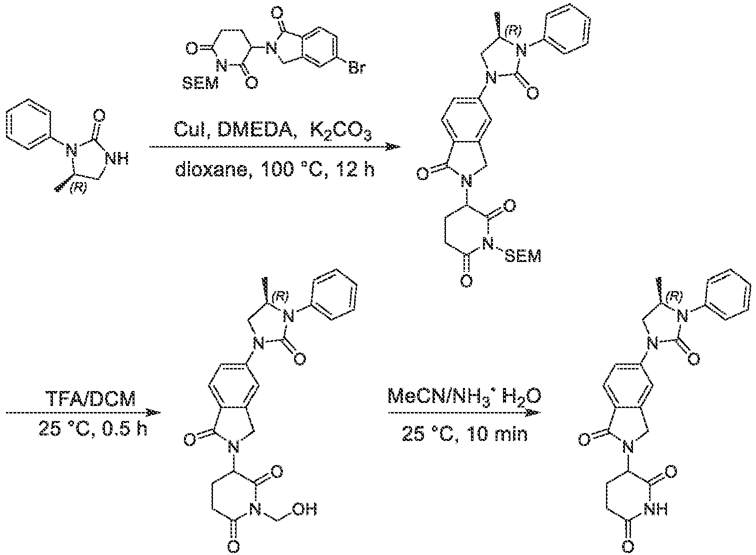

Step 1. To a solution of (*R*)-5-methyl-1-phenylimidazolidin-2-one (50.0 mg, 283 µmol, 1.00 *eq)* in dioxane (1.50 mL) was added 3-(5-bromo-1-oxoisoindolin-2-yl)-1-((2-(trimethylsilyl)ethoxy)methyl)piperidine-2,6-dione (154 mg, 340 µmol, 1.20 *eq*), potassium carbonate (117 mg, 851 µmol, 3.00 *eq*), *N,N*'-dimethylethylenediamine (12.5 mg, 141 µmol, 15.2 µL, 0.50 *eq*) and copper iodide (10.8 mg, 56.7 µmol, 0.200 *eq*) under nitrogen. The mixture was stirred at 100 °C for 12 h. The reaction mixture was diluted with water (30 mL) and exacted with ethyl acetate (2 × 30 mL). The organic phase was separated, washed with brine (10 mL), dried over sodium sulfate, filtered and concentrated under reduced pressure to give a residue. The residue was purified by reversed-phase HPLC (column: spherical C18, 20-45 um, 100Å, SW 80, mobile phase: [water(0.1%Formic Acid)-ACN]) and lyophilized to give 3-(5-((*R*)-4-methyl -2-oxo -3-phenylimidazolidin -1-yl)-1-oxoisoindolin-2-yl)-1-((2-(trimethylsilyl)ethoxy)methyl)piperidine-2,6-dione (300 mg, 546 µmol, 64% yield) as a yellow solid.

¹H NMR (400 MHz, DMSO-*d*₆) *δ* = 7.90 (s, 1H), 7.74 (q, *J* = 8.5 Hz, 2H), 7.53 (br d, *J =* 8.0 Hz, 2H), 7.41 (br t, *J =* 7.9 Hz, 2H), 7.21 - 7.12 (m, 1H), 5.22 (br dd, *J* = 4.9, 13.4 Hz, 1H), 5.06 (q, *J* = 9.7 Hz, 2H), 4.64 (br dd, *J* = 5.9, 14.3 Hz, 1H), 4.47 (br d, *J* = 17.0 Hz, 1H), 4.32 - 4.20 (m, 2H), 3.68 - 3.62 (m, 1H), 3.58 - 3.45 (m, 2H), 3.12 - 3.02 (m, 1H), 2.79 (br d, *J* = 16.9 Hz, 1H), 2.43 - 2.37 (m, 1H), 2.08 - 1.99 (m, 1H), 1.27 (br d, *J* = 6.0 Hz, 3H), 0.84 (br t, *J* = 7.6 Hz, 2H), -0.02 (s, 9H)

Step 2. To a solution of 3-(5-((*R*)-4-methyl-2-oxo-3-phenylimidazolidin-1-yl)-1-oxoisoindolin-2-yl)-1-((2- (trimethylsilyl)ethoxy)methyl)piperidine-2,6-dione (231 mg, 420 µmol, 1.00 *eq)* in trifluoroacetic acid (0.500 mL) and dichloromethane (5.00 mL). The mixture was stirred at 25 °C for 0.5 h. The reaction mixture was concentrated under reduced pressure to give 1-(hydroxymethy])-3-(5-((*R*)-4-methyl-2-oxo-3- phenylimidazolidin-1-yl)-1-oxoisoindolin-2-yl)piperidine-2,6-dione (188 mg, 419 µmol, 99% yield) as a yellow solid. MS (ESI) m/z 449.0 [M+H]⁺

Step 3. A solution of 1-(hydroxymethyl)-3-(5-((*R*)-4-methyl-2-oxo-3-phenylimidazolidin-1-yl)-1-oxoisoindolin -2-yl )piperidine-2,6-dione (188 mg, 419 µmol, 1.00 *eq*) in acetonitrile (10.0 mL) and ammonium hydroxide (0.500 mL) was stirred at 25 °C for 10 min. The mixture was purified by *prep*-HPLC (column: Phenomenex luna C18 150*25mm* 10um;mobile phase: [water(FA)-ACN];B%: 20%-50%,9min) and lyophilized to give 3-(5-((*R*)-4-methyl-2-oxo-3-phenylimidazolidin-1-yl)-1-oxoisoindolin-2-yl)piperidine-2,6-dione (142 mg, 339 µmol, 80 % yield) as a white solid.

¹H NMR (400 MHz, DMSO-*d*₆) *δ* = 10.98 (s, 1H), 7.88 (br d, *J=* 3.1 Hz, 1H), 7.79 - 7.74 (m, 1H), 7.73 - 7.69 (m, 1H), 7.53 (d, *J* = 7.8 Hz, 2H), 7.41 (t, *J* = 7.9 Hz, 2H), 7.19 - 7.14 (m, 1H), 5.10 (dd, *J* = 5.1, 13.3 Hz, 1H), 4.64 (td, .I = 5.9, 8.8 Hz, 1H), 4.48 - 4.39 (m, 1H), 4.36 - 4.29 (m, 1H), 4.24 (dt, *J* = 1.9, 9.0 Hz, 1H), 3.64 (ddd, *J* = 2.7, 5.8, 9.0 Hz, 1H), 2.98 - 2.85 (m, 1H), 2.63 - 2.57 (m, 1H), 2.40 (br dd, *J* = 4.4, 13.1 Hz, 1H), 2.04 - 1.95 (m, 1H), 1.27 (d, *J* = 6.1 Hz, 3H).

### Example 9. Synthesis of Compound 106

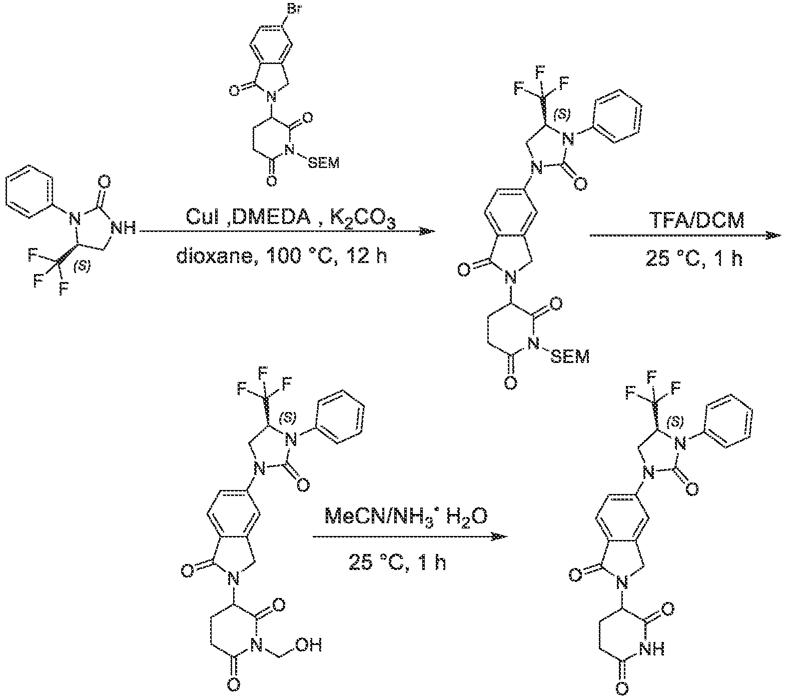

Step 1. To a solution of (*S*)-1-phenyl-5-(trifluoromethyl)imidazolidin-2-one (200 mg, 868 µmol, 1.97 *eq*) and 3-(5-bromo-1-oxo-isoindolin-2-yl)-1-(2-trimethylsilylethoxymethyl)piperidine-2,6-dione (200 mg, 441 µmol, 1.00 *eq*) in dioxane (2.00 mL) was added potassium carbonate (243 mg, 1.76 mmol, 4.00 *eq*), *N,N'-*dimethylethylenediamine (11.6 mg, 132 µmol, 14.2 µL, 0.300 *eq)* and cuprous iodide (16.8 mg, 88.2 µmol, 0.200 *eq*) under nitrogen. Then the mixture was stirred at 100 °C for 12 h. The mixture was diluted with water (50 mL) and extracted with ethyl acetate (3 × 30 mL). The combined organic layers were washed with brine (3 × 10 mL), dried over anhydrous sodium sulfate, filtered and concentrated under reduced pressure to give a residue. The residue was purified by purified by column chromatography (SiO₂, petroleum ether/ethyl acetate = 50/1 to 3/1) and concentrated in vacuum to afford 3-(1-oxo-5-((*S*)-2-oxo-3-phenyl-4-(trifluoromethyl)imidazolidin-1-yl)isoindolin-2-yl)-1-((2-(trimethylsilyl)ethoxy)methyl)piperidine-2,6-dione (200 mg, 328 µmol, 74% yield) as yellow oil.

¹H NMR (400 MHz, DMSO-*d*₆) *δ* = 7.95 (s, 1H), 7.86 - 7.82 (m, 1H), 7.79 - 7.74 (m, 1H), 7.61 (d, *J* = 8.0 Hz, 2H), 7.46 (t, *J* = 7.9 Hz, 2H), 7.31 - 7.24 (m, 1H), 5.68 - 5.60 (m, 1H), 5.24 (br dd, *J =* 4.8, 13.3 Hz, 1H), 5.07 (q, *J* = 9.8 Hz, 2H), 4.54 - 4.44 (m, 2H), 4.36 - 4.28 (m, 1H), 4.16 (td, *J* = 3.0, 10.7 Hz, 1H), 3.57 - 3.51 (m, 2H), 3.13 - 3.03 (m, 1H), 281 (br d, *J =* 16.3 Hz, 1H), 2.47 - 2.39 (m, 1H), 2.10 - 2.04 (m, 1H), 0.88 - 0.84 (m, 2H), 0.00 (s, 9H).

Step 2. To a solution of 3-(1-oxo-5-((*S*)-2-oxo-3-phenyl-4-(trifluoromethyl)imidazolidin-1-yl)isoindolin-2-yl)-1-((2-(trimethylsilyl)ethoxy)methyl)piperidine-2,6-dione (200 mg, 331 µmol, 1.00 *eq*) in dichloromethane (2.00 mL) was added trifluoroacetic acid (616 mg, 5.40 mmol, 400 µL, 16.2 *eq*). Then the mixture was stirred at 25 °C for 1 h. The mixture was concentrated in vacuum to give 1-(hydroxymethyl)-3-(1-oxo-5-((*S*)-2-oxo-3-phenyl-4-(trifluoromethyl)imidazolidin-1-yl)isoindolin-2-yl)piperidine-2,6-dione (160 mg, crude) as yellow oil.

Step 3. To a solution of 1-(hydroxymethyl)-3-(1-oxo-5-((*S*)-2-oxo-3-phenyl-4-(trifluoromethyl)imidazolidin-1-yl)isoindolin-2-yl)piperidine-2,6-dione (160 mg, 318 µmol, 1.00 *eq*) in acetonitrile (2.00 mL) was added ammonium hydroxide (400 µL). Then the mixture was stirred at 25 °C for 1 h. The mixture was adjusted pH to 5-6 by formic acid (0.2 mL) and filtered. The filtrate was concentrated in vacuum. The residue was purified by reversed phase column chromatography (C18, 40 g; condition: water/acetonitrile = 1/0 to 0/1, 0.1% formic acid) and lyophilized to afford 3-(1-oxo-5-((*S*)-2-oxo-3-phenyl-4-(trifluoromethyl)imidazolidin-1-yl)isoindolin-2-yl)piperidine-2,6-dione (41.09 mg, 86.1 µmol, 27% yield) as a white solid.

¹H NMR (400 MHz, DMSO-*d*₆) *δ* = 10.99 (s, 1H), 7.92 (br d, *J =* 7.3 Hz, 1H), 7.85 - 7.80 (m, 1H), 7.77 - 7.72 (m, 1H), 7.62 - 7.58 (m, 2H), 7.45 (t, *J* = 7.9 Hz, 2H), 7.30 - 7.24 (m, 1H), 5.67 - 5.59 (m, 1H), 5.11 (dd, *J* = 4.6 13.4 Hz, 1H), 4.50 - 4.42 (m, 2*H*), 4.37 - 4.29 (m, 1H), 4.14 (td, *J* = 3.2, 10.7 Hz, 1H), 2.97 - 2.87 (m, 1H), 2.60 (br dd, *J =* 2.4, 15.5 Hz, 1H), 2.47 - 2.39 (m, 1H), 2.05 - 1.97 (m, 1H).

### Example 10. Synthesis of Compound 113

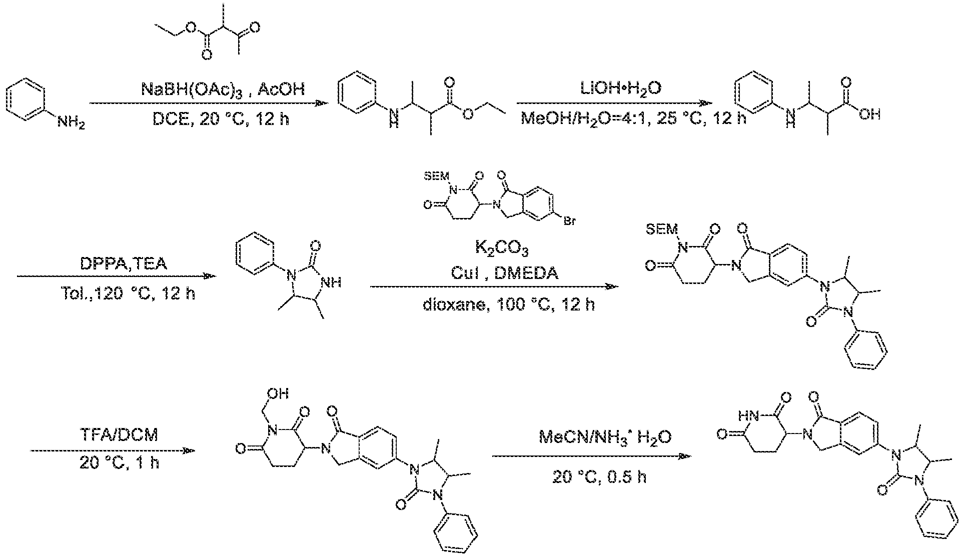

Step 1. To a mixture of aniline (1.00 g, 10.7 mmol, 980 µL, 1.00 *eq),* ethyl 2-methyl-3-oxo-butanoate (1.55 g, 10.7 mmol, 1.52 mL, 1.00 *eq*), and acetic acid (64.4 mg, 1.07 mmol, 61.4 µL, 0.100 *eq*) in 1, 2-dichloroethane (10.0 mL) was added sodium triacetoxyhydroborate (6.83 g, 32.2 mmol, 3.00 *eq*) in portions at 0 °C. The mixture was stirred at 20 °C for 12 h. The mixture was diluted with water (80 mL) and extracted with ethyl acetate (3 × 60 mL). The combined organic layer was washed with brine (60 mL), dried over sodium sulfate, filtered and concentrated in vacuum. The residue was purified by silica gel chromatography (petroleum ether/ethyl acetate = 10/1 to 1/1) and concentrated in vacuum to afford ethyl 3-anilino-2-methyl-butanoate (1.30 g, crude) as a yellow solid.

Step 2. To a solution of ethyl 3-anilino-2-methyl-butanoate (1.10 g, 4.97 mmol, 1.00 *eq*) in methanol (8.80 mL) and water (2.20 mL) was added lithium hydroxide (625 mg, 14.9 mmol, 3.00 *eq*), and then the mixture was stirred at 25 °C for 12 h. The mixture was concentrated in vacuum. The residue was quenched by water (100 mL) and extracted with ethyl acetate (3 ×50 mL). The aqueous phase was added hydrochloric acid (1 M, 100 mL) and extracted with ethyl acetate (3 × 50 mL). The combined organic layers were washed with brine (20 mL), dried over anhydrous sodium sulfate, filtered and concentrated under reduced pressure to give 3-anilino-2-methyl-butanoic acid (900 mg, 4.61 mmol, 92% yield) as yellow oil. MS (ESI) m/z 194.0 [M+H]⁺

Step 3. To a solution of 3-anilino-2-methyl-butanoic acid (800 mg, 4.14 mmol, 1.00 *eq*) in toluene (24.0 mL) was added diphenyl phosphoryl azide (18.2 g, 66.2 mmol, 14.3 mL, 16.0 *eq*) and triethylamine (502 mg, 4.97 mmol, 691 µL, 1.20 *eq*). Then the mixture was stirred at 120 °C for 12 h under nitrogen. The reaction mixture was diluted with water (50 mL) and extracted with ethyl acetate (3 × 20 mL). The combined organic layers were washed with brine (10 mL), dried over anhydrous sodium sulfate, filtered and concentrated under reduced pressure to give a residue, which was purified by column chromatography (SiO₂, petroleum ether/ethyl acetate = 50/1 to 1/1) and concentrated in vacuum to afford 4,5-dimethyl-1-phenyl-imidazolidin-2-one (600 mg, crude) as a yellow solid.

¹H NMR (400 MHz, DMSO-*d*₆) *δ* = 7.47 (br d, *J* = 7.8 Hz, 1H), 7.39 - 7.27 (m, 3H), 7.23 - 7.11 (m, 2H), 7.06 - 6.91 (m, 1H), 4.13 - 3.77 (m, 2H), 1.32 - 0.95 (m, 6H).

Step 4. To a solution of 3-(5-bromo-1-oxo-isoindolin-2-yl)-1-(2-trimethylsilylethoxymethyl)piperidine-2,6-dione (100 mg, 220 µmol, 1.00 *eq*) and 4,5-dimethyl-1-phenyl-imidazolidin-2-one (41.9 mg, 220 µmol, 1.00 *eq*) in dioxane (2.00 mL) was added potassium carbonate (91.4 mg, 661 µmol, 3.00 *eq*), cuprous iodide (4.20 mg, 22.0 µmol, 0.100 *eq*), and *N,N'*-dimethylethylenediamine (3.89 mg, 44.1 µmol, 4.75 µL, 0.200 *eq*). Then the mixture was stirred at 100°C for 12 h under nitrogen and the mixture was concentrated in vacuum. The residue was purified by reversed phase column chromatography (C18, 40 g; condition: water/acetonitrile = 1/0 to 0/1, 0.1% formic acid) and concentrated in vacuum to afford 3-(5-(4,5-dimethyl-2-oxo-3-phenylimidazolidin-1-yl)-1-oxoisoindolin-2-yl)-1-((2-(dimethylsilyl)ethoxy)methyl)piperidine-2,6-dione (60.0 mg, 106 µmol, 48% yield) as a yellow solid.

¹H NMR (400 MHz, DMSO-*d*₆) *δ* = 7.89 - 7.82 (m, 1H), 7.78 - 7.67 (m, 2H), 7.60 - 7.46 (m, 2H), 7.45 - 7.37 (m, 2H), 7.22 - 7.13 (m, 1H), 5.28 - 5.20 (m, 1H), 5.13 - 5.02 (m, 2H), 4.78 - 4.63 (m, 1H), 4.50 (br dd, *J* = 7.9, 16.9 Hz, 1H), 4.31 (br dd, *J* = 4.8, 17.4 Hz, 1H), 3.54 (br dd, *J* = 6.2, 7.4 Hz, 2H), 3.10 - 3.03 (m, 1H), 2.84 - 2.78 (m, 1H), 2.10 - 2.04 (m, 1H), 1.30 (br d, *J* = 5.4 Hz, 3H), 1.22 - 1.17 (m, 3H), 0.90 - 0.81 (m, 4H), 0.00 (s, 9H).

Step 5. To a solution of 3-(5-(4,5-dimethyl-2-oxo-3-phenylimidazolidin-1-yl)-1-oxoisoindolin-2-yl)-1-((2-(dimethylsilyl)ethoxy)methyl)piperidine-2,6-dione (100 mg, 177 µmol, 1.00 *eq*) in dichloromethane (2.00 mL) was added trifluoroacetic acid (616 mg, 5.40 mmol, 0.400 mL, 30.4 *eq*). Then the mixture was stirred at 25 °C for 1 h. The mixture was concentrated in vacuum to afford 3-(5-(4,5-dimethyl-2-oxo-3-phenylimidazolidin-1-yl)-1-oxoisoindolin-2-yl)-1-(hydroxymethyl)piperidine-2,6-dione (80.0 mg, crude) as a white solid. MS (ESI) m/z 463.3 [M+H]⁺

Step 6. A mixture of 3-(5-(4,5-dimethyl-2-oxo-3-phenylimidazolidin-1-yl)-1-oxoisoindolin-2-yl)-1-(hydroxymethyl)piperidine-2,6-dione (80.0 mg, 172 µmol, 1.00 *eq*) in acetonitrile (2.00 mL) and ammonium hydroxide (0.0400 mL) was stirred at 20 °C for 0.5 h. The mixture was adjusted to pH < 7 by formic acid (0.500 mL) and concentrated in vacuum. The residue was purified by reversed-phase HPLC (column: Phenomenex Synergi C18 150*25mm* 10µm; mobile phase: [water(0.225% formic acid)-acetonitrile]; B%: 35%-55%,8min) and lyophilized to afford 3-(5-(4,5-dimethyl-2-oxo-3-phenylimidazolidin-1-yl)-1-oxoisoindolin-2-yl)piperidine-2,6-dione (10.06 mg, 23.03 µmol, 13% yield) as a white solid.

¹H NMR (400 MHz, DMSO-*d*₆) δ = 10.99 (br s, 1H), 8.51 (s, 0,01H), 7.87 - 7.80 (m, 1H), 7.77 - 7.66 (m, 2H), 7.60 - 7.45 (m, 2H), 7.45 - 7.37 (m, 2H), 7.22 - 7.12 (m, 1H), 5.16 - 5.06 (m, 1H), 4.78 - 4.60 (m, 1H), 4.53 - 4.44 (d, *J* = 7.4 Hz, 1H), 4.39 - 4.29 (m, 1H), 4.28 - 4.15 (m, 1H), 2.97 - 2.88 (m, 1H), 2.63 - 2.59 (m, 1H), 2.47 - 2.37 (m, 1H), 2.01 (br dd, *J =* 4.9, 6.6 Hz, 1H), 1.18 (dd, *J* = 6.3, 16.8 Hz, 6H).

### Example 11. Synthesis of Compound 117

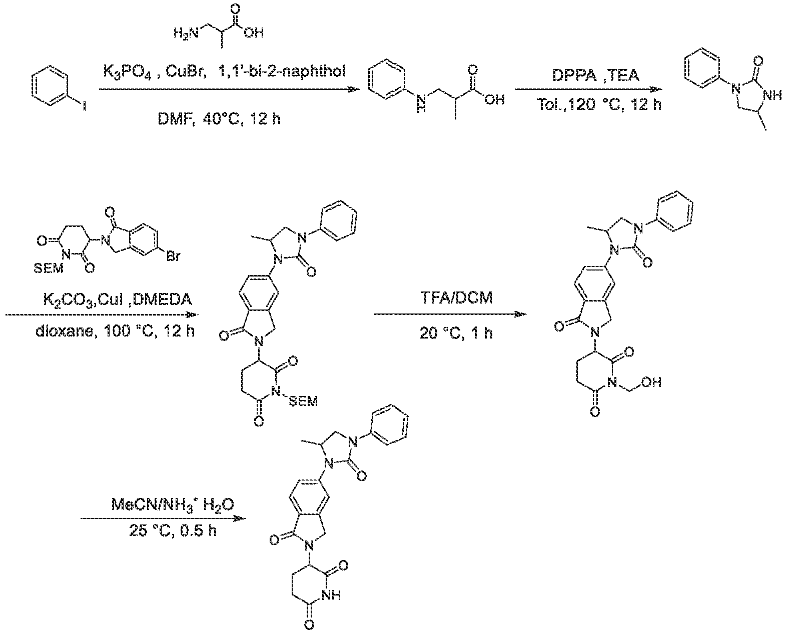

Step 1. To a solution of 3-amino-2-methyl-propanoic acid (1.52 g, 14.7 mmol, 1.50 *eq*), iodobenzene (2.00 g, 9.80 mmol, 1.09 mL, 1.00 *eq*), potassium phosphate (4.16 g, 19.6 mmol, 2.00 *eq*), 1-(2-hydroxy-1-naphthyl)naphthalen-2-ol (561 mg, 1.96 mmol, 0.200 *eq*) in dimethyformamide (40.0 mL) was added cuprous bromide (281 mg, 1.96 mmol, 59.7 µL, 0.200 *eq*) under nitrogen. The mixture was stirred at 40 °C for 12 h. The mixture was added water (250 mL) and extracted with ethyl acetate (3 × 100 mL). The aqueous phase was adjusted pH<7 with hydrochloric acid (1.00 M in water) (100 mL) and extracted with ethyl acetate (3 × 100 mL), the combined organic phase was washed with brine (50 mL), dried with anhydrous sodium sulfate, filtered and concentrated in vacuum to give 2-methyl-3-(phenylamino)propanoic acid (1.35 g, 7.53 mmol, 77% yield) as brown oil.

¹H NMR (400 MHz, DMSO-*d*₆) *δ* = 12.37 - 11.56 (m, 1H), 6.83 (dd, *J* = 7.4, 8.2 Hz, 2H), 6.34 (d, *J* = 8.5 Hz, 2H), 6.29 (t, *J* = 7.3 Hz, 1H), 5.85 - 4.95 (m, 1H), 3.05 (br d, J= 7.0 Hz, 1H), 2.76 (dd, *J* = 6.7, 13.1 Hz, 1H), 2.43 - 2.36 (m, 1H), 0.88 (d, *J* = 7.0 Hz, 3H).

Step 2. To a solution of 2-methyl-3-(phenylamino)propanoic acid (1.00 g, 5.58 mmol, 1.00 *eq*), diphenylphosphoryl azide (4.61 g, 16.7 mmol, 3.63 mL, 3.00 *eq*) in toluene (5.00 mL) was added triethylamine (1.13 g, 11.2 mmol, 1.55 mL, 2.00 *eq*) under nitrogen. The mixture was stirred at 120 °C for 12 h. The mixture was concentrated under reduced pressure. The residue was purified by silica gel chromatography (Petroleum ether/Ethyl acetate=1/0 to 2/1) to give 4-methyl-1-phenylimidazolidin-2-one (500 mg, 2.84 mmol, 51% yield) as a white solid.

¹H NMR (400 MHz, DMSO-*d*₆) *δ* = 7.53 (d, *J =* 8.6 Hz, 2H), 7.31 - 7.26 (m, 2H), 7.08 (br s, 1H), 7.00 - 6.92 (m, 1H), 3.99 - 3.89 (m, 1H), 3.86 - 3.71 (m, 1H), 3.36 (br d, *J* = 6.4 Hz, 1H), 1.19 (d, *J* = 6.0 Hz, 3H),

Step 3. To a solution of 4-methyl-1-phenyl-imidazolidin-2-one (50.0 mg, 283 µmol, 1.00 *eq*), 3-(5-bromo-1-oxoisoindolin-2-yl)-1-((2-(trimethylsilyl)ethoxy)methyl)piperidine-2,6-dione (142 mg, 312 µmol, 1*.*10 *eq*), and potassium carbonate (117 mg, 851 µmol, 3.00 *eq*) in dioxane (2.00 ml) was added copper iodide (5.40 mg, 28.4 µmol, 0.100 *eq*) and *N,N'*-dimethylethylenediamine (5.00 mg, 56.8 µmol, 6.11 µL, 0.200 *eq*) under nitrogen. The mixture was stirred at 100 °C for 12 h. The mixture was added water (50 mL) and extracted with ethyl acetate (3 × 40 mL), the combined organic phase was washed with brine (50 mL), dried with anhydrous sodium sulfate, filtered and concentrated in vacuum. The mixture was purified by reversed-phase HPLC (0.1% formic acid condition) and lyophilized to give 3-(5-(5-methyl-2-oxo-3-phenylimidazolidin-1-yl)-1-oxoisoindolin-2-yl)-1-((2-(trimethylsilyl)ethoxy)methyl)piperidine-2,6-dione (10.0 mg, 18.2 µmol, 6% yield) as a yellow solid.

¹H NMR (400 MHz, DMSO-*d*₆) *δ* = 7.86 (s, 1H), 7.72 (q, *J =* 8.1 Hz, 2H), 7.63 (d, *J =* 7.9 Hz, 2H), 7.38 (t, *J* = 8.1 Hz, 2H), 7.09 (t, *J* = 7.4 Hz, 1H), 5.23 (td, *J* = 4.4, 13.3 Hz, 1H), 5.10 - 5.01 (m, 2H), 4.78 - 4.68 (m, 1H), 4.49 (dd, *J* = 8.6, 17. 0 Hz, 1H), 4.30 (dd, *J* = 6.4, 17.1 Hz, 1H), 4.20 (t, *J* = 9.1 Hz, 1H), 3.67 - 3.58 (m, 1H), 3.56 - 3.50 (m, 2H), 3.11 - 3.04 (m, 1H), 2.83 - 2.74 (m, 1H), 2.40 (br dd, *J =* 3.9, 13.3 Hz, 1H), 2.09 - 2.03 (m, 1H), 1.32 (d, *J* = 6.0 Hz, 3H), 0.89 - 0.82 (m, 2H), -0.01 - -0.03 (m, 9H).

Step 4. A solution of 1-(hydroxymethyl)-3-(5-(5-methyl-2-oxo-3-phenylimidazolidin-1-yl)-1-oxcisoindolin-2-yl)piperidine-2,6-dione (5.00 mg, 11.2 µmol, 1.00 *eq*) in ammonium hydroxide (0.0200 mL) and acetonitrile (1.00 mL) was stirred at 25 °C for 0.5 h. The mixture was adjusted pH=3 with formic acid. The mixture was concentrated under reduced pressure. The residue was purified by reversed-phase HPLC ( 0.1%) formic acid condition) and lyophilized to give 3-(5-(5-methyl-2-oxo-3-phenylimidazolidin-1-yl)-1-oxoisoindolin-2-yl)piperidine-2,6-dione (2.33 mg, 4.97 µmol, 45%) yield) as a white solid.

¹H NMR (400 MHz, DMSO-*d*₆) *δ* = 11.10 - 10.87 (m, 1H), 8.34 (s, 0.4H), 7.85 (br s, 1H), 7.76 - 7.68 (rn, 2H), 7.63 (br d, *J* = 8.1 Hz, 2H), 7.38 (t, *J =* 7.6 Hz, 2H), 7.14 - 7.05 (m, 1H), 5.16 - 5.01 (m, 1H), 4.77 - 4.67 (m, 1H), 4.51 - 4.42 (m, 1H), 4.38 - 4.28 (m, 1H), 4.20 (t, *J =* 9.1 Hz, 1H), 3.61 (br dd, *J =* 4.9, 9.1 Hz, 1H), 2.97 - 2.88 (m, 1H), 2.58 (br d, *J* = 2. 0 Hz, 1H), 2.44 - 2.34 (m, 1H), 2.04 - 1.94 (m, 1H), 1.31 (d, *J* = 6. 0 Hz, 3H).

### Example 12. Synthesis of Compound 122

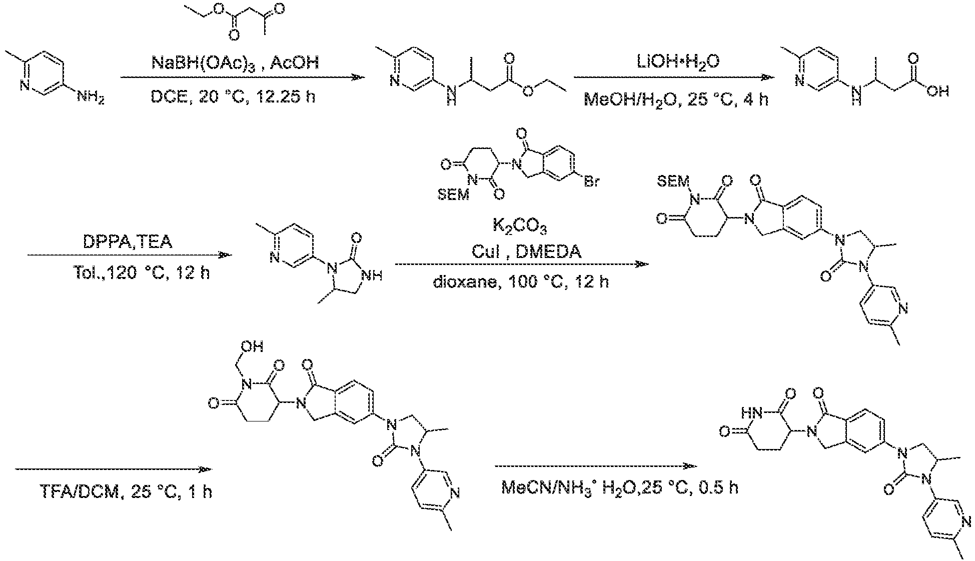

Step 1. To a solution of ethyl 3-oxobutanoate (7.22 g, 55.4 mmol, 7.01 mL, 1.20 *eq)* in 1, 2-dichloroethane (200 mL,) was added acetic acid (277 mg, 4.62 mmol, 264 µL, 0.100 *eq*). The mixture was stirred at 20 °C for 10 min. The 6-methylpyridin-3-amine (5.00 g, 46.2 mmol, 1.00 *eq*) was added into the mixture and the mixture was stirred at 20 °C for 5 min. At last, sodium triacetoxyhydroborate (29.4 g, 138 mmol, 3.00 *eq*) was added into the mixture and the mixture was stirred at 20 °C for 12 h under nitrogen. The reaction mixture was diluted with water (200 mL) and extract with ethyl acetate (2 × 300 mL). The organic phase was separated, washed with brine (50 mL), dried over sodium sulfate, filtered and concentrated under reduced pressure to give a residue which was purified by reversed-phase HPLC (column: spherical C18, 20-45 um, 100Å, SW 330, mobile phase: [water(0.1%Formic Acid)-ACN) to give ethyl 3-((6-methylpyridin-3-yl)amino)butanoate (2.70 g, 12.1 mmol, 26% yield) as yellow liquid.

¹H NMR (400 MHz, DMSO-*d*₆) *δ* = 7.83 (d, *J* = 2.3 Hz, 1H), 7.02 - 6.98 (m, 1H), 6.95 - 6.89 (m, 1H), 5.56 (br d, *J* = 7.3 Hz, 1H), 4.04 (q, *J* = 7.1 Hz, 2H), 3.87 - 3.75 (m, 1H), 2.54 (br d, *J* = 6.4 Hz, 1H), 2.37 (dd, *J* = 6.9, 15.0 Hz, 1H), 2.30 (s, 3H), 1.19 - 1.12 (m, 6H).

Step 2. To a solution of ethyl 3-((6-methylpyridin-3-yl)amino)butanoate (1.50 g, 6.75 mmol, 1.00 *eq*) in methanol (16.0 mL) and water (4.00 mL) was added lithium hydroxide monohydrate (991 mg, 23.6 mmol, 3.50 *eq*). The mixture was stirred at 25 °C for 4 h. The reaction mixture was concentrated under reduced pressure to give a residue which was purified by reversed-phase HPLC (column: spherical C18, 20-45 um, 100Å, SW 330, mobile phase: [water (0. 1%Formic Acid)- acetonitrile) to give 3-((6-methylpyridin-3-yl)amino)butanoic acid (1.40 g, crude) as colorless liquid.

¹H NMR. (400 MHz, DMSO-*d*₆) *δ* = 8.14 (s, 1H), 7.83 (d, *J* = 2.8 Hz, 1H), 7.03 - 6.99 (m, 1H), 6.95 - 6.91 (m, 1H), 5.74 - 5.46 (m, 1H), 3.81 - 3.72 (m, 1H), 2.30 (s, 3H), 2.07 (s, 2H), 1.14 (d, *J* = 6.3 Hz, 3H).

Step 3. To a solution of 3-((6-methylpyridin-3-yl)amino)butanoic acid (1.30 g, 6.69 mmol, 1.00 *eq),* diphenyl phosphoryl azide (5.53 g, 20.0 mmol, 4.35 mL, 3.00 *eq)* in toluene (20.0 mL) was triethylamine (2.03 g, 20.0 mmol, 2.79 mL, 3.00 *eq).* The mixture was stirred at 1 20 °C for 12 h. The reaction mixture was concentrated under reduced pressure to give a residue which was purified by reversed-phase HPLC (column: spherical C18, 20-45 µm, 100Å, SW 330, mobile phase: [water(0.1%Formic Acid)- acetonitrile) to give 5-methyl-1-(6-methylpyridin-3-yl)imidazolidin-2-one (200 mg, 1.05 mmol, 15% yield) as a yellow solid.

¹H NMR (400 MHz, DMSO-*d*₆) *δ* = 8.52 (d, *J* = 2.5 Hz, 1H), 7.19 (d, *J* = 8.5 Hz, 1H), 6.96 (br s, 1H), 5.67 (br s, 1H), 4.51 - 4.44 (m, 1H), 3.59 - 3.55 (m, 1H), 3.02 - 3.00 (m, 1H), 2.41 (s, 3H), 1.31 - 1.24 (m, 3H).

Step 4. To a solution of trifluoroacetic acid (1.00 mL) in dichloromethane (5.00 mL) was added 3-(5-(4-methyl-3-{6-methylpyridin-3-yl)-2-oxoimidazolidin-1-yl)-1-oxoisoindolin-2-yl)-1-((2-(trimethylsilyl)ethoxy)methyl) piperidine-2,6-dione (90.0 mg, 159 µmol, 1.00 *eq*)*.* The mixture was stirred at 25 °C for 1 h. The reaction mixture was concentrated under reduced pressure to give 1-(hydroxymethyl)-3-(5-(4-methyl-3-(6-methylpyridin-3-yl)-2-oxoimidazolidin-1-yl)-1-oxoisoindolin-2-yl)piperidine-2,6-dione (70.0 mg, crude) as a white solid. MS (ESI) m/z 464.4 [M-H]⁺

Step 5. To a solution of 1-(hydroxymethyl)-3-(5-(4-methyl-3-(6-methylpyridin-3-yl)-2-oxoimidazolidin-1-yl)-1-oxoisoindolin-2-yl)piperidine-2,6-dione (70.0 mg, 151 µmol, *1.00 eq*) was added acetonitrile (10.0 mL) and ammonia hydrate (0.200 mL) at 25 °C. The mixture was stirred at 25 °C for 0.5 h. The reaction mixture was concentrated under reduced pressure to give a residue which was purified by *prep*-HPLC (column: Phenomenex luna C18 150*25mm* 10um;mobile phase: [water(FA)-ACN];B%: 1%-28%,8min) to give 3-(5-(4-methyl-3-(6-methylpyridin-3-yl)-2-oxoimidazolidin-1-yl)-1-oxoisoindolin-2-yl)piperidine-2,6-dione (21.1 mg, 48.8 µmol, 32% yield) as an off-white solid.

¹H NMR (400 MHz, DMSO-*d*₆) *δ* = 10.97 (br s, 1H), 8.62 (d, *J* = 2.3 Hz, 1H), 7.88 (br s, 1H), 7.82 (dd, *J* = 2.8, 8.4 Hz, 1H), 7.79 - 7.74 (m, 1H), 7.73 - 7.70 (m, 1H), 7.30 (d, *J* = 8.5 Hz, 1H), 5.10 (dd, *J* = 5.1, 13.3 Hz, 1H), 4.64 (td, *J* = 6.0, 8.7 Hz, 1H), 4.49 - 4.41 (m, 1H), 4.35 - 4.29 (m, 1H), 4.26 (dt, *J* = 2.1, 9.2 Hz, 1H), 3.70 - 3.64 (m, 1H), 2.96 - 2.87 (m, 1H), 2.63 - 2.57 (m, 1H), 2.46 (s, 3H), 2.40 (br dd, *J* = 4.6, 13.3 Hz, 1H), 2.05 - 1.97 (m, 1H), 1.27 (d, *J* = 6.0 Hz, 3H).

### Example 13. Synthesis of Compound 121

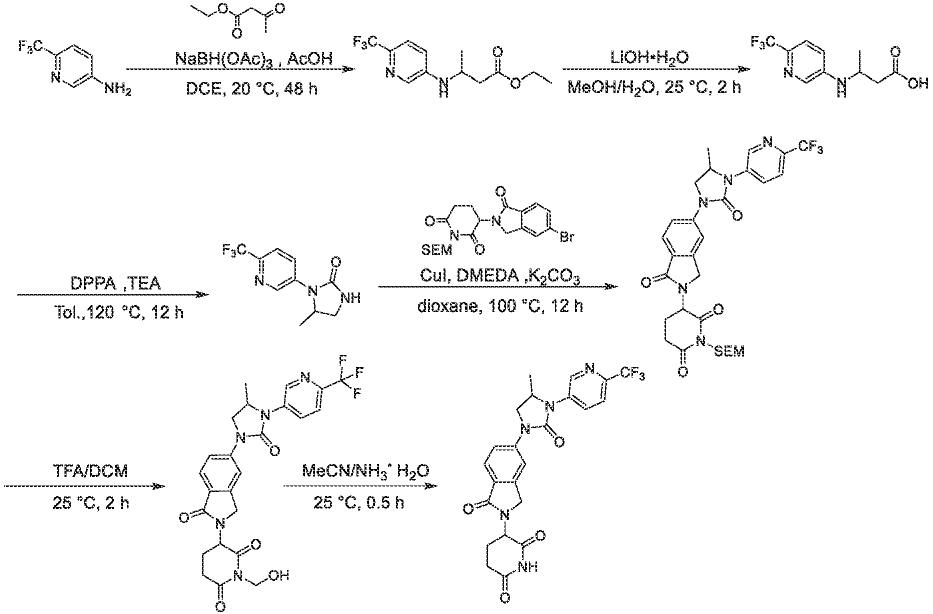

Step 1. To a solution of ethyl 3-oxobutanoate (1.93 g, 14.8 mmol, 1.87 mL, 1.20 *eq*), acetic acid (74.1 mg, 1.23 mmol, 70.6 µL, 0.100 *eq*), and 6-(trifluoromethyl)pyridin-3-amine (2.00 g, 12.3 mmol, 1.00 *eq)* in 1,2-dichloroethane (20.0 mL) was added sodium triacetoxyhydroborate (7.84 g, 37.0 mmol, 3.00 *eq*) at 0 °C. The mixture was stirred at 20 °C for 48 h. The mixture was added water (500 mL) and extracted with ethyl acetate (3 × 150 mL), the combined organic phase was washed with brine (200 mL), dried with anhydrous sodium sulfate, filtered and concentrated in vacuum. The residue was purified by column chromatography (silicon dioxide, Petroleum ether/Ethyl acetate=1/0 to 10/1) and further purified by reversed-phase HPLC (0.1% formic acid condition) and lyophilized to give ethyl 3-((6-(trifluoromethyl)pyridin-3-yl)amino)butanoate (1.60 g, 5.79 mmol, 47% yield) as yellow oil.

¹H NMR (400 MHz, DMSO-*d*₆) *δ* = 8.05 - 7.99 (m, 1H), 7.50 (dd, *J* = 5.1, 8.6 Hz, 1H), 6.63 - 6.52 (m, 1H), 4.04 (q, *J* = 6.7 Hz, 3H), 3.15 - 3.05 (m, 1H), 2.54 - 2.50 (m, 1H), 2.33 (d, *J* = 6.9 Hz, 1H), 1.20 - 1.14 (m, 6H).

Step 2. To a solution of ethyl 3-((6-(trifluoromethyl)pyridin-3-yl)amino)butanoate (1.40 g, 5.07 mmol, 1 .00 *eq*) in methanol (8.00 mL) and water (2.00 mL) was added lithium hydroxide monohydrate (851 mg, 20.3 mmol, 4.00 *eq).* The mixture was stirred at 25 °C for 2 h. The mixture was added water (50 mL) and extracted with ethyl acetate (3 × 30 mL). The aqueous phase was adjusted pH<7 with hydrochloric acid (1.00 M in water) (30 mL) and extracted with ethyl acetate (3 × 30 mL), the combined organic phase was washed with brine (20 mL), dried with anhydrous sodium sulfate, filtered and concentrated in vacuum to give 3-((6-(trifluoromethyl)pyridin-3-yl)amino)butanoic acid (350 mg, 1.34 mmol, 26 % yield) as a yellow solid.

¹H NMR. (400 MHz, DMSO-*d*₆) *δ* = 12.56 (s, 1H), 8.04 (d, *J* = 2.6 Hz, 1H), 7.52 (d, *J* = 8.6 Hz, 1H), 7.10 (d, *J* = 15.6 Hz, 1H), 6.68 - 6.43 (m, 1H), 3.97 - 3.78 (m, 1H), 2.46 (br s, 1H), 2.40 - 2.35 (m, 1H), 1.18 (d, *J* = 6.4 Hz, 3H).

Step 3. To a solution of 3-((6-(trifluoromethyl)pyridin-3-yl)amino)butanoic acid (300 mg, 1.21 mmol, 1.00 *eq*) in toluene (10.0 mL) was added diphenylphosphoryl azide (998 mg, 3.63 mmol, 786 µL, 3.00 *eq*) and triethylamine (245 mg, 2.42 mmol, 336 µL, 2.00 *eq*) under nitrogen. The mixture was stirred at 120 °C for 12 h. The mixture was added water (50 mL) and extracted with ethyl acetate (3 × 50 mL), the combined organic phase was washed with brine (20 mL), dried with anhydrous sodium sulfate, filtered and concentrated in vacuum. The mixture was purified by reversed-phase HPLC ( 0.1% formic acid condition) and lyophilized to give 5-methyl-1-(6-(trifluoromethyl)pyridin-3-yl)imidazolidin-2-one (160 mg, 652 µmol, 54% yield) as a white solid.

¹H NMR (400 MHz, DMSO-*d*₆) *δ* = 8.93 (d, *J* = 2.4 Hz, 1H), 8.20 (dd, *J* = 2.4, 8.8 Hz, 1H), 7.83 (d, *J* = 8.8 Hz, 1H), 7.42 - 7.34 (m, 1H), 4.63 (ddd, *J* = 4.3, 6.0, 8.5 Hz, 1H), 3.64 (t, *J* = 8.8 Hz, 1H), 3.07 (dd, *J* = 4.3, 8.9 Hz, 1H), 1.25 (d, *J* = 6.0 Hz, 3H).

Step 4. To a solution of 5-methyl-1-(6-(trifluoromethyl)pyridin-3-yl)imidazolidin-2-one (50.0 mg, 204 µmol, 1.00 *eq*)*,* 3-(5-bromo-1-oxoisoindolin-2-yl)-1-((2-(trimethylsilyl)ethoxy)methyl)piperidine-2,6-dione (102 mg, 224 µmol, 1.10 *eq*), and potassium carbonate (84.5 mg, 612 µmol, 3.00 *eq*) in dioxane (2.00 mL) was added copper iodide (3.88 mg, 20.4 µmol, 0.100 *eq*) and *N,N*'-dimethylethylenediamine (3.60 mg, 40.8 µmol, 4.39 µL, 0.200 *eq*) under nitrogen. The mixture was stirred at 100 °C for 12 h. The mixture was concentrated under reduced pressure. The residue was purified by reversed-phase HPLC ( 0.1% formic acid condition) and lyophilized to give 3-(5-(4-methyl-2-oxo-3-(6-(trifluoromethyl)pyridin-3-yl)imidazolidin-1-yl)-1-oxcisoindolin-2-yl)-1-((2-(trimethylsilyl)ethoxy)methyl)piperidine 2,6-dione (130 mg, 210 µmol, 34% yield) as yellow oil.

¹H NMR(400 MHz, DMSO-*d*₆) *δ* = 8.27 (dd, *J* = 2.1, 8.6 Hz, 1H), 7.94 (d, *J* = 8.9 Hz, 2H), 7.82 - 7.75 (m, 2H), 5.22 (dd, *J* = 4.8, 13.0 Hz, 1H), 5.10 - 5.03 (m, 2H), 4.85 - 4.78 (m, 1H), 4.56 - 4.47 (m, 1H), 4.34 - 4.27 (m, 2H), 3.78 - 3.72 (m, 1H), 3.53 (br dd, *J* = 3.1, 7.4 Hz, 2H), 3.12 - 3.01 (m, 2H), 2.87 - 2.76 (m, 2H), 2.10 - 2.03 (m, 1H), 1.35 (d, *J* = 6.1 Hz, 3H), 0.87 - 0.82 (m, 2H), 0.02 (s, 9H).

Step 5. A solution of 1-(hydroxymethyl)-3-(5-(4-methyl-2-oxo-3-(6-(trifluoromethyl)pyridin-3-yl)imidazolidin-1-yl)-1-oxoisoindolin-2-yl)piperidine-2,6-dione (50.0 mg, 96.6 µmol, 1.00 *eq*) in ammonium hydroxide (0.0200 mL) and acetonitrile (1.00 mL) was stirred at 25 °C for 0.5 h. The mixture was adjusted pH=3 with formic acid. The mixture was concentrated under reduced pressure. The mixture was purified by reversed-phase HPLC ( 0.1% formic acid condition) and lyophilized to give 3-(5-(4-methyl-2-oxo-3-(6-(trifluoromethyl)pyridin-3-yl)imidazolidin-1-yl)-1-oxoisoindolin-2-yl)piperidine-2,6-dione (39.0 mg, 81.2 µmol, 84% yield) as a white solid.

¹H NMR (400 MHz, DMSO-*d*₆) *δ* = 10.98 (br s, 1H), 9.05 (d, *J* = 2.3 Hz, 1H), 8.27 (dd, *J* = 2.3, 8.7 Hz, 1H), 7.96 - 7.87 (m, 2H), 7.82 - 7.70 (m, 2H), 5.14 - 5.06 (m, 1H), 4.85 - 4.75 (m, 1H), 4.50 - 4.41 (m, 1H), 4.38 - 4.28 (m, 2H), 3.78 - 3.70 (m, 1H), 2.98 - 2.88 (m, 1H), 2.60 (br dd, *J* = 2.1, 15.4 Hz, 1H), 2.44 - 2.35 (m, 1H), 2.05 - 1.97 (m, 1H), 1.35 (d, *J* = 6.0 Hz, 3H).

### Example 14. Synthesis of Compound 120

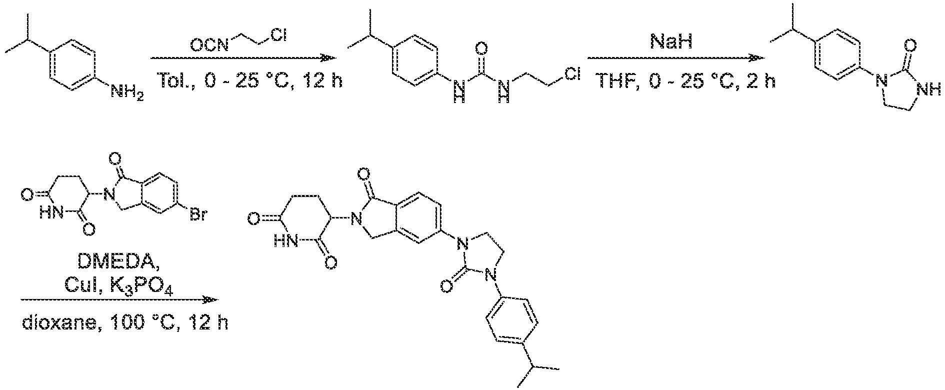

Step 1. To a solution of 4-isopropylaniline (500 mg, 3.70 mmol, 526 µL, 1.00 *eq*) in toluene (8.00 mL) was added 1-chloro-2-isocyanato-ethane (585 mg, 5.55 mmol, 1.50 *eq*) at 0 °C. The mixture was stirred at 25 °C for 12 h. The mixture was filtered to give a filter cake, then it was dried under reduced pressure to give 1-(2-chloroethyl)-3-(4-isopropylphenyl)urea (800 mg, 3.32 mmol, 90% yield) as a white solid.

¹H NMR (400 MHz, DMSO-*d*₆) *δ* = 8.50 (s, 1H), 7.33 - 7.26 (m, 2H), 7.13 - 7.04 (m, 2H), 6.37 - 6.25 (m, 1H), 3.69 - 3.59 (m, 2H), 3.47 - 3.36 (m, 2H), 2.85 - 2.73 (m, 1H), 1.21 - 1.10 (m, 6H).

Step 2. To a solution of 1-(2-chloroethyl)-3-(4-isopropylphenyl)urea (500 mg, 2.08 mmol, 1.00 *eq*) in tetrahydrofuran (10.0 mL) was added sodium hydride (125 mg, 3.12 mmol, 60% purity, 1.50 *eq*) at 0 °C under nitrogen atmosphere. The mixture was stirred at 25 °C for 2 h. The reaction mixture was quenched with methanol (10.0 mL). The mixture was concentrated under reduced pressure to give 1-(4-isopropylphenyl) imidazolidin-2-one (400 mg, 1.96 mmol, 94% yield) as a white solid.

¹H NMR (400 MHz, DMSO-*d*₆) *δ* = 7.52 - 7.33 (m, 2H), 7.07 (d, *J* = 8.6 Hz, 2H), 3.70 - 3.60 (m, 2H), 3.38 - 3.28 (m, 2H), 2.79 (quin, *J* = 6.9 Hz, 1H), 1.16 (d, *J* = 7.0 Hz, 6H).

Step 3. To a solution of 3-(5-bromo-1-oxo-isoindolin-2-yl)piperidine-2,6-dione (94.9 mg, 294 µmol, 1.20 *eq*), 1-(4-isopropylphenyl)imidazolidin-2-one (50.0 mg, 245 µmol, 1.00 *eq*) and potassium phosphate (156 mg, 734 µmol, 3.00 *eq*) in 1,4-dioxane (2.00 mL) was added copper(I) iodide (4.66 mg, 24.5 µmol, 0.100 *eq*) and *N,N'*-dimethylethylenediamine (4.32 mg, 48.9 µmol, 5.27 µL, 0.200 *eq*) under nitrogen atmosphere. The mixture was stirred at 100 °C for 12 h. The mixture was adjusted to pH=5-6 with formic acid and filtered, then the filtrate was concentrated under reduced pressure. The residue was purified by *Prep-*HPLC (column: Phenomenex luna C18 150*25nini* 10um; mobile phase: [water (formic acid) - acetonitrile]; B%: 40%-70%,9 min) to afford 3-(5-(3-(4-isopropylphenyl)-2-oxoimidazolidin-1-yl)-1-oxoisoindolin-2-yl)piperidine-2,6-dione (11.53 mg, 25.8 µmol, 5% yield) as an off-white solid.

¹H NMR (400 MHz, DMSO-*d*₆) *δ* = 10.98 (br s, 1H), 7.91 (s, 1H), 7.80 - 7.75 (m, 1H), 7.74 - 7.69 (m, 1H), 7.55 (d, *J* = 8.6 Hz, 2H), 7.25 (d, *J* = 8.6 Hz, 2H), 5.10 (dd, *J* = 5.1, 13.3 Hz, 1H), 4.49 - 4.42 (m, 1H), 4.36 - 4.28 (m, 1H), 4.07 - 3.97 (m, 4H), 2.97 - 2.84 (m, 2H), 2.64 - 2.57 (m, 1H), 2.43 - 2.37 (m, 1H), 2.05 - 1.96 (m, 1H), 1.20 (d, *J* = 6.9 Hz, 6H).

### Example 15. Synthesis of Compound 119

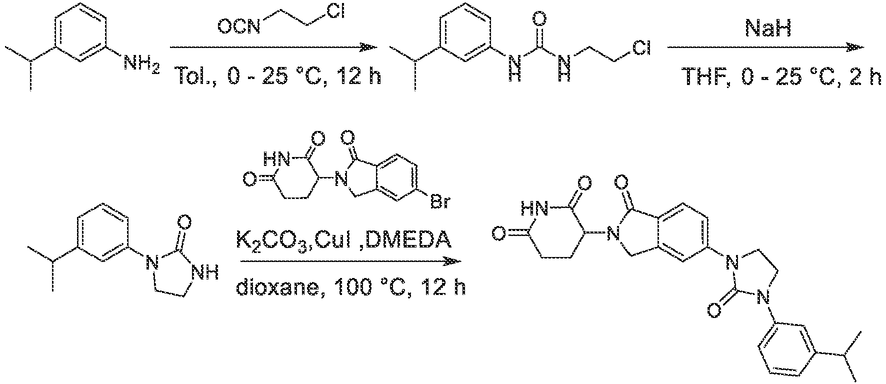

Step 1. To a solution of 3-isopropylaniline (3.00 g, 22.1 mmol, 3.13 mL, 1.00 *eq*) in toluene (30.0 mL) was added 1-chloro-2-isocyanatoethane (3.51 g, 33.2 mmol, 1.50 *eq*), and the resulting mixture was stirred at 25 °C for 12 h. The reaction mixture was filtered, and the filter cake was concentrated in vacuum to afford 1-(2-chloroethyl)-3-(3-isopropylphenyl)urea (5.00 g, 20.5 mmol, 92% yield) as a white solid.

¹H NMR (400 MHz, DMSO-*d*₆) *δ* = 7.28 (s, 1H), 7.22 - 7.10 (m, 3H), 6.79 (d, *J* = 7.5 Hz, 1H), 6.36 (br t, *J* = 5.8 Hz, 1H), 3.66 (t, *J* = 6.1 Hz, 2H), 3.42 (q, *J* = 6.1 Hz, 2H), 2.81 (td, *J* = 6.9, 13.8 Hz, 1H), 1.18 (d, *J* = 6.9 Hz, 6H).

Step 2. To a solution of 1-(2-chloroethyl)-3-(3-isopropylphenyl)urea (1.00 g, 4.15 mmol, 1.00 *eq*) in tetrahydrofuran (10.0 mL) was added sodium hydride (249 mg, 6.23 mmol, 60% purity, 1.50 *eq*) at 0 °C under nitrogen atmosphere, and then the mixture was stirred at 25 °C for 2 h. The reaction mixture was added in methanol (40 mL) and filtered. The filtrate was concentrated in vacuum to afford 1-(3-isopropylphenyl)imidazolidin-2-one (800 mg, 3.92 mmol, 94% yield) as a white solid.

¹H NMR(400 MHz, DMSO-*d*₆) *δ* = 7.52 (s, 1H), 7.23 (dd, *J* = 1.3, 8.2 Hz, 1H), 7.00 (t, *J* = 7.9 Hz, 1H), 6.52 (d, *J* = 7.5 Hz, 1H), 3.53 - 3.46 (m, 2H), 3.35 - 3.28 (m, 2H), 2.80 - 2.72 (m, 1H), 1.17 (d, *J* = 6.9 Hz, 6H).

Step 3. To a solution of 1-(3-isopropylphenyl)imidazolidin-2-one (34.7 mg, 170 µmol, 1.10 *eq*) and 3-(5-bromo-1-oxo-isoindolin-2-yl)piperidine-2,6-dione (50.0 mg, 154 µmol, 1.00 *eq*) in dioxane (2.00 mL) was added cuprous iodide (29.4 mg, 154 µmol, 1.00 *eq*), *N,N'*-dimethylethylenediamine (13.6 mg, 154 µmol, 16.6 µL, 1.00 *eq*), and potassium carbonate (64.1 mg, 464 µmol, 3.00 *eq*). Then the mixture was stirred at 100°C for 12 h under nitrogen atmosphere. The filtrate was purified by *prep*-HPLC (column: Phenomenex luna C18 150*25mm* 10um;mobile phase: [water(FA)-ACN];B%: 40%-70%,9min) and lyophilized to afford 3-(5-(3-(3-isopropylphenyl)-2-oxoimidazolidin-1-yl)-1-oxoisoindolin-2-yl)piperidine-2,6-dione (21.75 mg, 47.2 µmol, 6% yield) as a white solid.

¹H NMR (400 MHz, DMSO-*d*₆) *δ* = 11.09 (s, 1H), 8.04 (s, 1H), 7.91 - 7.86 (m, 1H), 7.85 - 7.80 (m, 1H), 7.70 (s, 1H), 7.52 (dd, *J* = 1.3, 8.3 Hz, 1H), 7.41 (t, *J* = 7.9 Hz, 1H), 7.10 (d, *J* = 7.5 Hz, 1H), 5.21 (dd, *J* = 5.1, 13.3 Hz, 1H), 4.60 - 4.53 (m, 1H), 4.47 - 4.40 (m, 1H), 4.15 (br s, 4H), 3.08 - 2.97 (m, 2H), 2.72 (br dd, *J* = 2.1, 15.4 Hz, 1H), 2.58 - 2.46 (m, 1H), 2.16 - 2.07 (m, 1H), 1.34 (d, *J* = 6.9 Hz, 6H).

### Example 16. Synthesis of Compound 116

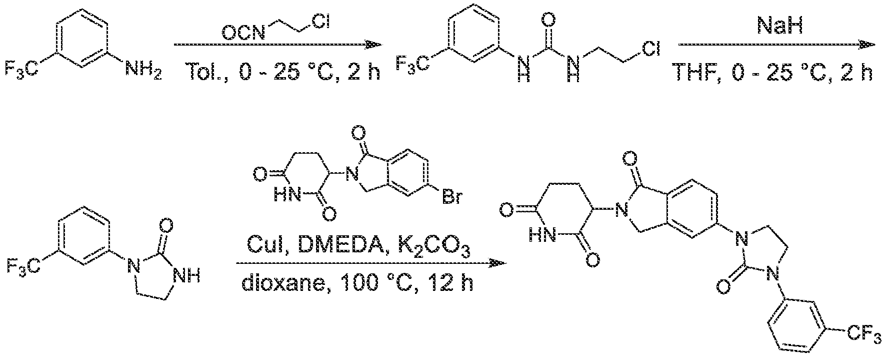

Step 1. A mixture of 3-(trifluoromethyl)aniline (5.00 g, 31.0 mmol, 3.88 mL, *1.00 eq)* in toluene (20.0 mL) was added 1-chloro-2-isocyanatoethane (3.93 g, 37.2 mmol, 3.17 mL, 1.20 *eq*) dropwise at 0 °C. The mixture was stirred at 25 °C for 2 h. The mixture was filterted and the filtrate was concentrated in vacuum to give 1-(2-chloroethyl)-3-(3-(trifluorornethyl)phenyl)urea (6.70 g, 25.1 mmol, 80% yield) as a white solid.

¹H NMR (400 MHz, DMSO-*d*₆) *δ* = 9.03 (s, 1H), 7.98 (s, 1H), 7.56 - 7.41 (m, 2H), 7.24 (br d, *J* = 7.4 Hz, 1H), 6.53 (br t, *J* = 5.6 Hz, 1H), 3.73 - 3.62 (m, 2H), 3.44 (q, *J* = 6.1 Hz, 2H).

Step 2. To a solution of 1-(2-chloroethyl)-3-(3-(trifluoromethyl)phenyl)urea (6.70 g, 25.1 mmol, 1.00 *eq*) in tetrahydrofuran (70.0 mL) was added sodium hydride (1.51 g, 37.7 mmol, 60.0% purity, 1.50 *eq*) at 0 °C under nitrogen. The mixture was stirred at 25 °C for 12 h. The reaction mixture was quenched with saturated ammonium chloride solution (50.0 mL) and extracted with ethyl acetate (3 × 20 mL). The combined organic layers were washed with brine (20 mL), dried over sodium sulfate, concentrated under reduced pressure. The residue was purified by reversed-phase HPLC (0.1% formic acid condition). The desired fraction was collected was extracted with ethyl acetate (3 × 20 mL). The combined organic layers were washed with brine (20 mL), dried over sodium sulfate, filtered and concentrated under reduced pressure to give 1-(3-(trifluoromethyl)phenyl)imidazolidin-2-one (120 mg, 521 µmol, 2% yield) as a white solid.

¹H NMR (400 MHz, DMSO-*d*₆) *δ* = 8.11 (s, 1H), 7.66 (br d, *J* = 8.3 Hz, 1H), 7.53 (t, *J* = 8.0 Hz, 1H), 7.30 (d, J = 7.6 Hz, 1H), 7.18 (br s, 1H), 3.90 (dd, *J* = 7.1, 8.9 Hz, 2H), 3.48 - 3.38 (m, 2H).

Step 3. To a mixture of 1-(3-(trifluoromethyl)phenyl)imidazolidin-2-one (28.5 mg, 124 µmol, 1.00 *eq*) and 3-(5-bromo-1-oxoisoindolin-2-yl)piperidine-2,6-dione (40.0 mg, 124 µmol, 1.00 *eq*) in dioxane (1.00 mL) was added *N,N'*-dimethylethylenediamine (2.18 mg, 24.8 µmol, 2.66 µL, 0.200 *eq*), copper iodide (2.36 mg, 12.4 µmol, 0.100 *eq*) and potassium carbonate (51.3 mg, 371 µmol, 3.00 *eq*). The mixture was stirred at 100 °C for 12 h under nitrogen. The mixture was concentrated in vacuum. The residue was added dimethyformamide (2.00 mL) and filterted. The filtrate was purified by reversed-phase HPLC (0.1% formic acid condition) and lyophilized to give 3-(1-oxo-5-(2-oxo-3-(3-(trifluoromethyl)phenyl)imidazolidin-1-yl)isoindolin-2-yl)piperidine-2,6-dione (20.0 mg, 42.3 µmol, 9% yield) as a white solid.

¹H NMR (400 MHz, DMSO-*d*₆) δ = 10.98 (br s, 1H), 8.21 (s, 1H), 7.95 (s, 1H), 7.80 - 7.76 (m, 2H), 7.75 - 7.71 (m, 1H), 7.63 (t, *J* = 8.0 Hz, 1H), 7.43 (d, *J* = 7.6 Hz, 1H), 5.10 (dd, *J* = 5.2, 13.3 Hz, 1H), 4.49 - 4.30 (m, 2H), 4.08 (s, 4H), 2.96 - 2.87 (m, 1H), 2.63 - 2.57 (m, 1H), 2.44 - 2.34 (m, 1H), 2.06 - 1.97 (m, 1H).

### Example 17. Synthesis of Compound 110

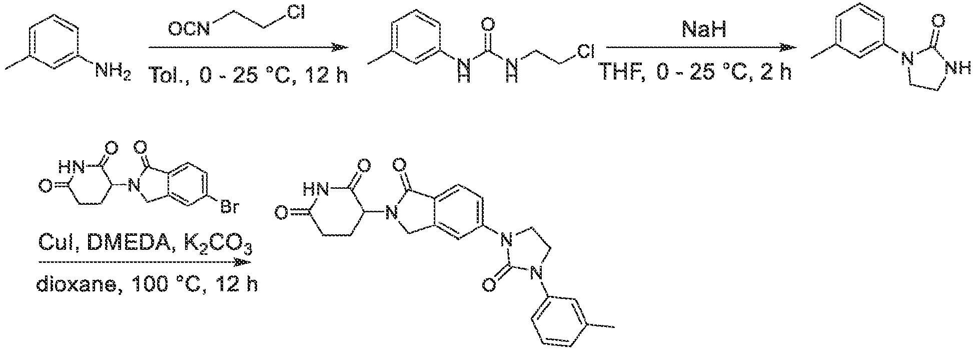

Step 1. To a mixture of *m*-toluidine (2.00 g, 18.6 mmol, 2.02 mL, 1.00 *eq)* in toluene (20.0 mL) was added dropwise 1-chloro-2-isocyanatoethane (2.36 g, 22.4 mmol, 1.91 mL, 1.20 *eq*) at 0 °C. The mixture was stirred at 25 °C for 2 h. The mixture was concentrated in vacuum to afford 1-(2-chloroethyl)-3-(m-tolyl)urea (3.46 g, 16.3 mmol, 87.1% yield) as a white solid which was used to the next step without further purification.

¹H NMR (400 MHz, DMSO-*d*₆) *δ* = 8.53 (s, 1H), 7.22 (s, 1H), 7.19 - 7.13 (m, 1H), 7.12 - 7.05 (m, 1H), 6.72 (br d, *J =* 7.3 Hz, 1H), 6.37 (br t, *J* = 5.2 Hz, 1H), 3.65 (t, *J* = 5.8 Hz, 2H), 3.40 (q, *J* = 5.8 Hz, 2H), 2.24 (s, 3H).

Step 2. To a mixture of 1-(2-chloroethyl)-3-(m-tolyl)urea (1.50 g, 7.05 mmol, *1.00 eq*) in tetrahydrofuran (15.0 mL) was added sodium hydride (423 mg, 10.5 mmol, 60.0% purity, 1.50 *eq*) at 0 °C. The mixture was stirred at 25 °C for 2 h under nitrogen atmosphere. The reaction mixture was quenched with saturated ammonium chloride solution (20 mL) and concentrated in vacuum to afford 1-(m-tolyl)imidazolidin-2-one (0.900 g, 5.11 mmol, 72% yield) as a white solid.

¹H NMR (400 MHz, DMSO-*d*₆) *δ* = 7.4 1 - 7.31 (m, 2H), 7.16 (t, *J* = 7.9 Hz, 1H), 6.92 (br s, 1H), 6.79 (d, *J* = 7.4 Hz, 1H), 3.85 - 3.76 (m, 2H), 3.40 - 3.35 (m, 2H), 2.27 (s, 3H).

Step 3. To a mixture of 1-(m-tolyl)imidazolidin-2-one (27.2 mg, 154 µmol, 1.00 *eq*) and 3-(5-bromo-1-oxoisoindolin-2-yl)piperidine-2,6-dione (50.0 mg, 154 µmol, 1.00 *eq*) in dioxane (1.00 mL) was added potassium carbonate (42.7 mg, 309 µmol, 2.00 *eq*)*, N,N'-*dimethylethylenediamine (2.73 mg, 30.9 µmol, 3.33 µL, 0.200 *eq*) and cuprous iodide (2.95 mg, 15.4 µmol, 0.100 *eq*)*.* The mixture was stirred at 100 °C for 12 h under nitrogen atmosphere. The mixture was concentrated in vacuum. The residue was diluted with dimethyformamide (5 mL) and filterted. The filtrate was purified by reversed-phase HPLC (0.1%) FA condition) and lyophilized to afford 3-(1-oxo-5-(2-oxo-3-(m-tolyl)imidazolidin-1-yl)isoindolin-2-yl)piperidine-2,6-dione (15.0 mg, 35.8 µmol, 6% yield) as a brown solid.

¹H NMR (400 MHz, DMSO-*d*₆) *δ* = 10.99 (br s, 1H), 8.51 - 8.38 (m, 0.1H), 7.93 (s, 1H), 7.79 - 7.69 (m, 2H), 7.51 - 7.42 (m, 2H), 7.26 (t, *J =* 7.8 Hz, 1H), 6.92 (br d, *J* = 7.6 Hz, 1H), 5.10 (br dd, *J* = 4.8, 13.3 Hz, 1H), 4.50 - 4.40 (m, 1H), 4.37 - 4.28 (m, 1H), 4.02 (br d, *J* = 5.5 Hz, 4H), 2.96 - 2.87 (m, 1H), 2.62 (br s, 1H), 2.40 (br dd, *J* = 3.9, 13.4 Hz, 1H), 2.33 (s, 3H), 2.06 - 1.97 (m, 1H).

### Example 18. Synthesis of Compound 111

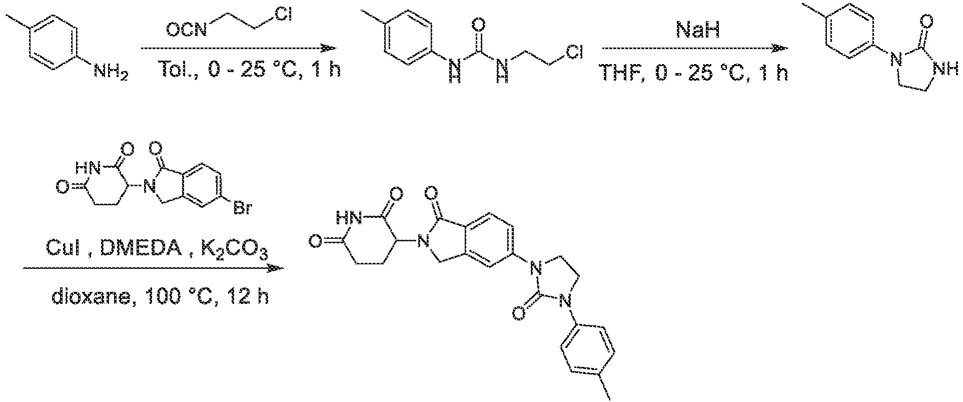

Step 1. To a mixture of *p*-toluidine (2.00 g, 18.7 mmol, 2.06 mL, *1.00 eq)* in toluene (20.0 mL) was added 1-chloro-2-isocyanatoethane (2.36 g, 22.4 mmol, 1.20 *eq)* dropwise at 0 °C. The mixture was stirred at 25 °C for 1 h. The mixture was filtered. The filter cake was washed with water (5 mL) and dried in vacuum to give 1-(2-chloroethyl)-3-(*p-*tolyl)urea (3.50 g, 16.5 mmol, 88% yield) as a white solid.

¹H NMR (400 MHz, DMSO-*d*₆) *δ* = 8.50 (s, 1H), 7.27 (d, *J* = 8.4 Hz, 2H), 7.02 (d, *J* = 8.4 Hz, 2H), 6.33 (t, *J =* 5.8 Hz, 1H). 3.64 (t, *J* = 6.1 Hz, 2H), 3.40 (q, *J* = 6.1 Hz, 2H), 2.21 (s, 3H).

Step 2. To a mixture of 1-(2-chloroethyl)-3-(*p*-tolyl)urea (1.50 g, 7.05 mmol, 1.00 *eq*) in anhydrous tetrahydrofuran (20.0 mL) was added sodium hydride (423 mg, 10.6 mmol, 60% purity, 1.50 *eq*) in portions at 0 °C. The mixture was stirred at 25 °C for 1 h under nitrogen atmosphere. The mixture was poured into methanol (100 mL) slowly and concentrated in vacuum. The residue was added methanol (20 mL) and filtered. The filter cake was washed acetonitrile (5 mL) and dried in vacuum to afford 1-(*p*-tolyl)imidazolidin-2-one (1.20 g, 6.81 mmol, 96% yield) as a white solid.

¹H NMR. (400 MHz, DMSO-*d*₆) *δ* = 7.42 (d, *J* = 8.6 Hz, 2H), 7.09 (d, *J* = 8.4 Hz, 2H), 3.79 (dd, *J* = 6.9, 8.9 Hz, 2H), 3.39 - 3.35 (m, 2H), 2.23 (s, 3H).

Step 3. To a mixture of 1-(*p*-tolyl)imidazolidin-2-one (32.7 mg, 186 µmol, 1.20 *eq*) and 3-(5-bromo-1- oxoisoindolin-2-yl)piperidine-2,6-dione (50.0 mg, 155 µmol, 1.00 *eq*)*,* potassium carbonate (64.2 mg, 464 µmol, 3.00 *eq*) in dioxane (2.00 mL) was added copper iodide (5.89 mg, 30.9 µmol, 0.200 *eq*) and *N,N*'-dimethylethylenediamine (5.46 mg, 61.9 µmol, 6.66 µL, 0.400 *eq*) in portions. The mixture was stirred at 100 °C for 12 h under nitrogen atmsophere. The mixture was concentrated in vacuum. The residue was added dimethylsulfoxide (4 mL) and filtered. The filtrate was purified by reverse phase chromatography (column: spherical C18, 20-45 µm, 100Å, SW 80, mobile phase: [water(0.1%Formic Acid)-ACN) and lyophilized to afford 3-(1-oxo-5-(2-oxo-3-(*p-*tolyl)imidazolidin-1-yl)isoindolin-2-yl) piperidine-2,6-dione (17.73 mg, 39.8 µmol, 6% yield) as an off-white solid.

¹H NMR (400 MHz, DMSO-*d*₆) *δ* = 10.96 (br s, 1H). 7.91 (s, 1H), 7.76 (s, 1H), 7.74 - 7.68 (m, 1H). 7.53 (d, *J* = 8.4 Hz, 2H), 7.19 (d, *J* = 8.4 Hz, 2H), 5.10 (dd, *J* = 5.1, 13.3 Hz, 1H), 4.49 - 4.40 (m, 1H). 4.38 - 4.27 (m, 1H). 4.08 - 3.96 (m, 4H), 2.97 - 2.86 (m, 1H), 2.64 - 2.57 (m, 1H), 2.47 - 2.35 (m, 1H). 2.29 (s, 3H), 2.07 - 1.96 (m, 1H).

### Example 19. Synthesis of Compound 112

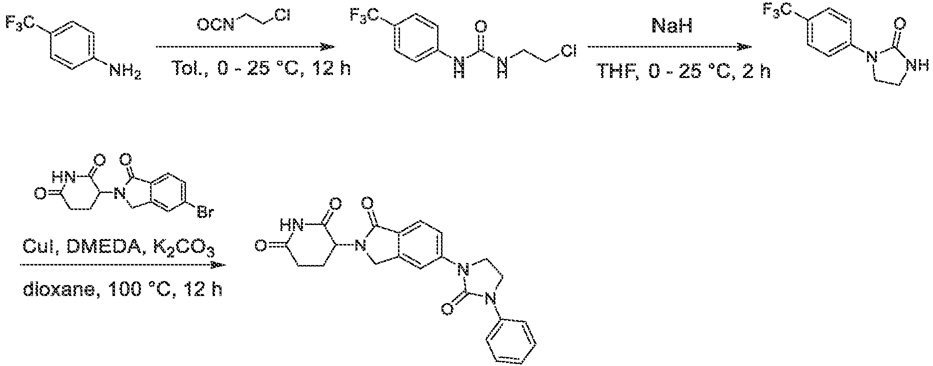

Step 1. To a solution of 4-(trifluoromethyl)aniline (2.00 g, 12.4 mmol, 1.54 mL, *1.00 eq)* in toluene (10.0 mL) was added 1-chloro-2-isocyanatoethane (1.57 g, 14.9 mmol, 1.27 mL, 1.20 *eq*) at 0 °C. The mixture was stirred at 25 °C for 12 h. The mixture was filtered to give a filter cake and concentrated under reduced pressure to afford 1-(2-chloroethyl)-3-(4-(trifluoromethyl)phenyl)urea (1.80 g, crude) as a white solid.

¹H NMR (400 MHz, DMSO-*d₆*) *δ* = 9. 07 (s, 1H), 7.62 - 7.58 (m, 2H), 7.58 (s, 2H), 6.54 (br t, *J* = 5.6 Hz,1H), 3.70 - 3.65 (m, 2H), 3.44 (q, *J =* 6.0 Hz, 2H)

Step 2. To a solution of 1-(2-chloroethyl)-3-(4-(trifluoromethyl)phenyl)urea (1.80 g, 6.75 mmol, 1.00 *eq*) in tetrahydrofuran (10.0 mL) was added sodium hydride (405 mg, 10.1 mmol, 60% purity, 1.50 *eq*) at 0 °C under nitrogen atmosphere. The mixture was stirred at 25 °C for 2 h. The mixture was quenched with saturated ammonium chloride solution (50 mL), then extracted with ethyl acetate (3 × 100 mL). The organic layers were collected and dried over anhydrous sodium sulfate and evaporated to afford 1-(4-(trifluoromethyl)phenyl)imidazolidin-2-one (2.00 g, crude) as a white solid.

¹H NMR (400 MHz, DMSO-*d*₆) *δ* = 7.75 (s, 1H). 7.72 (s, 1H), 7.58 (s, 1H), 7.56 (s, 1H), 3.80 (br t, *J* = 7.9 Hz, 2H), 3.41 (br t, *J* = 7.9 Hz, 2H)

Step 3. To a solution of 1-(4-(trifluoromethyl)phenyl)imidazolidin-2-one (50.00 mg, 155 umol, 1.00 *eq*) and 3-(5-bromo-1-oxoisoindolin-2-yl)piperidine-2,6-dione (35.6 mg, 155 umol, 1.00 *eq*) in dioxane (2.00 mL) was added potassium carbonate (64.2 mg, 464 umol, 3.00 *eq*) and dimethylethylenediamine (1.36 mg, 15.5 umol, 1.67 uL, 0.100 *eq*), cuprous iodide (2.95 mg, 15.5 µmol, 0.100 *eq).* The mixture was stirred at 100 °C for 12 h under nitrogen atmosphere. The mixture was filtered and the filtrate was purified by *Prep-*HPLC (column: Welch Ultimate C18150*25mm*5um;mobile phase: [water(FA)-ACN];B%. 33%-63%,10min). The desired fraction was collected and lyophilized to afford 3-(1-oxo-5-(2-oxo-3-(4-(trifluoromethyl)phenyl)imidazolidin-1-yl)isoindolin-2-yl)piperidine-2,6-dione (11.06 mg, 23.4 umol, 5% yield) as an off-white solid.

¹H NMR (400 MHz, DMSO-*d₆*) *δ* = 11.06 - 10.91 (m, 1H), 7.93 (s, 1H), 7.89 (s, 1H), 7.86 (s, 1H), 7.81 - 7.78 (m, 1H), 7.75 (d, *J* = 3.3 Hz, 2H), 7.73 (d, *J* = 2.9 Hz, 1H), 5.11 (dd, *J* = 5.3, 13.3 Hz, 1H). 4.50 - 4.44 (m, 1H), 4.36 - 4.31 (m, 1H), 4.08 (s, 4H), 2.97 - 2.86 (m, 1H), 2.63 - 2.57 (m, 1H), 2.40 (br dd, *J* = 4.8, 13.4 Hz, 1H), 2.04 - 1.98 (m, 1H)

### Example 20. Synthesis of Compound 118

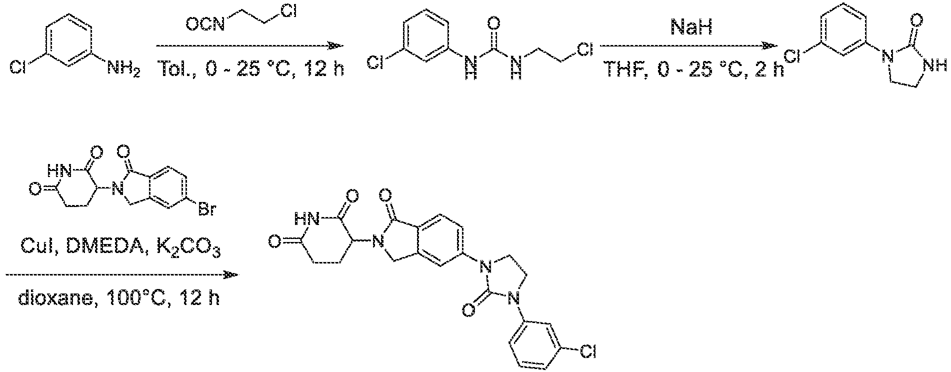

Step 1. To a solution of 3-chloroaniline (2.00 g, 15.7 mmol, 1.67 mL, 1.00 *eq*) in toluene (10.0 mL) was added 1-chloro-2-isocyanato-ethane (2.48 g, 23.5 mmol, 2.00 mL, 1.50 *eq*) at 0 °C. The mixture was stirred at 25 °C for 12 h. The mixture was filtered. The filter cake was concentrated under reduced pressure to afford 1-(2-chloroethyl)-3-(3-chlorophenyl)urea (3.50 g, crude) as a white solid.

¹H NMR (400 MHz, DMSO-*d₆*) *δ* = 8.86 (s, 1H), 7.67 (t, J= 2.0 Hz, 1H), 7.26 - 7.21 (m, 1H). 7.20 - 7.17 (m, 1H), 6.98 - 6.91 (m, 1H), 6.48 (br t, *J* = 5.7 Hz, 1H). 3.66 (t, *J =* 6.1 Hz, 2H), 3.42 (q, *J* = 6.1 Hz, 2H),

Step 2. To a solution of 1-(2-chloroethyl)-3-(3-chlorophenyl)urea (3.50 g, 15.0 mmol, 1.00 *eq*) in tetrahydrofuran (10.0 mL) was added sodium hydride (901 mg, 22.5 mmol, 60% purity, 1.50 *eq*) at 0 °C under nitrogen atmosphere. The mixture was stirred at 25 °C for 2 h. The mixture was quenched with saturated ammonium chloride solution (50 mL), then extracted with ethyl acetate (3 × 100 mL). The organic layers were collected and dried over anhydrous sodium sulfate and evaporated to give 1-(3-chlorophenyl)imidazolidin-2-one (3.80 g, crude) as a white solid.

¹H NMR (400 MHz, DMSO-*d₆*) *δ* = 7.79 (s, 1H), 7.39 - 7.34 (m, 1H). 7.32 - 7.27 (m, 1H), 6.98 (br d, *J =* 7.6 Hz, 1H), 3.80 (t, *J* = 7.9 Hz, 2H), 3.39 (t, *J =* 7.9 Hz, 2H).

Step 3. To a solution of 3-(5-bromo-1-oxoisoindolin-2-yl)piperidine-2,6-dione (50.0 mg, 155 µmol, 1.00 *eq*) and 1-(3-chlorophenyl)imidazolidin-2-one (30.4 mg, 155 umol, *1.00 eq*) in dioxane (1.00 mL) was added potassium carbonate (64.2 mg, 464 umol, 3.00 *eq*) and dimethylethylenediamine (1.36 mg, 15.5 µmol, 1.67 uL, 0.100 *eq).* After nitrogen purge, cuprous iodide (2.95 mg, 15.5 umol, 0.100 eq) was added into the mixture. The mixture was stirred at 100 °C for 12 h. The mixture was filtered and the filtrate was purified by *prep-*HPLC (column: Welch Ultimate C18 150*25mm*5um;mobile phase: [water(FA)-ACN];B%: 35%-65%,10min). The desired fraction was collected and the aqueous solution was lyophilized to afford 3-(5-(3-(3-chlorophenyl)-2-oxoimidazolidin-1-yl)-1-oxoisoindolin-2-yl)piperidine- 2,6-dione (29.98 mg, 68.3 umol, 11 % yield) as a yellow solid.

¹H NMR (400 MHz, DMSO-*d₆*) *δ* = 10.98 (br s, 1H), 7.92 (s, 1H), 7.86 (t, *J* = 2.0 Hz, 1H). 7.79 - 7.76 (m, 1H), 7.74 - 7.71 (m, 1H). 7.54 (dd, *J* = 1.4, 8.3 Hz, 1H). 7.41 (t, *J =* 8.1 Hz, 1H), 7.15 (dd, *J* = 1.4, 7.7 Hz, 1H), 5.10 (dd, *J* = 5.0, 13.4 Hz, 1H), 4.49 - 4.43 (m, 1H), 4.36 - 4.30 (m, 1H), 4.07 - 4.01 (m, 4H), 2.97 - 2.87 (m, 1H), 2.63 - 2.57 (m, 1H), 2.41 (br d, *J* = 8.6 Hz, 1H). 2.03 - 1.98 (m, 1H),

### Example 21. Synthesis of Compound 148

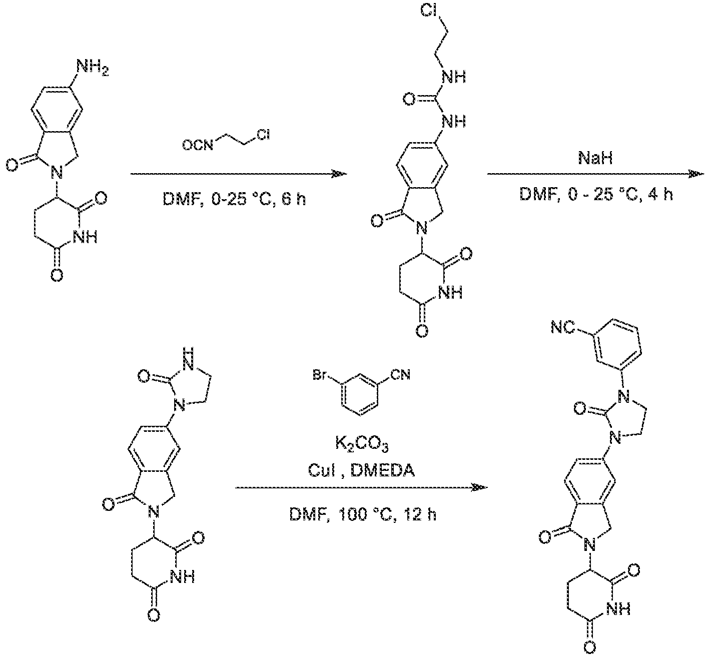

Step 1. To a solution of 3-(5-amino-1-oxoisoindolin-2-yl)piperidine-2,6-dione (4.00 g, 15.4 mmol, 1.00 *eq)* in dimethyl formamide (40.0 mL) was added 1-chloro-2-isocyanatoethane (2.44 g, 23.1 mmol, 1.97 mL, 1.50 *eq)* at 0 °C. The mixture was stirred at 25 °C for 6 h. The mixture was filtered to give a filter liquor which was purified by reversed-phase HPLC (column: spherical C18, 20-45 um, 100Å, SW 330, mobile phase: [water-acetonitrile) to give 1-(2-chloroethyl)-3-(2-(2,6-dioxopiperidin-3-yi}-1-oxoisoindolin-3-yl)urea (1.50 g, 4.11 mmol, 26% yield) as an off-white solid. ¹H NMR (400 MHz, DMSO-*d*₆) *δ* = 10.96 (br s, 1H), 9.20 (s, 1H), 7.77 (s, 1H), 7.57 (d, *J =* 8.3 Hz, 1H), 7.39 (dd, *J* = 1.8, 8.4 Hz, 1H). 6.63 (t, *J =* 5.8 Hz, 1H), 5.06 (dd, *J =* 5.1, 13.3 Hz, 1H), 4.42 - 4.35 (m, 1H), 4.28 - 4.22 (m, 1H), 3.70 - 3.64 (m, 2H), 3.63 - 3.52 (m, 2H), 2.95 - 2.84 (m, 1H). 2.59 (td, *J* = 1.9, 15.4 Hz, 1H). 2.41 - 2.32 (m, 1H), 2.03 - 1.92 (m, 1H). MS (ESI) m/z 365.0 [M+H]⁺

Step 2. To a solution of 1-(2-chloroethyl)-3-(2-(2,6-dioxopiperidin-3-yl)-1-oxoisoindolin-5-yl)urea (1.30 g, 3.56 mmol, 1.00 *eq*) in dimethyl formamide (10.0 mL) was added sodium hydride (213 mg, 5.35 mmol, 60% purity, 1.50 *eq*) at 0 °C under nitrogen. The mixture was stirred at 25 °C for 4 h. The reaction mixture was quenched with saturated ammonium chloride (10.00 mL) to give a solution. The solution was filtered. The filter cake was dried in vacuum to give 3-(1-oxo-5-(2-oxoimidazolidin-1-yl)isoindolin-2-yl)piperidine-2,6-dione (830 mg, 2.53 mmol, 71% yield) as a gray solid. ¹H NMR (400 MHz, DMSO-*d*₆) *δ* = 10.97 (br s, 1H), 7.82 (s, 1H), 7.72 - 7.68 (m, 1H), 7.67 - 7.63 (m, 1H), 7.20 (s, 1H), 5.08 (dd, *J* = 5.1, 13.3 Hz, 1H). 4.46 - 4.37 (m, 1H), 4.31 - 4.25 (m, 1H), 3.96 - 3.88 (m, 2H), 3.44 (br t, *J* = 8.0 Hz, 2H), 2.93 - 2.86 (m, 1H), 2.59 (td, *J* = 2.0, 15.4 Hz, 1H). 2.44 - 2.33 (m, 1H), 2.02 - 1.95 (m, 1H). MS (ESI) m/z 329.0 [M+H]⁺

Step 3. To a solution of 3-(1-oxo-5-(2-oxoimidazolidin-1-yl)isoindolin-2-yl)piperidine-2,6-dione (20.0 mg, 60.9 µmol, 1.00 eq), 3-bromobenzonitrile (16.6 mg, 91.3 umol, 1.50 *eq)* and potassium carbonate (25.2 mg, 182 µmol, 3.00 *eq*) in dimethyl formamide (1.00 mL) was added copper(I) iodide (2.32 mg, 12.1 µmol, 0.200 *eq*) and *N,N*'-dimethylethane-1,2-diamine (5.37 mg, 60.9 umol, 6.56 uL, 1.00 *eq)* in portions under nitrogen. The mixture was stirred at 100 °C for 12 h. The mixture was filtered to give a filter liquor which was purified by *prep*-HPLC (column: Phenomenex Luna C18 150*25mm*10um;mobile phase: [water(formic acid )- acetonitrile];B%: 22%-52%,8min) to give 3-(3-(2-(2,6-dioxopiperidin-3-yl)-1-oxoisoindolin-5-yl)-2-oxoimidazolidin-1-yl)benzonitrile (7.25 mg, 16.8 umol, 5% yield) as an off-white solid. ¹H NMR (400 MHz, DMSO-*d*₆) *δ* = 10.98 (s, 1H), 8.11 (s, 1H), 8.02 - 7.97 (m, 1H). 7.93 (s, 1H), 7.81 - 7.77 (m, 1H). 7.76 - 7.72 (m, 1H). 7.63 - 7.57 (m, 1H). 7.56 - 7.51 (m, 1H). 5.11 (dd, *J* = 5.0, 13.3 Hz, 1H), 4.51 - 4.42 (m, 1H). 4.38 - 4.29 (m, 1H), 4.11 - 4.02 (m, 4H), 2.97 - 2.87 (m, 1H). 2.60 (br d, J = 17.4 Hz, 1H), 2.44 - 2.36 (m, 1H). 2.06 - 1.96 (m, 1H). MS (ESI) m/z 430.0 [M+H]⁺

### Example 22. Synthesis of Compound 127

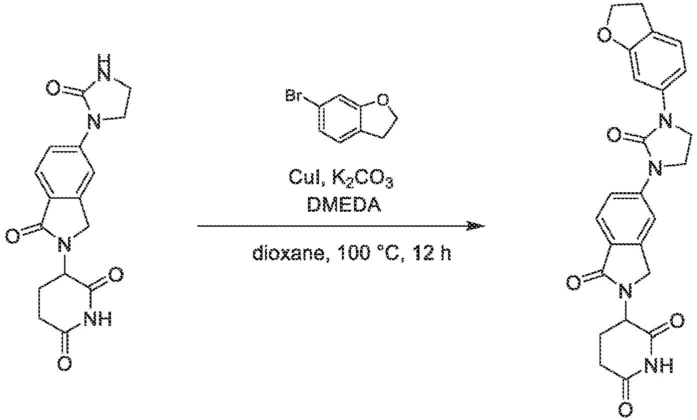

To a solution of 3-(1-oxo-5-(2-oxoimidazolidin-1-yl)isoindolin-2-yl)piperidine-2,6-dione (20.0 mg, 60.9 umol, 1.00 *eq*)*,* 6-bromo-2,3-dihydrobenzofuran (18.1 mg, 91.3 umol, 1.50 *eq*)*,* potassium carbonate (25.2 mg, 182 umol, 3.00 *eq*) in 1,4-dioxane (2.00 mL) was added -*N,N*'-dimethylethane-1,2-diamine (5.37 mg, 60.9 umol, 6.56 uL, 1.00 *eq*)*,* copper(I) iodide (2.32 mg, 12.1 umol, 0.200 *eq*) under nitrogen. The mixture was stirred at 100 °C for 12 h. The reaction mixture was concentrated under reduced pressure. The residue was purified by *prep*-HPLC (column: Welch Ultimate C18 150*25mm*5um;mobile phase: [water(formic acid)- acetonitrile];B%: 24%-54%,10min) to give 3-(5-(3-(2,3-dihydrobenzofuran-6-yl)-2-oxoimidazolidin-1-yl)-1-oxoisoindolin-2-yl)piperidine-2,6-dione (9.00 mg, 20.1 µmol, 8% yield) as a yellow solid. ¹H NMR (400 MHz, DMSO-*d*₆) *δ* = 10.98 (s, 1H), 7.93 - 7.86 (m, 1H). 7.79 - 7.74 (m, 1H), 7.73 - 7.69 (m, 1H), 7.23 - 7.19 (m, 2H), 7.01 (dd, *J* = 1.8, 8.2 Hz, 1H), 5.14 - 5.05 (m, 1H), 4.54 (t, *J* = 8.7 Hz, 2H), 4.48 - 4.42 (m, 1H). 4.35 - 4.29 (m, 1H). 4.06 - 3.93 (m, 4H), 3.15 (t, *J* = 8.6 Hz, 2H), 2.97 - 2.86 (m, 1H). 2.61 - 2.58 (m, 1H). 2.39 (br s, 1H), 2.03 - 1.96 (m, 1H). MS (ESI) m/z 447.3 [M+H]⁺

### Example 23. Synthesis of Compound 170

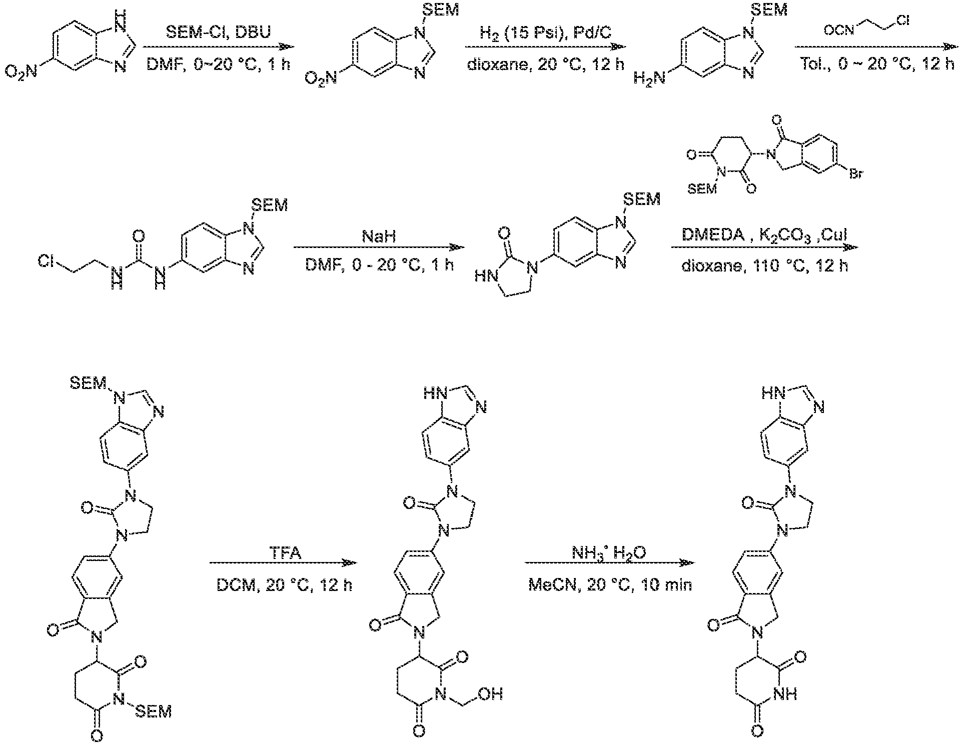

Step 1. To a solution of 5-nitro-1H-benzo[*d*]imidazole (2.00 g, 12.2 mmol, 1.00 *eq)* in dimethyl formamide (10 mL) was added 1,8-diazabicyclo[5.4.0]undec-7-ene (3.73 g, 24.5 mmol, 3.70 mL, 2.00 *eq*) and 2-(trimethylsilyl) ethoxymethyl chloride (3.68 g, 22.0 mmol, 3.91 mL, 1.80 *eq*) at 0 °C. The mixture was stirred at 20 °C for 1 h. The mixture was filtered. The filtrate was purified by reversed-phase HPLC (column: spherical C18, 20-45 um, 100Å, SW 330, mobile phase: [water(0.1%Formic Acid)-ACN]) to give 5-nitro-1-((2-(trimethylsilyl)ethoxy)methyl)-1*H*-benzo[*d*]imidazole (3.00 g, 10.2 mmol, 83% yield) as a yellow solid. ¹H NMR (400 MHz, DMSO-*d*₆) *δ* = 8.76 - 8.68 (m, 1H). 8.68 - 8.57 (m, 1H), 8.26 - 8.14 (m, 1H). 7.91 - 7.87 (m, 1H), 5.82 - 5.72 (m, 2H), 3.52 (dt, *J =* 2.2, 8.0 Hz, 2H), 0.83 (t, *J =* 8.0 Hz, 2H), -0.10 (d, *J =* 3.3 Hz, 9H). MS (ESI) m/z 294.2 [M+H]⁺

Step 2. To a solution of 5-nitro-1-((2-(trimethylsilyl)ethoxy)methyl)-1*H-*benzo[*d*]imidazole (3.00 g, 10.2 mmol, 1.00 *eq)* in dioxane (30 mL) was added palladium on carbon (100 mg, 10% purity) under nitrogen. The mixture was stirred at 20 °C for 12 h under hydrogen (15 Psi). The mixture was filtered. The filter liquor was concentrated under reduced pressure to give 1-((2-(trimethylsilyl)ethoxy)methyl)-1*H*-benzo[*d*]imidazol- 5-amine (2.50 g, crude) as yellow oil. MS (ESI) m/z 264.1 [M+H]⁺

Step 3. To a solution of 1-((2-(trimethylsilyl)ethoxy)methyl)-1*H-*benzo[d]imidazol-5-amine (2.50 g, 9.49 mmol, 1.00 *eq*) in toluene (30 mL) was added 1-chloro-2-isocyanatoethane (1.50 g, 14.2 mmol, 1.50 *eq*) at 0 °C. The mixture was stirred at 20 °C for 12 h. The reaction mixture was concentrated under reduced pressure. The residue was purified by column chromatography (SiO₂, Petroleum ether/Ethyl acetate=8/1 to 0/1) to give 1-(2-chloroethyl)-3-(1-((2-(trimethylsilyl)ethoxy)methyl)-1*H*-betizo[*d*]imidazol-5-yl)urea (3.00 g, 8.13 mmol, 85% yield) as yellow oil.

¹H NMR (400 MHz, DMSO-*d*₆) *δ* = 8.72 (s, 1H), 8.20 (s, 1H), 7.92 (d, *J* = 1.6 Hz, 1H), 7.50 (d, *J* = 8.5 Hz, 1H), 7.03 (dd, *J* = 1.9, 8.7 Hz, 1H), 6.38 (t, *J* = 5.8 Hz, 1H). 5.57 - 5.51 (m, 2H), 3.67 (t, *J =* 6.2 Hz, 2H), 3.49 - 3.42 (m, 4H), 0.87 - 0.81 (m, 2H), -0.08 (s, 9H). MS (ESI) m/z 369.3 [M+H]⁺

Step 4. To a solution of 1-(2-chloroethyl)-3-(1-((2-(trimethylsilyl)ethoxy)methyl)-1*H*-benzo[*d*]imidazol-5-yl)urea (3.00 g, 8.13 mmol, 1.00 *eq*) in dimethyl formamide (10 mL) was added sodium hydride (487 mg, 12.2 mmol, 60% purity, 1.50 *eq*) at 0 °C. The mixture was stirred at 20 °C for 1 h under nitrogen. The reaction mixture was adjust pH to 5-6 with formic acid. The solution was purified by reversed-phase HPLC (column: spherical C18, 20-45 um, 100Å, SW 330, mobile phase: [water(0.1%Formic Acid)-ACN]) to give 1-(1-((2-(trimethylsilyl)ethoxy)methyl)-1*H*-benzo[*d*]imidazol-5-yl)imidazolidin-2-one (2.50 g, 7.52 mmol, 92% yield) as a yellow solid.

¹H NMR (400 MHz, DMSO-*d*₆) *δ* = 8.27 (d, *J =* 18.6 Hz, 1H), 7.80 - 7.68 (m, 1H). 7.66 - 7.48 (m, 2H), 6.93 - 6.80 (m, 1H). 5.59 (d, *J* = 7.0 Hz, 2H), 3.94 - 3.88 (m, 2H), 3.49 (td, *J* = 4.0, 8.0 Hz, 2H), 3.43 - 3.40 (m, 2H), 0.83 (dt, *J* = 4.3, 8.0 Hz, 2H), -0.09 (d, *J* = 1.9 Hz, 9H).

Step 5. To a solution of 1-(1-((2-(trimethylsilyl)ethoxy)methyl)-1*H-*benzo[*d*]imidazol-5-yl)imidazolidin-2-one (150 mg, 451 µmol, 1.00 *eq*) in dioxane (4.00 mL) was added 3-(5-bromo-1-oxoisoindolin-2-yl)- 1-((2-(trimethylsilyl)ethoxy)methyl)piperidine-2,6-dione (204 mg, 451 µmol, 1.00 eq), potassium carbonate (187 mg, 1.35 mmol, 3.00 *eq), N,N*-dimethylethylenediamine (19.8 mg, 225 µmol, 24.2 uL, 0.500 *eq*) and copper(I) iodide (17.1 mg, 90.2 umol, 0.200 *eq*)*.* The mixture was stirred at 110 °C for 12 h under nitrogen. The reaction mixture was concentrated under reduced pressure. The residue was purified by reversed-phase HPLC (column: spherical C18, 20-45 um, 100Å, SW 40, mobile phase: [water(0.1%Formic Acid)-ACN]) to give 3-(1-oxo-5-(2-oxo-3-(1-((2-(trimethylsilyl)ethoxyl)methyl)-1*H*-benzo[*d*]imidazol-5-yl) imidazolidin-1-yl) isoindolin-2-yl)-1-((2-(trimethylsilyl)ethoxy)methyl)piperidine-2,6-dione (50.0 mg, 70.9 umol, 7% yield) as a yellow solid. MS (ESI) m/z 705.7 [M+H]⁺

Step 6. A solution of 3-(1-oxo-5-(2-oxo-3-(1-(trimethylsilyl)ethoxy)methyl)-1*H*-benzo[*d*]imidazol-5-yl) imidazolidin-1-yl)isoindolin-2-yl)-1-((2-(trimethylsilyl)ethoxy)methyl)piperidine-2,6-dione (50.0 mg, 70.9 µmol, 1.00 *eq)* in the mixture of dichloromethane (5.00 mL) and trifluoroacetic acid (770 mg, 6.75 mmol, 0.500 mL, 95.2 *eq)* was stirred at 20 °C for 12 h. The reaction mixture was concentrated under reduced pressure to give 3-(5-(3-(1*H*-benzo[*d*]imidazol-5-yl)-2-oxoimidazolidin-1-yl)-1-oxoisoindolin-2-yl)-1- (hydroxymethyl)piperidine-2,6-dione (40.0 mg, crude) as a yellow solid.

Step 6. To a solution of -(3-(1*H*-benzo[*d*]midazol-5-yl)-2-oxoimidazolidin-1-yl)-1-oxoisoindolin-2-yl)-1-(hydroxymethyl)piperidine-2,6-dione (40.0 mg, 79.2 µmol, 1.00 *eq*) in acetonitrile (5.00 mL) was added ammonium hydroxide (227 mg, 1.82 mmol, 0.250 mL, 28% purity, 22.9 *eq*)*.* The mixture was stirred at 20 °C for 10 min. The mixture was filtered. The filter liquor was purified by *Prep*-HPLC (column: Phenomenex luna C18 150*25mm* 10um;mobile phase: [water(FA)-ACN];B%: 0%-30%, 10min) and lyophilized to give 3-(5-(3-(1*H*-benzo[*d*]imidazol-5-yl)-2-oxoimidazolidin-1-yl)-1- oxoisoindolin-2-yl) piperidine-2,6-dione (18.0 mg, 40.5 umol, 51% yield) as a yellow solid.

¹H NMR (400 MHz, DMSO-*d*₆) *δ* = 12.41 (br s, 1H), 10.98 (s, 1H), 8.19 (br s, 1H), 7.92 (s, 1H), 7.87 - 7.77 (m, 2H), 7.74 - 7.69 (m, 1H), 7.68 - 7.37 (m, 2H), 5.10 (dd, *J* = 5.1, 13.3 Hz, 1H), 4.51 - 4.40 (m, 1H), 4.36 - 4.28 (m, 1H), 4.07 (s, 4H), 2.98 - 2.87 (m, 1H), 2.63 - 2.57 (m, 1H), 2.40 (dq, *J* = 4.4, 13.3 Hz, 1H), 2.06 - 1.95 (m, 1H).

¹H NMR (400 MHz, DMSO-*d*₆, T = 80 °C) *δ* = 12.37 - 11.97 (m, 1H), 10.70 (br s, 1H), 8.13 (s, 1H), 7.92 (d, *J* = 1.1 Hz, 1H), 7.83 (s, 1H), 7.81 - 7.77 (m, 1H), 7.73 - 7.69 (m, 1H), 7.64 - 7.41 (m, 2H), 5.06 (dd, *J* = 5.3, 13.0 Hz, 1H), 4.50 - 4.43 (m, 1H), 4.41 - 4.34 (m, 1H), 4.08 (s, 4H), 2.90 (ddd, *J* = 5.5, 13.4, 17.5 Hz, 1H), 2.67 - 2.60 (m, 1H), 2.42 (dq, *J* = 4.6, 13.1 Hz, 1H), 2.05 (dtd, *J* = 2.5, 5.3, 12.8 Hz, 1H). MS (ESI) m/z 445.3 [M+H]⁺

### Example 24. Synthesis of Compound 163

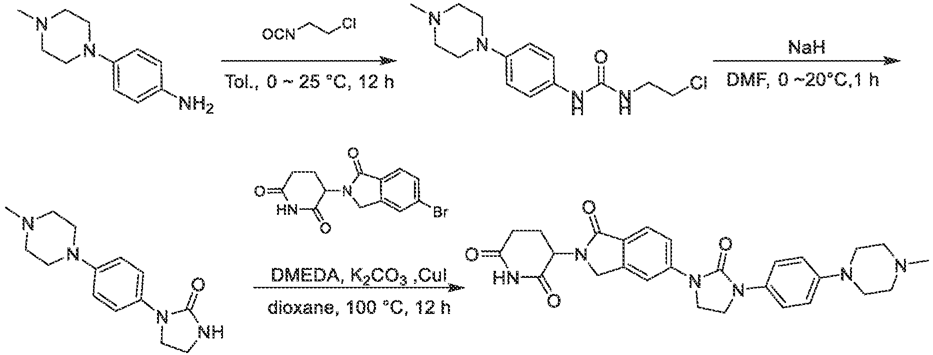

Step 1. To a solution of 4-(4-methylpiperazin-1-yl)aniline (3.00 g, 15.7 mmol, 1.00 *eq)* in toluene (50.0 mL) was added 1-chloro-2-isocyanatoethane (2.48 g, 23.5 mmol, *1.50 eq)* at 0 °C. The mixture was stirred at 25 °C for 12 h. The mixture was filtered to give a filter cake, then it was concentrated under reduced pressure to give 1-(2-chloroethyl)-3-(4-(4-methylpiperazin-1-yl)phenyl)urea (4.30 g, 14.5 mmol, 92% yield) as a grey solid.

¹H NMR (400 MHz, DMSO-*d*₆) *δ* = 8.33 (s, 1H), 7.22 (d, *J* = 9.0 Hz, 2H), 6.86 - 6.77 (m, 2H), 6.25 (t, *J* = 5.8 Hz, 1H), 3.63 (t, *J* = 6.2 Hz, 2H), 3.39 (q, *J* = 6.1 Hz, 2H), 3.05 - 2.96 (m, 4H), 2.47 - 2.40 (m, 4H), 2.21 (s, 3H).

Step 2. To a solution of 1-(2-chloroethyl)-3-(4-(4-methylpiperazin-1-yl)phenyl)urea (2.00 g, 6.74 mmol, 1.00 *eq)* in dimethyl formamide (10.0 mL) was added sodium hydride (404 mg, 10.1 mmol, 60% purity, 1.50 *eq)* at 0 °C. The mixture was stirred at 20 °C for 1 h. The reaction mixture was quenched with formic acid (2.00 mL) to give a solution, then it was concentrated under reduced pressure to give 1-(4-(4-methylpiperazin-1-yl) phenyl)imidazolidin-2-one (2.00 g, crude) as a brown solid. ¹H NMR (400 MHz, DMSO-*d*₆) *δ* = 7.38 (d, *J* = 8.9 Hz, 2H), 6.89 (d, *J* = 8.9 Hz, 2H), 6.74 (br s, 1H), 3.77 (t, *J* = 7.9 Hz, 2H), 3.36 (br t, *J* = 7.9 Hz, 2H), 3.10 - 3.04 (m, 4H), 2.55 (br s, 4H), 2.28 (s, 3H).

Step 3. To a solution of 1-(4-(4-methylpiperazin-1-yl)phenyl)imidazolidin-2-one (50.0 mg, 192 umol, 1.00 *eq)* in dioxane (1.50 mL) was added 3-(5-bromo-1-oxoisoindolin-2-yl)piperidine-2,6-dione (62.1 mg, 192 umol, 1.00 eq), potassium carbonate (79.6 mg, 576 umol, 3.00 eq), *N,N*-dimethylethylenediamine (8.47 mg, 96.0 umol, 10.3 uL, 0.500 *eq)* and copper(I) iodide (7.32 mg, 38.4 umol, 0.200 *eq).* The mixture was stirred at 100 °C for 12 h under nitrogen. The mixture was filtered. The filtrate was purified by *Prep*-HPLC (column: Phenomenex luna C18 150* 25mm* 10um;mobile phase: [water(FA)-ACN];B%: 5%-35%,58min) and lyophilized to give 3-(5-(3-(4-(4-methylpiperazin-1-yl)phenyl)-2-oxotmidazolidin-1-yl)-1- oxoisoindolin-2-yl)piperidine-2,6-dione (80.0 ing, 159 umol, 83% yield) as a gray solid. ¹H NMR (400 MHz, DMSO-*d*₆) *δ* = 10.98 (s, 1H), 7.93 - 7.84 (m, 1H), 7.81 - 7.74 (m, 1H), 7.73 - 7.68 (m, 1H), 7.49 (d, *J* = 9.1 Hz, 2H), 7.00 (d, *J* = 9.1 Hz, 2H), 5.15 - 5.05 (m, 1H), 4.49 - 4.40 (m, 1H), 4.34 - 4.27 (m, 1H), 4.11 - 4.00 (mm, 2H), 3.99 - 3.92 (m, 2H), 3.24 - 3.14 (m, 4H), 2.98 - 2.92 (m, 1H), 2.91 - 2.76 (m, 4H), 2.62 (br d, *J* = 2.4 Hz, 1H), 2.54 (br s, 3H), 2.40 (br dd, *J* = 4.4, 12.8 Hz, 1H), 2.04 - 1.97 (m, 1H). MS (ESI) m/z 503.3 [M+H]⁺

### Example 25. Synthesis of Compound 174

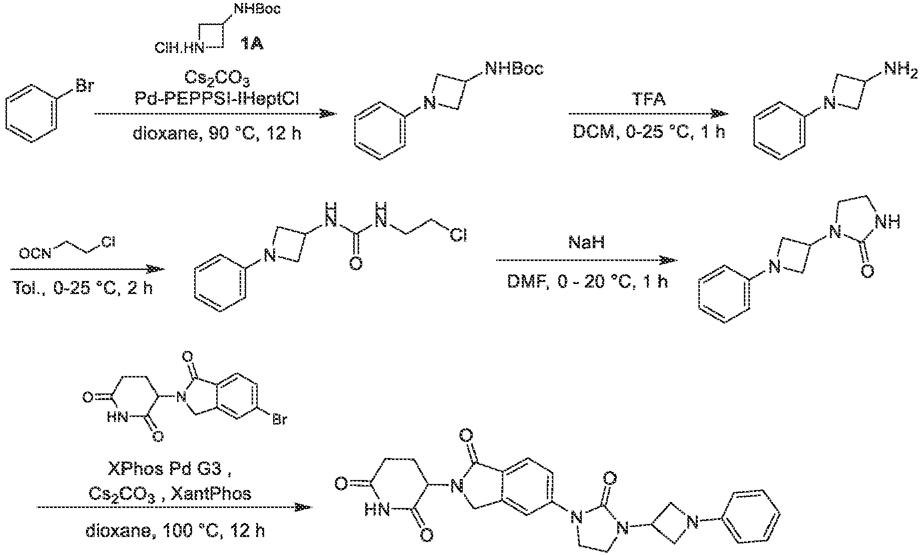

Step 1. To a solution of bromo benzene (1.00 g, 6.37 mmol, 671 uL, 1.00 *eq), tert-*butyl azetidin-3-ylcarbamate hydrochloride (1.20 g, 5.73 mmol, 0.900 *eq*, hydrochloride) in dioxane (20.0 mL) was added cesium carbonate (2.08 g, 6.37 mmol, 1.00 *eq*), 1,3-bis[2,6-bis(1-propylbutyl)phenyl]-4,5-dichloro-2*H*-imidazol -1-ium-2-ide;3-chloropyridine;dichloropalladium (310 mg, 318 umol, 0.0500 *eq)* under nitrogen atmosphere. The mixture was stirred at 90 °C for 12 h. The mixture was filtered to give a filtrate, then was concentrated under reduced pressur to give tert-butyl (1-phenylazetidin-3-yl)carbamate (1.50 g, 6.04 mmol, 95% yield) as a yellow solid. ¹H NMR (400 MHz, DMSO-*d*₆) *δ* = 7.15 (dd, *J* = 7.2, 8.4 Hz, 2H), 6.67 (s, 1H), 6.41 (d, *J* = 7.6 Hz, 2H), 4.46 - 4.34 (m, 1H), 4.04 (t, *J* = 7.6 Hz, 2H), 3.52 (t, *J* = 6.8 Hz, 2H), 1.39 (s, 9H)

Step 2. To a solution of *tert*-butyl (1-phenylazetidin-3-yl)carbamate (750 mg, 3.02 mmol, 1.00 *eq)* in dichloromethane (3.00 mL) was added drop-wise trifluoroacetic acid (1.00 mL) at 0 °C. The mixture was stirred at 25 °C for 1 h. The reaction mixture was concentrated under reduced pressure and purified by reversed-phase HPLC (column: spherical C18, 20-45 um, 100A, SW 120, mobile phase: [water(0.1%Formic Acid)-ACN]) and lyophilized to give 1-phenylazetidin-3-amine (500 mg, crude) as a yellow solid.

¹H NMR (400 MHz, DMSO-*d*₆) *δ* = 8.40 (br s, 2H), 7.19 (t, *J* = 7.6 Hz, 2H), 6.73 (t, *J* = 7.2 Hz, 1H), 6.50 (d, *J* = 8.0 Hz, 2H), 4.11 - 4.05 (m, 3H), 3.77 (br dd, *J* = 3.2, 7.2 Hz, 2H). MS (ESI) m/z 149.0 [M+H]⁺

Step 3. To a solution of 1-phenylazetidin-3-amine (500 mg, 3.37 mmol, 1.00 *eq*) in tetrahydrofuran (10.0 mL) was added 1-chloro-2-isocyanatoethane (534 mg, 5.06 mmol, 431 uL, 1.50 *eq*) at 0 °C. The mixture was stirred at 25 °C for 2 h. The mixture was filtered to give a filtrate, then was concentrated under reduced pressure and purified by reversed-phase HPLC (column: spherical C18, 20-45 um, 100Å, SW 120, mobile phase: [water(0.1%Formic Acid)-ACN]) and lyophilized to give 1-(2-chloroethyl)-3-(1-phenylazetidin-3-yl) urea (220 mg, 867 umol, 26% yield) as a white solid.

¹H NMR (400 MHz, DMSO-*d*₆) *δ* = 7.16 (dd, *J* = 7.2, 8.4 Hz, 2H), 6.74 (d, *J* = 7.6 Hz, 1H), 6.67 (t, *J* = 7.2 Hz, 1H), 6.43 (dd, *J* = 0.8, 8.4 Hz, 2H), 6.17 (br t, *J* = 6.0 Hz, 1H), 4.55 - 4.44 (m, 1H), 4.06 (t, *J* = 7.2 Hz, 2H), 3.57 (t, *J* = 6.4 Hz, 2H), 3.51 - 3.45 (m, 2H), 3.31 - 3.28 (m, 2H). MS (ESI) m/z 254.2 [M+H]⁺

Step 4. To a solution of 1-(2-chloroethyl)-3-(1-phenylazetidin-3-yl)urea (220 mg, 867umol, 1.00 *eq*) in tetrahydrofuran (10.0 mL) was added sodium hydride (52.0 mg, 1.30 mmol, 60% purity, 1.50 *eq*) in one portion at 0 °C. The mixture was stirred at 20 °C for 1 h under nitrogen atmosphere. The reaction mixture was adjust pH to 5-6 with formic acid to give a solution and concentrated under reduced pressure to give a residue. The residue was purified by reversed-phase HPLC (column: spherical C18, 20-45 um, 100A, SW 80, mobile phase: [water(0.1%Formic Acid)-ACN]) and lyophilized to give 1-(1-phenylazetidin-3 -yl) imidazolidin-2-one (60.0 mg, 276 umol, 32%) yield) as a white solid.

¹H NMR (400 MHz, DMSO-*d*₆) *δ* = 7.17 (dd, *J* = 7.5, 8.4 Hz, 2H), 6.68 (t, *J* = 7.3 Hz, 1H), 6.50 (br s, 1H), 6.45 (d, *J* = 7.6 Hz, 2H), 4.76 - 4.64 (m, 1H), 3.96 (t, *J* = 7.7 Hz, 2H), 3.87 - 3.77 (m, 2H), 3.52 - 3.45 (m, 2H), 3.29 - 3.24 (m, 2H). MS (ESI) m/z 218.1 [M+H]⁺

Step 5. To a solution of 1-(1-phenylazetidin-3-yl)imidazolidin-2-one (20.0 mg, 92.1 umol, 1.00 *eq*) in dioxane (1.50 mL) was added 3-(5-bromo-1-oxoisoindolin-2-yl)piperidine-2,6-dione (29.8 mg, 92.1 umol, 1.00 *eg*), cesium carbonate (90.0 mg, 276 umol, *3.00 eq*), 4,5-bis(diphenylphosphino)-9,9-dimethylxanthene (5.33 mg, 9.21 umol, 0.100 *eq*) and methanesulfonato[9,9-dimethyl-4,5-Bis(diphenylphosphino)xanthene][2-amino-1,1-biphenyl]palladium(II)dichloromethaneadduct (7.79 mg, 9.21 umol, 0.100 *eq*). The mixture was stirred under nitrogen atmosphere at 100 °C for 12 h. The mixture was filtered to give a filtrate, then was concentrated under reduced pressure and purified by reversed-phase HPLC (column: spherical C18, 20-45 um, 100Å, SW 40, mobile phase: [water(0.1%Formic Acid)-ACN]) and lyophilized to give 3-(1-oxo-5-(2-oxo-3-(1-phenylazetidin-3-yl)imidazolidin-1-yl)isoindolin-2-yl)piperidine-2,6-dione (11.0 mg, 23.9 umol, 8% yield, 99% purity) as a white solid.

¹H NMR (400 MHz, DMSO-*d*₆) *δ* = 10.97 (s, 1H), 7.83 (s, 1H), 7.76 - 7.71 (m, 1H), 7.69 - 7.65 (m, 1H), 7.19 (t, *J* = 7.6 Hz, 2H), 6.71 (t, *J* = 7.2 Hz, 1H), 6.48 (d, *J* = 7.6 Hz, 2H), 5.08 (dd, *J* = 5.2*,* 13.2 Hz, 1H), 4.93 - 4.82 (m, 1H), 4.47 - 4.38 (m, 1H), 4.34 - 4.22 (m, 1H), 4.08 - 4.02 (m, 2H), 3.97 - 3.90 (m, 4H), 3.74 - 3.68 (m, 2H), 2.99 - 2.84 (m, 1H), 2.64 - 2.59 (m, 1H), 2.40 - 2.36 (m, 1H), 2.03 - 1.95 (m, 1H). MS (ESI) m/z 460.3 [M+H]⁺

### Example 26. Synthesis of Compound 184

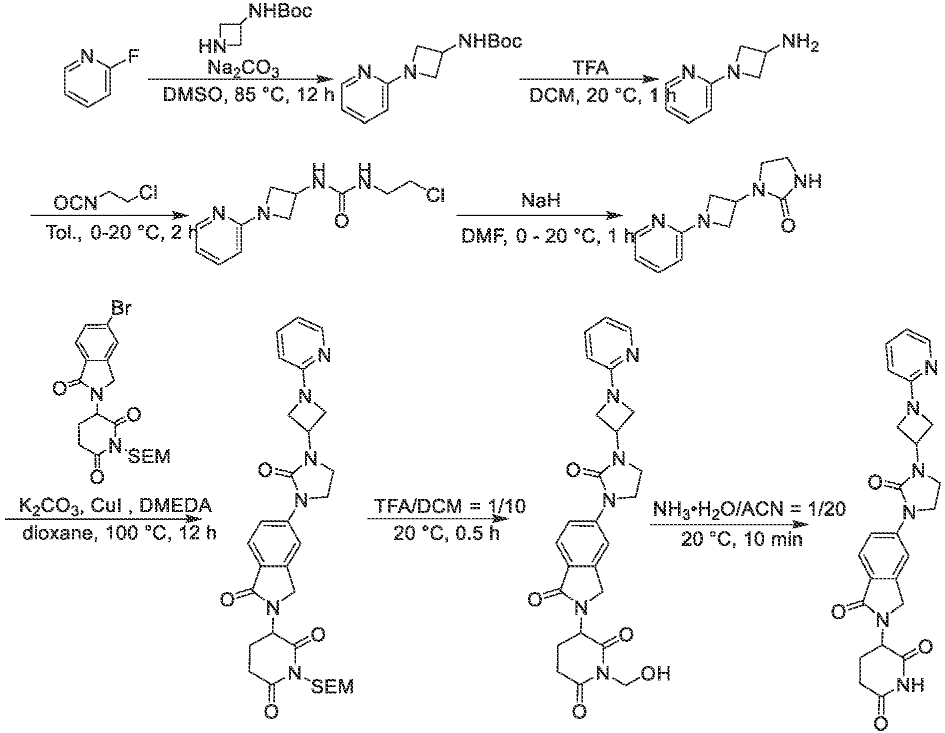

Step 1. Toa solution of 2-fluoropyridine (1.00 g, 10.3 mmol, 884 uL, *1.00 eq*) in dimethylsulfoxide (10.0 mL) was added *tert*-butyl azetidin-3-ylcarbamate (2.15 g, 10.3 mmol, 1.00 *eq,* hydrochloric acid) and sodium carbonate (2.18 g, 20.6 mmol, 2.00 *eq).* The mixture was stirred at 85 °C for 12 h. The mixture was filtered to give a filter liquor which was purified by reversed-phase HPLC (column: spherical C18, 20-45 um, 100Å, SW 330, mobile phase: [water(0.1%Formic Acid)-ACN]) to give *tert*-butyl (1-(pyridin-2-yl)azetidin-3-yl)carbamate (1.90 g, 7.62 mmol, 73% yield) as a yellow solid. ¹H NMR (400 MHz, DMSO-*d*₆) *δ* = 8.08 - 8.01 (m, 1H), 7.53 (br d, *J* = 7.6 Hz, 1H), 7.49 (ddd, *J* = 2.0, 7 2, 8.4 Hz, 1H), 6.62 (ddd, *J* = 0.8, 5.6, 6.8 Hz, 1H), 6.36 (d, *J* = 8.4 Hz, 1H), 4.46 - 4.35 (m, 1H), 4.12 (t, *J* = 8.0 Hz, 2H), 3.70 (dd, *J* = 6.0, 8.4 Hz, 2H), 1.39 (s, 9H). MS (ESI) m/z 250.3 [M+H]⁺

Step 2. To a solution of *tert*-butyl (1-(pyridin-2-yl)azetidin-3-yl)carbamate (1.90 g, 7.62 mmol, 1.00 *eq*) in dichloromethane (20.0 mL) was added trifluoroacetic acid (20.9 g, 183 mmol, 13.5 mL, 24.0 *eq).* The mixture was stirred at 20 °C for 1 h. The reaction mixture was concentrated under reduced pressure to give 1-(pyridin-2-yl)azetidin-3-amine (2.00 g, crude, trifluoroacetic acid) as a yellow solid.

Step 3. To a solution of 1-(pyridin-2-yl)azetidin-3-amine (2.00 g, 7.60 mmol, *1.00 eq*, trifluoroacetic acid) in toluene (30.0 mL) was added triethylamine (1.54 g, 15.2 mmol, 2.12 mL, 2.00 *eq*) and 1-chloro-2-isocyanatoethane (1.20 g, 11.4 mmol, 1.50 *eq*) at 0 °C. The mixture was stirred at 20 °C for 2 h under nitrogen. The reaction mixture was concentrated under reduced pressure to give a residue. The crude product was purified by reversed-phase HPLC (column: spherical C18, 20-45 um, 100Å, SW 330, mobile phase: [water(0.1%Formic Acid)-ACN]) to give 1-(2-chloroethyl)-3-(1-(pyridin-2-yl)azetidin-3-yl)urea (1.20 g, 4.71 mmol, 62% yield) as a yellow solid.

¹H NMR (400 MHz, DMSO-*d*₆) *δ* = 8.01 (dd, *J* = 0.8, 6.0 Hz, 1H), 7.84 (br t, *J* = 7.6 Hz, 1H), 7.26 - 6.97 (m, 1H), 6.85 - 6.81 (m, 1H), 6.76 (br d, *J* = 8.8 Hz, 1H), 6.41 (br s, 1H), 4.60 - 4.49 (m, 1H), 4.37 (br t, *J* = 8.4 Hz, 2H), 3.95 (br dd, *J* = 5.6, 8.8 Hz, 2H), 3.59 - 3.56 (m, 2H), 3.32 (q, *J* = 6.0 Hz, 2H). MS (ESI) m/z 255.2 [M+H]⁺

Step 4. To a solution of 1-(2-chloroethyl)-3-(1-(pyridin-2-yl)azetidin-3-yl)urea (0.600 g, 2.36 mmol, 1.00 *eq)* in dimethyl formamide (3.00 mL) was added sodium hydride (141 mg, 3.53 mmol, 60% purity, 1.50 *eq)* at 0 °C. The mixture was stirred at 20 °C for 1 h under nitrogen. The reaction mixture was adjust pH to 5-6 with formic acid to give a solution. The solution was purified by reversed-phase HPLC (column: spherical C18, 20-45 um, 100A, SW 80, mobile phase: [water (0.1%Formic Acid)-ACN]) to give 1-(1-(pyridin-2-yi) azetidin-3-yl)imidazolidin-2-one (500 mg, crude) as a yellow solid.

¹H NMR (400 MHz, DMSO-*d*₆) *δ* = 8.09 - 8.01 (m, 1H), 7.55 - 7.45 (m, 1H), 6.66 - 6.58 (m, 1H), 6.51 (br s, 1H), 6.41 - 6.35 (m, 1H), 4.74 - 4.67 (m, 1H), 4.06 (t, *J* = 8.4 Hz, 2H), 4.01 - 3.93 (m, 2H), 3.55 - 3.41 (m, 2H), 3.33 - 3.21 (m, 2H).

Step 5. To a solution of 1-(1-(pyridin-2-yl)azetidin-3-yl)imidazolidin-2-one (50.0 mg, 229 umol, 1.00 *eq*) in dioxane (1.00 mL) was added 3-(5-bromo-1-oxoisoindolin-2-yl)-1-((2-(trimethylsilyl)ethoxy)methyl)piperidine-2,6- dione (114 mg, 252 umol, 1.10 *eq*), potassium carbonate (63.3 mg, 458 umol, 2.00 *eq*), *N,N*-dimethylethylenediamine (10.1 mg, 114 umol, 12.3 uL, 0.500 *eq*) and copper(I) iodide (8.73 mg, 45.8 umol, 0.200 *eq*). The mixture was stirred at 100 °C for 12 h under nitrogen. The mixture was filtered to give a filter liquor. The filter liquor was purified by reversed-phase HPLC (column: spherical C18, 20-45 um, 100A, SW 120, mobile phase: [water(0.1%Formic Acid)-ACN]) to give 3-(1-oxo-5-(2-oxo-3 -(1-(pyridin-2-yl)azetidin-3-yl)imidazolidin-1-yl)isoindolin-2-yl)-1-((2-(trimethylsilyl)ethoxy)methyl)piperidine-2,6-dione (75.0 mg, 127 umol, 11% yield) as a yellow solid.

¹H NMR (400 MHz, DMSO-*d*₆) *δ* = 8.09 - 8.04 (m, 1H), 7.84 (s, 1H), 7.76 - 7.72 (m, 1H), 7.70 - 7.67 (m, 1H), 7.64 (br t, *J* = 7.8 Hz, 1H), 6.73 (br t, *J* = 6.0 Hz, 1H), 6.55 (br d, *J* = 8.4 Hz, 1H), 5.20 (dd, *J* = 5.2*,* 13.2 Hz, 1H), 5.05 (q, *J* = 9.6 Hz, 2H), 4.96 - 4.87 (m, 1H), 4.45 (br d, *J* = 17.2 Hz, 1H), 4.29 - 4.25 (m, 1H), 4.24 - 4.16 (m, 4H), 3.98 - 3.90 (m, 2H), 3.76 - 3.69 (m, 2H), 3.52 (dt, *J* = 5.2, 8.0 Hz, 2H), 3.06 - 3.00 (m, 1H), 2.83 - 2.76 (m, 1H), 2.41 - 2.33 (m, 1H), 2.06 - 1.99 (m, 1H), 0.87 - 0.80 (m, 2H), -0.02 (s, 9H).

Step 6. To a solution of 3-(1-oxo-5-(2-oxo-3-(1-(pyridin-2-yl)azetidin-3-yl)imidazolidin-1-yl)isoindolin-2-yl)-1-((2- (trimethylsilyl)ethoxy)methyl)piperidine-2,6-dione (75.0 mg, 126 umol, 1.00 *eq*) in dichloromethane (10 mL) was added trifluoroacetic acid (1.54 g, 13.5 mmol, 1 mL, 106 *eq*). The mixture was stirred at 20 °C for 0.5 h. The reaction mixture was concentrated under reduced pressure to give 1-(hydroxymethyl)-3-(1-oxo-5- (2-oxo-3-(1-(pyridin-2-yl)azetidin-3-yl)imidazolidin-1-yl)isoindolin-2-yl)piperidine-2,6-dione (60.0 mg, 122 umol, 96% yield) as a yellow solid.
MS (ESI) m/z 491.2 [M+H]⁻

Step 7. To a solution of 1-(hydroxymethyl)-3-(1-oxo-5-(2-oxo-3-(1-(pyridin-2-yl)azetidin-3-yl)imidazolidin-1-yl)isoindolin-2-yl)piperidine-2,6-dione (60.0 mg, 122 umol, 1.00 *eq*) in acetonitrile (10 mL) was added ammonium hydroxide (455 mg, 3.89 mmol, 0.500 mL, 30% purity, 31.8 *eq*). The mixture was stirred at 20 °C for 10 min. The reaction mixture was quenched with formic acid (0.1 ml) to give a solution. The solution was purified by *Prep*-HPLC (column: UniSil 3-100 C18 Ultra (150*25mm*3um);mobile phase: [water(FA)-ACN];B%: 3%-33%,7min) and lyophilized to give 3-(1-oxo-5-(2-oxo-3-(1-(pyridin-2-yl) azetidin-3-yl)imidazolidin-1-yl)isoindolin-2-yl)piperidine-2,6-dione (34.1 mg, 74.2 umol, 60% yield) as an off-white solid.

¹H NMR (400 MHz, DMSO-*d*₆) *δ* = 10.97 (s, 1H), 8.13 (s, 1H), 8.07 (d, *J* = 4.4 Hz, 1H), 7.83 (s, 1H), 7.76 - 7.71 (m, 1H), 7.70 - 7.64 (m, 2H), 6.74 (t, *J* = 6.4 Hz, 1H), 6.57 (br d, *J* = 8.0 Hz, 1H), 5.09 (dd, *J* = 5.2*,* 13.2 Hz, 1H), 4.97 - 4.87 (m, 1H), 4.48 - 4.38 (m, 1H), 4.29 (br d, *J* = 17.2 Hz, 1H), 4.27 - 4.18 (m, 4H), 3.99 - 3.90 (m, 2H), 3.76 - 3.68 (m, 2H), 2.97 - 2.84 (m, 1H), 2.60 (br d, *J* = 16.8 Hz, 1H), 2.39 (dq, *J* = 4.4, 13.2 Hz, 1H), 2.04 - 1.93 (m, 1H). MS (ESI) m/z 461.2 [M+H]⁺

### Example 27. Synthesis of Compound 129

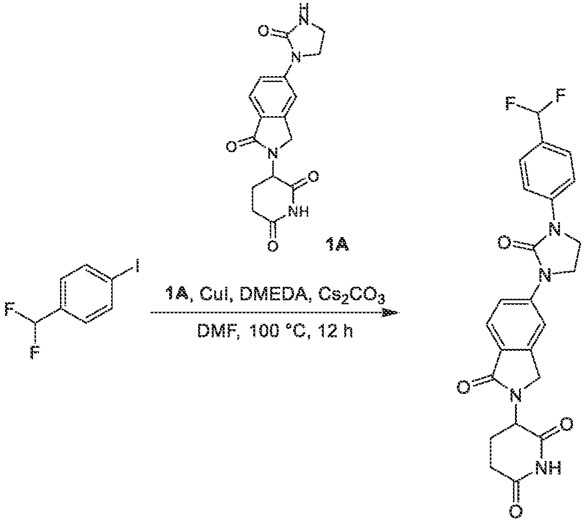

Step 1. To a solution of 1-(difluoromethyl)-4-iodobenzene (46.4 mg, 183 umol, *1.20 eq),* 3-(1-oxo-5-(2-oxoimidazolidin-1-yl)isoindolin-2-yl)piperidine-2,6-dione (50.0 mg, 152 umol, 1.00 *eq)* and cesium carbonate (99.2 mg, 305 umol, 2.00 *eq)* in dimethyl formamide (1.00 mL) was added copper iodide (2.90 mg, 15.2 umol, 0.100 *eq) and N*¹*,N*²-dimethylethane-1,2-diamine (6.71 mg, 76.1 umol, 8.20 uL, 0.500 *eq*), the mixture was stirred at 100 °C for 2 h. The reaction mixture was filtered to give a filtrate, the filtrate was concentrated to give a residue. The residue was purified by reversed-phase HPLC (0.1% FA condition) to give a crude product. The crude product was purified by *prep*-HPLC (column: Phenomenex C18 150 × 25 mm × 10 um; mobile phase: [water (NH4HCO3) - ACN]; B%: 22% - 52%, 14 min) to afford 3-(5-(3-(4-(difluoromethyl)phenyl)-2-oxoimidazolidin-1-yl)-1-oxoisoindolin-2-yl)piperidine-2,6-dione (10.64 mg, 23.4 umol, 5% yield) as a white solid.

¹H NMR (400 MHz, DMSO-*d*₆) *δ* = 11.10 - 10.82 (m, 1H), 7.96 - 7.92 (m, 1H), 7.83 - 7.77 (m, 3H), 7.76 - 7.72 (m, 1H), 7.63 - 7.57 (m, 2H), 7.15 - 6.86 (m, 1H), 5.15 - 5.07 (m, 1H), 4.51 - 4.44 (m, 1H), 4.38 - 4.31 (m, 1H), 4.11 - 4.04 (m, 4H), 2.96 - 2.89 (m, 1H), 2.69 - 2.63 (m, 1H), 2.40 - 2.36 (m, 1H), 2.05 - 1.99 (m, 1H). MS (ESI) m/z 455.0 [M+H]⁺

### Example 28. Synthesis of Compound 123

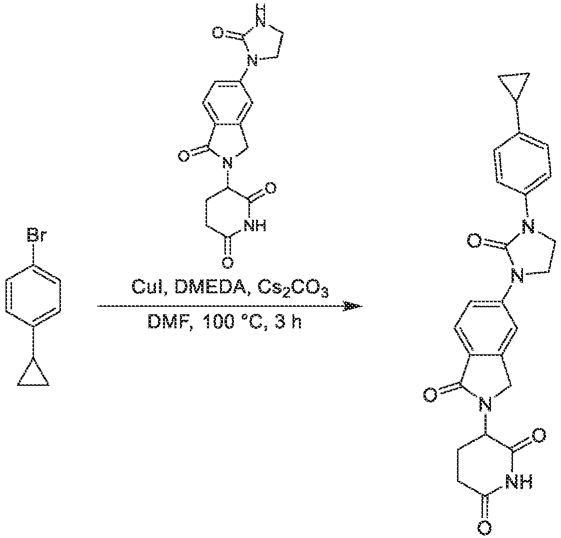

Step 1. To a mixture of 1-bromo-4-cyclopropylbenzene (50.0 mg, 253 umol, 1.00 *eq)* and 3-(1-oxo-5-(2-oxoimidazolidin-1-yl)isoindolin-2-yl)piperidine-2,6-dione (49.9 mg, 152 umol, 0.600 *eq)* in dimethyformamide (1.00 mL) was added cesium carbonate (165 mg, 507 umol, 2.00 *eq*), *n*,*n*'-dimethylethylenediamine (11.2 mg, 126 umol, 13.6 uL, 0.500 *eq*) and cuprous iodide (9.66 mg, 50.7 umol, 0.200 *eq*). The mixture was stirred at 100 °C for 2 h under nitrogen. The reaction mixture was filterted. The filtrate was purified by reversed phase HPLC (0.1% formic acid condition) and lyophilized. The reverse phase collection was filterted to give 3-(5-(3-(4-cyclopropylphenyl)-2-oxoimidazolidin-1-yl)-1-oxoisoindolin-2-yl)piperidine-2,6-dione (73.76 mg, 164 µmol, 6% yield, 99% purity) as a yellow solid.

¹H NMR (400 MHz, DMSO-*d*₆) *δ* = 10.98 (s, 1H), 7.90 (s, 1H), 7.79 - 7.68 (m, 2H), 7.51 (br d, *J =* 7.8 Hz, 2H), 7.09 (br d, *J* = 7.6 Hz, 2H), 5 10 (br dd, *J* = 4.9, 13.3 Hz, 1H), 4.49 - 4.41 (m, 1H), 4.35 - 4.27 (m, 1H), 4.00 (br dd, *J* = 5.2, 13.9 Hz, 4H), 2.96 - 2.86 (m, 1H), 2.60 (br d, *J* = 16.9 Hz, 1H), 2.40 (br dd, *J* = 3.8, 12.9 Hz, 1H), 2.00 (br dd, *J* = 5.4, 6.8 Hz, 1H), 1.93 - 1.85 (m, 1H), 0.96 - 0.87 (m, 2H), 0.68 - 0.60 (m, 2H). MS (ESI) m/z. 445.2 [M+H]⁺

### Example 29. Synthesis of Compound 146

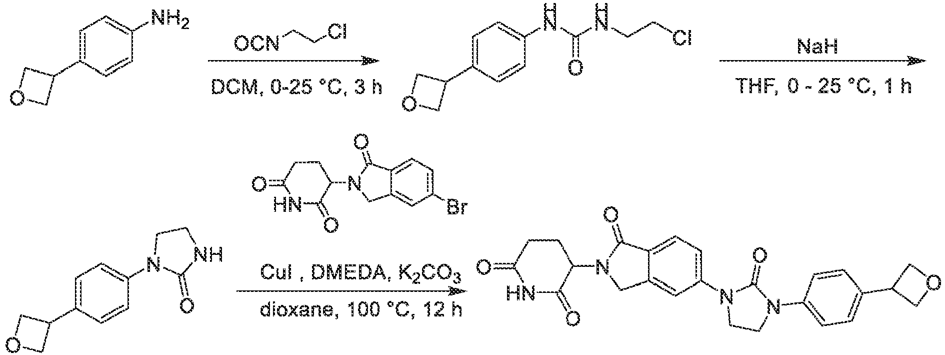

Step 1. To a solution of 4-(oxetan-3-yl)aniline (450 mg, 3.02 mmol, 1.00 *eq*) in dichloromethane (15.0 mL) was added 1-chloro-2-isocyanato-ethane (380 mg, 3.60 mmol, *1.19 eq*) at 0 °C. The mixture was stirred at 25 °C for 3 h. The mixture was filtered. The filter cake was concentrated under reduced pressure to afford 1-(2-chloroethyl)-3-(4-(oxetan-3-yl)phenyl)urea (1.50 g, crude) as a white solid.
¹H NMR. (400 MHz, DMSO-*d*₆) *δ* = 8.63 (s, 1H), 7.38 (d, *J* = 8.5 Hz, 2H), 7.26 (d, *J* = 8.5 Hz, 2H), 6.38 (t, *J* = 5.8 Hz, 1H), 4.89 (dd, *J* = 5.8, 8.4 Hz, 2H), 4.57 (t, *J* = 6.3 Hz, 2H), 4.15 (quin, *J* = 7.6 Hz, 1H), 3.65 (t, *J* = 6.2 Hz, 2H), 3.41 (q, *J* = 6.0 Hz, 2H). MS (ESI) m/z 255.3 [M+H]⁺
Step 2. To a solution of 1-(2-chloroethyl)-3-(4-(oxetan-3-yl)phenyl)urea (500 mg, 1.96 mmol, 1.00 *eq*) in tetrahydrofuran (8.00 mL) and dimethyl formamide (2.00 mL) was added sodium hydride (158 mg, 3.94 mmol, 60% purity, 2.01 *eq*) at 0 °C. The mixture was stirred at 25 °C for 1 h. The mixture was quenched with methanol (10 mL), then the mixture was adjusted to pH = 7 with hydrochloric acid. The mixture was concentrated under reduced pressure to afford 1-(4-(oxetan-3-yl)phenyl)imidazolidin-2-one (500 mg, crude) as a white solid. MS (ESI) m/z 219.1 [M+H]⁺
Step 3. To a solution of 1-(4-(oxetan-3-yl)phenyl)imidazolidin-2-one (50.0 mg, 229 umol, 1.00 *eq*) and 3-(5-bromo-l-oxoisoindolin-2-yl)piperidine-2,6-dione (111 mg, 344 umol, 1.50 *eq*) in dioxane (5.00 mL) was added *N*,*N*-dimethylethylenediamine (10.1 mg, 115 umol, 12.3 uL, 0.500 *eq*)*,* potassium carbonate (95.0 mg, 687 umol, 3.00 *eq*) and copper iodide (22.0 mg, 116 umol, 0.504 *eq*) in portions under nitrogen atmosphere. The mixture stirred at 100 °C for 12 h. The mixture was filtered. The filtrate was concentrated under reduced pressure to give the crude product which was purified by reversed-phase HPLC (column: spherical C18, 20-45 um, 100Å, SW 80, mobile phase: [water(0.1%Formic Acid)-ACN];B%: 5%-70%, 30 min) and further purified by *Prep*-NPLC (column: Welch Ultimate XB-CN 250*50*10um;mobile phase: [Hexane-IPA];B%: 20%-60%,21min). The desired fraction was collected and concentrated under pressure to give a residue. The residue was purified by *Prep*-HPLC (column: Phenomenex luna C18 150*25mm* 10um;mobile phase: [water(FA)-ACN];B%: 19%-49%,9min). The desired fraction was collected and lyophilized to afford 3-(5-(3-(4-(oxetan-3-yl)phenyl)-2-oxoimidazolidin-1-yl)-1-oxoisoindolin-2-yl)piperidine-2,6-dione (17.41 mg, 35.2 umol, 2% yield, 93% purity) as a yellow solid. ¹H NMR (400 MHz, DMSO-*d₆*) *δ* = 10.98 (br s, 1H), 7.92 (s, 1H), 7.81 - 7.75 (m, 1H), 7.74 - 7.69 (m, 1H), 7.65 (d, *J* = 8.6 Hz, 2H), 7.42 (d, *J* = 8.6 Hz, 2H), 5.10 (dd, *J* = 5.1, 13.3 Hz, 1H), 4.94 (dd, *J =* 5.9, 8.4 Hz, 2H), 4.62 (t, *J* = 6.3 Hz, 2H), 4.49 - 4.42 (m, 1H), 4.36 - 4.29 (m, 1H), 4.24 (quin, *J* = 7.6 Hz, 1H), 4.09 - 3.98 (m, 4H), 2.98 - 2.87 (m, 1H), 2.60 (br dd, *J* = 22, 15.3 Hz, 1H), 2.40 (dq, *J* = 4.7, 13.2 Hz, 1H), 2.05 - 1.96 (m, 1H). MS (ESI) m/z 461.1 [M+H]⁺

### Example 30. Synthesis of Compound 131

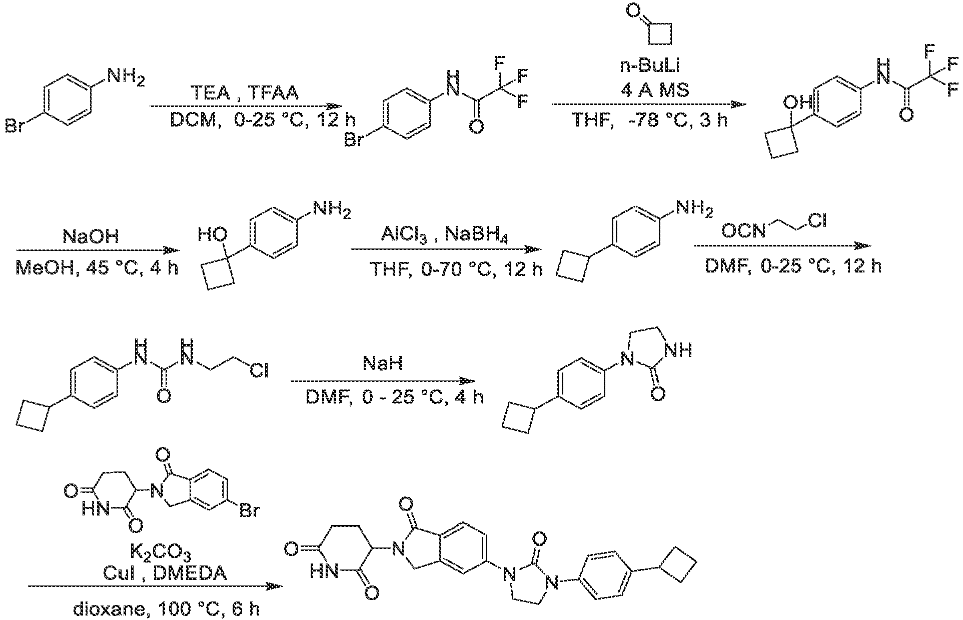

Step 1. To a solution of 4-bromoaniline (10.0 g, 58.1 mmol, 1.00 *eq*)*,* triethylamine (17.0 g, 168 mmol, 23.4 mL, 2.90 *eq*) in dichloromethane (30.0 mL) was added (2,2,2-trifluoroacetyl) 2,2,2-trifluoroacetate (18.3 g, 87.2 mmol, 12.1 mL, 1.50 *eq*) at 0 °C under nitrogen. The mixture was stirred at 25 °C for 12 h. The reaction mixture was diluted with sodium hydroxide solution (30.0 mL) and extracted with ethyl acetate (2 × 60 mL). The organic phase was separated, washed with brine (30 mL), dried over sodium sulfate, filtered and concentrated under reduced pressure to give a residue which was purified by column chromatography (SiO₂, Petroleum ether/Ethyl acetate=1/0 to 20/1) to give *N*-(4-bromophenyl)-2,2,2-trifluoroacetamide (15.0 g, 55.9 mmol, 96% yield) a yellow solid. ¹H NMR (400 MHz, DMSO-*d*₆) *δ* = 11.36 (br s, 1H), 7.68 - 7.56 (m, 4H).

Step 2. To a solution of *N*-(4-bromophenyl)-2,2,2-trifluoroacetamide (10.0 g, 37.3 mmol, 1.00 *eq*), Molecular sieves 4A (5.00 g, 37.3 mmol, 1.00 *eq*) in tetrahydrofuran (130 mL) was added butyllithium (2.5 M, 37.3 mL, 2.50 *eq*) dropwise under nitrogen at -78 °C. The mixture was stirred at -78 °C for 1 h. The cyclobutanone (2.86 g, 40.8 mmol, 3.05 mL, 1.09 *eq*) was added to the mixture at -78 °C under nitrogen. The mixture was stirred at - 78 °C for 2 h. The reaction mixture was quenched with saturated ammonium chloride solution (50.0 mL). The solution was diluted with water (20 mL) and extract with ethyl acetate (2 × 50 mL). The organic phase was separated, washed with brine (30 mL), dried over sodium sulfate, filtered and concentrated under reduced pressure to give 2,2,2-trifluoro-*N*-(4-(1-hydroxycyclobutyl)phenyl)acetamide (9.00 g, crude) as a yellow oil. ¹H NMR (400 MHz, DMSO-*d*₆) *δ* = 11.23 (br s, 1H), 7.62 (d, *J* = 8.6 Hz, 2H), 7.50 (d, *J* = 8.6 Hz, 2H), 5.51 (s, 1H), 2.40 - 2.34 (m, 2H), 2.30 - 2.24 (m, 2H), 1.92 - 1.85 (m, 2H).

Step 3. A solution of 2,2,2-trifluoro-*N*-(4-(1-hydroxycyclobutyl)phenyl)acetamide (9.00 g, 34.7 mmol, 1.00 *eq*) in methanol (30.0 mL), sodium hydroxide (30.0 mL, 40% purity) was stirred at 45 °C for 4 h. The reaction mixture was concentrated under reduced pressure to give a residue. The residue was purified by column chromatography (SiO₂, Petroleum ether/Ethyl acetate=1/0 to 0/1) to give 1-(4-aminophenyl)cyclobutanol (4.50 g, 27.5 mmol, 79% yield) as a yellow solid.

Step 4. To a solution of 1-(4-aminophenyl)cyclobutanol (4.50 g, 27.5 mmol, 1.00 eq), sodium borohydride (5.74 g, 151 mmol, 5.50 *eq*) in tetrahydrofuran (60.0 mL) was added aluminum trichloride (11.0 g, 82.7 mmol, 4.52 mL, 3.00 *eq*) under nitrogen at 0 °C. The mixture was stirred at 70 °C for 12 h under nitrogen. The reaction mixture was diluted with water (100 mL) and extracted with ethyl acetate (2 × 200 mL). The organic phase was separated, washed with brine (80 mL), dried over sodium sulfate, filtered and concentrated under reduced pressure to give 4-cyclobutylaniline (3.85 g, 26.1 mmol, 94% yield) as a yellow solid.

¹H NMR (400 MHz, DMSO-*d*₆) *δ* = 6.96 (d, *J* = 8.1 Hz, 2H), 6.62 (d, *J* = 8.1 Hz, 2H), 6.30 - 5.71 (m, 2H), 3.35 (quin, *J* = 8.5 Hz, 1H), 2.25 - 2.13 (m, 2H), 2.03 - 1.94 (m, 2H), 1.92 - 1.83 (m, 1H), 1.81 - 1.71 (m, 1H). MS (ESI) m/z 147.9 [M+H]⁺

Step 5. To a solution of 4-cyclobutylaniline (2.00 g, 13.5 mmol, 1.00 *eq*) in dimethyl formamide (20.0 mL) was added 1-chloro-2-isocyanatoethane (2.15 g, 20.3 mmol, 1.73 mL, 1.50 *eq*) at 0 °C. The mixture was stirred at 25 °C for 12 h. The reaction mixture was concentrated under reduced pressure to give a residue which was added dimethyl formamide (10.0 mL). Then the solution was added into water (80 mL) dropwise. Then the mixture was filtered to give filter cake. The filter cake was added water (20 mL) and lyophilized to give 1-(2-chloroethyl)-3-(4-cyclobutylphenyl)urea (2.80 g, 11.0 mmol, 81% yield) as a yellow solid.

¹H NMR (400 MHz, DMSO-*d*₆) *δ* = 8.54 (s, 1H), 7.30 (d, *J* = 8.4 Hz, 2H), 7.08 (d, *J* = 8.5 Hz, 2H), 6.36 - 6.28 (m, 1H), 3.64 (t, *J* = 6.2 Hz, 2H), 3.44 - 3.37 (m, 3H), 2.28 - 2.19 (m, 2H), 2.07 - 1.99 (m, 2H), 1.89 (br d, *J* = 2.3 Hz, 1H), 1.82 - 1.74 (m, 1H), MS (ESI) m/z 252.8 [M+H]⁺

Step 6. To a solution of 1-(2-chloroethyl)-3-(4-cyclobutylphenyl)urea (1.00 g, 3.96 mmol, 1.00 *eq*) in dimethyl formamide (15.0 mL) was added sodium hydride (237 mg, 5.93 mmol, 60% purity, 1.50 *eq*) at 0 °C under nitrogen. The mixture was stirred at 25 °C for 4 h. The reaction mixture was quenched with saturated ammonium chloride (10 mL) to give a solution which was diluted with water (20 mL) and extracted with ethyl acetate (2 × 50 mL). The organic phase was separated, washed with brine (10 mL), dried over sodium sulfate, filtered and concentrated under reduced pressure to give 1-(4-cyclobutylphenyl)imidazolidin-2-one (650 mg, 3.01 mmol, 75% yield) as a yellow solid.

¹H NMR (400 MHz, DMSO-*d*₆) *δ* = 7.46 (d, *J* = 8.6 Hz, 2H), 7.15 (d, *J* = 8.6 Hz, 2H), 6.90 - 6.82 (m, 1H), 3.81 (dd, *J* = 6.9, 8.9 Hz, 2H), 3.45 (t, *J =* 8.8 Hz, 1H), 3.41 - 3.35 (m, 2H), 2.29 - 2.22 (m, 2H), 2.10 - 2.00 (m, 2H), 1.98 - 1.89 (m, 1H), 1.84 - 1.75 (m, 1H). MS (ESI) m/z 216.9 [M+H]⁺

Step 7. To a solution of 1-(4-cyclobutylphenyl)imidazolidin-2-one (20.0 mg, 92.4 umol, 1.00 *eq*), 3-(5-bromo-1-oxoisoindolin-2-yl)piperidine-2,6-dione (44.8 mg, 138 umol, *1.50 eq*) and potassium carbonate (38.3 mg, 277 umol, 3.00 *eq*) in 1,4-dioxane (2.00 mL) was added copper(I) iodide (3.52 mg, 18.4 umol, 0.2 *eq*) and *N*,*N*'-dimethylethane-1,2-diamine (4.08 mg, 46.2 umol, 4.98 uL, 0.500 *eq)* in portions under nitrogen. The mixture was stirred at 100 °C for 6 h. The mixture was filtered to give a filter liquor, then it was concentrated under reduced pressure to give a residue. The residue was purified by *prep*-HPLC (column: Phenomenex luna C18 150*25mm* 10um;mobile phase: [water(formic acid)-acetonitrile];B%: 41%-71%, 10min) to give 3-(5-(3-(4-cyclobutylphenyl)-2-oxoimidazolidin-1-yl)-1-oxoisoindolin-2-yl)piperidine-2,6-dione (14.0 mg, 30.5 umol, 5% yield) as a white solid.

¹H NMR (400 MHz, DMSO-*d*₆) *δ* = 10.98 (s, 1H), 7.91 (s, 1H), 7.80 - 7.75 (m, 1H), 7.74 - 7.69 (m, 1H), 7.56 (d, *J* = 8.6 Hz, 2H), 7.25 (d, *J =* 8.5 Hz, 2H), 5.10 (dd, *J =* 5.1, 13.3 Hz, 1H), 4.48 - 4.41 (m, 1H), 4.35 - 4.29 (m, 1H), 4.08 - 3.96 (m, 4H), 3.55 - 3.45 (m, 1H), 2.97 - 2.86 (m, 1H), 2.68 - 2.56 (m, 1H), 2.40 (br dd, *J* = 4.5, 13.0 Hz, 1H), 2.31 (br d, *J* = 2.8 Hz, 2H), 2.13 - 2.04 (m, 2H), 2.04 - 1.94 (m, 2H), 1.86 - 1.75 (m, 1H). MS (ESI) m/z 459.0 [M+H]⁺

### Example 31. Synthesis of Compound 144

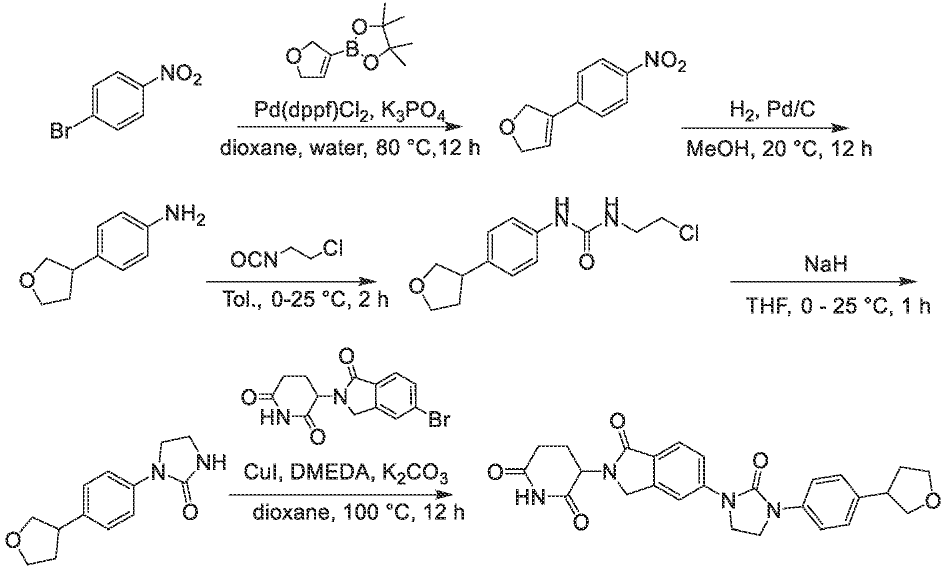

Step 1. To a solution of 1-bromo-4-nitrobenzene (618.2 mg, 3.06 mmol, 1.20 *eq*) in dioxane (2.00 mL) and water (0.20 mL) was added 2-(2,5-dihydrofuran-3-yl)-4,4,5,5-tetramethyl-1,3,2-dioxaborolane (500 mg, 2.55 mmol, 1.00 *eq*), potassium phosphate (1.62 g, 7.65 mmol, 3.00 *eq*) and [1,1'-bis (diphenylphosphino) ferrocene] dichloropalladium(II) (186 mg, 255 umol, 0.10 *eq*) under nitrogen. The mixture was stirred at 80 °C for 12 h. The reaction mixture was diluted with water (30 mL) and exacted with ethyl acetate (2 × 30 mL). The organic phase was separated, washed with brine (10 mL), dried over sodium sulfate, filtered and concentrated under reduced pressure to give a residue. The residue was purified by reversed-phase HPLC (column: spherical C18, 20-45 um, 100Å, SW 80, mobile phase: [water(0.1%Formic Acid)-ACN]) and lyophilized to give 3-(4-nitrophenyl)-2,5-dihydrofuran (436 mg, 2.28 mmol, 89% yield) was obtained as a white solid. ¹H NMR (400 MHz, DMSO-*d*₆) *δ =* 8.22 (d, *J* = 8.8 Hz, 2H), 7.69 (d, *J* = 8.6 Hz, 2H), 6.87 - 6.82 (m, 1H), 4.94 (dt, *J* = 1.9, 4.8 Hz, 2H), 4.83 - 4.73 (m, 2H).

Step 2. To a solution of 3-(4-nitrophenyl)-2,5-dihydrofuran (436 mg, 2.28 mmol, *1.00 eq*) in methanol (50.0 mL) was added palladium on carbon (50.0 mg, 10% purity) under hydrogen(15 Psi) . The mixture was stirred at 25 °C for 12 h. The reaction mixture was filtered to give a liquor, then was concentrated under reduced pressure to give 4-tetrahydrofuran-3-ylaniline (350 mg, crude) as a black solid. ¹H NMR (400 MHz, DMSO-*d*₆) *δ* = 6.98 (br d, *J* = 8.3 Hz, 1H), 6.94 (br s, 1H), 6.67 - 6.53 (m, 1H), 6.48 (dd, *J* = 8.4, 10.9 Hz, 1H), 5.04 - 4.72 (m, 1H), 4.00 - 3.86 (m, 2H), 3.79 - 3.71 (m, 1H), 3.21 - 3.14 (m, 2H), 2.63 (d, *J* = 5.1 Hz, 1H), 2.25 - 2.13 (m, 1H), 1.88 - 1.76 (m, 1H).

Step 3. To a solution of 4-(tetrahydrofuran-3-yl)aniline (320 mg, 1.96 mmol, 1.00 *eq)* in toluene.(5.00 mL) was added 1-chloro-2-isocyanatoethane (310 mg, 2.94 mmol, 1.50 eq) at 0 °C. The mixture was stirred at 25 °C for 2 h. The mixture was filtered. The filter cake was concentrated under reduced pressure to give 1-(2-chloroethyl)-3-(4-(tetrahydrofuran-3-yl)phenyl)urea (411 mg, crude) as a yellow solid.

¹H NMR (400 MHz, DMSO-*d*₆) *δ* = 8.61 (s, 1H), 7.32 (d, *J* = 8.5 Hz, 2H), 7.12 (d, J= 8.5 Hz, 2H), 6.38 (br t, *J* = 5.7 Hz, 1H), 3.99 (t, *J* = 7.8 Hz, 1H), 3.91 (dt, *J =* 4.4, 8.3 Hz, 1H), 3.77 (q, *J* = 8.0 Hz, 1H), 3.64 (t, *J* = 6.1 Hz, 2H), 3.47 (t, *J* = 8.1 Hz, 1H), 3.40 (q, *J* = 6.0 Hz, 2H), 3.32 - 3.24 (m, 1H), 2.30 - 2.19 (m, 1H), 1.86 (qd, *J* = 8.2, 12.1 Hz, 1H).

Step 4. To a solution of 1-(2-chloroethyl)-3-(4-(tetrahydrofuran-3-yl)phenyl)urea (411 mg, 1.53 mmol, 1.00 *eq*) in tetrahydrofuran (10.0 mL) was added sodium hydride (91.7 mg, 2.29 mmol, 60% purity, 1.50 *eq*) at 0 °C under nitrogen. The mixture was stirred at 25 °C for 1 h. The reaction mixture was quenched with formic acid (1.00 ml) to give a solution. The solution was diluted with water (30 mL) and extracted with ethyl acetate (2 × 30 mL). The organic phase was separated, washed with brine (10 mL), dried over sodium sulfate, filtered. The residue was purified by column chromatography (SiO2, petroleum ether/ethyl acetate=8/1 to 0/1) to give 1-(4-(tetrahydrofuran-3-yl)phenyl)imidazolidin-2-one (400 mg, crude) as a white solid. ¹H NMR (400 MHz, DMSO-*d*₆) *δ =* 7.47 (d, *J* = 8.6 Hz, 2H), 7.20 (d, *J* = 8.5 Hz, 2H), 6.87 (br s, 1H), 4.06 - 3.88 (m, 2H), 3.87 - 3.73 (m, 3H), 3.49 (t, *J* = 7.9 Hz, 1H), 3.38 (br t, *J* = 7.9 Hz, 2H), 3.31 - 3.27 (m, 1H), 2.31 - 2.22 (m, 1H), 1.92 - 1.82 (m, 1H).

Step 5. To a solution for 1-(4-(tetrahydrofuran-3-yl)phenyl)imidazolidin-2-one (50 mg, 215 umol, 1.00 *eq*) in dioxane (1.00 mL) was added 3-(5-bromo-1-oxoisoindolin-2-yl)piperidine-2,6-dione (90.43 mg, 279 umol, 1.30 *eq*), potassium carbonate (89.2 mg, 645.7 umol, 3.00 eq), *N*,*N*'-dimethylethylenediamine (1.90 mg, 21.53 umol, 2.32 uL, 0.10 *eq*) and copper iodide (4.10 mg, 21.5 umol, 0.100 *eq*) under nitrogen. The mixture was stirred at 100 °C for 12 h. The mixture was filtered to give a filter cake and liquor. The liquor was purified by reversed-phase HPLC (column: spherical C18, 20-45 um, 100Å, SW80, mobile phase: [water(0.1%Formic Acid)-ACN]) and further purified by *prep*-HPLC (column: Phenomenex Luna C18 150*25mm*10um;mobile phase: [water(FA)-ACN];B%: 24%-54%,10min) and lyophilized. The filter cake was purified by *prep*-HPLC (column: Phenomenex luna C18 150*25mm* 10um;mobile phase: [water(FA)-ACN];B%: 23%-53%, 10min) and lyophilized to give 3-(1-oxo-5-(2-oxo-3- (4- (tetrahydrofuran-3-yl)phenyl)imidazolidin-1-yl)isoindolin-2-yl)piperidine-2,6-dione (20.10 mg, 42.36 umol, 4.92% yield) as a white solid. ¹H NMR (400 MHz, DMSO-*d*₆) *δ* = 10.98 (br s, 1H), 7.91 (s, 1H), 7.80 - 7.75 (m, 1H), 7.74 - 7.69 (m, 1H), 7.58 (d, *J* = 8.6 Hz, 2H), 7.29 (d, *J* = 8.6 Hz, 2H), 5.10 (dd, *J* = 5.0, 13.3 Hz, 1H), 4.52 - 4.40 (m, 1H), 4.37 - 4.27 (m, 1H), 4.07 - 3.92 (m, 6H), 3.80 (q, *J* = 7.8 Hz, 1H), 3.53 (br t, *J* = 7.9 Hz, 2H), 2.97 - 2.86 (m, 1H), 2.58 (br s, 1H), 2.40 (br dd, *J* = 4.3, 13.3 Hz, 1H), 2.34 - 2.26 (m, 1H), 2.04 - 1.96 (m, 1H), 1.95 - 1.86 (m, 1H). MS (ESI) m/z 475.2 [M+H]⁺

### Example 32. Synthesis of Compound 176

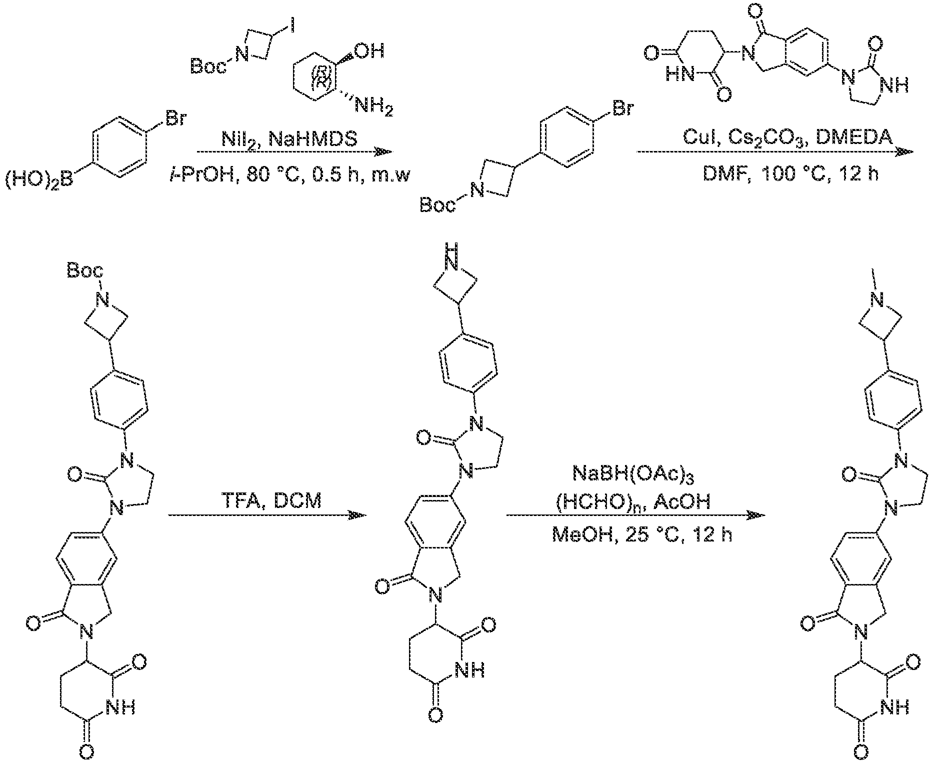

Step 1. To a solution of (4-bromophenyl)boronic acid (1.42 g, 7.06 mmol, 2.00 *eq*), tert-butyl 3-iodoazetidine-1-carboxylate (1.00 g, 3.53 mmol, 1.00 *eq*) in isopropanol (6.00 mL) was added sodium bis(trimethylsilyl)amide (1 M, 7.06 mL, 2.00 *eq*), diiodonickel (66.2 mg, 212 umol, 11.4 uL, 0.0600 *eq*) and (1*R*,2*R*)-2-aminocyclohexanol;hydrochloride (32.1 mg, 212 umol, 0.0600 *eq*)*.* The mixture was stirred at 25 °C for 30 min and heated at 80 °C for 30 min under microwave under nitrogen atmosphere. After cooling to room temperature, the mixture was quenched with saturated ammonium chloride (80 mL) and extracted with ethyl acetate (3 × 100 mL). The combined organic layers were washed with brine (50 mL), dried over anhydrous sodium sulfate and concentrated in vacuum. The mixture was purified by column chromatography (SiO₂, Petroleum ether/Ethyl acetate=200/1 to 20/1) to get *tert*-butyl 3-(4-bromophenyl)azetidine-1-carboxylate (3.00 g, 9.61 mmol, 68% yield) as white oil. ¹H NMR (400 MHz, CDCl₃) *δ* = 7.50 - 7.45 (m, 2H), 7.20 (d, *J* = 8.4 Hz, 2H), 4.33 (t, *J* = 8.8 Hz, 2H), 3.93 (dd, *J* = 6.0, 8.4 Hz, 2H), 3.74 -3.63 (m, 1H), 1.47 (s, 9H).

Step 2. To a solution of *tert*-butyl 3-(4-bromophenyl)azetidine-1-carboxylate (50.0 mg, 160 umol, 1.00 *eq*), 3-(1-oxo-5-(2-oxoimidazolidin-1-yl)isoindolin-2-yl)piperidine-2,6-dione (31.5 mg, 96.1 umol, 0.600 *eq*), cesium carbonate (104 mg, 320 umol, 2.00 *eq*) in *N*,*N*-dimethyl formamide (1.00 mL) was added copper iodide (6.10 mg, 32.0 umol, 0.200 *eq*) and *N*,*N*-dimethylethylenediamine (14.1 mg, 160 umol, 17.2 uL, 1.00 *eq*)*.* The mixture was stirred at 100 °C for 12 h under nitrogen atmosphere. The mixture was adjusted to pH=5-6 with formic acid and diluted with water (30 mL) and extracted with ethyl acetate (3 × 30 mL). The organic phase was separated, washed with brine (30 mL), dried over sodium sulfate, filtered and concentrated under reduced pressure. The mixture was purified by reversed-phase HPLC(column: spherical C18, 20-45 um, 100Å, SW 120, mobile phase: [water(0.1%formic acid) - acetonitrile]) and lyophilized to give *tert*-butyl 3-(4-(3-(2-(2,6-dioxopiperidin-3-yl)-1-oxoisoindolin-5-yl)-2-oxoimidazolidin-1-yl)phenyl)azetidine-1-carboxylate (100 mg, 179 umol, 11% yield) as a yellow solid. ¹H NMR (400 MHz, DMSO-*d*₆) *δ* = 10.97 (br s, 1H), 7.92 (s, 1H), 7.80 - 7.75 (m, 1H), 7.74 - 7.70 (m, 1H), 7.63 (d, *J =* 8.4 Hz, 2H), 7.35 (d, *J* = 8.8 Hz, 2H), 5.10 (dd, *J* = 5.2, 13.2 Hz, 1H), 4.49 - 4.40 (m, 1H), 4.36 - 4.29 (m, 1H), 4.24 (br s, 1H), 4.07 - 3.99 (m, 3H), 3.87 - 3.76 (m, 3H), 2.96 - 2.86 (m, 1H), 2.64 - 2.61 (m, 1H), 2.40 (br dd, *J* = 4.4, 13.2 Hz, 1H), 2.05 - 1.96 (m, 1H), 1.41 (s, 9H). MS (ESI) m/z 560.3 [M+H]⁺

Step 3. To a solution of *tert*-butyl 3-(4-(3-(2-(2,6-dioxopiperidin-3-yl)-1-oxoisoindolin-5-yl)-2-oxoimidazolidin-1-yl)phenyl)azetidine-1-carboxylate (100 mg, 179 umol, 1.00 *eq*) in trifluoroacetic acid (0.500 mL) and dichloromethane (1.50 mL) was stirred at 25 °C for 1 h. The mixture was concentrated under reduced pressure to give 3-(5-(3-(4-(azetidin-3-yl)phenyl)-2-oxoimidazolidin-1-yl)-1-oxoisoindolin-2-yl)piperidine-2,6-dione (80.0 mg, 174 umol, 97% yield) as yellow oil. MS (ESI) m/z 460.1 [M+H]⁺

Step 4. To a solution of 3-(5-(3-(4-(azetidin-3-yl)phenyl)-2-oxoimidazolidin-1-yl)-1-oxoisoindolin-2-yl)piperidine-2,6-dione (80.0 mg, 174 umol, 1.00 *eq*) in methanol (1.00 mL) was added formaldehyde solution (42.4 mg, 522 umol, 38.9 uL, 37% purity, 3.00 *eq*), acetic acid (1.05 mg, 17.4 umol, 9.96^{e-1} uL, 0.100 *eq*)*.* After stirring for 30 min, sodium triacetoxy borohydride (73.8 mg, 348 umol, 2.00 *eq*) was added to the reaction mixture. The mixture was stirred at 25 °C for 12 h. The mixture was filtered. The filtrate was purified by reversed phase HPLC (column: spherical C 18, 20-45 um, 100 Å, SW 120, mobile phase: [water (0.1%Formic Acid)-acetonitrile) and lyophilized to get a residue. The residue was purified by reversed phase HPLC (column: Phenomenex luna C18 150*25mm* 10um;mobile phase: [water(Formic Acid)- acetonitrile]; B%: 0%-29%,7 min) and lyophilized to afford 3-(5-(3-(4-(1-methylazetidin-3-yl)phenyl)-2-oxoimidazolidin-1-yl)-1-oxoisoindolin-2-yl)piperidine-2,6-dione (16.6 mg, 32.21 umol, 19% yield, 92% purity) as a white solid.

¹H NMR (400 MHz, DMSO-*d*₆) δ = 10.70 (br s, 1H), 8.16 (s, 1H), 7.90 (d, *J* = 1.2 Hz, 1H), 7.80 - 7.74 (m, 1H), 7.74 - 7.66 (m, 1H), 7.59 (d, *J =* 8.4 Hz, 2H), 7.34 (d, *J =* 8.4 Hz, 2H), 5.12 - 5.04 (m, 1H), 4.52 - 4.32 (m, 2H), 4.11 - 3.97 (m, 4H), 3.69 - 3.54 (m, 3H), 3.13 (br d, *J* = 6.4 Hz, 2H), 2.91 - 2.84 (m, 1H), 2.69 - 2.60 (m, 1H), 2.44 - 2.36 (m, 1H), 2.36 - 2.25 (m, 3H), 2.12 - 1.99 (m, 1H), MS (ESI) m/z 474.1 [M+H]⁺

### Example 33. Synthesis of Compound 177

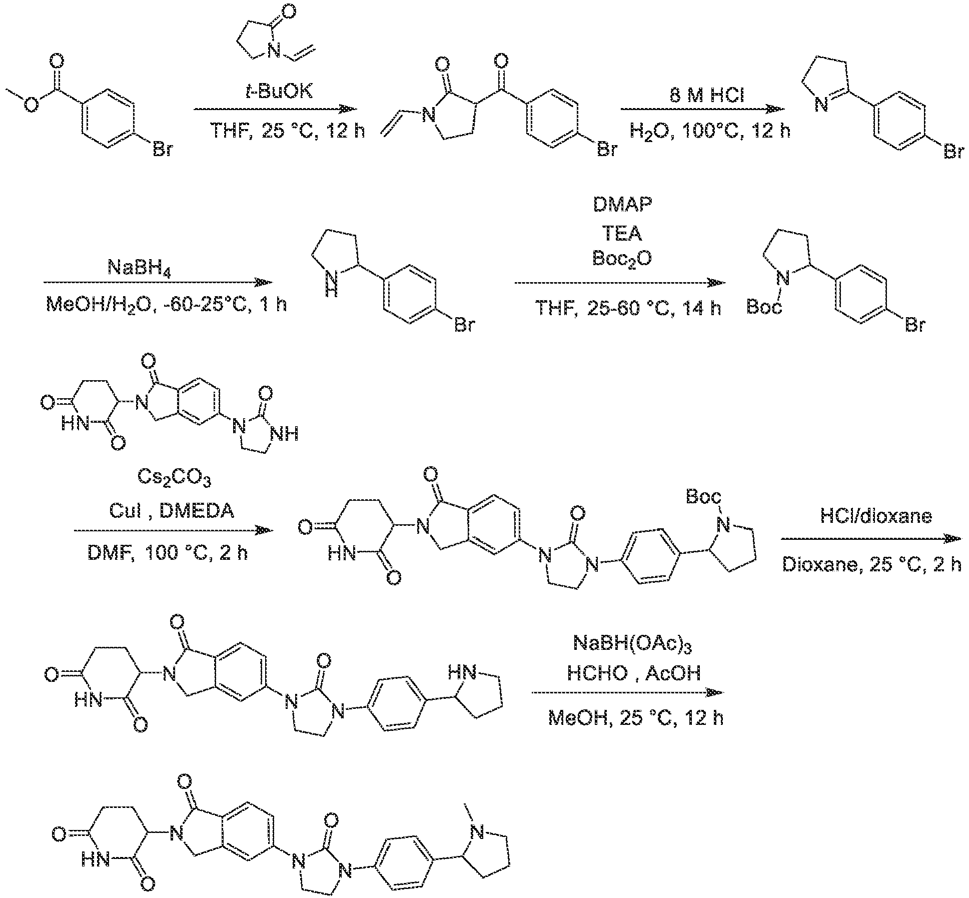

Step 1. To a solution of methyl 4-bromobenzoate (9.40 g, 43.7 mmol, 1.00 *eq*) and 1-vinylpyrrolidin-2-one (4.30 g, 38.6 mmol, 4.13 mL, 0.88 *eq*) in tetrahydrofuran (100 mL) was added potassium *tert*-butoxide (4.93 g, 43.9 mmol, 1.00 *eq*) under nitrogen atmosphere (15 *psi*). The mixture was stirred at 25 °C for 12 h. The water (300 mL) was added and the pH adjusted to 7 with hydrochloric acid (1.00 M). The resulting mixture was extracted with ethyl acetate (3 × 200 mL). The combined organic layers were dried over anhydrous sodium sulfate, filtered and concentrated in vacuum to give a residue. The residue was purified by column chromatography on silica gel eluted with petroleum ether/ethyl acetate = 10/1 to 1/1. The desired fraction was collected and concentrated reduced pressure to afford 3-(4-bromobenzoyl)-1-vinylpyrrolidin-2-one (8.20 g, 27.8 mmol, 63% yield) as a brown solid. ¹H NMR (400 MHz, CHLOROFORM-d) *δ* = 7.96 - 7.88 (m, 1H), 7.65 (br d,*J* = 8.1 Hz, 1H), 7.11 (dd, *J* = 9.1, 16.0 Hz, 1H), 4.71 - 4.25 (m, 3H), 3.79 - 3.57 (m, 1H), 3.44 (dt, *J* = 3.8, 9.2 Hz, 1H), 2.75 - 2.60 (m, 1H), 2.34 - 2.20 (m, 1H).

Step 2. A solution of 3-(4-bromobenzoyl)-1-vinylpyrrolidin-2-one (2.50 g, 8.50 mmol, 1.00 *eq)* in hydrochloric acid (8.00 M, 37.5 mL, 35.3 *eq)* was stirred at 100 °C for 12 h under nitrogen atmosphere (15 *psi*)*.* The mixture was cooled to ambient temperature and extracted with ethyl acetate (50.0 mL). The aqueous layer was basified to pH = 13 with sodium hydroxide (15% aqueous solution) and extracted with dichloromethane (3 × 50.0 mL). The combined organic layers were dried over anhydrous sodium sulfate. After filtration, the filtrate was concentrated under reduced pressure to afford 5-(4-bromophenyl)-3,4-dihydro-2*H*-pyrrole (2.00 g, crude) as a yellow solid. ¹H NMR (400 MHz, CHLOROFORM-*d*) 6 = 7.71 (d, *J* = 8.5 Hz, 2H), 7.59 - 7.52 (m, 2H), 4.11 - 4.00 (m, 2H), 2.99 - 2.86 (m, 2H), 2.10 - 2.05 (m, 2H).

Step 3. To a solution of 5-(4-bromophenyl)-3,4-dihydro-2*H*-pyrrole (1.50 g, 6.69 mmol, 1.00 *eq)* in methanol (10.0 mL) and water (2.50 mL) was added sodium borohydride (506 mg, 13.3 mmol, 2.00 *eq)* at -60 °C. The mixture was stirred at 25 °C for 1 h. The mixture was quenched in 1M hydrochloric acid (10.0 mL). Then the mixture was concentrated under reduced pressure to give a residue. The residue was triturated with methylsulfinylmethane and water. Then the mixture was filtered to afford 2-(4-bromophenyl)pyrrolidine (1.30 g, 5.75 mmol, 85% yield) as a black solid. ¹H NMR (400 MHz, DMSO-*d₆*) *δ* = 7.65 - 7.58 (m, 2H), 7.57 - 7.53 (m, 2H), 4.47 (br dd, *J* = 6.9, 10.0 Hz, 1H), 3.25 - 3.20 (m, 2H), 2.35 - 2.32 (m, 1H), 2.09 - 2.04 (m, 1H), 1.98 (s, 2H). MS (ESI) m/z 227.8 [M+H]⁺

Step 4. To a solution of 2-(4-bromophenyl)pyrrolidine (490 mg, 2.17 mmol, 1.00 *eq)* in tetrahydrofuran (2.00 mL) was added di-*tert*-butyl dicarbonate (472 mg, 2.17 mmol, 497 uL, 1.00 *eq)* 4-dimethylaminopyridin (26.4 mg, 216 umol, 0.10 *eq)* and triethylamine (657 mg, 6.50 mmol, 904 uL, 3.00 *eq).* The mixture was stirred at 25 °C for 2 h. Then the mixture was stirred 60 °C for 12 h. The reaction mixture was diluted with hydrochloric acid (1.00 M, 30.0 mL) and extracted with ethyl acetate (2 × 30.0 mL). The organic phase was separated, washed with brine (10.0 mL), dried over sodium sulfate, filtered and concentrated under reduced pressure to afford tert-butyl 2-(4-bromophenyl)pyrrolidine-1-carboxylate (240 mg, 654 umol, 30% yield, 89% purity) as yellow oil. ¹H NMR (400 MHz, DMSO-*d₆*) *δ* = 7.49 (br d, *J* = 8.1 Hz, 2H), 7.13 (br d, *J* = 7.5 Hz, 2H), 4.84 - 4.61 (m, 1H), 3.56 - 3.45 (m, 2H), 2.34 - 2.19 (m, 1H), 1.86 - 1.74 (m, 2H), 1.67 (br d, *J* = 6.8 Hz, 1H), 1.42 - 1.33 (m, 5H), 1.11 (br s, 4H).

Step 5. Six reaction were carried out in parallel. To a solution of *tert*-butyl 2-(4-bromophenyl)pyrrolidine-1-carboxylate (40.0 mg, 122 umol, 1.00 *eq)* and 3-(1-oxo-5-(2-oxoimidazolidin-1-yl)isoindolin-2-yl)piperidine-2,6-dione (20.1 mg, 61.3 umol, 0.50 *eq*) in *N,N*-dimethyl formamide (1.00 mL) was added cuprous iodide (4.67 mg, 24.5 umol, 0.20 *eq),* cesium carbonate (79.9 mg, 245 umol, 2.00 *eq)* and *N,N'*-dimethylethane-1,2-diamine (10.8 mg, 122 umol, 13.2 uL, 1.00 *eq).* The mixture was stirred at 100 °C for 2 h under nitrogen atmosphere (15 *psi*). The mixture was filtered to give the filtrate and the filtrate was purified by reversed-phase HPLC (C₁₈, 80 g; condition: water/acetonitrile = 1/0 to 0/1, 0.1% formic acid). The desired fraction was collected and lyophilized to afford *tert*-butyl 2-(4-(3-(2-(2,6-dioxopiperidin-3-yl)-1-oxoisoindolin-5-yl)-2-oxoimidazolidin-1-yl)phenyl)pyrrolidine-1-carboxylate (80.0 mg, 133 umol, 18% yield, 96% purity) as a white solid. ¹H NMR (400 MHz, DMSO-*d₆*) *δ* = 10.98 (br s, 1H), 7.91 (s, 1H), 7.81 - 7.75 (m, 1H), 7.74 - 7.69 (m, 1H), 7.63 - 7.53 (m, 2H), 7.17 (br d, *J* = 8.1 Hz, 2H), 5.10 (dd, *J* = 5.1, 13.3 Hz, 1H), 4.88 - 4.65 (m, 1H), 4.51 - 4.40 (m, 1H), 4.37 - 4.26 (m, 1H), 4.07 - 3.94 (m, 4H), 3.56 - 3.43 (m, 2H), 2.98 - 2.85 (m, 1H), 2.60 (br dd, *J* = 2.3, 15.1 Hz, 1H), 2.40 - 2.32 (m, 1H), 2.05 - 1.95 (m, 1H), 1.81 (br s, 2H), 1.75 - 1.66 (m, 1H), 1.41 - 1.32 (m, 4H), 1.13 (br s, 5H).

Step 6. To a solution of *tert*-butyl 2-(4-(3-(2-(2,6-dioxopiperidin-3-yl)-1-oxoisoindolin-5-yl)-2-oxoimidazolidin-1-yl)phenyl)pyrrolidine-1-carboxylate (80.0 mg, 139 umol, 1.00 *eq)* in dioxane (3.00 mL) was added hydrochloride/dioxane (4.00 M, 3.00 mL, 86.0 *eq).* The mixture was stirred at 25 °C for 2 h. The mixture was concentrated under reduced pressure to afford 3-(1-oxo-5-(2-oxo-3-(4-(pyrrolidin-2-yl)phenyl)imidazolidin-1-yl)isoindolin-2-yl)piperidine-2,6-dione (140 mg, crude) as a yellow solid. MS (ESI) m/z 474.0 [M+H]⁺

Step 7. To a solution of 3-(1-oxo-5-(2-oxo-3-(4-(pyrrolidin-2-yl)phenyl)imidazolidin-1-yl)isoindolin-2-yl)piperidine-2,6-dione (140 mg, 295 umol, 1.00 *eq)* in methanol (5.00 mL) was added formaldehyde (26.6 mg, 886 umol, 24.4 uL, 3.00 *eq),* sodium triacetoxy borohydride (125 mg, 591 umol, 2.00 *eq)* and acetic acid (1.78 mg, 29.5 umol, 1.69 uL, 0.10 *eq).* Then the mixture was stirred at 25 °C for 12 h. The mixture was concentrated under reduced pressure to give a residue. The residue was purified by *prep-*HPLC (column: Phenomenex luna C18 150*25mm* 10um;mobile phase: [water(formic acid)- acetonitrile];B%: 1%-32%, 15min). The desired fraction was collected and lyophilized to afford 3-(5-(3-(4-(1-methylpyrrolidin-2-yl)phenyl)-2-oxoimidazolidin-1-yl)-1-oxoisoindolin-2-yl)piperidine-2,6-dione (12.47 mg, 25.32 umol, 8% yield, 99% purity) as a yellow solid.

¹H NMR (400 MHz, METHANOL-*d₄*) δ = 8.49 (s, 1H), 7.97 (s, 1H), 7.82 - 7.73 (m, 4H), 7.50 (d, *J* = 8.6 Hz, 2H), 5.33 - 5.05 (m, 2H), 4.57 - 4.43 (m, 2H), 4.13 - 4.05 (m, 4H), 3.65 (td, *J* = 5.8, 11.5 Hz, 1H), 3.15 - 3.01 (m, 1H), 3.00 - 2.89 (m, 1H), 2.88 - 2.74 (m, 1H), 2.61 (s, 3H), 2.56 - 2.41 (m, 2H), 2.30 - 2.15 (m, 4H). MS (ESI) m/z 488.1 [M+H]⁺

### Example 34. Synthesis of Compound 126

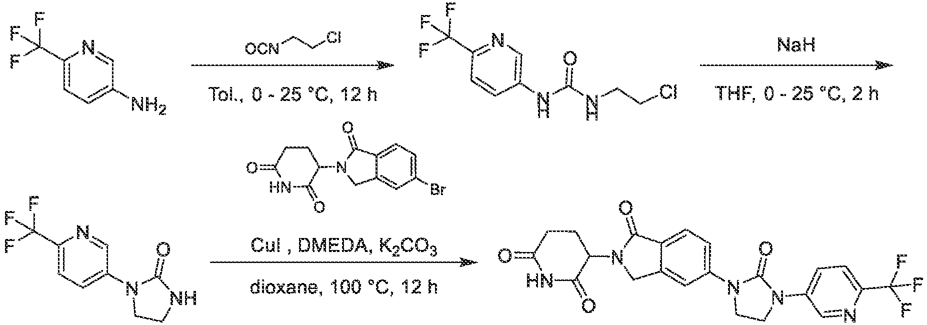

Step 1. To a solution of 6-(trifluoromethyl)pyridin-3-amine (1.00 g, 6.17 mmol, *1.00 eq)* in toluene (5.00 mL) was added 1-chloro-2-isocyanatoethane (976 mg, 9.25 mmol, 787 uL, 1.50 *eq)* at 0 °C . The mixture was stirred at 25 °C for 12 h. The mixture was filtered. The filter cake was concentrated under reduced pressure to afford 1-(2-chloroethyl)-3-(6-(trifluoromethylpyridin-3-yl)urea (700 mg, crude) as a white solid.

¹H NMR (400 MHz, DMSO-*d₆*) *δ* = 9.32 (s, 1H), 8.67 (d, *J* = 2.3 Hz, 1H), 8.15 (dd, *J* = 2.3, 8.6 Hz, 1H), 7.76 (d, *J* = 8.6 Hz, 1H), 6.72 (br t, *J* = 5.6 Hz, 1H), 3.72 - 3.65 (m, 2H), 3.45 (q, *J* = 6.0 Hz, 2H).

Step 2. To a solution of 1-(2-chloroethyl)-3-(6-(trifluoromethyl)pyridin-3-yl)urea (600 mg, 2.24 mmol, 1.00 *eq)* in tetrahydrofuran (5.00 mL) was added sodium hydride (135 mg, 3.36 mmol, 60% purity, 1.50 *eq)* at 0 °C under nitrogen atmosphere. The mixture was stirred at 25 °C for 2 h. The mixture was quenched with saturated ammonium chloride in aqueous solution (50 mL), then extracted with ethyl acetate (3 × 100 mL). The organic layers were collected and dried over anhydrous sodium sulfate, evaporated to afford 1-(6-(trifluoromethyl)pyridin-3-yl)imidazolidin-2-one (700 mg, crude) as a white solid. ¹H NMR (400 MHz, DMSO-*d₆*) *δ* = 8.91 (d, *J* = 2.1 Hz, 1H), 8.27 - 8.06 (m, 1H), 7.72 (d, *J* = 8.8 Hz, 1H), 3.79 (br d, *J* = 8.3 Hz, 2H), 3.48 - 3.38 (m, 2H).

Step 3. To a solution of 1-(6-(trifluoromethyl)pyridin-3-yl)imidazolidin-2-one (35.8 mg, 155 umol, 1.00 *eq)* and 3-(5-bromo-1-oxoisoindolin-2-yl)piperidine-2,6-dione (50.0 mg, 155 umol, 1.00 *eq)* in dioxane (1.00 mL) was added potassium carbonate (64.2 mg, 464 umol, 3.00 eq), dimethylethylenediamine (1.36 mg, 15.5 umol, 1.67 uL, 0.100 *eq)* and cuprous iodide (2.95 mg, 15.5 umol, 0.100 *eq)* under nitrogen atmosphere. The mixture was stirred at 100 °C for 12 h. The mixture was filtered. The filtrate was purified by *prep*-HPLC (column: Phenomenex Luna C18 200*40mm*10um; mobile phase: [water(FA)-ACN];B%: 28%-58%,10min). The desired fraction was collected and the aqueous solution was lyophilized to afford 3-(1-oxo-5-(2-oxo-3-(6-(trifluoromethyl)pyridin-3-yl)imidazolidin-1-yl)isoindolin-2-yl)piperidine-2,6-dione (24.3 mg, 54.9 umol, 8% yield) as an off-white solid. ¹H NMR (400 MHz, DMSO-*d₆*) *δ* = 10.99 (s, 1H), 9.07 (d, *J* = 2.5 Hz, 1H), 8.3 1 (dd, *J* = 2.4, 8.6 Hz, 1H), 7.96 - 7.91 (m, 2H), 7.84 - 7.79 (m, 1H), 7.77 - 7.74 (m, 1H), 5.11 (dd, *J* = 5.0, 13.3 Hz, 1H), 4.51 - 4.42 (m, 1H), 4.39 - 4.30 (m, 1H), 4.13 (s, 4H), 2.97 - 2.87 (m, 1H), 2.58 (br d, *J* = 2.4 Hz, 1H), 2.42 (br d, *J* = 4.9 Hz, 1H), 2.04 - 1.98 (m, 1H). MS (ESI) m/z 474.0 [M+H]⁺

### Example 35. Synthesis of Compound 124

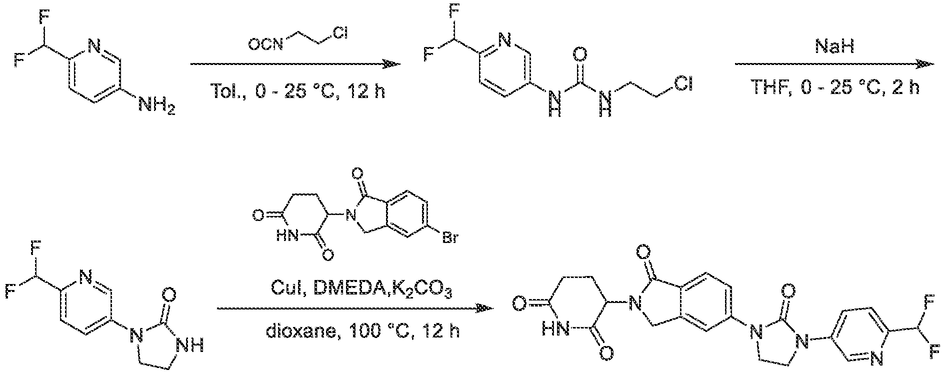

Step 1. To a solution of 6-(difluoromethyl)pyridin-3-amine (500 mg, 3.47 mmol, *1.00 eq)* in toluene (10.0 mL) was added 1-chloro-2-isocyanato-ethane (549 mg, 5.20 mmol, *1.50 eq)* at 0 °C. The mixture was stirred at 25 °C for 12 h. The mixture was filtered to give a filter cake, then it was dried under reduced pressure to give 1-(2-chloroethyl)-3-(6-(difluoromethyl)pyridin-3-yl)urea (700 mg, 2.80 mmol, 81% yield) as a pink solid.

¹H NMR (400 MHz, DMSO-*d*₆) *δ* = 9.15 (s, 1H), 8.62 (d, *J* = 2.5 Hz, 1H), 8.06 (dd, *J* = 2.4, 8.6 Hz, 1H), 7.57 (d, *J* = 8.5 Hz, 1H), 6.85 (t, *J* = 55.3 Hz, 1H), 6.69 - 6.60 (m, 1H), 3.70 - 3.65 (m, 2H), 3.48 - 3.41 (m, 2H). MS (ESI) m/z 250.2 [M+H]⁺

Step 2. To a solution of 1-(2-chloroethyl)-3-(6-(difluoromethyl)pyridin-3-yl)urea (700 mg, 2.80 mmol, *1.00 eq)* in tetrahydrofuran (10.0 mL) was added sodium hydride (168 mg, 4.21 mmol, 60% purity, 1.50 *eq)* at 0 °C under nitrogen atmosphere. The mixture was stirred at 25 °C for 2 h. The reaction mixture was quenched with methanol (10.0 mL) and concentrated under reduced pressure to give a crude product. The crude product was triturated with dichloromethane (3.00 mL) at 25 °C for 10 min and filtered to give 1-(6-(difluoromethyl)pyridin-3-yl)imidazolidin-2-one (620 mg, crude) as a yellow solid.

¹H NMR (400 MHz, DMSO-*d*₆) *δ* = 8.83 (d, *J* = 2.5 Hz, 1H), 8.14 (dd, *J* = 2.6, 8.7 Hz, 1H), 7.61 (d, *J* = 8.8 Hz, 1H), 7.05 - 6.69 (t, *J* = 55.2 Hz, 1H), 3.88 (dd, *J =* 6.9, 8.9 Hz, 2H), 3.46 - 3.45 (m, 2H). MS (ESI) m/z 214.3 [M+H]⁺

Step 3. To a solution of 1-(6-(difluoromethyl)pyridin-3-yl)imidazolidin-2-one (40.0 mg, 187 umol, 1.00 *eq),* 3-(5-bromo-1-oxo-isoindolin-2-yl)piperidine-2,6-dione (72.7 mg, 225 umol, 1.20 *eq),* potassium carbonate (25.9 mg, 187 umol, 1.00 *eq)* in 1,4-dioxane (2.00 mL) was added copper(I) iodide (3.57 mg, 18.7 umol, 0.100 *eq)* and *N,N*'-dimethylethylenediamine (3.31 mg, 37.5 umol, 4.04 uL, 0.200 *eq)* under nitrogen atmosphere. The mixture was stirred at 100 °C for 12 h. The pH of the mixture was adjusted to 5-6 with formic acid and filtered, then the filtrate was concentrated under reduced pressure to get a residue. The residue was purified by *Prep*-HPLC (column: Phenomenex luna C18 150*25mm* 10um; mobile phase: [water (formic acid) - acetonitrile]; B%: 23%-43%, 9 min) to get a crude product. The crude product was dissolved with formic acid and recrystallized with water, then filtered and lyophilized to afford 3-(5-(3-(6-(difluoromethyl)pyridin-3-yl)-2-oxoimidazolidin-1-yl)-1-oxoisoindolin-2-yl)piperidine-2,6-dione (12.6 mg, 26.8 umol, 3% yield, 97% purity) as a white solid. ¹H NMR (400 MHz, DMSO-*d*₆) *δ* = 10.99 (s, 1H), 8.99 (d, *J* = 1.6 Hz, 1H), 8.30 - 8.17 (m, 1H), 7.94 (s, 1H), 7.84 - 7.78 (m, 1H), 7.77 - 7.70 (m, 2H), 7.10 - 6.78 (t, *J* = 55.2 Hz, 1H), 5.11 (br dd, *J* = 5.1, 13.2 Hz, 1H), 4.51 - 4.43 (m, 1H), 4.38 - 4.30 (m, 1H), 4.11 (br s, 4H), 3.00 - 2.84 (m, 1H), 2.61 (br d, *J* = 17.1 Hz, 1H), 2.43 - 2.34 (m, 1H), 2.06 - 1.95 (m, 1H). MS (ESI) m/z 456.3 [M+H]⁺

### Example 36. Synthesis of Compound 130

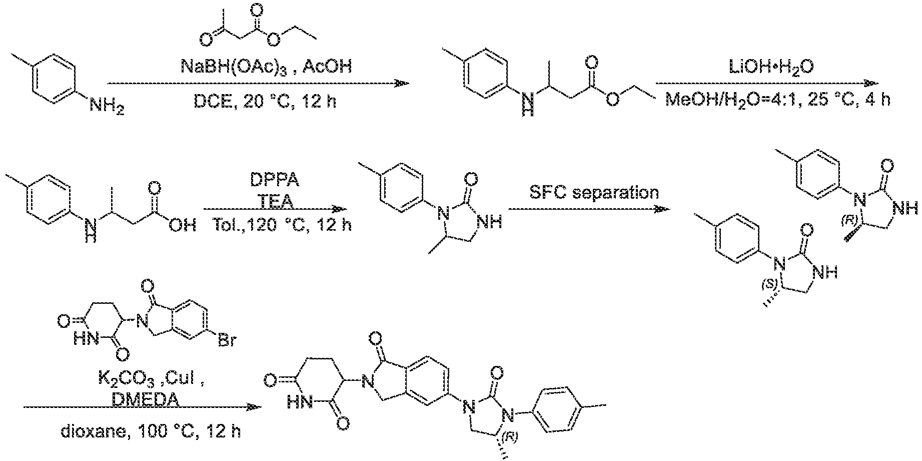

Step 1. To a solution of ethyl 3-oxobutanoate (7.29 g, 60.0 mmol, 7.07 mL, 1.20 *eq)* in 1, 2-dichloroethane (200 mL) was added acetic acid (280 mg, 4.67 mmol, 267 uL, *0.100 eq).* The mixture was stirred at 20 °C for 10 min. The *p*-toluidine (5.00 g, 46.7 mmol, 5.14 mL, 1.00 *eq)* was added into the mixture and the mixture was stirred at 20 °C for 5 min. At last, sodium triacetoxyhydroborate (29.7 g, 140mmol, 3.00 *eq)* was added into the mixture and the mixture was stirred at 20 °C for 12 h. The reaction mixture was diluted with water (200 mL) and extracted with ethyl acetate (2 × 300 mL). The organic phase was separated, washed with brine (50.0 mL), dried over sodium sulfate, filtered and concentrated under reduced pressure to give a residue. Then residue was purified by column chromatography (SiO₂, petroleum ether/ethyl acetate = 1/0 to 8/1) to give ethyl 3-(p-tolylamino)butanoate (10.0 g, 45.2 mmol, 48 % yield) as yellow oil. ¹H NMR (400 MHz, DMSO-*d*₆) *δ* = 6.89 (d,*J* = 8.1 Hz, 2H), 6.51 - 6.45 (m, 2H), 5.14 (d, *J* = 9.3 Hz, 1H), 4.05 - 4.00 (m, 2H), 3.82 - 3.71 (m, 1H), 2.58 - 2.52 (m, 1H), 2.29 (dd, *J* = 7.4, 14.8 Hz, 1H), 2.14 (s, 3H), 1.19 - 1.16 (m, 3H), 1.15 - 1.12 (m, 3H). MS (ESI) m/z 222.1 [M+H]⁺

Step 2. To a solution of ethyl 3-(*p*-tolylamino)butanoate (5.00 g, 22.6 mmol, 1.00 *eq)* in methanol (28.0 mL) and water (7.00 mL) was added lithium hydroxide monohydrate (3.32 g, 79.1 mmol, 3.50 *eq).* The mixture was stirred at 25 °C for 4 h. The reaction mixture was concentrated under reduced pressure and purified by reversed-phase HPLC (column: spherical C18, 20-45 um, 100Å, SW 330, mobile phase: [water (0.1%Formic Acid)-ACN]) to give 3-(*p*-tolylamino)butanoic acid (10.0 g, crude) as a white solid. MS (ESI) m/z 194.3 [M+H]⁺

Step 3. To a solution of 3-(*p*-tolylamino)butanoic acid (3.00 g, 15.5 mmol, 1.00 *eq),* diphenyl phosphoryl azide (12.8 g, 46.6 mmol, 10.1 mL, 3.00 eq) in toluene (50.0 mL) was added triethylamine (4.71 g, 46.6mmol, 6.48 mL, 3.00 *eq).* The mixture was stirred at 120 °C for 12 h. The mixture was filtered. The filtrate was concentrated under reduced pressure. The residue was purified by reversed-phase HPLC (column: spherical C18, 20-45 um, 100Å, SW 330, mobile phase: [water(0.1% NH₃•H₂O condition)-ACN]) and lyophilized to give 5-methyl-1-(*p*-tolyl)imidazolidin-2-one (0.600 g, 3.15 mmol, 20 % yield) as a white solid. ¹H NMR (400 MHz, DMSO-*d*₆) *δ* = 7.3 1 (d, *J* = 8.5 Hz, 2H), 7.11 (d, *J* = 8.3 Hz, 2H), 6.75 (s, 1H), 4.40 (quind, *J* = 6.0, 8.6 Hz, 1H), 3.54 (dt, *J* = 0.9, 8.7 Hz, 1H), 2.96 (ddd, *J* = 0.9, 5.7, 8.7 Hz, 1H), 2.25 (s, 3H), 1.19 - 1.09 (m, 3H).

Step 4. The product was further separated by SFC (column: DAICEL CHIRALPAK AS(250mm*30mm,10um); mobile phase: [0.1%NH₃H₂O EtOH];B%: 30%-30%,C6.0;108min) . The desired fraction was collected and concentrated reduced pressure to afford (5*R*)-5-methyl-1-(*p*-tolyl)imidazolidin-2-one (0.200 g, 1.05 mmol, 33% yield) as a white solid and (5*S*)-5-methyl-1-(*p*-tolyl)imidazolidin-2-one (0.200 g, 1.05 mmol, 33% yield) as a white solid.
¹H NMR (400 MHz, DMSO-*d*₆) *δ* = 7.31 (d, *J* = 8.5 Hz, 2H), 7.11 (d, *J* = 8.3 Hz, 2H), 6.75 (s, 1H), 4.40 (quind, *J* = 6.0, 8.5 Hz, 1H), 3.54 (dt, *J* = 0.6, 8.6 Hz, 1H), 3.01 - 2.88 (m, 1H), 2.25 (s, 3H), 1.15 (d, *J* = 6.1 Hz, 3H). MS (ESI) m/z 191.1 [M+H]⁺
¹H NMR (400 MHz, DMSO-*d*₆) *δ* = 7.32 (d, *J* = 8.4 Hz, 2H), 7.11 (d, *J* = 8.4 Hz, 2H), 6.75 (br s, 1H), 4.47 - 4.32 (m, 1H), 3.54 (t, *J* = 8.7 Hz, 1H), 2.96 (dd, *J* = 5.8, 8.6 Hz, 1H), 2.25 (s, 3H), 1.15 (d, *J* = 6.0 Hz, 3H). MS (ESI) m/z 191.2 [M+H]⁺

Step 5. To a solution of (*R*)-5-methyl-1-(*p*-tolyl)imidazolidin-2-one (50.0 mg, 263 umol, 1.00 *eq)* in dioxane (1.50 mL) was added 3-(5-bromo-1-oxo-isoindolin-2-yl)piperidine-2,6-dione (84.9 mg, 263 umol, 1.00 *eq*), *N,N*-dimethylethylenediamine (23.2 mg, 263 umol, 28.3 uL, 1.00 *eq),* copper iodide (50.1 mg, 263 umol, 1.00 *eq)* and potassium carbonate (109 mg, 788 umol, 3.00 *eq).* The mixture was stirred under nitrogen atmosphere at 100 °C for 12 h. The mixture was filtered. The filtrate was purified by *Prep*-HPLC (column: Phenomenex luna C18 150*25mm* 10um;mobile phase: [water(FA)-ACN];B%: 34%-64%,58min) and lyophilized to give 3-(5-((*R*)-4-methyl-2-oxo-3-(*p*-tolyl) imidazolidin-1-yl)-1-oxoisoindolin-2-yl) piperidine-2,6-dione (14.04 mg, 32.46 umol, 28.08% yield) as a yellow solid.

¹H NMR (400 MHz, DMSO-*d*₆) *δ* = 10.98 (s, 1H), 7.92 - 7.83 (m, 1H), 7.79 - 7.73 (m, 1H), 7.72 - 7.67 (m, 1H), 7.39 (d, *J* = 8.5 Hz, 2H), 7.21 (br d, *J* = 8.3 Hz, 2H), 5.10 (dd, *J* = 4.8, 13.1 Hz, 1H), 4.58 (td, *J* = 5.5, 8.5 Hz, 1H), 4.49 - 4.39 (m, 1H), 4.36 - 4.28 (m, 1H), 4.27 - 4.18 (m, 1H), 3.69 - 3.59 (m, 1H), 2.98 - 2.85 (m, 1H), 2.70 - 2.59 (m, 1H), 2.40 (br dd, *J* = 4.2, 13.3 Hz, 1H), 2.30 (s, 3H), 2.05 - 1.95 (m, 1H), 1.24 (d, *J* = 6.1 Hz, 3H). MS (ESI) m/z 433.4 [M+H]⁺

### Example 37. Synthesis of Compound 151

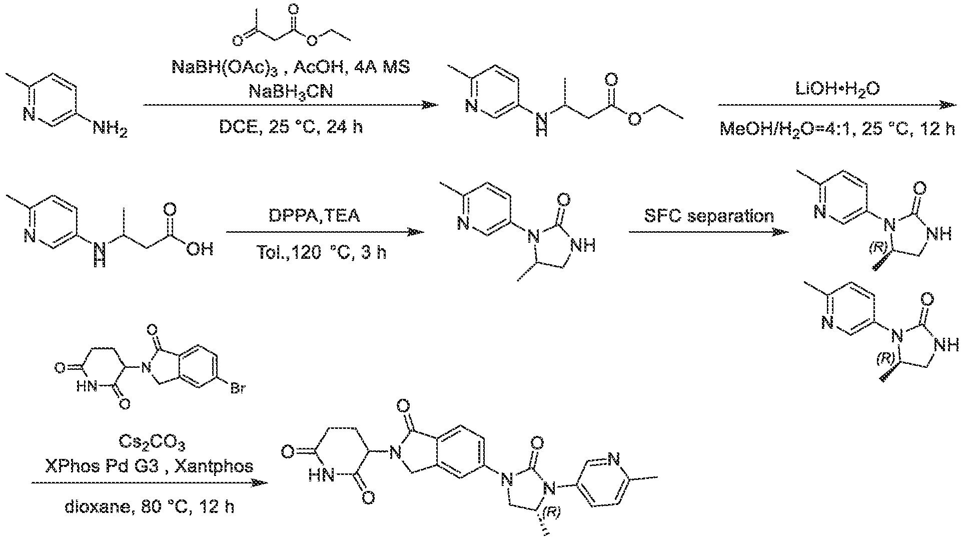

Step 1. To a solution of 6-methylpyridin-3-amine (20.0 g, 184 mmol, 1.00 *eq),* 4A MS (10.0 g) in 1, 2-dichloroethane (600 mL) was added ethyl 3-oxobutanoate (72.2 g, 554 mmol, 70.1 mL, 3.00 *eq)* and acetic acid (1.11 g, 18.4 mmol, 1.06 mL, 0.100 *eq*) *to* adjust pH=5. The mixture was stirred at 25 °C for 12 h. At last, sodium triacetoxy borohydride (117 g, 554 mmol, 3.00 *eq),* sodium cyanoborohydride (11.6 g, 184 mmol, 1.00 eq) was added into the mixture and the mixture was stirred at 25 °C for 12 h under nitrogen. The mixture was filtered to give a filter liquor, then was concentrated under reduced pressure to give a residue which was purified by column chromatography (SiO₂, Petroleum ether/Ethyl acetate=1/0 to 0/1) to give ethyl 3-((6-methylpyridin-3-yl)amino)butanoate (74.0 g, crude) as a black solid. MS (ESI) m/z 223.2 [M+H]⁺

Step 2. To a solution of ethyl 3-((6-methylpyridin-3-yl)amino)butanoate (85.0 g, 152 mmol, 40% purity, 1.00 *eq)* in water (62.5 mL), methanol (250 mL) was added lithium hydroxide hydrate (22.4 g, 535 mmol, 3.50 *eq).* The mixture was stirred at 25 °C for 12 h. The reaction mixture was concentrated under reduced pressure to give a residue which was purified by reversed-phase HPLC (column: spherical C18, 20-45 um, 100Å, SW 330, mobile phase: [water (0.1%Formic Acid)- acetonitrile) to give 3-((6-methylpyridin-3-yl)amino)butanoic acid (73.0 g, crude) as a colorless oil. ¹H NMR (400 MHz, DMSO-*d*₆) *δ* = 8.37 (s, 1H), 7.80 (d, *J* = 2.4 Hz, 1H), 6.92 - 6.87 (m, 1H), 6.81 (dd, *J* = 2.6, 8.3 Hz, 1H), 3.67 (br dd, *J* = 6.2, 12.1 Hz, 1H), 2.26 (s, 3H), 2.10 - 1.94 (m, 2H), 1.10 (d, *J* = 6.1 Hz, 3H).

Step 3. To a solution of 3-((6-methylpyridin-3-yl)amino)butanoic acid (30.0 g, 154 mmol, 1.00 *eq),* diphenylphosphorylazide (127 g, 463 mmol, 100 mL, 3.00 *eq)* in toluene (200 mL) was added triethylamine (46.8 g, 463 mmol, 64.4 mL, 3.00 *eq).* The mixture was stirred at 120 °C for 3 h. The reaction mixture was concentrated under reduced pressure to give a residue, which was diluted with saturated sodium bicarbonate (200 mL) and extract with ethyl acetate (2 × 300 mL). The organic phase was separated, washed with brine (100 mL), dried over sodium sulfate, filtered and concentrated under reduced pressure to give a residue which was purified by column chromatography (SiO₂, Petroleum ether/Ethyl acetate=1/0 to 0/1) followed by reversed-phase HPLC (column: spherical C18, 20-45 um, 100Å, SW 330, mobile phase: [water(0.1%NH3·H2O)- acetonitrile) to give 5-methyl-1-(6-methylpyridin-3-yl)imidazolidin-2-one (2.00 g, 10.4 mmol, 3% yield) as a white solid. ¹H NMR (400 MHz, DMSO-*d*₆) *δ* = 8.53 (d, *J* = 2.5 Hz, 1H), 7.77 (dd, *J* = 2.7, 8.4 Hz, 1H), 7.20 (d, *J* = 8.4 Hz, 1H), 6.97 (s, 1H), 4.47 (quind, *J* = 6.0, 8.6 Hz, 1H), 3.58 (t, *J* = 8.8 Hz, 1H), 3.00 (dd, *J* = 5.7, 8.8 Hz, 1H), 2.41 (s, 3H), 1.17 (d, *J* = 6.1 Hz, 3H). MS (ESI) m/z 192.2 [M+H]⁺

Step 4. The 5-methyl-1-(6-methylpyridin-3-yl)imidazolidin-2-one (1.04 g, 5.23 mmol, 96% purity, 1.00 eq) was separated by SFC (column: Phenomenex-Cellulose-2 (250mm*30mm,10um);mobile phase: [Neu-MeOH];B%: 20%-20%,A4.3;130min) to afford (*R*)-5-methyl-1-(6-methylpyridin-3-yl)imidazolidin-2-one (400 mg, 2.09 mmol, 40% yield) as a white solid and (S)-5-methyl-1-(6-methylpyridin-3-yl)imidazolidin-2-one (400 mg, 2.09 mmol, 40% yield). MS (ESI) m/z 192.1 [M+H]⁺

Step 5. To a solution of 3-(5-bromo-1-oxo-isoindolin-2-yl)piperidine-2,6-dione (50.0 mg, 154 umol, 1.00 *eq),* (R)-5-methyl-1-(6-methylpyridin-3-yl)imidazolidin-2-one (29.5 mg, 154 umol, 1.00 *eq),* cesium carbonate (100 mg, 309 umol, 2.00 *eq)* in 1,4-dioxane (2.00 mL) was added methanesulfonato[9,9-dimethyl-4,5-Bis(diphenylphosphino)xanthene][2-amino-1,1-biphenyl]palladium(II)dichloromethaneadduct (13.1 mg, 15.4 umol, 0.100 *eq),* 4,5-bis(diphenylphosphino)-9,9-dimethylxanthene (17.9 mg, 30.9 umol, 0.200 *eq*) under nitrogen. The mixture was stirred at 80 °C for 12 h. The mixture was filtered to give a filter liquor, then was concentrated under reduced pressure to give a residue which was purified by reversed-phase HPLC (column: spherical C18, 20-45 um, 100Å, SW 80, mobile phase: [water(0.1%Formic Acid)- acetonitrile) to give 3-(5-((*R*)-4-methyl-3-(6-methylpyridin-3-yl)-2-oxoimidazolidin-1-yl)-1-oxoisoindolin-2-yl)piperidine-2,6-dione (42.4 mg, 97.9 umol, 15% yield) as a white solid. ¹H NMR (400 MHz, DMSO-*d*₆) *δ* = 10.98 (s, 1H), 8.62 (d, *J =* 2.5 Hz, 1H), 7.88 (br s, 1H), 7.82 (dd, *J = 2.*6, 8.4 Hz, 1H), 7.79 - 7.74 (m, 1H), 7.73 - 7.69 (m, 1H), 7.30 (d, *J* = 8.5 Hz, 1H), 5.16 - 5.05 (m, 1H), 4.69 - 4.60 (m, 1H), 4.49 - 4.42 (m, 1H), 4.35 - 4.29 (m, 1H), 4.29 - 4.22 (m, 1H), 3.71 - 3.63 (m, 1H), 2.97 - 2.87 (m, 1H), 2.62 - 2.57 (m, 1H), 2.46 (s, 3H), 2.42 - 2.37 (m, 1H), 2.05 - 1.96 (m, 1H), 1.27 (d, *J =* 6.0 Hz, 3H). MS (ESI) m/z 434.2 [M+H]⁺

### Example 38. Synthesis of Compound 157

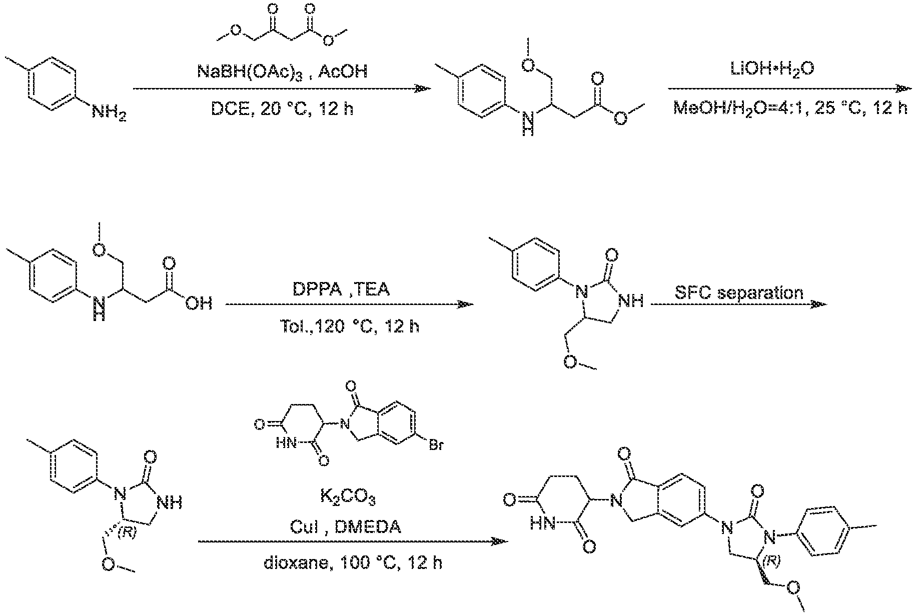

Step 1. To a solution of 4-methylaniline (10.0 g, 93.3 mmol, 10.2 mL, 1.00 *eq*) in 1, 2-dichloroethane (100 mL) was added methyl 4-methoxy-3-oxo-butanoate (13.6 g, 93.3 mmol, 12.0 mL, 1.00 *eq*), acetic acid (560 mg, 9.33 mmol, 533 uL, 0.100 *eq*) and sodium triacetoxy borohydride (39.5 g, 186 mmol, 2.00 *eq*) at 0 °C. Then the mixture was stirred at 20 °C for 12 h. The mixture was diluted with **water (100** mL) and extracted with ethyl acetate (3 × 15**0** mL). The combined organic layers were washed with brine (3 × 50 ml), dried over sodium sulfate, filtered and concentrated under reduced pressure to give a residue. The residue was purified by column chromatography (SiO2, **Petroleum ether/Ethyl acetate=50/1** to 20/**1**) and concentrated in vacuum to afford methyl 4-methoxy-3-(*p-*tolylamino)butanoate (11.0 g, 43.1 mmol, 46% yield, 93% purity) as yellow oil. ¹H NMR. (400 MHz, DMSO-*d*₆) *δ* = 6.89 (d, *J =* 8.3 Hz, 2H), 6.51 (d, *J =* 8.4 Hz, 2H), 5.20 (d, *J =* 9.4 Hz, 1H), 3.95 - 3.82 (m, 1H), 3.57 (s, 3H), 3.41 - 3.36 (m, 1H), 3.30 - 3.27 (m, 1H), 3.25 (s, 3H), 2.59 - 2.52 (m, 1H), 2.48 - 2.40 (m, 1H), 2.14 (s, 3H).

Step 2. To a solution of methyl 4-methoxy-3-(4-methylanilino)butanoate (11.0 g, 46.3 mmol, 1.00 *eq*) in methanol (8.80 mL) and water (2.20 mL) was added lithium hydroxide (5.84 g, 139 mmol, 3.00 *eq*). Then the mixture was stirred at 25 °C for 12 h. The reaction mixture was concentrated in vacuum. The residue was diluted with water (200 mL) and extracted with ethyl acetate (3 × 100 mL). The aqueous phase was adjust pH to 5-6 by hydrochloric acid (1M, 100 mL) and extracted with ethyl acetate (3 × 100 mL). The combined organic layers were washed with brine (3 × 20 mL), dried over sodium sulfate, filtered and concentrated under reduced pressure to afford 4-methoxy-3-(*p-*tolylamino)butanoic acid (10.0 g, crude) as yellow oil. ¹H NMR (400 MHz, DMSO-*d*₆) *δ* = 6.85 (d, *J* = 8.1 Hz, 2H), 6.47 (d, *J =* 8.4 Hz, 2H), 3.71 - 3.67 (m, 1H), 3.37 - 3.33 (m, 2H), 3.23 (s, 3H), 2.20 - 2.02 (m, 5H), 1.66 (s, 2H).

Step 3. To a solution of 4-methoxy-3-(*p*-tolylamino)butanoic acid (5.00 g, 22.3 mmol, 1.00 *eq*) in toluene (80.0 mL) was added diphenyl phosphoryl azide (18.4 g, 67.1 mmol, 14.5 mL, 3.00 *eq*) and triethylamine (3.40 g, 33.5 mmol, 4.68 mL, 1.50 *eq*). Then the reaction mixture was stirred at 120 °C for 12 h under nitrogen atmosphere. The reaction mixture was filtered. The filtrate was concentrated in vacuum. The residue was added *N,N-*dimethylformamide (30 mL) and filtered. The filtrate was purified by reversed phase column chromatography (C18, 330 g; condition: water/acetonitrile = 1/0 to 0/1, 0.1% formic acid) and lyophilized to afford 5-(methoxymethyl)-1-(*p*-tolyl)imidazolidin-2-one (1.50 g, 6.40 mmol, 28% yield, 94% purity) as a yellow solid. ¹H NMR (400 MHz, DMSO-*d*₆) *δ* = 7.35 (d, *J* = 8.5 Hz, 2H), 7.12 (d, *J =* 8.3 Hz, 2H), 6.79 (s, 1H), 4.50 (qd, *J =* 4.7, 9.3 Hz, 1H), 3.50 (t, *J* = 9.2 Hz, 1H), 3.36 (d, *J* = 4.8 Hz, 2H), 3.26 - 3.22 (m, 1H), 3.21 (s, 3H), 2.26 (s, 3H). MS (ESI) m/z 221.2 [M+H]⁺

Step 4. The 5-(methoxymethyl)-1-(p-tolyl)imidazolidin-2-one (1.10 g, 4.99 mmol, 1.00 *eq*) was separated by SFC (column: DAICEL CHIRALPAK AD(250mm*30mm,10um);mobile phase: [0.1%NH₃H₂O IPA];B%: 25%-25%,A3.7;55min) and concentrated in vacuum to afford (*R*)-5-(methoxymethyl)-1-(p-tolyl)imidazolidin-2-one (530 mg, 2.38 mmol, 47% yield, 99% purity) as a white solid and (*S*)-5-(methoxymethyl)-1-(p-tolyl)imidazolidin-2-one (530 mg, 2.38 mmol, 47% yield, 99% purity) as a white solid.
¹H NMR (400 MHz, DMSO-*d*₆) *δ* = 7.35 (d, *J* = 8.4 Hz, 2H), 7.11 (d, *J* = 8.3 Hz, 2H), 6.78 (s, 1H), 4.49 (qd, *J* = 4.6, 9.2 Hz, 1H), 3.50 (t, *J =* 9.0 Hz, 1H), 3.35 (d, *J =* 4.6 Hz, 2H), 3.25 - 3.21 (m, 1H), 3.21 - 3.18 (m, 3H), 2.25 (s, 3H). MS (ESI) m/z. 221.2 [M+H]⁺
¹H NMR (400 MHz, DMSO-*d*₆) *δ* = 7.35 (d, *J* = 8.4 Hz, 2H), 7.11 (d, *J* = 8.4 Hz, 2H), 6.78 (s, 1H), 4.49 (qd, *J* = 4.7, 9.2 Hz, 1H), 3.50 (t, *J* = 9.1 Hz, 1H), 3.35 (d, *J* = 4.6 Hz, 2H), 3.26 - 3.21 (m, 1H), 3.20 (s, 3H), 2.25 (s, 3H). MS (ESI) m/z. 221.2 [M+H]⁺

Step 5. To a solution of (*R*)-5-(methoxymethyl)-1-(p-tolyl)imidazolidin-2-one (34.0 mg, 154 umol, 1.00 *eq*) and 3-(5-bromo-1-oxo-isoindolin-2-yl)piperidine-2,6-dione (75.0 mg, 232 umol, 1.50 *eq*) in dioxane (1.00 mL) was added potassium carbonate (64.1 mg, 464 umol, 3.00 *eq*), copper iodide (2.95 mg, 15.4 umol, 0.100 *eq*) and *N¹*,*N²*-dimethylethane-1,2-diamine (2.73 mg, 30.9 umol, 3.33 uL, 0.200 *eq*). Then the mixture was stirred at 100 °C for 12 h under nitrogen atmosphere. The reaction mixture was adjust pH to 5-6 by formic acid (0.3 mL) and concentrated in vacuum. The residue was purified by reversed phase column chromatography (C18, 80 g; condition: water/acetonitrile = 1/0 to 0/1, 0.1% formic acid) and lyophilized. The residue was purified by reversed phase column chromatography (C18, 40 g; condition: water/acetonitrile = 1/0 to 0/1, 0.1% formic acid) and lyophilized to afford 3-(5-((*R*)-4-(methoxymethyl)-2-oxo-3-(p-tolyl)imidazolidin-1-yl)-1-oxoisoindolin-2-yl)piperidine-2,6-dione (18.07 mg, 38.7 umol, 5% yield, 99% purity) as a white solid . ¹H NMR (400 MHz, DMSO-*d*₆) *δ =* 11.07 - 10.91 (m, 1H), 7.90 (br s, 1H), 7.84 - 7.77 (m, 1H), 7.70 (d, *J* = 8.5 Hz, 1H), 7.43 (d, *J* = 8.0 Hz, 2H), 7.22 (d, *J =* 8.1 Hz, 2H), 5.10 (dd, *J* = 4.8, 13.3 Hz, 1H), 4.67 (br dd, *J =* 4.1, 8.6 Hz, 1H), 4.50 - 4.41 (m, 1H), 4.36 - 4.27 (m, 4.18 (br t, *J =* 9.3 Hz, 1H), 3.89 - 3.80 (m, 1H), 3.44 (br d, *J =* 3.1 Hz, 2H), 3.21 (s, 3H), 2.97 - 2.87 (m, 1H), 2.61 (br d, *J =* 17.6 Hz, 1H), 2.40 (br dd, *J* = 4.3, 13.1 Hz, 1H), 2.31 (s, 3H), 2.05 - 1.97 (m, 1H). MS (ESI) m/z 463.1 [M+H]⁺

### Example 39. Synthesis of Compound 143

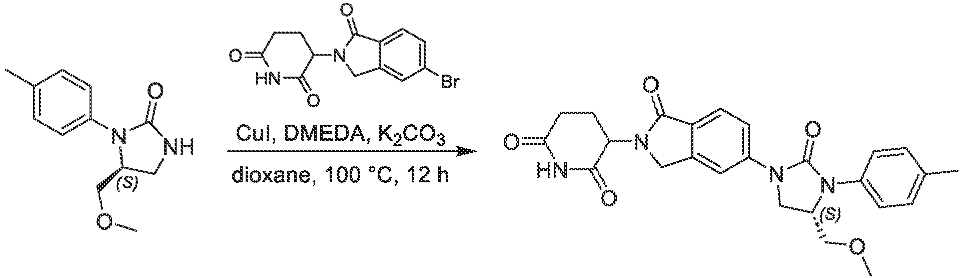

Step 1. To a mixture of (*S*)-5-(methoxymethyl)-1-(p-tolyl)imidazolidin-2-one (100 mg, 454 umol, 1.00 *eq*) and 3-(5-bromo-1-oxoisoindolin-2-yl)piperidine-2,6-dione (220 mg, 681 umol, 1.50 *eq*) in dioxane (1.00 mL) was added potassium carbonate (188 mg, 1.36 mmol, 3.00 *eq*), *N*,*N*'-dimethylethylenediamine (4.00 mg, 45.4 umol, 4.89 uL, 0.100 *eq*) and cuprous iodide (8.65 mg, 45.4 umol, 0.100 *eq*) under nitrogen. The mixture was stirred at 100 °C for 12 h. The mixture was concentrated in vacuum. The residue was added dimethyformamide (4.00 mL) and filterted. The filtrate was purified by *prep*-HPLC (column: Phenomenex luna C18 150*25mm* 10um;mobile phase: [water(FA)-ACN];B%: 30%-60%, 10min) and lyophilized to give 3-(5-((*S*)-4-(methoxymethyl)-2-oxo-3-(p-tolyl)imidazolidin-1-yl)-1-oxcisoindolin-2-yl)piperidine-2,6-dione (13.45 mg, 28.8 umol, 3% yield, 99% purity) as a white solid.

¹H NMR (400 MHz, DMSO-*d*₆) *δ* = 10.98 (br s, 1H), 7.89 (br s, 1H), 7.79 (br d, *J* = 3.6 Hz, 1H), 7.69 (d, *J* = 8.5 Hz, 1H), 7.42 (d, *J* = 8.3 Hz, 2H), 7.21 (d, *J* = 8.1 Hz, 2H), 5.10 (dd, *J =* 5.1, 13.1 Hz, 1H), 4.66 (br dd, *J =* 4.4, 8.9 Hz, 1H), 4.48 - 4.39 (m, 1H), 4.35 - 4.26 (m, 1H), 4.17 (br t, *J =* 9.4 Hz, 1H), 3.89 - 3.80 (m, 1H), 3.43 (br d, *J* = 3.4 Hz, 2H), 3.20 (s, 3H), 2.96 - 2.86 (m, 1H), 2.60 (br d, *J* = 16.8 Hz, 1H), 2.44 - 2.36 (m, 1H), 2.30 (s, 3H), 2.04 - 1.95 (m, 1H). MS (ESI) m/z 463.3 [M+H]⁺

### Example 40. Synthesis of Compound 141

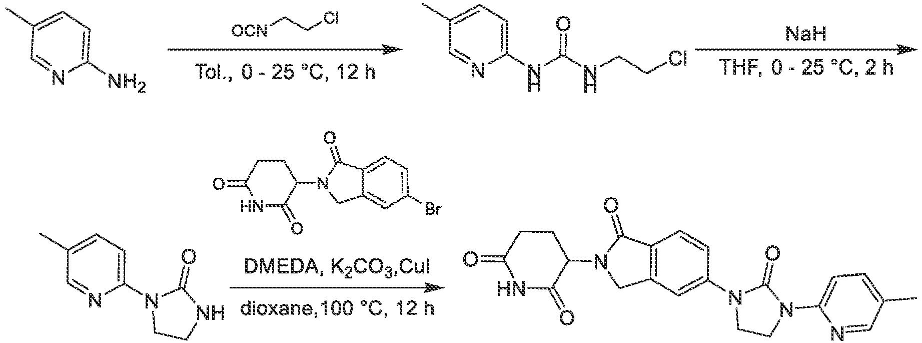

Step 1. To a mixture of 5-methylpyridin-2-amine (5.00 g, 46.2 mmol, 1.00 *eq*) in toluene (30.0 mL) was added 1-chloro-2-isocyanato-ethane (5.85 g, 55.5 mmol, 4.72 mL, 1.20 *eq*) at 0 °C. The mixture was stirred at 25 °C for 2 h. The mixture was filterted. The filter cake was concentrated in vacuum to give 1-(2-chloroethyl)-3-(5-methylpyridin-2-yl)urea (8.40 g, 39.3 mmol, 85% yield) as a white solid. ¹H NMR (400 MHz, DMSO-*d*₆) *δ* = 9.23 (s, 1H), 8.48 (br s, 1H), 8.00 (d, *J* = 1.4 Hz, 1H), 7.50 (dd, *J* = 2.1, 8.4 Hz, 1H), 7.23 (d, *J* = 8.4 Hz, 1H), 3.72 - 3.65 (m, 2H), 3.50 (q, *J =* 6.0 Hz, 2H), 2.18 (s, 3H).

Step 2. To a mixture of 1-(2-chloroethyl)-3-(5-methylpyridin-2-yl)urea (4.00 g, 18.7 mmol, 1.00 *eq*) in tetrahydrofuran (40.0 mL) was added sodium hydride (1.12 g, 28.1 mmol, 60% purity, 1.50 *eq*) at 0 °C. The mixture was stirred at 25 °C for 2 h under nitrogen. The reaction mixture was was quenched with saturated ammonium chloride aquenous (100 mL) and filterted. The filter cake was concentrated in vacuum to give 1-(5-methylpyridin-2-yl)imidazolidin-2-one 3.60 g, crude) as a white solid. ¹H NMR (400 MHz, DMSO-*d*₆) *δ* = 8.12 - 8.01 (m, 2H), 7.50 (dd, *J* = 2.1, 8.6 Hz, 1H), 7.14 (br s, 1H), 3.98 - 3.89 (m, 2H), 3.39 (s, 1H), 3.35 (s, 1H), 2.20 (s, 3H).

Step 3. To a mixture of 1-(5-methylpyridin-2-yl)imidazolidin-2-one (65.8 mg, 371 umol, 1.20 *eq*) and 3-(5-bromo-1-oxoisoindolin-2-yl)piperidine-2,6-dione (100 mg, 309 umol, 1.00 *eq*) in dioxane (1.00 mL) was added potassium carbonate (128 mg, 928 umol, 3.00 *eq*), *N*,*N*'-dimethylethylenediamine (5.46 mg, 61.9 umol, 6.66 uL, 0.200 *eq*) and cuprous iodide (5.89 mg, 30.9 umol, 0.100 *eq*) under nitrogen. The mixture was stirred at 100 °C for 12 h. The mixture was concentrated in vacuum. The residue was added dimethyformamide (5.00 mL) and filterted. The filtrate was purified by reversed-phase HPLC (0.1% formic acid condition) and lyophilized. The residue was added water (10 mL) and filtered. The filter cake was added water (10 mL) and lyophilized to give 3-(5-(3-(5-methylpyridin-2-yl)-2-oxoimidazolidin-1-yl)-1-oxoisoindolin-2-yl)piperidine-2,6-dione (14.6 mg, 34.4 umol, 5% yield, 99% purity) as a white solid. ¹H NMR (400 MHz, DMSO-*d*₆) *δ* = 10.98 (s, 1H), 8.20 (s, 1H), 8.13 (d, *J =* 8.5 Hz, 1H), 7.92 (s, 1H), 7.82 - 7.70 (m, 2H), 7.64 (dd, *J =* 1.9, 8.6 Hz, 1H), 5.10 (dd, *J* = 5.1, 13.3 Hz, 1H), 4.51 - 4.42 (m, 1H), 4.38 - 4.29 (m, 1H), 4.13 - 4.00 (m, 4H), 2.98 - 2.86 (m, 1H), 2.60 (br d, *J =* 17.0 Hz, 1H), 2.46 - 2.37 (m, 1H), 2.26 (s, 3H), 2.04 - 1.97 (m, 1H). MS (ESI) m/z. 420.2 [M+H]⁺

### Example 41. Synthesis of Compound 156

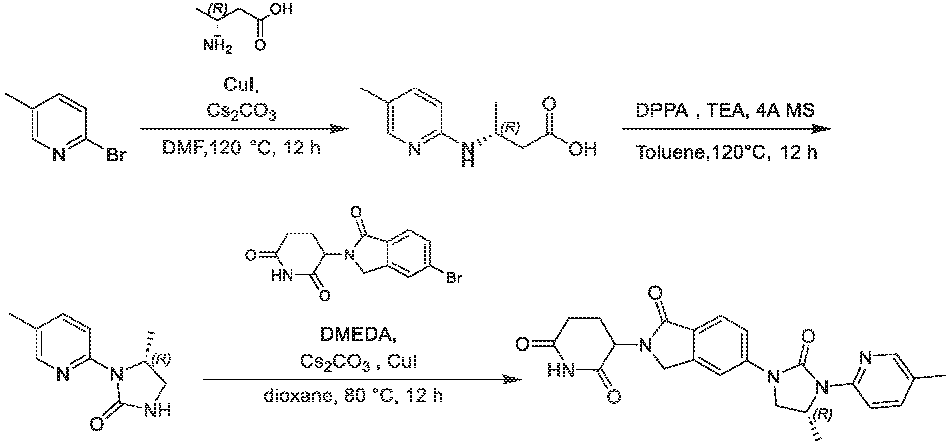

Step 1. To a solution of 2-bromo-5-methyl-pyridine (2.00 g, 11.6 mmol, 1.00 *eq*) in *N,N*-dimethylformamide (30.0 mL) was added copper iodide (221 mg, 1.16 mmol, 0.100 *eq*), cesium carbonate (7.58 g, 23.2 mmol, 2.00 *eq*) and (*R*)-3-aminobutanoic acid (2.40 g, 23.2 mmol, 2.00 *eq*) . Then the reaction mixture was stirred at 120 °C for 12 h under nitrogen atmosphere. The reaction mixture was concentrated in vacuum. The residue was purified by reversed phase column chromatography (C18, 330 g; condition: water/acetonitrile = 1/0 to 0/1, 0.1% formic acid) and concentrated in vacuum to afford (*R*)-3-((5-methylpyridin-2-yl)amino)butanoic acid (1.50 g, crude) as yellow oil. ¹H NMR (400 MHz, DMSO-*d*₆) *δ* = 7.74 (d, *J* = 1.6 Hz, 1H), 7.14 (dd, *J* = 2.1, 8.4 Hz, 1H), 6.87 - 6.42 (m, 1H), 6.27 (d, *J =* 8.4 Hz, 1H), 3.95 - 3.81 (m, 1H), 2.05 (s, 4H), 2.01 - 1.93 (m, 1H), 1.06 (d, *J* = 6.3 Hz, 3H).

Step 2. To a solution of (*R*)-3-((5-methylpyridin-2-yl)amino)butanoic acid (500 mg, 2.57 mmol, 1.00 *eq*) and 4A MS (2.00 g) in toluene (15.0 mL) was added diphenyl phosphoryl azide (2.13 g, 7.72 mmol, 1.67 mL, 3.00 *eq*) and triethylamine (781 mg, 7.72 mmol, 1.07 mL, 3.00 *eq*). Then the reaction mixture was stirred at 120 °C for 12 h under nitrogen atmosphere. The reaction mixture was filtered. The filtrate was concentrated in vacuum. The residue was diluted with water (100 mL) and extracted with ethyl acetate (3 × 100 mL). The combined organic layers were washed with brine (3 × 20 mL), dried over sodium sulfate, filtered and concentrated under reduced pressure to give a residue. The residue was purified by column chromatography (Si02, Petroleum ether/Ethyl acetate = 5/1 to 1/1) and concentrated in vacuum to afford (*R*)-5-methyl-1-(5-methylpyridin-2-yl)imidazolidin-2-one(750 mg, 2.75 mmol, 53% yield, 70% purity) as a yellow solid. ¹H NMR (400 MHz, DMSO-*d*₆) δ = 8.11 (s, 1H), 7.98 (d, *J* = 8.4 Hz, 1H), 7.08 - 7.03 (m, 1H), 5.48 (br d, *J =* 7.6 Hz, 1H), 4.73 - 4.62 (m, 1H), 3.56 (t, *J* = 8.8 Hz, 1H), 2.97 (dd, *J =* 3.7, 8.8 Hz, 1H), 2.22 (s, 3H), 1.29 (d, *J* = 6.0 Hz, 3H).

STEp 3. To a solution of (*R*)-5-methyl-1-(5-methylpyridin-2-yl)imidazolidin-2-one (30.0 mg, 156 umol, 1.69 *eq*) and 3-(5-bromo-1-oxo-isoindolin-2-yl)piperidine-2,6-dione (30.0 mg, 92.8 umol, 1.00 *eq*) in dioxane (1.00 mL) was added cesium carbonate (60.5 mg, 185. umol, 2.00 *eq*), copper iodide (17.6 mg, 92.8 umol, 1.00 *eq*) and *N¹*,*N²*-dimethylethane-1,2-diamine (8.18 mg, 92.8 umol, 9.99 uL, 1.00 *eq*). Then the reaction mixture was stirred at 80 °C for 12 h under nitrogen atmosphere. The reaction mixture was adjust pH to 5-6 by formic acid 0.2 mL) and concentrated in vacuum. The residue was added *N,N-*dimethylformamide (4 mL) and filtered. The filtrate was purified by *Prep*-HPLC (column: Phenomenex luna C18 150*25mm* 10um;mobile phase: [water(FA)-ACN];B%: 20%-50%,10min) and lyophilized to afford 3-(5-((*R*)-4-methyl-3-(5-methylpyridin-2-yl)-2-oxoimidazolidin-1-yl)-1-oxoisoindolin-2-yl)piperidine-2,6-dione(26.25 mg, 60.7 umol, 6% yield, 94% purity) as a yellow solid. ¹H NMR (400 MHz, DMSO-*d*₆) is = 11.07 - 10.87 (m, 1H), 8.22 (d, *J* = 2.4 Hz, 1H), 8.06 - 8.01 (m, 1H), 7.90 (br d, *J* = 5.2 Hz, 1H), 7.80 - 7.75 (m, 1H), 7.74 - 7.70 (m, 1H), 7.68 - 7.62 (m, 1H), 5.10 (dd, *J* = 5.2, 13.2 Hz, 1H), 4.88 - 4.80 (m, 1H), 4.49 - 4.43 (m, 1H), 4.36 - 4.29 (m, 1H), 4.23 (dt, *J* = 2.2, 9.2 Hz, 1H), 3.64 (br dd, *J* = 3.8, 9.2 Hz, 1H), 2.94 - 2.87 (m, 1H), 2.60 (br d, *J =* 12.8 Hz, 1H), 2.43 - 2.38 (m, 1H), 2.27 (s, 3H), 2.03 - 1.98 (m, 1H), 1.38 (d, *J =* 6.0 Hz, 3H). MS (ESI) m/z. 443.0 [M+H]⁺

### Example 42. Synthesis of Compound 140

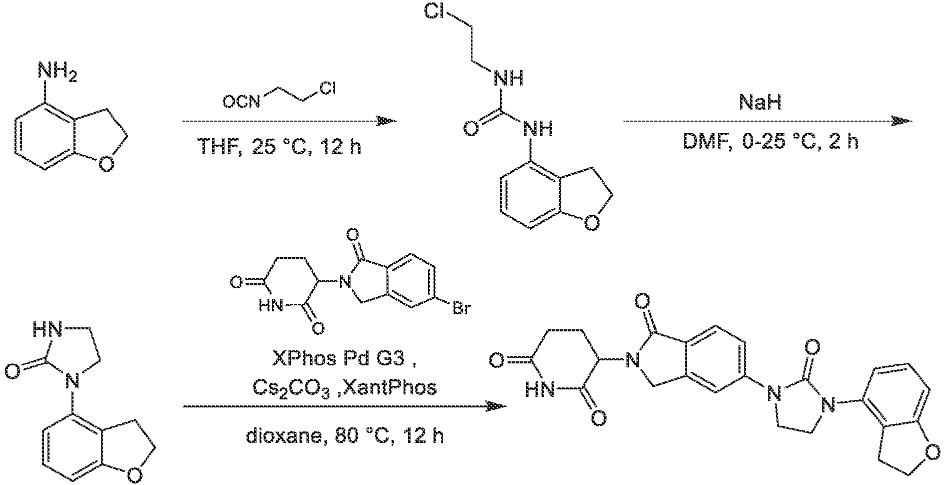

Step 1. To a solution of 2, 3-dihydrobenzofuran-4-amine (1.00 g, 7.40 mmol, 1.00 *eq*) in tetrahydrofuran (10.0 mL) was added 1-chloro-2-isocyanatoethane (1.17 g, 11.1 mmol, 944 uL, 1.50 *eq*). The mixture was stirred at 25 °C for 12 h. The mixture was filtered. The filter cake was concentrated under reduced pressure to afford 1-(2-chloroethyl)-3-(2, 3-dihydrohenzofuran-4-yl)urea (1.40 g, crude) as a white solid. ¹H NMR (400 MHz, DMSO-*d₆*) *δ* = 8.05 (s, 1H), 7.41 (d, *J =* 8.1 Hz, 1H), 6.95 (t, *J* = 8.1 Hz, 1H), 6.72 (br t, *J* = 5.7 Hz, 1H), 6.36 (d, *J =* 7.8 Hz, 1H), 4.51 (t, *J =* 8.7 Hz, 2H), 3.66 (t, *J =* 6.0 Hz, 2H), 3.42 (q, *J* = 6.0 Hz, 2H), 3.05 (t, *J* = 8.7 Hz, 2H),

Step 2. To a solution of 1-(2-chloroethyl)-3-(2,3-dihydrobenzofuran-4-yl)urea (1.40 g, 5.82 mmol, 1.00 *eq*) in *N*,*N*-dimethyl formamide (2.00 mL) was added sodium hydride (465 mg, 11.6 mmol, 60 % purity, 2.00 *eq*) at 0 °C under nitrogen atmosphere. The mixture was stirred at 25 °C for 2 h. The mixture was quenched with saturated ammonium chloride in aqueous solution (50.0 mL), then extracted with ethyl acetate (3 × 100 mL). The organic layers were collected and dried over anhydrous sodium sulfate and evaporated to afford 1-(2, 3-dihydrobenzofuran-4-y])imidazolidin-2-one (700 mg, crude) as a white solid. ¹H NMR (400 MHz, DMSO-*d₆*) *δ* = 7.05 (t, *J* = 7.9 Hz, 1H), 6.81 - 6.75 (m, 2H), 6.56 (d, *J* = 7.9 Hz, 1H), 4.48 (t, *J =* 8.8 Hz, 2H), 3.82 (dd, *J* = 7.1, 8.6 Hz, 2H), 3.40 (br s, 2H), 3.17 (t,*J* = 8.7 Hz, 2H).

Step 3. To a solution of 1-(2,3-dihydrobenzofuran-4-yl)imidazolidin-2-one (200 mg, 979 umol, 1.00 *eq*) and 3-(5-bromo-1-oxoisoindolin-2-yl)piperidine-2,6-dione (316 mg, 979 umol, 1.00 *eq*) in dioxane (10.0 mL) was added cesium carbonate (957 mg, 2.94 mmol, 3.00 *eq*), 4,5-*bis*(diphenylphosphino)-9,9-dimethylxanthene (56.7 mg, 97.9 umol, 0.100 *eq*), and methanesulfonato(2-dicyclohexylphosphino-2,4,6-tri-i-propyl-1,1-biphenyl)(2-amino-1,1-biphenyl-2-yl)palladium (82.9 mg, 97.9 umol, 0.100 *eq*) under nitrogen atmosphere. The mixture was stirred at 80 °C for 12 h. The mixture was filtered to give a residue. The residue was purified by reversed-phase HPLC (column: spherical C18, 20-45 um, 100Å, SW 120, mobile phase: [water (0.1 % Formic Acid) - acetonitrile]; B%: 15% - 45%, 30 min) and *prep-*HPLC (column: Phenomenex luna C18 150*25 mm* 10 um; mobile phase:[water (formic acid) - acetonitrile]; B %: 19 %- 49 %, 10 min) to afford 3-(5-(3-(2,3-dihydrobenzofuran-4-yl)-2-oxoimidazolidin-1-yl)-1-oxoisoindolin-2-yl)piperidine 2,6-dione (8.4 mg, 18.8 umol, 1% yield) as a white solid. After combined with another batch (EC3042-537). 3-(5-(3-(2,3-dihydrobenzofuran-4-yl)-2-oxoimidazolidin-1-yl)-1- oxoisoindolin-2-yl)piperidine-2,6-dione (9.74 mg, 92% purity) was obtained. ¹H NMR (400 MHz, DMSO-*d*₆) *δ* = 10.98 (s, 1H), 7.88 (s, 1H), 7.79 - 7.74 (m, 1H), 7.74 - 7.69 (m, 1H), 7.18 - 7.09 (m, 1H), 6.88 (d, *J* = 8.0 Hz, 1H), 6.67 (d, *J* = 8.0 Hz, 1H), 5.10 (dd, *J =* 5.2, 13.2 Hz, 1H), 4.52 (t, *J* = 8.8 Hz, 2H), 4.48 - 4.41 (m, 1H), 4.35 - 4.28 (m, 1H), 4.09 - 3.97 (m, 4H), 3.22 (t, *J =* 8.8 Hz, 2H), 2.97 - 2.85 (m, 1H), 2.60 (br d, *J* = 17.2 Hz, 1H), 2.39 (br dd, *J* = 4.4, 13.2 Hz, 1H), 2.06 - 1.95 (m, 1H). MS (ESI) m/z 447.3 [M+H]⁺

### Example 43. Synthesis of Compound 175

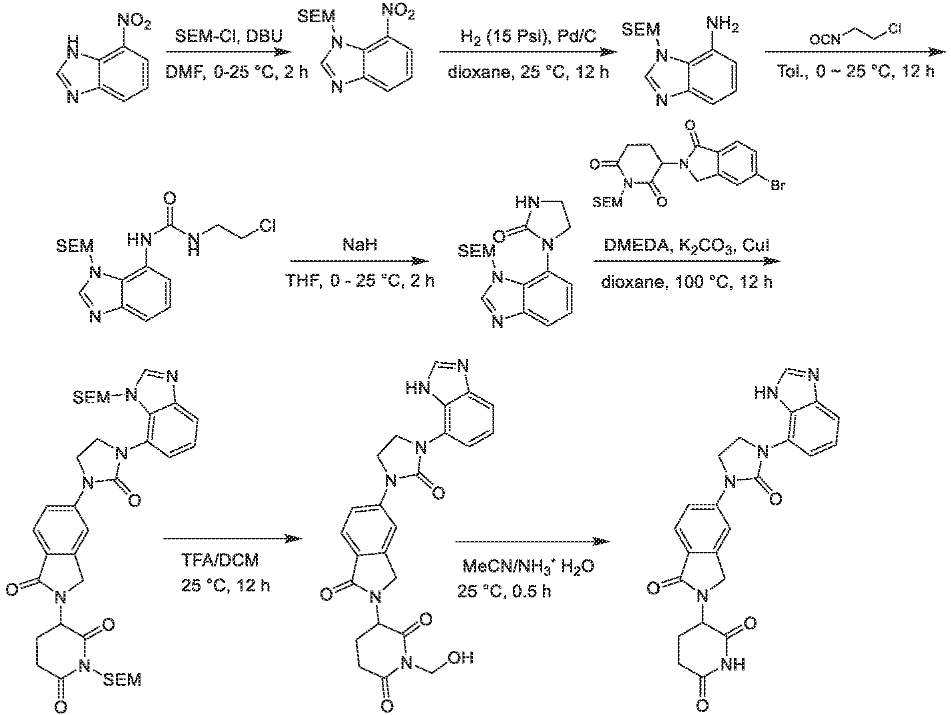

Step 1. To a mixture of 7-nitro-1*H*-benzo[*d*]imidazole (2.00 g, 12.3 mmol, 1.00 *eq*) and 1,8-diazabicyclo[5.4.0]undec-7-ene (3.73 g, 24.5 mmol, 3.70 mL, 2.00 *eq*) in dimethyformamide (20.0 mL) was dropwise added 2-(trimethylsilyl)ethoxymethyl chloride (3.68 g, 22.1 mmol, 3.91 mL, 1.80 *eq*) at 0 °C. The mixture was stirred at 25 °C for 2 h. The reaction mixture was diluted with water (100 mL) and extracted with ethyl acetate (3 × 100 mL). The combined organic layers were washed with brine (100 mL), dried over sodium sulfate, filtered and concentrated under reduced pressure to give a residue. The residue was purified by column chromatography (SiO2, Petroleum ether/Ethyl acetate = 20/1 to 0/1) and concentrated in vacuum to give 7-nitro-1-((2-(trimethylsilyl)ethoxy)methyl)-1*H-*benzo[d]imidazole (1.10 g, 3.75 mmol, 30% yield) as a yellow solid. ¹H NMR (400 MHz, DMSO-*d*₆) *δ* = 8.70 (s, 1H), 8.11 (ddd, *J* = 0.9, 8.0, 16.4 Hz, 2H), 7.51 (t, *J =* 8.0 Hz, 1H), 5.76 (s, 2H), 3.54 - 3.48 (m, 2H), 0.86 - 0.81 (m, 2H), -0.08 - 0.13 (m, 9H).

Step 2. A mixture of 7-nitro-1-((2-(trimethylsilyl)ethoxy)methyl)-1*H-*benzo[*d*]imidazole (1.10 g, 3.75 mmol, 1.00 *eq*) in dioxane (20.0 mL) was degassed and purged with nitrogen for 3 times, and then the mixture was added palladium on carbon (200 mg, 10% purity) and stirred at 25 °C for 12 h under hydrogen atmosphere. The mixture was filtered. The filtrate was concentrated in vacuum to give 1-((2-(trimethylsilyl)ethoxy) rnethyl)-1*H*-benzo[d]imidazol-7-amine (900 mg, 3.42 mmol, 91% yield) as yellow oil. ¹H NMR (400 MHz, DMSO-*d*₆) *δ* = 8.11 (s, 1H), 6.98 - 6.93 (m, 1H), 6.76 (br dd, *J* = 0.8, 8.0 Hz, 1H), 6.42 - 6.38 (m, 1H), 5.52 (s, 2H), 5.28 (s, 2H), 3.51 - 3.45 (m, 2H), 0.86 - 0.80 (m, 2H), -0.08 (s, 9H).

Step 3. To a solution of 1-((2-(trimethylsilyl)ethoxy)methyl)-1*H-*benzol[*d*]imidazol-7-amine (900 mg, 3.42 mmol, 1.00 *eq)* in toluene (10.0 mL) was added 1-chloro-2-isocyanato-ethane (540 mg, 5.13 mmol, 436 uL, 1.50 *eq)* at 0 °C. The mixture was stirred at 25 °C for 12 h. The mixture was filtered. The filter cake was concentrated in vacuum to give 1-(2-chloroethyl)-3-(1-((2-(trimethylsilyl)ethoxy)methyl)-1*H-*benzo[d]imidazol-7-yl)urea (700 mg, crude) as a white solid. ¹H NMR (400 MHz, DMSO-*d*₆) *δ* = 8.29 (s, 1H), 8.14 (s, 1H), 7.46 (dd, *J* = 1.1, 7.8 Hz, 1H), 7.23 - 7.13 (m, 2H), 6.76 (br t, *J* = 5.7 Hz, 1H), 5.64 (s, 2H), 3.66 (t, *J* = 6.3 Hz, 2H), 3.44 - 3.41 (m, 2H), 3.37 - 3.29 (m, 2H), 0.87 - 0.79 (m, 2H), -0.08 (s, 9H).

Step 4. To a mixture of 1-(2-chloroethyl)-3-(1-((2-(trimethylsilyl)ethoxy)methyl)-1*H*-benzo[*d*]imidazol-7-yl)urea (700 mg, 1.90 mmol, 1.00 *eq)* in dimethyformamide (5.00 mL) was added sodium hydride (114 mg, 2.85 mmol, 60% purity, 1.50 *eq)* at 0 °C. The mixture was stirred at 25 °C for 1 h. The reaction mixture was quenched with saturated ammonium chloride (50.0 mL) and filtered. The filter cake was purified by reversed phase (column: spherical C18, 20-45 um, 100Å, SW 80, mobile phase: [water(0.1%Formic Acid)-ACN) and lyophilized to give 1-(1-((2-(trimethylsilyl)ethoxy)methyl)-1*H*-benzo[*d*]imidazol-7- yl)imidazolidin-2-one (200 mg, 602 umol, 31% yield) as a white solid. ¹H NMR (400 MHz, DMSO-d₆) δ = 8.31 (s, 1H), 7.62 (d, *J* = 7.4 Hz, 1H), 7.35 (d, *J* = 7.6 Hz, 1H), 7.25 - 7.17 (m, 1H), 6.83 (s, 1H), 5.62 (s, 2H), 4.40 (t, *J* = 8.0 Hz, 2H), 3.52 - 3.42 (m, 4H), 0.84 (t, *J* = 8.0 Hz, 2H), -0.08 (s, 9H).

Step 5. To a mixture of 1-(1-((2-(trimethylsilyl)ethoxy)methyl)-1*H-*benzo[*d*]imidazol-7-yl)imidazolidin-2-one (90.0 mg, 270 umol, 1.00 *eq)* and 3-(5-bromo-1-oxoisoindolin-2-yl) -1-((2-(trimethylsilyl)ethoxy)methyl) piperidine-2,6-dione (122 mg, 270 umol, 1.00 *eq)* in dioxane (1.50 mL) was added potassium carbonate (112 mg, 812 umol, *3.00 eq),* n,n'-dimethylethylenediamine (11.9 mg, 135 umol, 14.5 uL, 0.500 *eq*) and cuprous iodide (10.3 mg, 54.1 umol, 0.200 *eq).* The mixture was stirred at 100 °C for 12 h under nitrogen. The mixture was concentrated in vacuum. The residue was dissolved in dimethyformamide (5.00 mL) and filtered. The crude product was purified by reversed phase (column: spherical C18, 20-45 um, 100Å, SW 40, mobile phase: [water(0.1%Formic Acid)-ACN) and lyophilized to give 3-(1-oxo-5-(2-oxo-3-(1-((2- (trimethylsilyl)ethoxy)methyl)-1*H*-benzo[d]imidazol-7-yl)imidazolidin-1-yl)isoindolin-2-yl)-1-((2-(trimethylsilyl)ethoxy)methyl)piperidine-2,6-dione (40.0 mg, 56.7 umol, 20% yield) as a white solid. ¹H NMR (400 MHz, DMSO-*d*₆) *δ* = 8.40 (s, 1H), 7.95 (s, 1H), 7.85 - 7.80 (m, 1H), 7.77 - 7.72 (m, 1H), 7.62 (d, *J* = 7.0 Hz, 1H), 7.50 (dd, *J* = 0.6, 8.3 Hz, 1H), 7.35 - 7.28 (m, 1H), 5.67 (s, 2H), 5.27 - 5.20 (m, 1H), 5.11 - 5.02 (m, 2H), 4.54 - 4.47 (m, 3H), 4.30 (d, *J* = 17.0 Hz, 1H), -4.17 - 4.09 (m, 2H), 3.57 - 3.50 (m, 4H), 3.13 - 3.01 (m, 1H), 2.85 - 2.75 (m, 1H), 2.43 - 2.37 (m, 1H), 2.11 - 2.02 (m, 1H), 0.90 - 0.82 (m, 4H), 0.02 - -0.03 (m, 9H), -0.06 (s, 9H).

Step 6. To a mixture of 3-(1-oxo-5-(2-oxo-3-(1-((2-(trimethylsilyl) ethoxy)methyl)-1*H*-benzo[*d*]imidazol-7-yl) imidazolidin-1-yl)isoindolin-2-yl)-1-((2-(trimethylsilyl)ethoxy)methyl)piperidine-2,6-dione (60.0 mg, 85.1 umol, 1.00 *eq)* in trifluoroacetic acid (1.00 mL) and dichloromethane (5.00 mL). The mixture was stirred at 25 °C for 12 h. The mixture was concentrated in vacuum to give 3-(5-(3-(1*H*-benzo[d]imidazol-7-yl) -2-oxoimidazolidin-1-yl)-1-oxoisoindolin-2-yl)-1-(hydroxymethyl)piperidine-2,6-dione (40.0 mg, crude) as yellow oil. MS (ESI) m/z. 475.2 [M+H]⁺

Setp 7. A mixture of 3-(5-(3-(1H-benzo[*d*]imidazol-7-yl) -2-oxoimidazolidin-1-yl)-1-oxoisoindolin-2-yl) -1-hydroxypiperidine-2,6-dione (40 mg, 79.3 umol, 1.00 *eq)* in ammonium hydroxide (0.250mL) and acetonitrile (5.00 mL). The mixture was stirred at 25 °C for 30 min. The mixture was quenched by formic acid (0.200 mL) and concentrated in vacuum. The residue was added dimethyformamide (1.00 mL). The crude product was purified by reversed phase (column: spherical C18, 20-45 um, 100Å, SW 40, mobile phase: [water(0.1%Formic Acid)-ACN) and lyophilized to give 3-(5-(3-(1*H*-benzo[d]imidazol-7-yl) -2-oxoimidazolidin-1-yl) -1-oxoisoindolin-2-yl) piperidine -2,6-dione (12.64 mg, 28.4 umol, 35% yield as a white solid. ¹H NMR (400 MHz, DMSO-*d*₆) *δ* = 12.62 (br d, *J* = 2.4 Hz, 1H), 11.03 - 10.92 (m, 1H), 8.22 (br s, 1H), 7.92 (s, 1H), 7.86 - 7.77 (m, 1H), 7.76 - 7.68 (m, 1H), 7.59 - 7.49 (m, 1H), 7.44 - 7.33 (m, 1H), 7.22 (t, *J =* 7.8 Hz, 1H), 5.09 (dd, *J =* 5.1, 13.3 Hz, 1H), 4.46 (br d, *J* = 17.3 Hz, 2H), 4.36 - 4.29 (m, 1H), 4.11 (br s, 3H), 2.94 - 2.86 (m, 1H), 2.58 (br s, 1H), 2.40 (br dd, *J* = 4.5, 13.3 Hz, 1H), 2.04 - 1.96 (m, 1H). MS (ESI) m/z. 445.3 [M+H]⁺

### Example 44. Synthesis of Compound 172

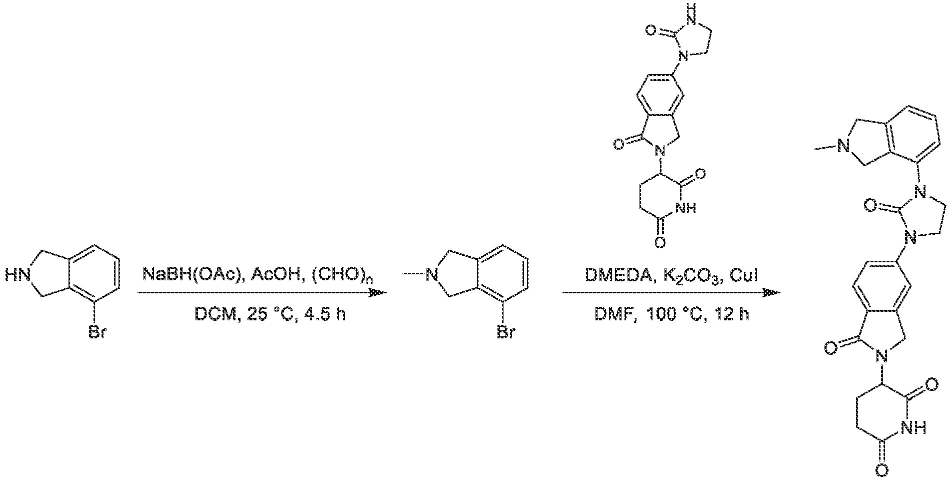

Step 1. To a solution of 4-bronioisoindoline (1.00 g, 5.05 mmol, 1.00 *eq)* in dichloromethane (20.0 mL) was added acetic acid (152 mg, 2.52 mmol, 144 uL, 0.500 *eq)* and formaldehyde (819 mg, 10.1 mmol, 752 uL, 37.0% purity, 2.00 *eq).* The mixture was stirred at 25 °C for 0.5 h, then was added sodium triacetoxy borohydride (3.21 g, 15.2 mmol, *3.00 eq)* and stirred at 25 °C for 4 h. The mixture was filtered to give a filtrate, then was concentrated under reduced pressure to give a residue. The residue was diluted with water (30.0 mL) and extracted with dichloromethane (2 × 30.0 mL). The organic phase was separated, washed with brine (10.0 mL), dried over sodium sulfate, filtered and concentrated under reduced pressure to give 4-bromo-2-methylisoindoline (800 mg, 3.77 mmol, 75% yield) as a red oil. ¹H NMR (400 MHz, CDCl₃) *δ* = 7.32 (d, *J* = 8.0 Hz, 1H), 7.14 - 7.10 (m, 1H), 7.09 - 7.03 (m, 1H), 4.00 (s, 2H), 3.94 (s, 2H), 2.59 (s, 3H).

Step 2. To a solution of 4-bromo-2-methylisoindoline (50.0 mg, 236 umol, 1.00 *eq)* and 3-(1-oxo-5-(2- oxoimidazolidin-1-yl)isoindolin-2-yl)piperidine-2,6-dione (77.4 mg, 236 umol, 1.00 *eq)* in dimethyl formamide (2.00 mL) was added *N,N-*dimethylethylenediamine (10.4 mg, 118 umol, 12.7 uL, 0.500 *eq*), potassium carbonate (97.8 mg, 707 umol, 3.00 *eq)* and copper(I) iodide (8.98 mg, 47.2 umol, 0.200 *eq*)*.* The mixture was stirred under nitrogen atmosphere at 100 °C for 12 h. The mixture was filtered to give a filtrate and purified by *Prep*-HPLC (column: Phenomenex luna C18 150*25mm* 10um;mobile phase: [water(FA)-ACN];B%: 0%-27%,10min) and lyophilized to give 3-(5-(3-(2-methylisoindolin-4-yl)-2- oxoimidazolidin-1-yl)-1-oxoisoindolin-2-yl)piperidine-2,6-dione (30.0 mg, 65.2 umol, 4.62% yield, 94% purity) as a yellow solid. ¹H NMR (400 MHz, DMSO-*d*₆) *δ* = 10.97 (s, 1H), 7.87 (s, 1H), 7.79 - 7.75 (m, 1H), 7.73 - 7.69 (m, 1H), 7.30 - 7.25 (m, 1H), 7.24 - 7.20 (m, 1H), 7.15 (d, *J* = 7.2 Hz, 1H), 5.10 (dd, *J* = 5.0, 13.2 Hz, 1H), 4.50 - 4.41 (m, 1H), 4.37 - 4.27 (m, 1H), 4.07 - 3.97 (m, 4H), 3.97 - 3.86 (m, 4H), 2.94 - 2.87 (m, 1H), 2.63 - 2.58 (m, 1H), 2.49 - 2.48 (m, 3H), 2.42 - 2.36 (m, 1H), 2.04 - 1.96 (m, 1H).

¹H NMR (400 MHz, CDCl₃) *δ* = 8.00 (s, 1H), 7.89 (d, *J* = 8.4 Hz, 1H), 7.45 (s, 1H), 7.33 (s, 1H), 7.14 (dd, *J* = 7.6, 11.2 Hz, 2H), 5.22 (dd, *J* = 5.2, 13.2 Hz, 1H), 4.48 (s, 1H), 4.36 (br d, *J* = 16.0 Hz, 1H), 4.30 (br d, *J* = 16.0 Hz, 4H), 4.11 - 4.01 (m, 4H), 2.98 - 2.90 (m, 1H), 2.86 (br dd, *J* = 5.2, 12.8 Hz, 1H), 2.79 (s, 3H), 2.37 (dd, *J* = 4.8, 12.8 Hz, 1H), 2.27 - 2.20 (m, 1H). MS (ESI) m/z 460.1[M+H]⁺

### Example 45. Synthesis of Compound 165

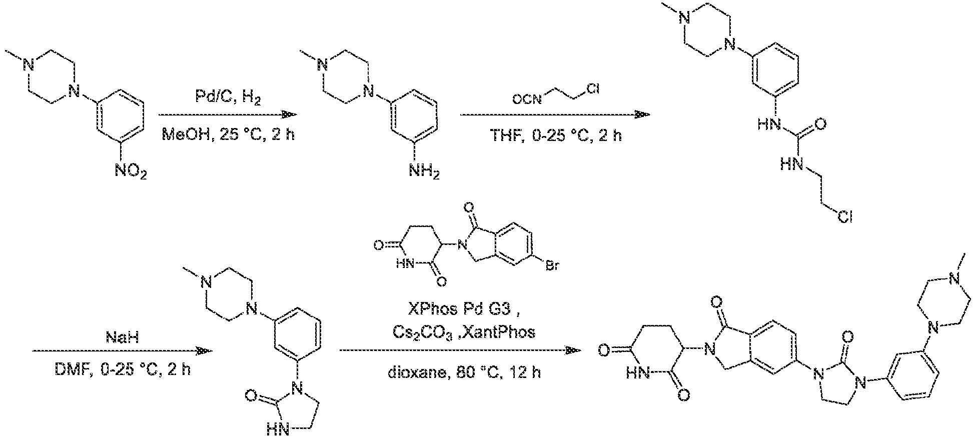

Step 1. To a solution of 1-methyl-4-(3-nitrophenyl)piperazine (2.00 g, 9.04 mmol, 1.00 *eq)* in methanol (10.0 mL) was added palladium on activated carbon (100 mg, 10%) purity) under hydrogen. The mixture was stirred at 25 °C for 2 h. The reaction mixture was filtered through diatomaceous earth, and the filter cake was washed with methanol (30 mL). The filtrate was concentrated under reduced pressure to afford 3-(4-methylpiperazin-1-yl)aniline (1.8 g, crude) as a black solid.

¹H NMR (400 MHz, DMSO-*d₆*) *δ* = 6.83 (t, *J* = 7.9 Hz, 1H), 6.17 - 6.06 (m, 2H), 6.05 - 5.99 (m, 1H), 3.34 (br s, 2H), 3.04 - 2.98 (m, 4H), 2.43 - 2.38 (m, 4H), 2.20 (s, 3H)

Step 2. To a solution of 3-(4-methylpiperazin-1-yl)aniline (1.80 g, 9.41 mmol, 1.00 *eq)* in tetrahydrofuran (10.0 mL) was added 1-chloro-2-isocyanatoethane (1.49 g, 14.1 mmol, 1.20 mL, 1.50 *eq)* at 0 °C. The mixture was stirred at 25 °C for 2 h. The mixture was filtered. The filter cake was concentrated under reduced pressure to afford 1-(2-chloroethyl)-3-(3-(4-methylpiperazin-1-yl)phenyl)urea (1.40 g, crude) as a white solid. ¹H NMR (400 MHz, DMSO-*d₆*) *δ* = 8.50 (s, 1H), 7.10 (s, 1H), 7.03 (t, *J* = 8.1 Hz, 1H), 6.78 - 6.68 (m, 1H), 6.50 (dd, *J* = 1.9, 8.2 Hz, 1H), 6.34 (t, *J* = 5.8 Hz, 1H), 3.64 (t, *J* = 6.1 Hz, 2H), 3.40 (q, *J* = 6.0 Hz, 2H), 3.11 - 3.02 (m, 4H), 2.46 - 2.42 (m, 4H), 2.21 (s, 3H)

Step 3. To a solution of 1-(2-chloroethyl)-3-(3-(-4-methylpiperazin-1-yl)phenyl)urea (1.40 g, 4.72 mmol, 1.00 *eq)* in N,N-dimethyl formamide (10.0 mL) was added sodium hydride (377 mg, 9.43 mmol, 60% purity, 2.00 *eq)* at 0 °C under nitrogen atmosphere. The mixture was stirred at 25 °C for 2 h. The mixture was quenched with saturated ammonium chloride in aqueous solution (50 mL), then extracted with ethyl acetate (3 × 100 mL). The organic layers were collected and dried over anhydrous sodium sulfate and evaporated to afford 1-(3-(4-methylpiperazin-1-yl)phenyl)imidazolidin-2-one (1.00 g, crude) as a white solid.

¹H NMR (400 MHz, DMSO-*d₆*) *δ* = 7.21 (s, 1H), 7.14 - 7.08 (m, 1H), 6.90 (dd, *J* = 1.3, 8.2 Hz, 1H), 6.86 (s, 1H), 6.57 (dd, *J* = 1.9, 8.3 Hz, 1H), 3.87 - 3.78 (m, 2H), 3.37 (br d, *J* = 8.5 Hz, 2H), 3.13 - 3.05 (m, 4H), 2.46 - 2.39 (m, 4H), 2.21 (s, 3H)

Step 4. To a solution of 1-(3-(4-methylpiperazin-1-yl)phenyl)imidazolidin-2-one (200 mg, 768 umol, 1.00 *eq)* and 3-(5-bromo-1-oxoisoindolin-2-yl)piperidine-2,6-dione (248 mg, 768 umol, 1.00 *eq)* in dioxane (1.00 mL) was added 4,5-bis(diphenylphosphino)-9,9-dimethylxanthene (44.5 mg, 76.8 umol, 0.100 *eq)* and cesium carbonate (751 mg, 2.30 mmol, *3.00* eq), methanesulfonato(2-dicyclohexylphosphino-2,4,6-tri-i-propyl-1,1
-biphenyl)(2-amino-1,1-biphenyl-2-yl)palladium (65.0 mg, 76.8 umol, 0.100 *eq)* under nitrogen atmosphere. The mixture was stirred at 80 °C for 12 h. The mixture was filtered. The filtrate was purified by reversed-phase HPLC (column: spherical C18, 20-45 um, 100Å, SW 120, mobile phase: [water(0.1%Formic Acid)-ACN];B%: 15%-45%, 30 min) and triturated with acetonitrile at 25 °C for 30 min to afford 3-(5-(3-(3-(4-methylpiperazin-1-yl)phenyl)-2-oxoimidazolidin-1-yl)-1-oxoisoindolin-2-yl)piperidine-2,6-dione (25.2 mg, 49.2 umol, 49% yield, 95% purity) as a white solid..

¹H NMR (400 MHz, DMSO-*d₆*) *δ* = 10.96 (br s, 1H), 8.30 (s, 0.1H), 7.92 (s, 1H), *7.79* - 7.75 (m, 1H), 7.74 - 7.67 (m, 1H), 7.34 (s, 1H), 7.24 - 7.16 (m, 1H), 6.96 (br d, *J =* 7.9 Hz, 1H), 6.69 (br d, *J* = 8.6 Hz, 1H), 5.10 (dd, *J* = 4.8, 13.1 Hz, 1H), 4.52 - 4.42 (m, 1H), 4.37 - 4.27 (m, 1H), 4.02 (br s, 4H), 3.18 - 3.12 (m, 4H), 2.97 - 2.84 (m, 1H), 2.60 (br d, *J* = 18.6 Hz, 1H), 2.47 - 2.44 (m, 4H), 2.40 (br dd, *J* = 4.5, 13.6 Hz, 1H), 2.23 (s, 3H), 2.04 - 1.97 (m, 1H), MS (ESI) m/z 503.2 [M+H]⁺

### Example 46. Synthesis of Compound 145

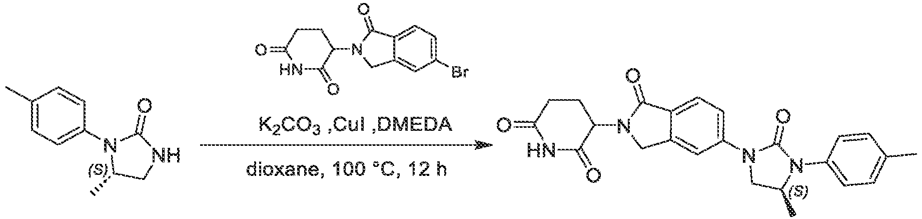

To a solution of (*S*)-5-methyl-1-(*p*-tolyl)imidazolidin-2-one (20.0 mg, 105 umol, 1.00 *eq)* and 3-(5-bromo) -1-oxoisoindolin-2-yl)piperidine-2,6-dione (34.0 mg, 105 umol, 1.00 *eq)* in dioxane (1.50 mL) was added *N,N*-dimethylethylenediamine (4.63 mg, 52.6 umol, 5.66 uL, 0.500 *eq*), potassium carbonate (43.6 mg, 315 umol, 3.00 *eq)* and copper(I) iodide (4.00 mg, 21.0 umol, 0.200 *eq*). The mixture was stirred under nitrogen atmosphere at 100 °C for 12 h. The reaction mixture was diluted with water (30.0 mL) and extracted with ethyl acetate (2 × 30.0 mL). The organic phase was separated, washed with brine (10.0 mL), dried over sodium sulfate, filtered and concentrated under reduced pressure to give a residue. The residue was purified by *Prep*-HPLC (column: Phenomenex luna C18 150*25mm* 10um;mobile phase: [water(FA)- ACN];B%: 32%-62%,10min) and lyophilized to afford 3-(5-((*S*)-4-methyl-2-oxo-3-(*p*-tolyl)imidazolidin -1-yl) -1-oxoisoindolin-2-yl)piperidine-2,6-dione (14.0 mg, 32.4 umol, 8% yield) as a white solid.

¹H NMR (400 MHz, DMSO-*d*₆) *δ* = 10.91 (br s, 1H), 7.87 (br d, *J* = 2.8 Hz, 1H), 7.78 - 7.72 (m, 1H), 7.72 - 7.67 (m, 1H), 7.39 (d, *J* = 8.4 Hz, 2H), 7.21 (br d, *J* = 8.0 Hz, 2H), 5.10 (dd, *J* = 5.2, 13.2 Hz, 1H), 4.63 - 4.54 (m, 1H), 4.48 - 4.40 (m, 1H), 4.36 - 4.28 (m, 1H), 4.26 - 4.19 (m, 1H), 3.63 (dt, *J* = 3.2, 6.0 Hz, 1H), 2.96 - 2.87 (m, 1H), 2.59 (br d, *J* = 17.6 Hz, 1H), 2.44 - 2.36 (m, 1H), 2.30 (s, 3H), 2.04 - 1.97 (m, 1H), 1.24 (d, *J* = 6.0 Hz, 3H). MS (ESI) m/z 433.2 [M+H]⁺

### Example 47. Synthesis of Compound 166

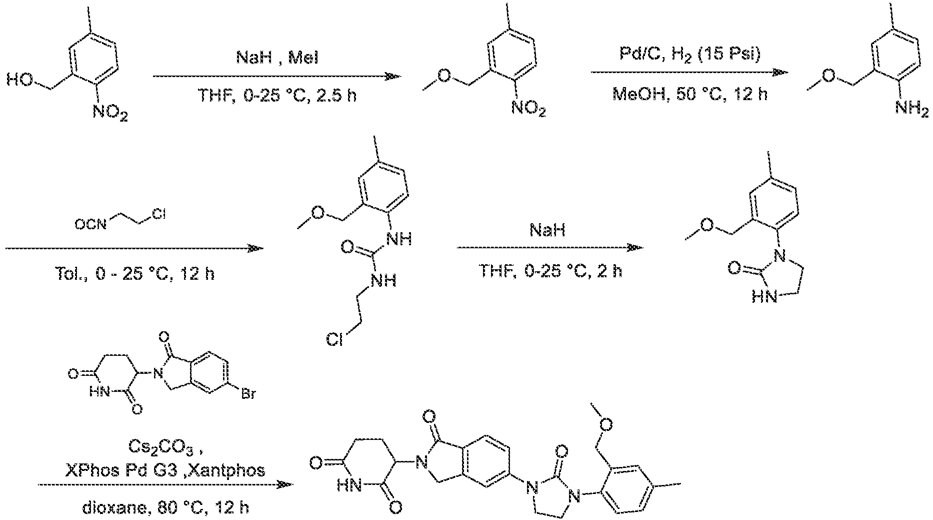

Step 1. To a solution of (5-methyl-2-nitrophenyl)methanol (4.00 g, 23.9 mmol, *1.00 eq)* in tetrahydrofuran (20.0 mL) was added sodium hydride (1.91 g, 47.8 mmol, 60% purity, 2.00 *eq)* at 0 °C under nitrogen. The mixture was stirred at 0 °C for 0.5 h. The iodomethane (13.5 g, 95.7 mmol, 5.96 mL, 4.00 *eq)* was added to the solution under nitrogen atmosphere. The mixture was stirred at 25 °C for 2 h. The reaction mixture was quenched with saturated ammonium chloride (40.0 mL) to give a solution which was diluted with water (20.0 mL) and extract with ethyl acetate (2 × 60 mL). The organic phase was separated, washed with brine (30 mL), dried over sodium sulfate, filtered and concentrated under reduced pressure to give a residue which was purified by column chromatography (SiO₂, Petroleum ether/Ethyl acetate = 1/0 to 40/1) to give 2-(methoxymethyl)-4-methyl-1-nitrobenzene (1.70 g, 9.38 mmol, 39% yield) as a yellow oil. ¹H NMR (400 MHz, CDCl₃) *δ* = 8.01 (d, *J* = 8.3 Hz, 1H), 7.58 (s, 1H), 7.23 - 7.19 (m, 1H), 4.83 (s, 2H), 3.51 (s, 3H), 2.47 - 2.45 (m, 3H),

Step 2. To a solution of 2-(methoxymethyl)-4-methyl-]-nitrobenzene (1.00 g, 5.52 mmol, 1.00 *eq)* in methanol (10.0 mL) was added palladium on carbon (500 mg, 10% purity) under nitrogen. The mixture was stirred at 50 °C for 12 h under hydrogen (15 Psi) atmosphere. The mixture was filtered to give a filter liquor, then was concentrated under reduced pressure to give 2-(methoxymethyl)-4-methylaniline (1.47 g, 9.72 mmol, 88% yield) as a yellow oil. ¹H NMR (400 MHz, DMSO-*d₆*) *δ* = 6.85 - 6.79 (m, 2H), 6.56 (dd, *J* = 2.6, 7.9 Hz, 1H), 4.68 (s, 2H), 4.28 (d, *J* = 1.4 Hz, 2H), 3.24 (d, *J* = 0.9 Hz, 3H), 2.14 (s, 3H)

Step 3. To a solution of 2-(methoxymethyl)-4-methylaniline (1.47 g, 9.72 mmol, 1.00 *eq)* in toluene (20.0 mL) was added 1-chloro-2-isocyanatoethane (1.54 g, 14.5 mmol, 1.24 mL, 1.50 *eq*) at 0 °C. The mixture was stirred at 25 °C for 12 h. The reaction mixture was concentrated under reduced pressure to give 1-(2-chloroethyl)-3-(2-(methoxymethyl)-4-methylphenyl)urea (2.49 g, 9.70 mmol, 99% yield) as a white solid. MS (ESI) m/z 257.1 [M+H]⁺

Step 4. To a solution of 1-(2-chloroethyl)-3-(2-(methoxymethyl)-4-methylphenyl)urea (2.00 g, 7.79 mmol, 1.00 *eq)* in tetrahydrofuran (30.0 mL) was added sodium hydride (467 mg, 11.6 mmol, 60% purity, 1.50 *eq)* at 0 °C under nitrogen. The mixture was stirred at 25 °C for 2 h. The reaction mixture was quenched with methanol (20.0 mL) to give a solution which was concentrated under reduced pressure to give a residue and the residue was purified by column chromatography (SiO₂, Petroleum ether/Ethyl acetate = 1/0 to 0/1) to give 1-(2-(methoxymethyl)-4-methylphenyl)imidazolidin-2-one (1.10 g, 4.99 mmol, 64% yield) as a white solid. ¹H NMR (400 MHz, DMSO-*d*₆) *δ* = 7.24 (s, 1H), 7.14 - 7.08 (m, 2H), 6.63 (s, 1H), 4.35 (s, 2H), 3.73 - 3.67 (m, 2H), 3.39 (t, *J* = 7.8 Hz, 2H), 3.29 (s, 3H), 2.30 (s, 3H). MS (ESI) m/z 221.1 [M+H]⁺

Step 5. To a solution of 3-(5-bromo-1-oxoisoindolin-2-yl)piperidine-2,6-dione (50.0 mg, 154 umol, 1.00 *eq*), 1-(2-(methoxymethyl)-4-methylphenyl)imidazolidin-2-one (34.0 mg, 154 umol, 1.00 *eq*), cesium carbonate (100 mg, 309 umol, 2.00 *eq*) in 1,4-dioxane (2.00 mL) was added methanesulfonato (2-dicyclohexylphosphino-2,4,6-tri-i-propyl-1,1-biphenyl)(2-amino-1,1-biphenyl-2-yl)palladium(II) (13.1 mg, 15.4 umol, 0.100 *eq)* and 4,5-bis(diphenylphosphino)-9,9-dimethylxanthene (17.9 mg, 30.9 umol, 0.200 *eq)* under nitrogen. The mixture was stirred at 80 °C for 12 h. The solution was added formic acid to adjust pH = 5 and concentrated under reduced pressure to give a residue which was purified by reversed-phase HPLC (column: spherical C18, 20-45 um, 100Å, SW 80, mobile phase: [water(0. 1%Formic Acid)- acetonitrile) to give 3-(5-(3-(2-(methoxymethyl)-4-methylphenyl)-2-oxoimidazolidin-1-yl)-1-oxoisoindolin-2-yl)piperidine-2,6-dione (179 mg, 387 umol, 25% yield, 97% purity) as a white solid.

¹H NMR (400 MHz, DMSO-*d*₆) *δ* = 10.97 (s, 1H), 7.87 (s, 1H), 7.78 - 7.73 (m, 1H), 7.73 - 7.68 (m, 1H), 7.28 (d, *J* = 8.0 Hz, 2H), 7.18 (dd, *J* = 1.6, 8.1 Hz, 1H), 5.09 (dd, *J* = 5.1, 13.3 Hz, 1H), 4.47 - 4.41 (m, 1H), 4.40 (s, 2H), 4.34 - 4.27 (m, 1H), 4.10 - 4.02 (m, 2H), 3.91 - 3.83 (m, 2H), 3.28 (s, 3H), 2.97 - 2.85 (m, 1H), 2.60 (br dd, *J* = 2.1, 15.5 Hz, 1H), 2.45 - 2.36 (m, 1H), 2.34 (s, 3H), 2.04 - 1.96 (m, 1H). MS (ESI) m/z 463.1 [M-H]⁺

### Example 48. Synthesis of Compound 169

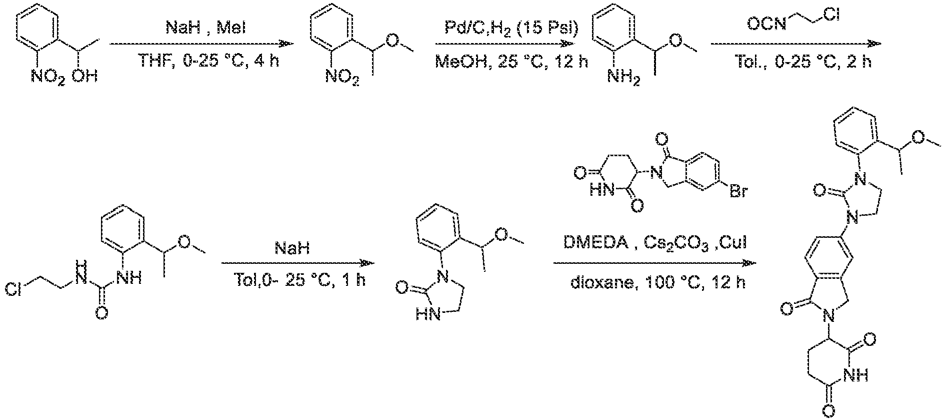

Step 1. To a solution of 1-(2-nitrophenyl)ethanol (5.00 g, 29.9 mmol, 1.00 *eq)* in tetrahydrofuran (50.0 mL) was added sodium hydride (1.79 g, 44.8 mmol, 60% purity, 1.50 *eq*) at 0°C. Then the reaction mixture was stirred at 25 °C for 0.5 h under nitrogen atmosphere. Then the reaction mixture was added iodomethane (12.7 g, 89.7 mmol, 5.59 mL, 3.00 *eq*) at 0 °C. Then the reaction mixture was stirred at 25 °C for 3 h under nitrogen atmosphere. The mixture was quenched with ammonium chloride (200 mL) and extracted with dichloromethane (3 × 100 mL). The combined organic layers were washed with brine (3 × 20 mL), dried over sodium sulfate, filtered and concentrated under reduced pressure to give 1-(1-methoxyethyl)-2--nitrobenzene (5.30 g, 28.6 mmol, 95% yield, 98% purity) as yellow oil.

¹H NMR (400 MHz, CDCl₃) *δ* = 7.75 (d, *J* = 8.0 Hz, 1H), 7.61 - 7.56 (m, 1H), 7.49 (t, *J* = 8.0 Hz, 1H), 7.25 (t, *J* = 8.0 Hz, 1H), 4.72 (q, *J*= 6.0 Hz, 1H), 3.05 (s, 3H), 1.36 (d, *J* = 6.0 Hz, 3H).

Step 2. To a solution of 1-(1-methoxyethyl)-2-nitro-benzene (2.80 g, 15.4 mmol, 1.00 *eq)* in methanol (75.0 mL) was added palladium on carbon (600 mg, 10% purity). Then the reaction mixture was stirred at 25 °C for 12 h under hydrogen atmosphere (15 Psi). The reaction mixture was filtered. The filtrate was concentrated in vacuum to afford 2-(1-methoxyethyl)aniline (1.80 g, 11.5 mmol, 74% yield, 97% purity) as yellow oil.

¹H NMR (400 MHz, CDCl₃) *δ* = 7.09 (dt, *J* = 1.6, 7.6 Hz, 1H), 7.00 (dd, *J* = 1.6, 7.6 Hz, 1H), 6.70 (dt, *J* = 1.2, 7.6 Hz, 1H), 6.65 (dd, *J* = 0.8, 8.0 Hz, 1H), 4.40 (q, *J* = 6.4 Hz, 1H), 3.28 (s, 3H), 1.54 (d, *J* = 6.4 Hz, 3H).

Step 3. To a solution of 2-(1-methoxyethyl)aniline (800 mg, 5.29 mmol, 1.00 *eq*) in toluene (8.00 mL) was added 1-chloro-2-isocyanato-ethane (837 mg, 7.94 mmol, 675 uL, 1.50 *eq*) at 0 °C. Then the reaction mixture was stirred at 25 °C for 12 h. The reaction mixture was fitered. The filter cake was concentrated in vacuum and the filtrate was concentrated in vacuum to give 1-(2-chloroethyl)-3-(2-(1-methoxyethyl)phenyl)urea (1.30 g, 4.91 mmol, 92% yield, 97% purity) as a white solid. ¹H NMR (400 MHz, DMSO-*d*₆) δ = 7.91 (s, 1H), 7.73 (dd, *J* = 0.8, 8 Hz, 1H), 7.06 - 7.01 (m, 2H), 4.52 (q, *J* = 6.4 Hz, 1H), 3.66 (t, *J* = 6.0 Hz, 2H), 3.44 - 3.39 (m, 2H), 3.13 (s, 3H), 1.34 (d, *J* = 6.4 Hz, 3H).

Step 4. To a solution of 1-(2-chloroethyl)-3-(2-(1-methoxyethyl)phenyl)urea (500 mg, 1.95 mmol, 1.00 *eq*) in toluene (10 mL) was added sodium hydride (116 mg, 2.92 mmol, 60% purity, 1.50 *eq*) at 0 °C. Then the reaction mixture was stirred at 25 °C for 1 h. The reaction mixture was quenched by addition methanol (20 mL) and concentrated in vacuum to afford 1-(2-(1-methoxyethyl)phenyl)imidazolidin-2-one (400 mg, 1.76 mmol, 90% yield, 97% purity) as a yellow solid. ¹H NMR (400 MHz, DMSO-*d*₆) *δ* = 7.27 (dd, *J* = 1.6, 7.6 Hz, 1H), 7.16 - 7.11 (m, 1H), 7.11 - 7.07 (m, 1H), 7.06 - 7.01 (m, 1H), 4.83 (q, *J* = 6.4 Hz, 1H), 3.47 - 3.38 (m, 2H), 3.31 - 3.25 (m, 2H), 3.02 (s, 3H), 1.31 (d, *J* = 6.4 Hz, 3H).

Step 5. To a solution of 1-(2-(1-methoxyethyl)phenyl)imidazolidin-2-one (34.0 mg, 154 umol, 1.00 *eq*) and 3-(5-bromo-1-oxo-isoindolin-2-yl)piperidine-2,6-dione (50.0 mg, 154 umol, 1.00 *eq*) in dioxane (1.00 mL) was added cesium carbonate (151 mg, 464 umol, *3.00 eq*), copper iodide (2.95 mg, 15.4 umol, 0.100 *eq*), *N¹,N²*-dimethylethane-1,2-diamine (2.73 mg, 30.9 umol, 3.33 uL, 0.200 *eq*). Then the reaction mixture was stirred at 100 °C for 12 h under nitrogen atmosphere. The reaction mixture was adjusted pH to 5-6 with formic acid (0.1 mL) and concentrated in vacuum. The residue was diluted with water (100 mL) and extracted with ethyl acetate mL (3 × 50 mL). The combined organic layers were washed with brine (3 × 20 mL), dried over sodium sulfate, filtered and concentrated under reduced pressure to give a residue. The residue was purified by reversed phase column chromatography (C18, 330 g; condition: water/acetonitrile = 1/0 to 0/1, 0.1% formic acid) and lyophili zed to afford 3-(5-(3-(2-(1-methoxyethyl)phenyl)-2-oxoimidazolidin-1-yl)-1-oxoisoindolin-2-yl)piperidine-2,6-dione (29.88 mg, 62.6 umol, 4% yield, 97% purity) as a yellow solid. ¹H NMR (400 MHz, DMSO-*d*₆) δ = 11.14 - 10.86 (m, 1H), 7.88 (s, 1H), 7.80 - 7.75 (m, 1H), 7.74 - 7.69 (m, 1H), 7.51 - 7.46 (m, 1H), 7.45 - 7.37 (m, 3H), 5.14 - 5.06 (m, 1H), 4.61 - 4.53 (m, 1H), 4.49 - 4.41 (m, 1H), 4.36 - 4.28 (m, 1H), 4.10 (br d, *J* = 7.6 Hz, 2H), 3.97 - 3.86 (m, 2H), 3.09 (s, 3H), 2.98 - 2.87 (m, 1H), 2.63 - 2.59 (m, 1H), 2.41 (br dd, *J* = 9.2, 13.2 Hz, 1H), 2.05 - 1.97 (m, 1H), 1.35 (d, *J* = 6.4 Hz, 3H). MS (ESI) m/z. 463.1 [M+H]⁺

### Example 49. Synthesis of Compound 168

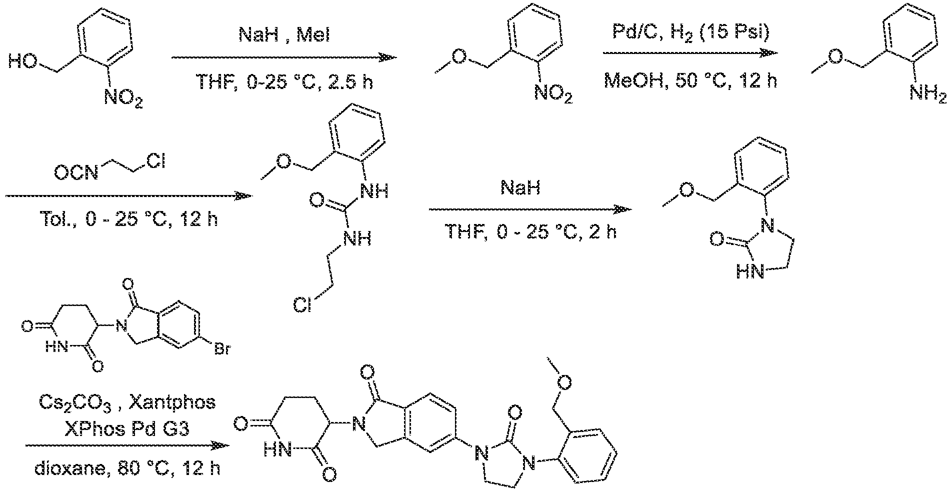

Step 1. To a solution of (2-nitrophenyl)methanol (5.00 g, 32.6 mmol, 1.00 *eq*) in tetrahydrofuran (80.0 mL) was added sodium hydride (2.61 g, 65.3 mmol, 60% purity, 2.00 *eq*) at 0 °C under nitrogen. The mixture was stirred at 0 °C for 0.5 h. The methyl iodide (18.5 g, 130 mmol, 8.13 mL, 4.00 *eq*) was added to the solution under nitrogen. The mixture was stirred at 25 °C for 2 h. The reaction mixture was quenched with saturated ammonium chloride (30.00 ml) to give a solution. The reaction mixture was diluted with water (20.0 mL) and extract with ethyl acetate (2 × 60 mL). The organic phase was separated, washed with brine (30 mL), dried over sodium sulfate, filtered and concentrated under reduced pressure to give a residue which was purified by column chromatography (SiO₂, Petroleum ether/Ethyl acetate=1/0 to 100/1) to give 1-(methoxymethyl)-2-nitrobenzene (2.20 g, 13.1 mmol, 40% yield) as a colorless oil. ¹H NMR (400 MHz, CDCl₃) *δ* = 8.06 (d, *J* = 8.3 Hz, 1H), 7.77 (d, *J* = 7.8 Hz, 1H), 7.64 (t, *J* = 7.6 Hz, 1H), 7.43 (t, *J* = 7.8 Hz, 1H), 4.83 (s, 2H), 3.49 (s, 3H).

Step 2. To a solution of 1-(methoxymethyl)-2-nitrobenzene (2.00 g, 11.9 mmol, 1.00 *eq*) in methanol (20.0 mL) was added palladium on carbon (1.00 g, 10% purity) under nitrogen. The mixture was stirred at 50 °C for 12 h under hydrogen (15 Psi). The mixture was filtered to give a filter liquor, then was concentrated under reduced pressure to give 2-(methoxymethyl)aniline (1.20 g, crude) as a yellow oil. ¹H NMR (400 MHz, CDCl₃) *δ* = 7.39 (t, *J* = 7.6 Hz, 1H), 7.32 (d, *J* = 7.0 Hz, 1H), 6.99 - 6.95 (m, 1H), 6.94 - 6.92 (m, 1H), 4.73 (s, 2H), 4.39 (br s, 2H), 3.59 (d, *J* = 0.8 Hz, 3H).

Step 3. To a solution of 2-(methoxymethyl)aniline (1.10 g, 8.02 mmol, 1.00 *eq*) in toluene (20.0 mL) was added 1-chloro-2-isocyanatoethane (1.27 g, 12.0 mmol, 1.02 mL, 1.50 *eq*) at 0 °C . The mixture was stirred at 25 °C for 12 h. The reaction mixture was concentrated under reduced pressure to give 1-(2-chloroethyl)-3-(2-(methoxymethyl)phenyl)urea (1.96, crude) as a white solid. ¹H NMR. (400 MHz, DMSO-*d*₆) *δ* = 7.84 - 7.79 (m, 2H), 7.24 (t, *J* = 7.4 Hz, 2H), 7.17 - 7.12 (m, 1H), 6.99 - 6.94 (m, 1H), 4.39 (s, 2H), 3.68 - 3.65 (m, 2H), 3.44 - 340 (m, 2H), 3.34 - 3.33 (m, 3H).

Step 4. To a solution of 1-(2-chloroethyl)-3-(2-(methoxymethyl)phenyl)urea (0.980 g, 4.04 mmol, 1.00 *eq*) in tetrahydrofuran (10.0 mL) was added sodium hydride (242 mg, 6.06 mmol, 60% purity, 1.5 *eq*) at 0 °C under nitrogen. The mixture was stirred at 25 °C for 2 h. The reaction mixture was quenched with methanol (10.0 mL) to give a solution. The reaction mixture was concentrated under reduced pressure to give a residue which was purified by column chromatography (SiO₂, Petroleum ether/Ethyl acetate=1/0 to 0/1) to give 1-(2-(methoxymethyl)phenyl)imidazolidin-2-one (1.10 g, 5.33 mmol, 66% yield) as a white solid. ¹H NMR (400 MHz, DMSO-*d*₆) *δ* = 7.43 (br d, *J* = 7.4 Hz, 1H), 7.30 (br d, *J* = 7.4 Hz, 1H), 7.28 - 7.26 (m, 1H), 7.21- 7.16 (m, 1H), 6.70 (s, 1H), 4.40 (s, 2H), 3.78 - 3.72 (m, 2H), 3.42 (d, *J* = 8.0 Hz, 2H), 3.30 (s, 3H)

Step 5. To a solution of 1-(2-(methoxymethyl)phenyl)imidazolidin-2-one (50.0 mg, 154 umol, 1.00 *eq*) , 3-(5-bromo-1-oxoisoindolin-2-yl)piperidine-2,6-dione (31.9 mg, 154 umol, 1.00 *eq*) , cesium carbonate (100 mg, 309 umol, 2.00 *eq*) in 1,4-dioxane (2.00 mL) was added methanesulfonato(2-dicyclohexylphosphino-2,4,6-tri-i-propyl-1,1-biphenyl)(2-amino-1,1-biphenyl-2-yl)palladium(II) (13.1 mg, 15.4 umol, 0.100 *eq*) and 4,5-bis(diphenylphosphino)-9,9-dimethylxanthene (8.95 mg, 15.4 umol, 0.100 *eq*) under nitrogen. The mixture was stirred at 80 °C for 12 h. The solution was added formic acid to adjust pH = 5 and concentrated under reduced pressure to give a residue which was purified by reversed-phase HPLC (column: spherical C18, 20-45 um, 100Å, SW 80, mobile phase: [water(0.1%Formic Acid)- acetonitrile). The crude product was added dimethyl formamide (5.00 mL) and filtered to give 3-(5-(3-(2-(methoxymethyl)phenyl)-2-oxoimidazolidin-1-yl)-1-oxoisoindolin-2-yl)piperidine-2,6-dione (70.2 mg, 156 umol, 10% yield) as a white solid.

¹H NMR (400 MHz, DMSO-*d*₆) *δ* = 10.97 (s, 1H), 7.88 (s, 1H), 7.79 - 7.74 (m, 1H), 7.74 - 7.69 (m, 1H), 7.52 - 7.47 (m, 1H), 7.43 - 7.33 (m, 3H), 5.10 (dd, *J* = 5.0, 13.3 Hz, 1H), 4.49 - 4.40 (m, 3H), 4.35 - 4.28 (m, 1H), 4.12 - 4.05 (m, 2H), 3.95 - 3.88 (m, 2H), 3.29 (s, 3H), 2.97 - 2.86 (m, 1H), 2.60 (br dd, *J* = 2.1, 15.4 Hz, 1H), 2.43 - 2.34 (m, 1H), 2.04 - 1.96 (m, 1H). MS (ESI) m/z 449.1 [M+H]⁺

### Example 50. Synthesis of Compound 136

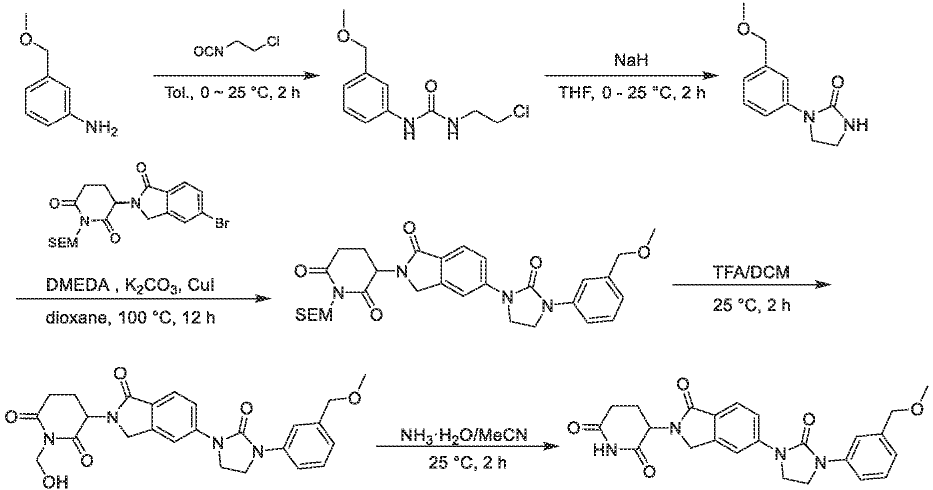

Step 1. To a solution of 3-(methoxymethyl)aniline (3.00 g, 21.9 mmol, 1.00 *eq*) in toluene (50.0 mL) was added 1-chloro-2-isocyanatoethane (2.38 g, 21.9 mmol, 1.92 mL, 97% purity, 1.00 *eq*) dropwise at 0 °C under nitrogen. The mixture was stirred at 25 °C for 2 h. The mixture was filtered to give 1-(2-chloroethyl)-3-(3-(rnethoxymethyl)phenyl)urea (3.00 g, curde) as a white solid. ¹H NMR (400 MHz, DMSO-*d*₆) *δ* = 8.65 (s, 1H), 7.39 (s, 1H), 7.30 - 7.25 (m, 1H), 7.21 - 7.15 (m, 1H), 6.84 (d, *J* = 7.6 Hz, 1H), 6.38 (t, *J* = 5.8 Hz, 1H), 4.34 (s, 2H), 3.69 - 3.62 (m, 2H), 3.45 - 3.39 (m, 2H), 3.27 (s, 3H).

Step 2. To a solution of 1-(2-chloroethyl)-3-(3-(methoxymethyl)phenyl)urea (3.00 g, 12.4 mmol, 1.00 *eq*) in tetrahydrofuran (20.0 mL) was added sodium hydride (741 mg, 18.5 mmol, 60% purity, 1.50 *eq*) at 0°C under nitrogen. The mixture was stirred at 25 °C for 2 h under nitrogen. The mixture was filtered and the filtrate was diluted with water (100 mL), extracted with ethyl acetate (3 × 100 mL). The combined organic layer was washed with brine (100 mL) and dried over sodium sulfate, filtered and concentrated to give crude product, which was purified by column chromatography (SiO₂, petroleum ether/ethyl acetate = 50/1 to 1/1) to afford 1-(3-(methoxymethyl)phenyl)imidazolidin-2-one (1.80 g, 8.73 mmol, 71% yield) as a white solid. ¹H NMR (400 MHz, DMSO-*d*₆) *δ* = 7.52 (s, 1H), 7.43 (br d, *J =* 8.3 Hz, 1H), 7.20 (t, *J* = 7.9 Hz, 1H), 6.82 (br d, *J* = 7.4 Hz, 1H), 4.34 (s, 2H), 3.72 (t, *J* = 7.9 Hz, 2H), 3.39 - 3.33 (m, 2H), 3.26 (s, 3H).

Step 3. To a solution of 1-(3-(methoxymethyl)phenyl)imidazolidin-2-one (45.5 mg, 220 umol, 1.00 *eq*)*,* 3-(5-bromo-1-oxcisoindolin-2-yl)-1-((2-(trimethylsilyl)ethoxy)methyl)piperidine-2,6-dione (100 mg, 220 umol, 1.00 *eq*), potassium carbonate (91.5 mg, 662 umol, 3.00 *eq*) in dioxane (2.00 mL) was added copper iodide (8.40 mg, 44.1 umol, 0.200 *eq*) and *N,N'*-dimethylethylenediamine (3.89 mg, 44.1 umol, 4.75 uL, *0.200 eq*). The mixture was stirred at 100 °C for 12 h. The mixture was concentrated under reduced pressure. The mixture was purified by reversed-phase HPLC (column: spherical C 18, 20-45 um, 100 Å, SW 80, mobile phase: [water(0.1%formic acid)-acetonitrile) and lyophilized to give 3-(5-(3-(3-(methoxymethyl)phenyl)-2-oxoimidazolidin-1-yl)-1-oxoisoindolin-2-yl)-1-((2-(trimethylsilyl)ethoxy)methyl)piperidine-2,6-dione (65.0 mg, 112 umol, 8% yield) as a white solid. ¹H NMR (400 MHz, DMSO-*d*₆) *δ* = 7.94 (s, 1H), 7.79 - 7.71 (m, 2H), 7.66 (s, 1H), 7.54 (br d, *J* = 8.1 Hz, 1H), 7.36 (t, *J* = 7.9 Hz, 1H), 7.04 (d, *J* = 7.5 Hz, 1H), 5.22 (dd, *J* = 5.1, 13.4 Hz, 1H), 5.06 (q, *J* = 9.7 Hz, 2H), 4.48 (d, *J* = 17.0 Hz, 1H), 4.43 (s, 2H), 4.35 - 4.25 (m, 1H), 4.08 - 3.97 (m, 4H), 3.59 - 3.48 (m, 2H), 3.31 - 3.30 (m, 3H), 3.13 - 3.00 (m, 1H), 2.80 (br d, *J* = 16.8 Hz, 1H), 2.42 - 2.31 (m, 1H), 2.12 - 1.99 (m, 1H), 0.89 - 0.80 (m, 2H), -0.02 (s, 9H).

Step 4. A solution of 3-(5-(3-(3-(methoxymethyl)phenyl)-2-oxoimidazolidin-1-yl)-1-oxoisoindolin-2-yl)-1-((2-(trimethylsilyl)ethoxy)methyl)piperidine-2,6-dione (60.0 mg, 104 umol, 1.00 *eq*) in trifluoroacetic acid (0.400 mL) and dichloromethane (2.00 mL) was stirred at 25 °C for 2 h. The mixture was concentrated under reduced pressure to give 1-(hydroxymethyl)-3-(5-(3-(3-(methoxymethyl)phenyl)-2-oxoimidazolidin-1-yl)-1-oxoisoindolin-2-yl)piperidine-2,6-dione (60 mg, curde) as brown oil. MS (ESI) m/z. 479.1 [M+H]⁺

Step 5. A solution of 1-(hydroxymethyl)-3-(5-(3-(3-(methoxymethyl)phenyl)-2-oxoimidazolidin-1-yl)-1-oxoisoindolin-2-yl)piperidine-2,6-dione (60 mg, 125.39 umol, 1.00 *eq*) in ammonium hydroxide (0.100 mL) and acetonitrile (2.00 mL) was stirred at 25 °C for 2 h. The mixture was added water (20.0 mL) and filtered to give a white solid. The solid was purified by reversed-phase HPLC (column: spherical C18, 20-45 um, 100Å, SW 40, mobile phase: [water(O. 1%formic acid)-acetonitrile]) and lyophilized to give 3-(5-(3-(3-(methoxymethyl)phenyl)-2-oxoimidazolidin-1-yl)-1-oxoisoindolin-2-yl)piperidine-2,6-dione (20.7 mg, 45.2 umol, 36% yield, 98% purity) as a white solid.

¹H NMR (400 MHz, DMSO-*d*₆) *δ* = 10.97 (br s, 1H), 7.93 (s, 1H), 7.80 - 7.70 (m, 2H), 7.66 (s, 1H), 7.54 (br d, *J* = 8.3 Hz, 1H), 7.36 (t, *J* = 7.9 Hz, 1H), 7.04 (d, *J* = 7.4 Hz, 1H), 5.10 (dd, *J* = 5.1, 13.2 Hz, 1H), 4.50 - 4.42 (m, 3H), 4.36 - 4.29 (m, 1H), 4.09 - 4.00 (m, 4H), 3.31 (s, 3H), 2.98 - 2.86 (m, 1H), 2.62 - 2.56 (m, 1H), 2.45 - 2.35 (m, 1H), 2.08 - 1.95 (m, 1H). MS (ESI) m/z 449.1 [M+H]⁺

### Example 51. Synthesis of Compound 139

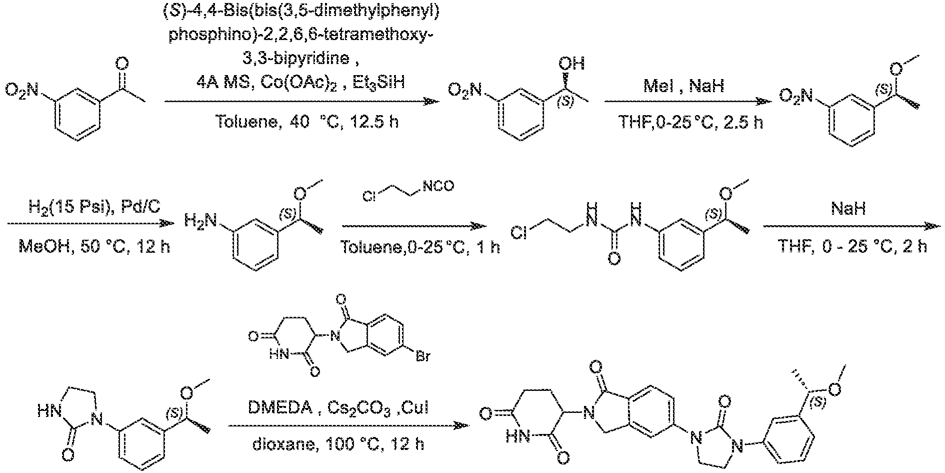

Step 1. To a solution of diacetoxycobalt (128 mg, 726 umol, 0.0600 *eq*), 4A MS (2.00 g) and (*S*)-4,4-Bis(bis(3,5-dimethylphenyl) (100 mg, 242 umol, 0.0200 *eq*) in toluene (20.0 mL) was stirred at 40 °C for 0.5 h. Then the reaction mixture was added 1-(3-nitrophenyl)ethanone (2.00 g, 12.1 mmol, 1.00 *eq*) and phenylsilane (1.57 g, 14.5 mmol, 1.79 mL, 1.20 *eq*). Then the reaction mixture was stirred at 40 °C for 12 h. The reaction mixture was quenched by addition hydrochloric acid (1M, 100 mL)and extracted with ethyl acetate (3 × 50 mL). The combined organic layers were washed with brine (3 × 20 mL), dried over anhydrous sodium sulfate, filtered and concentrated under reduced pressure to give a residue. The residue was purified by column chromatography (SiO2, Petroleum ether/Ethyl acetate = 20/1 to 5/1) and concentrated in vacuum to afford (*S*)-1-(3-nitrophenyl)ethanol (1.50 g, 8.88 mmol, 73% yield, 99% purity) as a white solid. ¹H NMR (400 MHz, CDCl₃) *δ* = 8.26 (s, 1H), 8.14 (dd, *J* = 1.2, 8.1 Hz, 1H), 7.73 (d, *J* = 7.6 Hz, 1H), 7.53 (t, *J* = 8.0 Hz, 1H), 5.04 (q, *J* = 6.4 Hz, 1H), 1.55 (d, *J* = 6.4 Hz, 3H).

Step 2. To a solution of (*S*)-1-(3-nitrophenyl)ethanol (1.40 g, 8.38 mmol, 1.00 *eq*) in tetrahydrofuran (15.0 mL) was added sodium hydride (502 mg, 12.5 mmol, 60% purity, *1.50 eq*) at 0 °C. Then the reaction mixture was stirred at 25 °C for 0.5 h under nitrogen atmosphere. Then the reaction mixture was added methyl iodide (3.57 g, 25.1 mmol, 1.56 mL, 3.00 *eq*) at 0 °C. Then the reaction mixture was stirred at 25 °C for 2 h under nitrogen atmosphere. The reaction mixture was quenched by addition saturated ammonium chloride (100 mL) and extracted with ethyl acetate (3 × 100 mL). The combined organic layers were washed with brine (20 mL), dried over anhydrous sodium sulfate, filtered and concentrated under reduced pressure to give a residue. The residue was purified by column chromatography (SiO₂, Petroleum ether/Ethyl acetate = 30/1 to 10/1) and concentrated in vacuum to afford (*S*)-1-(1-methoxyethyl)-3-nitrobenzene(1.50 g, 8.11 mmol, 96% yield, 98% purity) as yellow oil. 1H NMR (400 MHz, CDCl₃) *δ* = 8.19 (s, 1H), 8.15 (br d, J = 8,0 Hz, 1H), 7.67 (d, J = 7.6 Hz, 1H), 7.56 - 7.51 (m, 1H), 4.42 (q, J = 6.4 Hz, 1H), 3.28 (s, 3H), 1.47 (d, J = 6.4 Hz, 3H).

Step 3. To a solution of (S)-1-(1-methoxyethyl)-3-nitrobenzene (1.50 g, 8.28 mmol, 1.00 *eq*) in methanol (75.0 mL) was added palladium on activated carbon (750 mg, 10% purity). Then the reaction mixture was stirred at 50 °C for 12 h under hydrogen atmosphere (15 Psi). The reaction mixture was filtered. The filtrate was concentrated in vacuum to afford (S)-3-(1-methoxyethyl)aniline(1.20 g, 7.14 mmol, 86% yield, 90% purity) as yellow oil. ¹H NMR (400 MHz, DMSO-*d*₆) *δ* = 6.98 (t, *J* = 7.6 Hz, 1H), 6.51 (s, 1H), 6.47 - 6.40 (m, 2H), 5.03 (s, 2H), 4.11 (q, *J* = 6.4 Hz, 1H), 3.09 (s, 3H), 1.28 (d, *J* = 6.4 Hz, 3H).

Step 4. To a solution of (*S*)-3-(1-methoxyethyl)aniline (600 mg, 3.97 mmol, 1.00 *eq*) in toluene (10.0 mL) was added 1-chloro-2-isocyanato-ethane (628 mg, 5.95 mmol, 506 uL, 1.50 *eq*) at 0 °C. Then the reaction mixture was stirred at 25 °C for 2 h. The reaction mixture was concentrated in vacuum to afford (*S*)-1-(2-chloroethyl)-3-(3-(1-methoxyethyl)phenyl)urea (900 mg, 3.16 mmol, 79% yield, 90% purity) as a yellow solid.

MS (ESI) m/z. 257.1 [M+H]⁺

Step 5. To a solution of (*S*)-1-(2-chloroethyl)-3-(3-(1-methoxyethyl)phenyl)urea (900 mg, 3.51 mmol, 1.00 *eq*) in tetrahydrofuran (10.0 mL) was added sodium hydride (210 mg, 5.26 mmol, 60% purity, 1.50 *eq*) at 0 °C . Then the reaction mixture was stirred at 25 °C for 2 h under nitrogen atmosphere. The reaction mixture was quenched by addition saturated ammonium chloride (100 mL) and extracted with ethyl acetate (3 × 50 mL). The combined organic layers were washed with brine (3 × 20 mL), dried over anhydrous sodium sulfate, filtered and concentrated under reduced pressure to give a residue to afford (*S*)-1-(3-(1-methoxyethyl)phenyl)imidazolidin-2-one (700 mg, 2.99 mmol, 85% yield, 94% purity) as a yellow solid. ¹H NMR (400 MHz, DMSO-*d*₆) *δ* = 7.53 (s, 1H), 7.35 (dd, *J* = 1.3, 8.3 Hz, 1H), 7.08 (t, *J* = 7.8 Hz, 1H), 6.61 (d, *J* = 7.4 Hz, 1H), 4.16 (q, *J* = 6.4 Hz, 1H), 3.54 - 3.49 (m, 2H), 3.34 - 3.29 (m, 2H), 3.09 (s, 3H), 1.30 (d, *J* = 6.4 Hz, 3H).

Step 6. To a solution of (*S*)-1-(3-(1-methoxyethyl)phenyl)imidazolidin-2-one (34.0 mg, 154 umol, 1.00 *eq*) and 3-(5-bromo-1-oxo-isoindolin-2-yl)piperidine-2,6-dione (50.0 mg, 154 umol, 1.00 *eq*) in dioxane (2.00 mL) was added cesium carbonate (100 mg, 309 umol, 2.00 *eq*)*,* copper iodide (29.4 mg, 154 umol, 1.00 *eq*) and *N¹,N²*-dimethylethane-1,2-diamine (13.6 mg, 154 umol, 16.6 uL, 1.00 *eq*). Then the reaction mixture was stirred at 100 °C for 12 h under nitrogen atmosphere. The reaction mixture was adjust pH to 5-6 by formic acid (0.2 mL) and concentrated in vacuum. The residue was added *N,N*-dimethyl formamide (7.00 mL) and filtered. The filtrate was purified by *Prep*-HPLC (column: Phenomenex Luna C18 150*25mm* 10um;mobile phase: [water(FA)-ACN];B%: 28%-58%,10min) and lyophilized to afford 3-(5-(3-(3-((S)-1-methoxyethyl)phenyl)-2-oxoimidazolidin-1-yl)-1-oxoisoindolin-2-yl)piperidine-2,6-dione(42.35 mg, 86.0 umol, 5% yield, 94% purity) as a white solid.

¹H NMR (400 MHz, DMSO-*d*₆) *δ* = 10.98 (br s, 1H), 7.94 (s, 1H), 7.80 - 7.75 (m, 1H), 7.73 - 7.70 (m, 1H), 7.67 (s, 1H), 7.49 (dd, *J* = 1.4, 8.2 Hz, 1H), 7.37 (t, *J =* 7.9 Hz, 1H), 7.03 (d, *J* = 7.5 Hz, 1H), 5.10 (dd, *J* = 5.1, 13.3 Hz, 1H), 4.48 - 4.42 (m, 1H), 4.34 (d, *J* = 6.9 Hz, 1H), 4.31 (d, *J* = 3.9 Hz, 1H), 4.04 (br s, 4H), 3.14 (s, 3H), 2.97 - 2.87 (m, 1H), 2.63 - 2.58 (m, 1H), 2.40 (br d, *J* = 8.8 Hz, 1H), 2.04 - 1.98 (m, 1H), 1.35 (d, *J* = 6.4 Hz, 3H). MS (ESI) m/z. 463.2 [M+H]⁺

### Example 52. Synthesis of Compound 149

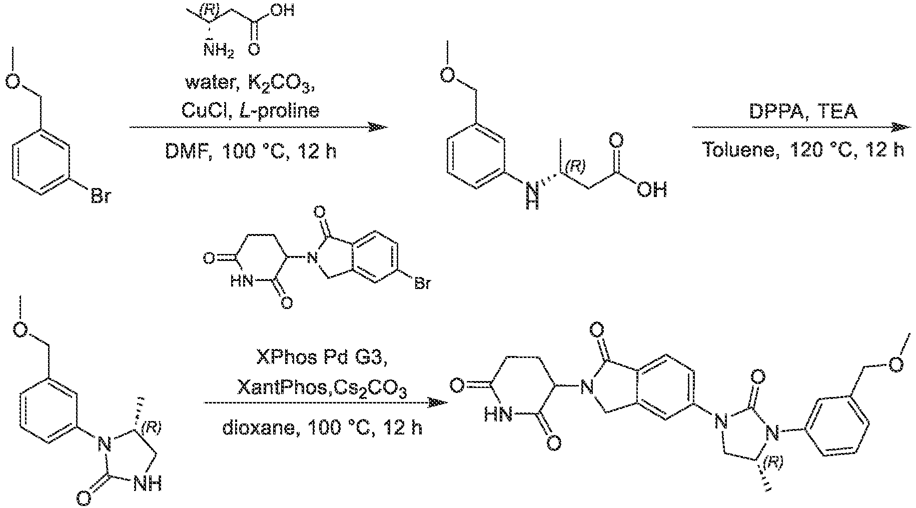

Step 1. To a solution of 1-bromo-3-(methoxymethyl)benzene (1.00 g, 4.97 mmol, 1.00 *eq*), (3*R*)-3-aminobutanoic acid (769 mg, 7.46 mmol, 1.50 *eq*), potassium carbonate (1.72 g, 12.4 mmol, 2.50 *eq*), water (224 mg, 12.4 mmol, 224 uL, 2.50 *eq*) in *N,N-*dimethyformamide (8.00 mL) was added cuprous chloride (49.2 mg, 497 umol, 11.9 uL, *0.100 eq*) and L-proline (57.3 mg, 497 umol, 0.100 *eq*) under nitrogen atmosphere. The mixture was stirred at 100 °C for 12 h. The mixture was filtered. The mixture was purified by reversed phase HPLC (column: spherical C 18, 20-45 um, 100 Å, SW 120, mobile phase: [water (0.1%Formic Acid)-acetonitrile) and lyophilized to get (*R*)-3-((3-(methoxymethyl)phenyl)amino)butanoic acid (650 mg, 2.91 mmol, 58% yield) as brown oil. ¹H NMR (400 MHz, DMSO-*d*₆) *δ* = 7.03 (br t, *J* = 7.2 Hz, 1H), 6.54 - 6.38 (m, 3H), 4.27 (s, 2H), 3.89 - 3.67 (m, 2H), 3.25 (s, 3H), 2.45 - 2.36 (m, 1H), 1.13 (br s, 3H). MS (ESI) m/z 224.3 [M+H]⁺

Step 2. To a solution of (*R*)-3-((3-(methoxymethyl)phenyl)amino)butanoic acid (600 mg, 2.69 mmol, 1.00 *eq*) in toluene (10.0 mL) was added diphenylphosphoryl azide (2.22 g, 8.06 mmol, 1.75 mL, 3.00 *eq*) and triethylamine (326 mg, 3.22 mmol, 449 uL, 1.20 *eq*). The mixture was stirred at 120 °C for 12 h under nitrogen atmosphere. The mixture was concentrated under reduced pressure to get a residue. The residue was purified by column chromatography (SiO₂, Petroleum ether/Ethyl acetate=5/1 to 1/1) to give (*R*)-1-(3-(methoxymethyl)phenyl)-5-methylimidazolidin-2-one (420 mg, crude) as yellow oil. ¹H NMR (400 MHz, DMSO-d₆) δ = 7.44 (s, 1H), 7.38 - 7.34 (m, 1H), 7.31 - 7.26 (m, 1H), 6.96 (d, *J =* 7.4 Hz, 1H), 6.84 (s, 1H), 4.50 - 4.43 (m, 1H), 4.38 (s, 2H), 3.56 (t, *J* = 8.7 Hz, 1H), 3.28 (s, 3H), 2.98 (br s, 1H), 1.19 - 1.16 (m, 3H).

Step 3. To a solution of (*R*)-1-(3-(methoxymethyl)phenyl)-5-methylimidazolidin-2-one (53.2 mg, 241 umol, 1.30 *eq*), 3-(5-bromo-1-oxo-isoindolin-2-yl)piperidine-2,6-dione (60.0 mg, 186 umol, 1.00 *eq*), cesium carbonate (121 mg, 371 umol, 2.00 *eq*) in dioxane (2.00 mL) was added methanesulfonato(2-dicyclohexylphosphino-2,4,6-tri-i-propyl-1,1-biphenyl)(2-amino-1,1-biphenyl-2-yl)palladium(H) (15.7 mg, 18.6 umol, 0.100 *eq*) and 4,5-bis(diphenylphosphino)-9,9-dimethylxanthene (21.5 mg, 37.1 umol, 0.200 *eq*)*.* The mixture was stirred at 100 °C for 12 h under nitrogen atmosphere. The mixture was adjusted to pH = 5-6 with formic acid and filtered. The filtrate was purified by reversed phase HPLC (column: spherical C 18, 20-45 um, 100 Å, SW 120, mobile phase: [water (0.1%Formic Acid)-acetonitrile) and lyophilized to get a residue. The residue was purified by reversed phase HPLC (column: spherical C 18, 20-45 um, 100 Å, SW 40, mobile phase: [water (0.1%Formic Acid)-acetonitrile) and lyophilized to afford 3-(5-((*R*)-3-(3-(methoxymethyl)phenyl)-4-methyl-2-oxoimidazolidin-1-yl)-1-oxoisoindolin-2-yl)piperidine-2,6-dione (22.5 mg, 46.6 umol, 6% yield, 96% purity) as a yellow solid. ¹H NMR (400 MHz, DMSO-d₆) δ = 10.97 (br s, 1H), 7.92 - 7.88 (m, 1H), 7.78 - 7.73 (m, 1H), 7.72 - 7.69 (m, 1H), 7.53 (s, 1H), 7.45 - 7.41 (m, 1H), 7.41 - 7.36 (m, 1H), 7.10 (d, *J* = 7.2 Hz, 1H), 5.10 (dd, *J* = 4.8, 13.2 Hz, 1H), 4.69 - 4.59 (m, 1H), 4.47 - 4.41 (m, 3H), 4.35 - 4.28 (m, 1H), 4.27 - 4.20 (m, 1H), 3.67 - 3.61 (m, 1H), 3.31 (s, 3H), 2.97 - 2.86 (m, 1H), 2.63 - 2.57 (m, 1H), 2.43 -2.38 (m, 1H), 2.04 - 1.96 (m, 1H), 1.27 (d, *J* = 6.0 Hz, 3H.

¹H NMR (400 MHz, CDCl₃) δ = 8.15 (br d, *J* = 3.2 Hz, 1H), 7.98 (br s, 1H), 7.87 (d, *J* = 8.4 Hz, 1H), 7.51 - 7.46 (m, 1H), 7.42 - 7.36 (m, 3H), 7.17 (br d, *J* = 6.4 Hz, 1H), 5.22 (br dd, *J* = 5.6, 13.2 Hz, 1H), 4.59 - 4.52 (*m,* 1H), 4.50 (s, 2H), 4.46 (s, 1H), 4.37 - 4.31 (m, 1H), 4.24 - 4.16 (m, 1H), 3.65 - 3.58 (m, 1H), 3.42 (s, 3H), 2.98 - 2.82 (m, 2H), 2.44 - 2.32 (m, 1H), 2.28 - 2.19 (m, 1H), 1.41 (d, *J* = 6.0 Hz, 3H). MS (ESI) m/z 463.1 [M+H]⁺

### Example 53. Synthesis of Compound 134

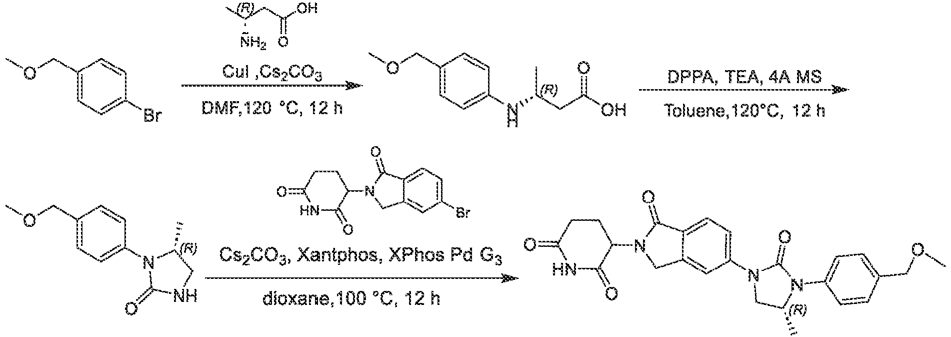

Step 1. To a solution of 1-bromo-4-(methoxymethyl)benzene (2.00 g, 9.95 mmol, 1.00 *eq*) in dimethyl formamide (2.00 mL) was added (*R*)-3-aminobutanoic acid (2.05 g, 19.8 mmol, 2.00 *eq*), cesium carbonate (6.48 g, 19.8 mmol, 2.00 *eq*) and copper iodide (189 mg, 994 umol, 0.100 *eq*) under nitrogen atmosphere. The mixture was stirred at 120 °C for 12 h. The reaction mixture was concentrated under reduced pressure to give a residue. The residue was purified by reversed-phase HPLC (column: spherical C18, 20-45 um, 100Å, SW 120, mobile phase: [water-acetonitrile]) and lyophilized to give (*R*)-3-((4-(methoxymethyl)phenyl)amino) butanoic acid (3.20 g, crude) as a brown oil.

Step 2. To a solution of (*R*)-3-((4-(methoxymethyl)phenyl)amino)butanoic acid (3.20 g, 14.3 mmol, 1.00 *eq*) in toluene (10.0 mL) was added 4A MS (2.00 g), diphenylphosphoryl azide (11.8 g, 43.0 mmol, 9.32 mL, 3.00 *eq*) and triethylamine (4.35 g, 43.0 mmol, 5.98 mL, 3.00 *eq*) under nitrogen, the mixture was stirred at 120 °C for 12 h, The reaction mixture was diluted with water (30 mL) and exacted with ethyl acetate (2 × 30 mL). The organic phase was separated, washed with brine (10 mL), dried over sodium sulfate, filtered and concentrated under reduced pressure to give a residue. The residue was purified by column chromatography (SiO₂, Petroleum ether/Ethyl acetate = 8/1 to 0/1) to give (*R*)-1-(4-(methoxymethyl) phenyl)-5-methylimidazolidin-2-one (1.80 g, 5.72 mmol, 39% yield, 70% purity) as a white solid.

Step 3. To a solution of (*R*)-1-(4-(methoxymethyl)phenyl)-5-methylimidazolidin-2-one (50.0 mg, 227 umol, 1.00 *eq*) in dioxane (1.00 mL) was added 3-(5-bromo-1-oxoisoindolin-2-yl)piperidine-2,6-dione (88.0mg, 272 umol, 1.20 *eq*), cesium carbonate (73.9 mg, 227 umol, 1.00 *eq*), 4,5-bis(diphenylphosphino)-9,9-dimethylxanthene (26.2 mg, 45.4 umol, 0.200 *eq*), and methanesulfonato(2-dicyclohexylphosphino-2,4,6-tri-i-propyl-1, 1-biphenyl)(2-amino-1,1-biphenyl-2-yl)palladium(II) (19.2 mg, 22.7 umol, 0.100 *eq)* under nitrogen atmosphere. The mixture was stirred at 100 °C for 12 h. The reaction mixture was concentrated under reduced pressure to give a residue. The residue was purified by reversed-phase HPLC (column: spherical C18, 20-45 um, 100Å, SW 80, mobile phase: [water(0.1%Formic Acid)-ACN]) and lyophilized to give 3-(5-((R)-3-(4-(methoxymethyl)phenyl)-4-methyl-2-oxoimidazolidin-1-yl)-1-oxoisoindolin-2-yl)piperidine-2,6-dione (500 mg, 108 umol, 16% yield) as a white solid. ¹H NMR (400 MHz, DMSO-d₆) δ = 10.98 (s, 1H), 7.88 (br d, *J* = 4.9 Hz, 1H), 7.75 (br dd, *J* = 1.8, 5.2 Hz, 1H), 7.73 - 7.68 (m, 1H), 7.52 (d, *J* = 8.5 Hz, 2H), 7.35 (d, *J* = 8.5 Hz, 2H), 5.10 (dd, *J* = 5.1, 13.3 Hz, 1H), 4.63 (td, *J* = 5.8, 8.8 Hz, 1H), 4.50 - 4.41 (m, 1H), 4.40 (s, 2H), 4.35 - 4.28 (m, 1H), 4.24 (dt, *J* = 2.0, 9.1 Hz, 1H), 3.68 - 3.61 (m, 1H), 3.30 (s, 3H), 2.97 - 2.85 (m, 1H), 2.68 - 2.58 (m, 1H), 2.42 - 2.32 (m, 1H), 2.05 - 1.95 (m, 1H), 1.27 (d, *J* = 6.1 Hz, 3H). MS (ESI) m/z 463.1 [M+H]⁺

### Example 54. Synthesis of Compound 150

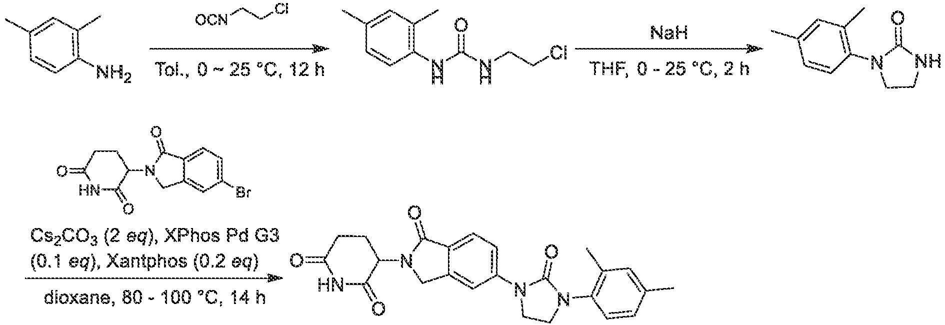

Step 1. To a mixture of 2,4-dimethylaniline (3.00 g, 24.8 mmol, 1.00 *eq)* in toluene (30.0 mL) was added 1-chloro-2-isocyanato-ethane (3.92 g, 37.1 mmol, 3.16 mL, *1.50 eq*) at 0 °C. The mixture was stirred at 25 °C for 12 h. The mixture was filterted. The filter cake was concentrated in vacuum to give 1-(2-chloroethyl)-3-(2,4-dimethylphenyl)urea (6.80 g, crude) as a white solid. ¹H NMR (400 MHz, DMSO-d₆) *δ =* 7.72 (s, 1H), 7.59 (d, *J* = 8.1 Hz, 1H), 6.93 (s, 1H), 6.89 (br d, *J* = 8.3 Hz, 1H), 6.71 (br t, *J* = 5.6 Hz, 1H), 3.65 (t, *J* = 6.1 Hz, 2H), 3.41 (q, *J* = 6.0 Hz, 2H), 2.20 (s, 3H), 2.13 (s, 3H).

Step 2. To a mixture of 1-(2-chloroethyl)-3-(2,4-dimethylphenyl)urea (2.00 g, 8.82 mmol, 1.00 *eq*) in tetrahydrofuran (20.0 mL) was added sodium hydride (529 mg, 13.2 mmol, 60% purity, 1.50 *eq*) at 0 °C. The mixture was stirred at 25 °C for 2h. The reaction mixture was quenched by addition saturated ammonium chloride (80.0 mL) aquenous solution, then the mixture was filterted. The filter cake was concentrated in vacuum. The residue was purified by column chromatography (Si02, Petroleum ether/Ethyl acetate = 10/1 to 0/1) and concentrated in vacuum to give 1-(2,4-dimethylphenyl)imidazolidin-2-one (850 mg, 4.47 mmol, 50% yield) as a white solid. ¹H NMR (400 MHz, DMSO-*d*₆) δ = 7.07 - 7.02 (m, 2H), 7.01 - 6.93 (m, 1H), 6.57 (s, 1H), 3.68 - 3.62 (m, 2H), 3.42 - 3.38 (m, 2H), 2.25 (s, 3H), 2.15 (s, 3H).

Step 3. To a mixture of 1-(2,4-dimethylphenyl)imidazolidin-2-one (300 mg, 1.58 mmol, 1.00 *eq*) and 3-(5-bromo-1-oxoisoindolin-2-yl)piperidine-2,6-dione (611 mg, 1.89 mmol, 1.20 *eq*) in dioxane (1.00 mL) was added cesium carbonate (1.03 g, 3.15 mmol, 2.00 eq), (2-dicyclohexylphosphino-2',4',6'-triisopropyl-1,1'-biphenyl)[2-(2'-amino-1,1'-biphenyl)]palladium(ii) methanesulfonate (133 mg, 158 umol, 0.100 *eq*) and 4,5-bis(diphenylphosphino)-9,9-dimethylxanthene (182 mg, 315 umol, 0.200 *eq*). The mixture was stirred at 80 °C for 12 h. The mixture was stirred at 100 °C for 2 h under nitrogen. The mixture was concentrated in vacuum. Then the residue was dissolved in dimethyformamide and filterted. The filtrate was purified by reversed-phase HPLC (column: spherical C18, 20-45 um, 100Å, SW 120, mobile phase: [water(0.1%Formic Acid)-ACN) and lyophilized to give 3-(5-(3-(2,4-dimethylphenyl)-2-oxoimidazolidin-1-yl)-1-oxoisoindolin-2-yl)piperidine-2,6-dione (132.52 mg, 312 umol, 19% yield) as a white solid. ¹H NMR (400 MHz, DMSO-*d*₆) δ = 10.96 (br s, 1H), 7.87 (s, 1H), 7.75 (br d, *J* = 1.6 Hz, 1H), 7.72 - 7.66 (m, 1H), 7.22 (d, *J* = 8.0 Hz, 1H), 7.12 (s, 1H), 7.07 (br d, *J* = 8.3 Hz, 1H), 5.09 (dd, *J* = 5.1, 13.3 Hz, 1H), 4.48 - 4.40 (m, 1H), 4.35 - 4.26 (m, 1H), 4.11 - 4.02 (m, 2H), 3.89 - 3.82 (m, 2H), 2.91 (br s, 1H), 2.63 - 2.56 (m, 1H), 2.39 (br dd, *J =* 4.4, 13.1 Hz, 1H), 2.29 (s, 3H), 2.20 (s, 3H), 2.04 - 1.97 (m, 1H).

### Example 55. Synthesis of Compound 167

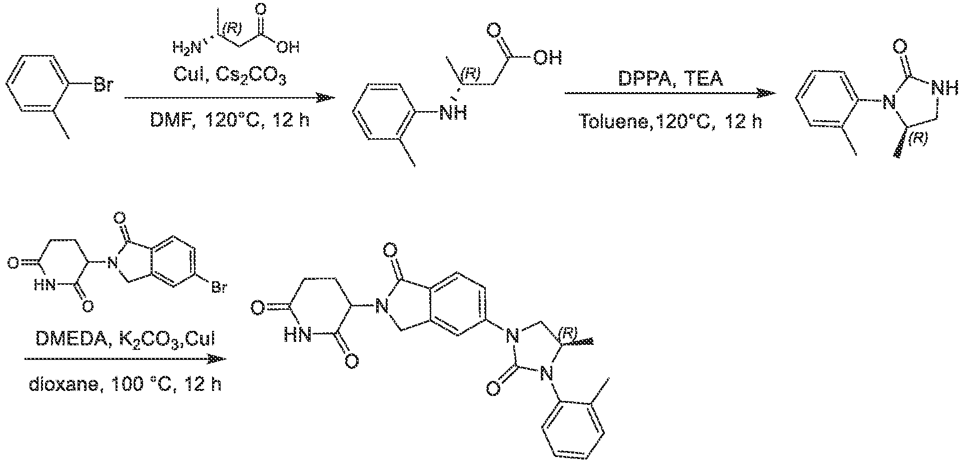

Step 1. To a solution of 1-bromo-2-methylbenzene (1.00 g, 5.85 mmol, 704 uL, 1.00 *eq*) and (*R*)-3-(o-tolylarnino)butanoic acid (1.21 g, 11.7 mmol, 2.00 *eq)* in dimethylformamide (10.0 mL) was added cesium carbonate (3.81 g, 11.7 mmol, 2.00 *eq)* and copper iodide (223 mg, 1.17 mmol, 0.200 *eq*). The mixture was stirred at 120 °C for 12 h under nitrogen atmosphere. The mixture was filterted. The filtrate was concentrated in vacuum. The residue was dissolved in water (20.0 mL). The crude product was purified by reversed phase (0.1% ammonium hydroxide) and lyophilized to give (*R*)-3-(o-tolylamino)butanoic acid (8.30 g, crude) as blue oil. MS (ESI) m/z 192.1 [M-H]⁺

Step 2. To a solution of (*R*)-3-(o-tolylamino)hutanoic acid (1.00 g, 5.17 mmol, *1.00 eq*) in toluene (10.0 mL) was added diphenylphosphoryl azide (1.42 g, 5.17 mmol, 1.12 mL, 1.00 *eq*) and triethylamine (524 mg, 5.17 mmol, 720 uL, 1.00 *eq*), molecular sieves, 13x (2.00 g, 5.17 mmol, 1.00 *eq*). The mixture was stirred at 120 °C for 12 h. The mixture was filtered and concentrated in vacuum. The crude product was purified by reversed-phase (column: spherical C18, 20-45 um, 120A, SW 120, mobile phase: [water(0.1%Formic Acid)-ACN) and lyophilized to give (*R*)-5-methyl-1-(o-tolyl)imidazolidin-2-one (300 mg, 1.58 mmol, 30% yield) as a white solid. ¹H NMR (400 MHz, DMSO-*d*₆) δ = 7.27 - 7.12 (m, 4H), 6.57 (br s, 1H), 4.21 - 4.12 (m, 1H), 3.60 - 3.54 (m, 1H), 3.01 (dt, J= 1.3, 8.5 Hz, 1H), 2.18 (s, 3H), 1.03 (d, J = 6.1 Hz, 3H).

Step 3. To a mixture of (*R*)-5-methyl-1-(o-tolyl)imidazolidin-2-one (50.0 mg, 263 umol, 1.00 *eq*) and 3-(5-bromo-1-oxoisoindolin-2-yl)piperidine-2,6-dione (119 mg, 368 umol, 1.40 *eq)* in dioxane (1.00 mL) was added cesium carbonate (171 mg, 526 umol, 2.00 *eq*), *N*,*N*'-dimethylethylenediamine(11.6 mg, 131 umol, 14.1 uL, 0.500 *eq*) and copper iodide (10.0 mg, 52.6 umol, 0.200 *eq*). The mixture was stirred at 100 °C for 12 h under nitrogen atmosphere. The mixture was filtered. The crude product was purified by reversed phase (0.1% formic acid condition), *Prep*-HPLC (column: Phenomenex luna C18 150*25mm* 10um; mobile phase: [water(Formic Acid)-ACN]; B%: 25%-55%, 8min ) and lyophilized to give 3-(5-((*R*)-4-methyl-2-oxo-3-(o-tolyl)imidazolidin -1-yl)-1-oxoisoindolin-2-yl)piperidine-2,6-dione (18.12 mg, 46.2 umol, 3% yield) as a white solid. ¹H NMR (400 MHz, DMSO-*d*₆) *δ* = 10.97 (br s, 1H), 7.86 (s, 1H), 7.78 - 7.74 (m, 1H), 7.71 - 7.67 (m, 1H), 7.34 - 7.26 (m, 4H), 5.09 (br dd, *J* = 5.0, 13.1 Hz, 1H), 4.46 - 4.26 (m, 4H), 3.66 (br t, *J* = 7.6 Hz, 1H), 2.93 - 2.86 (m, 1H), 2.63 - 2.57 (m, 1H), 2.39 (br dd, *J* = 4.2, 13.1 Hz, 1H), 2.24 (s, 3H), 2.03 - 1.97 (m, 1H), 1.16 (br d, *J* = 6.0 Hz, 3H). MS (ESI) m/z 433.1 [M+H]⁺

### Example 56. Synthesis of Compound 182

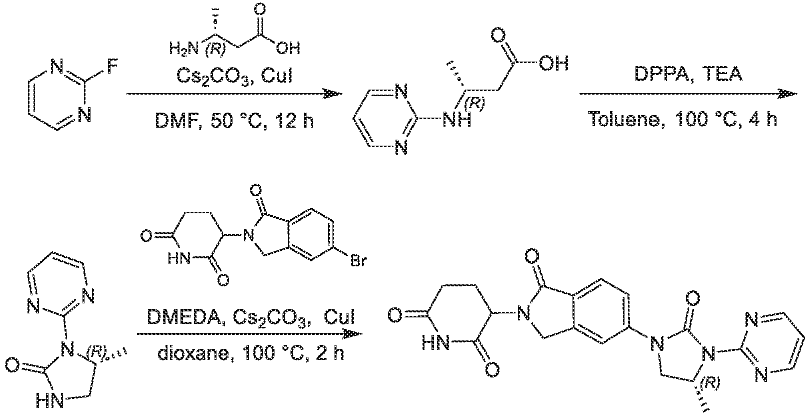

Step 1. To a mixture of 2-fluoropyrimidine (2.00 g, 20.4 mmol, 1.00 *eq*) and (*R*)-3-aminobutanoic acid (4.21 g, 40.7 mmol, 2.00 *eq*) in dimethyformamide (20.0 mL) was added cesium carbonate (13.3 g, 40.8 mmol, 2.00 *eq*) and cuprous iodide (776 mg, 4.08 mmol, 0.200 *eq).* The mixture was stirred at 50 °C for 12 h under nitrogen. The mixture was filterted. The filtrate was concentrated in vacuum. The residue was dissolved in water (20 mL). The crude product was purified by reversed phase (0.1% ammonium hydroxide) and lyophilized to give (*R*)-3-(pyrimidin-2-ylamino)butanoic acid (3.50 g, crude) as blue oil. ¹H NMR (400 MHz, DMSO-*d*₆) δ = 8.21 (br s, 2H), 7.52 (br s, 1H), 6.47 (br s, 1H), 4.13 (br s, 1H), 2.20 (br s, 2H), 1.14 (br s, 3H).

Step 2. To a mixture of (*R*)-3-(pyrimidin-2-ylamino)butanoic acid (3.50 g, 19.3 mmol, 1.00 *eq*) in toluene (30.0 mL) was added molecular sieves, 13 x (1.00 g), triethylamine (5.86 g, 57.9 mmol, 8.07 mL, 3.00 *eq)* and diphenylphosphoryl azide (15.9 g, 57.9 mmol, 12.5 mL, 3.00 *eq*). The mixture was stirred at 100 °C for 4 h under nitrogen. The reaction mixture was filterted and was washed with saturated sodium bicarbonate (50 mL) aquenous solution. The reaction mixture was diluted with saturated sodium bicarbonate aquenous solution (100 mL) and extracted with ethyl acetate (3 × 200 mL). The combined organic layers were washed with brine (200 mL), dried over sodium sulfate, filtered and concentrated under reduced pressure to give a residue. The residue was purified by reversed phase (0.1% ammonium hydroxide) and lyophilized. The reversed phase collection was purified by reversed phase (0.1% formic acid condition) and lyophilized to give (*R*)-5-methyl-1-(pyrimidin-2-yi)imidazolidin-2-one (170 mg, 954 µmol, 4% yield) as a yellow solid. MS (ESI) m/z 179.0 [M+H]⁺

Step 3. To a mixture of (*R*)-5-methyl-1-(pyrimidin-2-yl)imidazolidin-2-one (30.0 mg, 168 µmol, 1.00*eq*) and 3-(5-bromo-1-oxoisoindolin-2-yl)piperidine-2,6-dione (54.4 mg, 168 µmol, 1.00 *eq*) in dioxane (1.00 mL) was added cesium carbonate (164 mg, 505 µmol, *3.00 eq*)*,* n,n'-dimethylethylenediamine (7.42 mg, 84.2 µmol, 9.06 µL, 0.500 *eq)* and cuprous iodide (6.41 mg, 33.7 µmol, 0.200 *eq*). The mixture was stirred at 100 °C for 2 h under nitrogen. The reaction mixture was concentrated in vacuum. The crude product was dissolved in dimethyformamide (3.00 mL) and filterted. The crude product was purified by *prep*-HPLC (column: Phenomenex luna C18 150*25mm* 10um;mobile phase: [water(formic acid)- acetonitrile];B%: 8%-38%, 10min) and lyophilized to give 3-(5-((*R*)-4-methyl-2-oxo-3-(pyrimidin-2-yl)imidazolidin-1-yl)-1-oxoisoindolin-2-yl)piperidine-2,6-dione (8.66 mg, 20.6 µmol, 2 % yield) as as a white solid.

¹H NMR (400 MHz, DMSO-*d*₆) *δ* = 10.98 (br s, 1H), 8.73 (d, *J* = 4.8 Hz, 2H), 7.90 (br d, *J* = 4.3 Hz, 1H), 7.80 - 7.70 (m, 2H), 7.21 (t, *J* = 4.8 Hz, 1H), 5.11 (dd, *J* = 5.1, 13.3 Hz, 1H), 4.79 - 4.69 (m, 1H), 4.50 - 4.42 (m, 1H), 4.36 - 4.30 (m, 1H), 4.23 (dt, *J* = 2.3, 8.9 Hz, 1H), 3.71 - 3.59 (m, 1H), 2.98 - 2.87 (m, 1H), 2.63 - 2.57 (m, 1H), 2.40 - 2.34 (m, 1H), 2.03 - 1.96 (m, 1H), 1.42 (d, *J* = 6.1 Hz, 3H). MS (ESI) m/z 421.2 [M+H]⁺

### Example 57. Synthesis of Compound 132

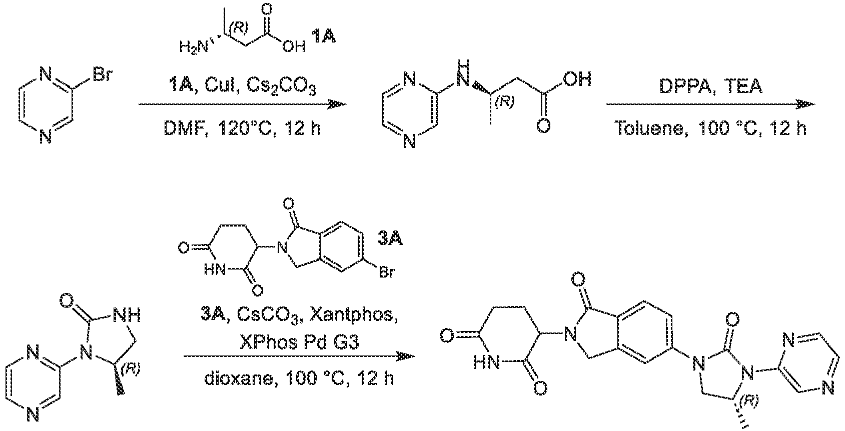

Step 1. To a solution of 2-bromopyrazine (1.00 g, 6.29 mmol, 1.00 *eq*) and (*R*)-3-aminobutanoic acid (778 mg, 7.55 mmol, 1.20 *eq*) in dimethyl formamide (10.0 mL) was added copper iodide (240 mg, 1.26 mmol, 0.200 *eq*) and cesium carbonate (4.10 g, 12.6 mmol, 2.00 *eq*), the mixture was stirred at 120 °C for 1'' h. The reaction mixture was concentrated under reduced pressure to give a residue. The residue was purified by reversed - phase HPLC (0.1% formic acid condition) to give (*R*)-3-(pyrazin-2-ylamino) butanoic acid (10.0 g, crude) as a white solid.

Step 2. To a solution of (*R*)-3-(pyrazin-2-ylamino)butanoic acid (8.00 g, 44.2 mmol, 1.00 *eq*) in toluene (80.0 mL) was added diphenylphosphoryl azide (36.5 g, 132 mmol, 28.7 mL, 3.00 *eq*), triethylamine (13.4 g, 132 mmol, 18.4 mL, 3.00 *eq*) and Molecular sieves pack 4A power (10.0 g), the mixture was stirred 100 °C for 12 h. The mixture was filtered to give filter, then the filtrate was diluted with water (30 ml) and exacted with ethyl acetate (3 × 100 ml). The organic phase was separated, washed with brine (100 ml), dried over anhydrous sodium sulfate, filtered and concentrated under reduced pressure to give a residue. The residue was purified by reversed - phase HPLC (0.1% ammonium hydroxide) to give (*R*)-5-methyl-1-(pyrazin-2-yl)imidazolidin-2-one (1.00 g, 5.61 mmol, 13%) yield) as a white solid. ¹H NMR (400 MHz, DMSO-d₆) *δ* = 9.41 - 9.33 (m, 1H), 8.38 - 8.29 (m, 1H), 8.23 - 8.13 (m, 1H), 7.47 - 7.38 (m, 1H), 4.70 - 4.60 (m, 1H), 3.68 - 3.61 (m, 1H), 3.08 - 3.03 (m, 1H), 1.32 (br d, *J* = 6.0 Hz, 3H).

Step 3. To a solution of (*R*)-5-methyl-1-(pyrazin-2-yl)imidazolidin-2-one (50.0 mg, 281 umol, 1.00 *eq)* in dioxane (2.00 mL) was added 3-(5-bromo-1-oxoisoindolin-2-yl)piperidine-2,6-dione (90.7 mg, 281 umol, 1.00 *eq*), cesium carbonate (183 mg, 561 umol, 2.00 *eq*), 4,5-bis(diphenylphosphino)-9,9-dimethylxanthene (32.5 mg, 56.1 umol, 0.200 *eq*) and Methanesulfonato(2-dicyclohexylphosphino-2',4',6'-tri-i-propyl-1,1'-biphenyl) (2'-amino-1,1'-biphenyl-2-yl)palladium(II) (23.8 mg, 28.1 umol, 0.100 *eq*), the mixture was stirred at 100 °C for 12 h under nitrogen. The reaction mixture was filtered and concentrated under reduced pressure to give a residue. The residue was dissolved in dimethyl formamide (3.00 ml) and purified by *Prep*-HPLC (column: Phenomenex luna C18 150 × 25 mm × 10 um; mobile phase: [water (formic acid) - acetonitrile]; B%: 16% - 46%, 10 min). The desired fraction was collected and concentrated to remove acetonitrile. The residual aqueous was lyophilized to give 3-(5-((R)-4-methyl-2-oxo-3-(pyrazin-2-yl)imidazolidin- 1-yl)-1-oxoisoindolin-2-yl)piperidine-2,6-dione (12.74 mg, 30.3 umol, 5% yield) as a yellow solid. ¹H NMR (400 MHz, DMSO-*d*₆) δ = 11.05 - 10.95 (m, 1H), 9.48 - 9.40 (m, 1H), 8.49 - 8.43 (m, 1H), 8.38 - 8.30 (m, 1H), 7.97 - 7.89 (m, 1H), 7.83 - 7.74 (m, 2H), 5.17 - 5.09 (m, 1H), 4.86 - 4.78 (m, 1H), 4.53 - 4.44 (m, 1H), 4.38 - 4.28 (m, 2H), 3.76 - 3.71 (m, 1H), 2.97 - 2.89 (m, 1H), 2.70 - 2.64 (m, 1H), 2.41 - 2.33 (m, 1H), 2.07 - 1.99 (m, 1H), 1.46 - 1.39 (m, 3H). MS (ESI) m/z 421.1 [M+H]⁺

### Example 58. Synthesis of Compound 180

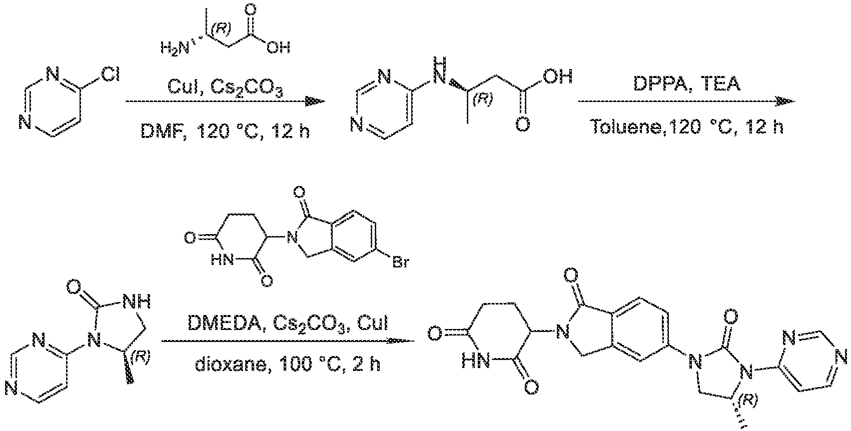

Step 1. To a solution of 4-chloropyrimidine (2.50 g, 21.8 mmol, 1.00 *eq*) and (3*R*)-3-aminobutanoic acid (2.25 g, 21.8 mmol, 1.00 *eq*) in dimethylformamide (20.0 mL) was added cesium carbonate (14.2 g 43.6 mmol, 2.00 *eq*) and copper iodide (831 mg, 4.37 mmol, 0.200 *eq*). The mixture was stirred at 120 °C for 12 h under nitrogen atmosphere. The reaction mixture was diluted with water (100 mL) and extracted with ethyl acetate (3 × 150 mL). The combined organic layers were washed with brine (300 mL ), dried over anhydrous sodium sulfate, and concentrated under reduced pressure to give a residue. The crude product was purified by reversed phase (column: spherical C18, 20-45 um, 100Å, SW 200, mobile phase: [water(0.1% ammonium hydroxide)-ACN) and lyophilized to give (R)-3-(pyrimidin-4-ylamino)butanoic acid as a green solid. MS (ESI) m/z 179.9 [M+H]⁺

Step 2. To a mixture of (3*R*)-3-(pyrimidin-4-ylamino)butanoic acid (7.00 g, 38.6 mmol, 1.00 *eq*) in toluene (70.0 mL) was added 4A molecular sieves (7.00 g) , diphenylphosphoryl azide (31.9 g, 115 mmol, 25.0 mL, 3.00 *eq*) and triethylamine (11.7 g, 115 mmol, 16.1 mL, 3.00 *eq*). The mixture was stirred at 120 °C for 12 h under nitrogen atmosphere. The solid was filtered, and the filtrate was mixed with saturated sodium carbonate (200 mL) and extracted with dichloromethane:isopropanol(3:1) (3 × 200 mL). The organic layer was washed with brine (500 mL), dried over anhydrous sodium sulfate, and concentrated under reduced pressure to give a residue. The residuewas purified by reversed phase (0.1%ammonium hydroxide condition) to give a crude product. The crude product was purified by column chromatography (SiO2, Petroleum ether/Ethyl acetate=20/1 to 0/1) to give (R)-5-methyl-1-(pyrimidin-4-yl)imidazolidin-2-one (1.00 g, 5.61 mmol, 14% yield) as an orange solid. ¹H NMR (400 MHz, DMSO-d₆) δ = 8.80 (d, *J* = 0.9 Hz, 1H), 8.46 (d, *J* = 6.0 Hz, 1H), 8.13 (dd, *J* = 1.1, 6.0 Hz, 1H), 4.68 (dt, *J* = 3.1, 5.9 Hz, 1H), 3.60 (t, *J* = 8.9 Hz, 1H), 3.01 (td, *J* = 1.3, 9.1 Hz, 1H), 1.33 (d, *J* = 6.1 Hz, 3H).

Step 3. To a mixture of (5*R*)-5-methyl-1-pyrimidin-4-yl-imidazolidin-2-one (41.3 mg, 232 µmol, 1.50 *eq*) and 3-(5-bromo-1-oxo-isoindolin-2-yl)piperidine-2,6-dione (50.0 mg, 154 µmol, 1.00 *eq*) in dioxane (1.00 mL) was added cesium carbonate (100 mg, 309 µmol, *2.00 eq*), *N*,*N*'-dimethylethylenediamine (6.82 mg, 77.3 µmol, 8.33 µL, 0.500 *eq*) and copper iodide (5.89 mg, 30.9 umol, 0.200 *eq*), The mixture was stirred at 100 °C for 2 h under nitrogen atmosphere. The mixture under reduced pressure evaporated to dryness to get a green solid. Dimethylformamide (8.00 mL) was added to dissolve the solid and then filtered to collect the filtrate. The filtrate was purified by prep-HPLC (column: Phenomenex luna C18 150*25mm* 10um;mobile phase: [water(formic acid)-ACN];B%: 7%-37%,2min)and lyophilized to give 3-(5-((*R*)-4-methyl-2-oxo-3 -(pyrimidin-4-yl)imidazolidin-1-yl)-1-oxoisoindolin-2-yl)piperidine-2,6-dione (30.2 mg, 76.1 µmol, 4% yield) as a yellowish solid. ¹H NMR (400 MHz, DMSO-d₆) δ = 11.02 - 10.92 (m, 1H), 8.98 (br d, *J =* 2.5 Hz, 1H), 8.73 - 8.53 (m, 1H), 8.24 (s, 1H), 7.91 (d, *J* = 7.4 Hz, 1H), 7.83 - 7.69 (m, 2H), 5.11 (dd, *J* = 4.4, 13.6 Hz, 1H), 4.85 (dt, *J* = 2.2, 6.0 Hz, 1H), 4.52 - 4.44 (m, 1H), 4.40 - 4.17 (m, 2H), 3.79 - 3.64 (m, 1H), 2.98 - 2.85 (m, 1H), 2.68 - 2.62 (m, 1H), 2.33 (br dd, *J* = 1.9, 5.2 Hz, 1H), 2.06 - 1.97 (m, 1H), 1.45 (d, *J* = 6.3 Hz, 3H). MS (ESI) m/z 421.2 [M+H]⁺

### Example 59. Synthesis of Compound 152

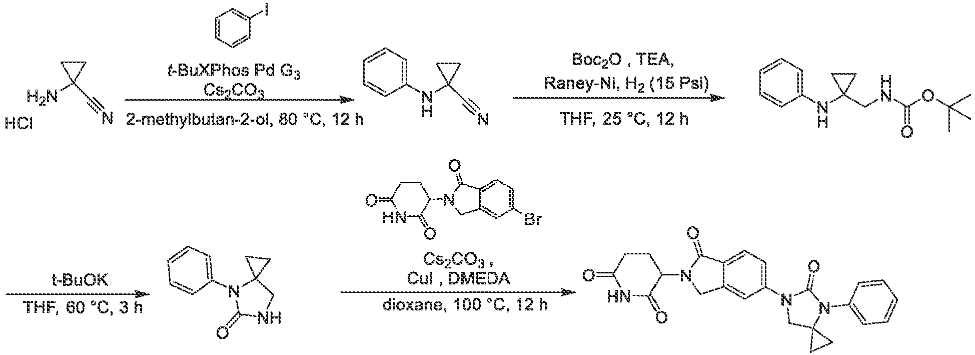

Step 1. To a solution of iodobenzene (1.18 g, 5.78 mmol, 644 uL, 1.00 *eq*), 1-aminocyclopropanecarbonitrile;hydrochloride (1.03 g, 8.68 mmol, 1.50 *eq*) and cesium carbonate (5.65 g, 17.3 mmol, 3.00 *eq)* in 2-methyl-2-butanol (20.0 mL) was added methanesulfonato(2-di-t-butylphosphino-2,4,6-tri-i-propyl-1,1-biphenyl)(2-amino-1,1-biphenyl-2-yl)palladium(ii) (459 mg, 578 umol, 0.100 *eq*)*.* The mixture was stirred at 80 °C for 12 h. The solution was concentrated under reduced pressure to give a residue. The residue was purified by reversed-phase HPLC(column: spherical C18, 20-45 um, 100-Å, SW 330, mobile phase:[water(0.1%Formic Acid)-acetonitrile) and lyophilized to get 1-(phenylamino)cyclopropane-1-carbonitrile (1.90 g, 12.0 mmol, 69% yield) as a white solid. ¹H NMR (400 MHz, DMSO-*d*₆) δ = 7.21 (t, *J* = 8.0 Hz, 2H), 6.86 (s, 1H), 6.79 (d, *J* = 7.6 Hz, 2H), 6.77 - 6.72 (m, 1H), 1.55 - 1.49 (m, 2H), 1.18 - 1.13 (m, 2H).

Step 2. To a solution of 1-anilinocyclopropanecarbonitrile (600 mg, 3.79 mmol, 1.00 *eq*) in tetrahydrofuran (20.0 mL) was added triethylamine (1.15 g, 11.3mmol, 1.58 mL, 3.00 *eq*), di-tert-butyldicarbonate (2.48 g, 11.3 mmol, 2.61 mL, 3.00 *eq*) and aluminum-nickel alloy (324 mg, 3.79 mmol, 1.00 *eq*) under nitrogen atmosphere. The mixture was sitrred at 25 °C for 12 h under hydrogen (15 Psi) atmosphere. The mixture was filtered to give a filter liquor, then was concentrated under reduced pressur to give a residue. The residue was purified by reversed-phase HPLC(column: spherical C18, 20-45 um, 100Å, SW 80, mobile phase:[water(0.1%Formic Acid)-acetonitrile) and lyophilized to get *tert*-butyl ((1-(phenylamino)cyclopropyl)methyl)carbamate (1.30 g, 4.96 mmol, 65% yield) as a white solid. ¹H NMR (400 MHz, DMSO-*d*₆) δ = 7.09 - 7.02 (m, 2H), 6.81 - 6.72 (m, 1H), 6.67 (br d, *J* = 8.0 Hz, 2H), 6.53 (br t, *J* = 7.2 Hz, 1H), 5.99 (s, 1H), 3.14 (br d, *J* = 5.6 Hz, 2H), 1.37 (s, 9H), 0.75 - 0.72 (m, 2H), 0.57 - 0.52 (m, 2H).

Step 3. To a solution of *tert-*butyl ((1-(phenylamino)cyclopropyl)methyl)carbamate (700 mg, 2.67 mmol, 1.00 *eq*) and potassium *tert*-butoxide (898 mg, 8.00 mmol, 3.00 *eq*) in tetrahydrofuran (8.00 mL) was stirred at 60 °C for 3 h under nitrogen atmosphere. The reaction mixture was diluted with water (100 mL) and extracted withethyl acetate(3 × 50 ml). The combined organic layers were washed with brine (3 × 20 mL), dried over anhydrous sodium sulfate, filtered and concentrated under reduced pressure to give a residue. The residue was purified by column chromatography (SiO₂, Petroleum ether/Ethyl acetate=20/1 to 2/1) and concentrated in vacuum to get 4-phenyl-4,6-diazaspiro[2.4]heptan-5-one (500 mg, 2.31 mmol, 86% yield, 87% purity) as a white solid. MS (ESI) m/z. 189.0 [M+H]⁺

Step 4. To a solution of 4-phenyl-4,6-diazaspiro[2.4]heptan-5-one (30.0 mg, 159 umol, 1.00 *eq*), 3-(5-bromo-1-oxo-isoindolin-2-yl)piperidine-2,6-dione (41.2 mg, 127 umol, *0.800 eq*), cesium carbonate (155 mg, 478 umol, 3.00 *eq*) in dioxane (2.00 mL) was added copper iodide (3.04 mg, 15.9 umol, 0.100 eq) and *N,N*-dimethylethane-1,2-diamine (2.81 mg, 31.8 umol, 3.43 uL, 0.200 *eq).* The mixture was stirred at 100 °C for 12 h under nitrogen atmosphere. The mixture was adjusted to pH=5-6 with formic acid and filtered and concentrated in vacuum. The residue was purified by *prep*-HPLC(column: Phenomenex luna C18 150*25mm* 10um;mobile phase: [water(FA)-acetonitrile];B%: 27%-57%,10 min) and lyophilized to afford 3-(1-oxo-5-(5-oxo-4-phenyl-4,6-diazaspiro[2.4]heptan-6-yl)isoindoin-2-yl)piperidine-2,6-dione (17.1 mg, 38.5 umol, 2% yield, 97%) purity) as a white solid. ¹H NMR (400 MHz, DMSO-*d*₆) δ = 10.97 (br s, 1H), 7.86 (s, 1H), 7.77 - 7.69 (m, 2H), 7.49 - 7.43 (m, 2H), 7.41 - 7.36 (m, 1H), 7.23 (d, *J* = 7.2 Hz, 2H), 5.09 (dd, *J* = 5.1, 13.2 Hz, 1H), 4.49 - 4.40 (m, 1H), 4.37 - 4.25 (m, 1H), 4.16 (s, 2H), 2.96 - 2.86 (m, 1H), 2.61 (br d, *J* = 2.4 Hz, 1H), 2.46 - 2.37 (m, 1H), 2.04 - 1.95 (m, 1H), 0.86 - 0.80 (m, 2H), 0.70 - 0.63 (m, 2H). MS (ESI) m/z. 431.1 [M+H]⁺

### Example 60. Synthesis of Compound 142

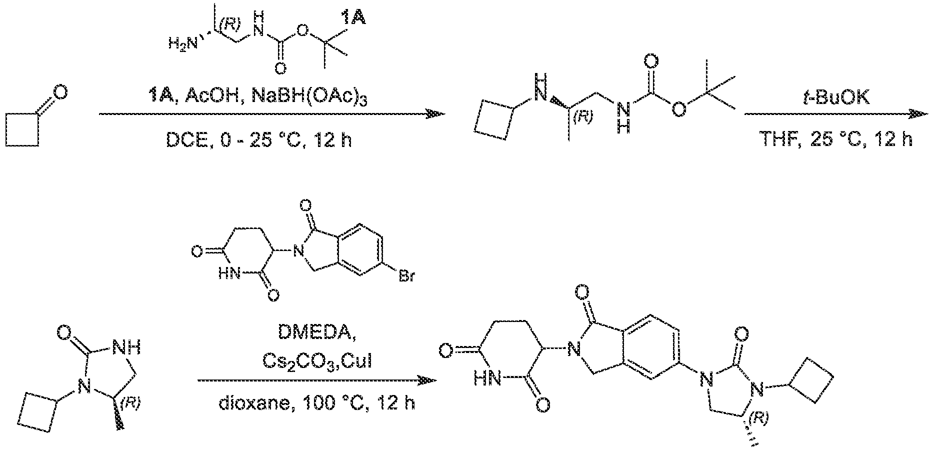

Step 1. To a solution of cyclobutanone (287 mg, 4.10 mmol, 306 uL, 1.10 *eq), tert*-butyl (R)-(2-aminopropyl)carbamate (650 mg, 3.73 mmol, 1.00 *eq)* in 1,2-dichloroethane (50.0 mL) was added acetic acid (672 mg, 11.1 mmol, 640 uL, 3.00 *eq*) and sodium triacetoxy borohydride (3.95 g, 18.6 mmol, 5.00 *eq*) at 0 °C. The mixture was stirred at 25 °C for 12 h. The reaction mixture was diluted with water (30 mL) and extracted with dichloromethane (3 × 30 mL). The organic phase was separated, washed with brine(3 × 10 mL), dried over anhydrous sodium sulfate, filtered and concentrated under reduced pressure to give a residue. The residue was purified by column chromatography (SiO₂, Ethyl acetate : Methanol=10/1 to 5/1) and concentrated to get *tert*-butyl (*R*)-(2-(cyclobutylamino)propyl)carbamate (530 mg, 2.32 mmol, 62% yield) as yellow oil. ¹H NMR (400 MHz, DMSO-*d*₆) *δ* = 6.69 (br s, 1H), 3.23 - 3.16 (m, 1H), 2.89 - 2.82 (m, 1H), 2.78 - 2.71 (m, 1H), 2.58 (br dd, *J* = 6.0, 12.4 Hz, 1H), 2.14 - 2.03 (m, 2H), 1.66 - 1.53 (m, 4H), 1.38 (s, 9H), 0.87 (d, *J* = 6.4 Hz, 3H).

Step 2. To a solution of *tert*-butyl (*R*)-(2-(cyclobutylamino)propyl)carbamate (400 mg, 1.75 mmol, 1.00 *eq*) in tetrahydrofuran (8.00 mL) was added potassium *tert-*butoxide (589 mg, 5.26 mmol, 3.00 *eq*)*.* Then the reaction mixture was stirred at 25 °C for 12 h. The reaction mixture was diluted with water (50 mL) and extracted with ethyl acetate (3 × 50 mL). The combined organic layers were washed with brine (3 × 20 mL), dried over anhydrous sodium sulfate, filtered and concentrated under reduced pressure to get (*R*)-1-cyclobutyl-5-methylimidazolidin-2-one (290 mg, 1.17 mmol, 66% yield, 62% purity) as yellow oil. ¹H NMR (400 MHz, DMSO-*d*₆) *δ* = 6.26 - 6.09 (m, 1H), 3.96 (br t, *J* = 8.8 Hz, 1H), 3.76 - 3.66 (m, 1H), 3.30 (br s, 1H), 2.74 (dd, *J* = 6.4, 8.0 Hz, 1H), 2.37 - 2.20 (m, 2H), 2.04 - 1.93 (m, 2H), 1.64 - 1.52 (m, 2H), 1.17 (d, *J* = 6.0 Hz, 3H).

Step 3. To a solution of (5*R*)-1-cyclobutyl-5-methyl-imidazolidin-2-one (19.8 mg, 128 umol, 1.00 *eq*) and 3-(5-bromo-1-oxo-isoindolin-2-yl)piperidine-2,6-dione (25.0 mg, 77.3 umol, 0.600 *eq*) in dioxane (1.00 mL) was added cesium carbonate (84.0 mg, 257 umol, 2.00 *eq*)*,* copper iodide (2.46 mg, 12.8 umol, 0.100 *eq)* and *N¹,N²*-dimethylethane-1,2-diamine (2.27 mg, 25.7 umol, 2.78 uL, 0.200 *eq*). Then the reaction mixture was stirred at 100 °C for 12 h under nitrogen atmosphere. The reaction mixture was adjusted pH to 5-6 by formic acid (0.2 ml) and concentrated in vacuum. The residue was added *N,N-*dimethylformamide (4 mL) and filtered. The filtrate was purified by *prep*-HPLC(column: Phenomenex luna C18 150*25mm* 10um;mobile phase: [water(formic acid)- acetonitrile]; B%: 22%-52%, 58 min) and lyophilized to afford 3-(5-((R)-3-cyclobutyl-4-methyl-2-oxoimidazolidin-1-yl)-1-oxoisoindolin-2-yl)piperidine-2,6-dione (18.5 mg, 42.4 umol, 2% yield, 91% purity) as a yellow solid.

¹H NMR. (400 MHz, CDCl₃) *δ* = 8.11 (br s, 1H), 7.93 (br d, *J* = 1.2 Hz, 1H), 7.82 (d, *J* = 8.4 Hz, 1H), 7.33 - 7.27 (m, 1H), 5.21 (ddd, *J* = 2.4, 5.2, 13.2 Hz, 1H), 4.48 - 4.40 (m, 1H), 4.35 - 4.27 (m, 1H), 4.24 - 4.16 (m, 1H), 4.03 - 3.91 (m, 2H), 3.35 (s, 1H), 2.96 - 2.80 (m, 2H), 2.49 - 2.33 (m, 3H), 2.31 - 2.20 (m, 3H), 1.83 - 1.73 (m, 2H), 1.38 (d, *J* = 6.0 Hz, 3H).

¹H NMR. (400 MHz, DMSO-*d*₆) δ = 10.95 (s, 1H), 7.87 - 7.77 (m, 1H), 7.72 - 7.63 (m, 2H), 5.09 (dd, *J* = 4.8, 13.2 Hz, 1H), 4.46 - 4.38 (m, 1H), 4.36 - 4.21 (m, 1H), 4.19 - 4.07 (m, 1H), 4.04 - 3.88 (m, 2H), 3.41 (br d, *J* = 3.2 Hz, 1H), 2.94 - 2.87 (m, 1H), 2.61 - 2.56 (m, 2H), 2.41 (br d, *J* = 2.4 Hz, 2H), 2.15 - 2.08 (m, 2H), 2.02 - 1.96 (m, 1H), 1.73 - 1.64 (m, 2H), 1.28 (d, *J* = 6.0 Hz, 3H).

### Example 62. Synthesis of Compound 138

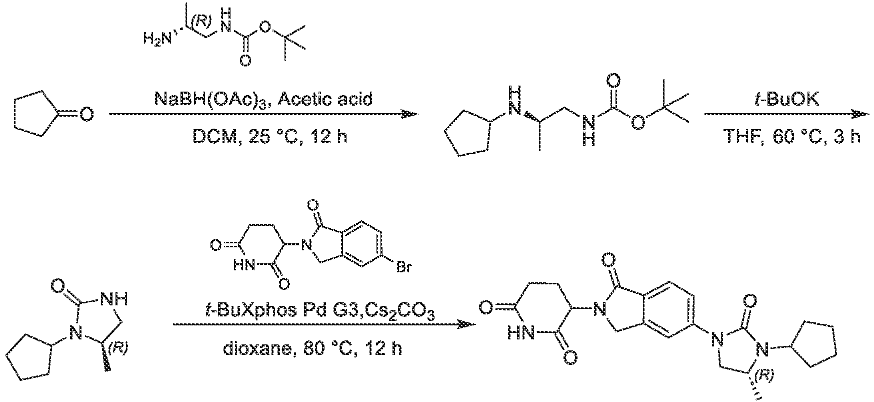

Step 1. To a solution of *tert*-butyl (*R*)-(2-aminopropyl)carbamate (1.00 g, 5.74 mmol, 1.00 *eq)* and cyclopentanone (579 mg, 6.89 mmol, 610 uL, 1.20 *eq)* in dichloromethane (20.0 mL) was added sodium triacetoxyborohydride (6.08 g, 28.7 mmol, 5.00 *eq*) and acetic acid (475 mg, 1.72 mmol, 0.300 *eq),* the mixture was stirred at 25 °C for 12 h. The mixture was concentrated under reduced pressure to give a residue. The residue was purified by reversed-phase HPLC (C18, 40 g; condition: water/ acetonitrile = 1/0 -0/1, 0.1% NH₃·H₂O) and lyophilized to afford *tert*-butyl (*R*)-(2-(cyclopentylamino)propyl)carbamate (485 mg, 1.70 mmol, 29% yield) as a brown oil. ¹H NMR (400 MHz, DMSO-*d₆*) *δ* = 6.68 (br t, *J =* 5.4 Hz, 1H), 3.07 (quin, *J* = 6.4 Hz, 1H), 2.92 - 2.82 (m, 1H), 2.81 - 2.73 (m, 1H), 2.66 - 2.57 (m, 1H), 1.78 - 1.63 (m, 2H), 1.62 - 1. 53 (m, 2H), 1.50 - 1.41 (m, 2H), 1.37 (s, 9H), 1.28 - 1.12 (m, 2H), 0.89 (d, *J* = 6.3 Hz, 3H).

Step 2. To a solution of *tert*-butyl (*R*)-(2-(cyclopentylamino)propyl)carbamate (485 mg, 2.00 mmol, 1.00 *eq)* in tetrahydrofuran (2.00 mL) was added potassium *tert-*butoxide (674 mg, 6.00 mmol, 3.00 *eq),* the mixture was stirred at 60 °C for 3 h. The reaction mixture was filtered. The filtrate was diluted with water (50.0 mL), the aqueous phase was extracted with ethyl acetate (3 × 100 mL). The combined organic phase was washed with brine (3 × 50 mL), dried with anhydrous anhydrous sodium sulfate, filtered and concentrated in vacuum to afford (*R*)-1-cyclopentyl-5-methylimidazolidin-2-one (370 mg, 1.63 mmol, 81% yield,) as an off-white solid . ¹H NMR (400 MHz, DMSO-*d₆*) *δ* = 6.11 (br s, 1H), 3.81 - 3.73 (m, 1H), 3.73 - 3.64 (m, 1H), 3.36 - 3.27 (m, 2H), 2.79 - 2.71 (m, 1H), 1.81 - 1.71 (m, 2H), 1.70 - 1.54 (m, 4H), 1.52 - 1.42 (m, 2H), 1.18 - 1.14 (m, 3H).

Step 3. To a solution of (*R*)-1-cyclopentyl-5-methylimidazolidin-2-one (31.2 mg, 186 umol, 1.20 *eq*) and 3-(5-bromo-1-oxoisoindolin-2-yl)piperidine-2,6-dione (50 mg, 155 umol, 1.00 *eq*) in dioxane (2.00 mL) was added cesium carbonate (151 mg, 464 umol, 3.00 *eq)* and [2-(2-aminophenyl)phenyl]-methylsulfonyloxy-palladium;ditert-butyl-[2-(2,4,6-triisopropylphenyl)phenyl]phosphane (12.3 mg, 15.5 umol, 0.100 *eq*), the mixture was stirred at 80 °C for 12 h under nitrogen atmosphere. The mixture was diluted with dioxane (40.0 mL) and filtered. The filtrate was purified by *Prep*-HPLC (column: Phenomenex luna C18 150*25mm* 10um;mobile phase: [water(FA)-ACN];B%: 35%-65%,7min) and lyophilized to afford 3-(5-((*R*)-3-cyclopentyl-4-methyl-2-oxoimidazolidin-1-yl)-1-oxoisoindolin-2-yl)piperidine-2,6-dione (70.0 mg, 159 umol, 11% yield) as a yellow solid. ¹H NMR (400 MHz, DMSO-*d₆*) *δ* = 10.96 (s, 1H), 7.81 (br s, 1H), 7.71 - 7.61 (m, 2H), 5.08 (dd, *J* = 5.0, 13.3 Hz, 1H), 4.45 - 4.35 (m, 1H), 4.32 - 4.23 (m, 1H), 4.05 - 3.96 (m, 1H), 3.95 - 3.85 (m, 2H), 3.45 - 3.38 (m, 1H), 2.98 - 2.83 (m, 1H), 2.59 (br d, *J* = 17.0 Hz, 1H), 2.45 - 2.35 (m, 1H), 2.04 - 1.93 (m, 1H), 1.91 - 1.81 (m, 2H), 1.80 - 1.67 (m, 4H), 1.60 - 1.49 (m, 2H), 1.28 (d, *J* = 6.0 Hz, 3H). MS (ESI) m/z 411.4 [M+H]⁺

### Example 63. Synthesis of Compound 161

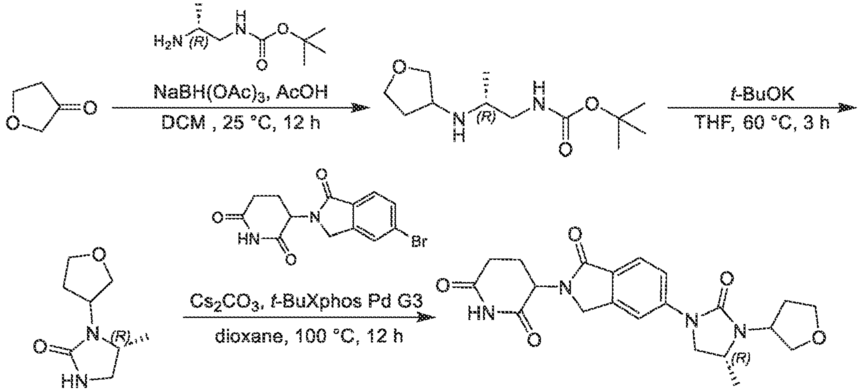

Step 1. To a solution of dihydrofuran-3(2*H*)-one (177 mg, 2.07 mmol, 1.20 *eq*) and *tert*-butyl (*R*)-(2-arninopropyl)carbamate (300 mg, 1.72 mmol, 1.00 *eq*) in dichloromethane (5.00 mL) was added sodium triacetoxyhydroborate (1.82 g, 8.61 mmol, 5.00 *eq*) and acetic acid (1.03 g, 17.2 mmol, 985 µL, 10.0 *eq).* The reaction was stirred at 25 °C for 12 h. The reaction was filtered to give filtrate and concentrated under reduced pressure to give a residue. The residue was purified by column chromatography (SiO2, dichloromethane: methanol = 10:1) to afford *tert-*butyl ((2*R*)-2-((tetrahydrofuran-3-yl)amino)propyl)carbamate (250 mg, 1.02 mmol, 59.43% yield) as yellow oil. ¹H NMR (400 MHz, DMSO-*d₆*) *δ* = 6.81 - 6.63 (m, 1H), 4.32 - 4.24 (m, 1H), 3.74 - 3.61 (m, 4H), 3.47 (dd, *J* = 1.6, 9.2 Hz, 1H), 3.30 (br dd, *J* = 4.8, 8.4 Hz, 1H), 2.96 - 2.77 (m, 2H), 2.69 - 2.60 (m, 1H), 1.61 - 1.53 (m, 1H), 1.40 - 1.37 (m, 9H), 0.92 (t, *J* = 6.8 Hz, 3H). (The spectra comes from the pilot run)

Step 2. To a solution of *tert*-butyl ((2*R*)-2-((tetrahydrofuran-3-yl)amino)propyl)carbamate (250 mg, 1.02 mmol, 1.00 *eq)* in tetrahydrofuran (5.00 mL) was added potassium *tert*-butoxide (344 mg, 3.07 mmol, 3.00 *eq).* The reaction was stirred at 60 °C for 3 h. The reaction was filtered to give a residue. The residue was purified by column chromatography (SiO2, methanol /Ethyl acetate = 0/1 to 10/1) to afford (5*R*)-5-methyl-1-(tetrahydrofuran-3-yl)imidazolidin-2-one (60.0 mg, 352 µmol, 34% yield) as colourless oil. ¹H NMR (400 MHz, DMSO-*d₆*) *δ* = 6.30 (br s, 1H), 4.32 - 4.01 (m, 1H), 3.95 - 3.83 (m, 1H), 3.76 - 3.55 (m, 4H), 2.83 - 2.73 (m, 1H), 2.16 - 1.78 (m, 3H), 1.17 (dd, *J* = 6.4, 9.6 Hz, 3H).

Step 3. To a solution of (5*R*)-5-methyl-1-(tetrahydrofuran-3-yl)imidazolidin-2-one (30.0 mg, 176 µmol, 1.00 *eq)* and 3-(5-bromo-1-oxoisoindolin-2-yl)piperidine-2,6-dione (56.9 mg, 176 µmol, 1.00 *eq*) in dioxane (1.00 mL) was added cesium carbonate (172 mg, 528 µmol, 3.00 *eq*) and [2-(2-aminophenyl)phenyl]-methylsulfonyloxy-palladium;ditert-butyl-[2-(2,4,6-triisopropylphenyl)phenyl]phosphane (14.0 mg, 17.6 µmol, 0.100 eq) under nitrogen atmosphere. The reaction was stirred at 100 °C for 12 h. The reaction was filtered to give a residue. The residue was purified by *Prep*-HPLC (column: Phenomenex luna C18 150*25mm* 10um;mobile phase: [water(FA)-ACN];B%: 20%-40%, 10min) to afford 3-(5-((4*R*)-4-methyl-2-oxo-3-(tetrahydrofuran-3-yl)imidazolidin-1-yl)-1-oxoisoindolin-2-yl)piperidine-2,6-dione (37.77 mg, 87.0 µmol, 25% yield, 95% purity) as a white solid. ¹H NMR (400 MHz, DMSO-*d₆*) *δ* = 10.98 (s, 1H), 7.82 (br s, 1H), 7.73 - 7.62 (m, 2H), 5.09 (dd, *J* = 5.2, 13.2 Hz, 1H), 4.46 - 4.22 (m, 3H), 4.07 - 3.91 (m, 3H), 3.85 - 3.81 (m, 1H), 3.80 - 3.65 (m, 2H), 3.46 (br dd, *J* = 3.2, 8.4 Hz, 1H), 2.97 - 2.87 (m, 1H), 2.60 (br d, *J* = 17.2 Hz, 1H), 2.44 - 2.31 (m, 1H), 2.27 - 2.04 (m, 2H), 2.00 (br dd, *J* = 5.6, 12.6 Hz, 1H), 1.30 (dd, *J* = 5.6, 9.6 Hz, 3H). MS (ESI) m/z 413.1 [M+H]⁺

### Example 64. Synthesis of Compound 160

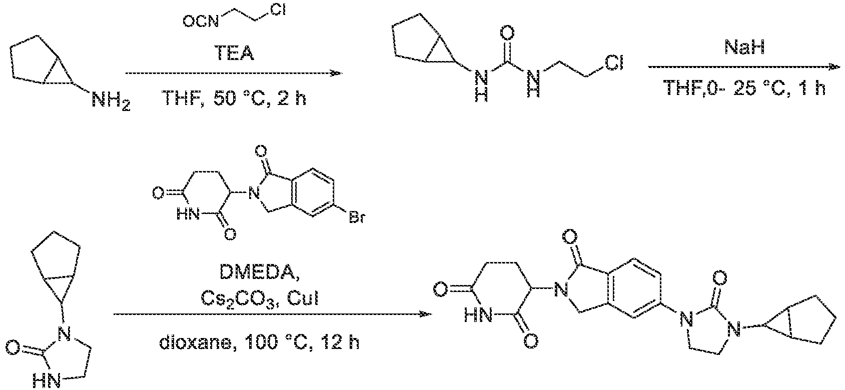

Step 1. A mixture of bicyclo[3.1.0]hexan-6-amine (0.720 g, 7.41 mmol, 1.00 *eq)* and triethylamine (2.25 g, 22.2 mmol, 3.10 mL, 3.00 *eq)* in tetrahydrofuran (30.0 mL)was added 1-chloro-2-isocyanato-ethane (1.17 g, 11.1 mmol, 946 uL, 1.50 *eq*). The mixture was stirred at 50.0 °C for 2 h. The reaction mixture was poured into water (50.0 mL) and extracted with ethyl acetate (3 × 50.0 mL). The combined organic phase was separated, washed with brine (50.0 mL), dried over anhydrous sodium sulfate, filtered and concentrated to afford 1-(6-bicyclo[3.1.0]hexanyl)-3-(2-chloroethyl)urea (1.530 g, crude) as a light yellow solid. ¹H NMR (400 MHz, DMSO-*d6*) *δ* = 6.33 (br t, *J* = 5.0 Hz, 2H), 3.58 - 3.56 (m, 3H), 3.30 (s, 2H), 1.81 - 1.72 (m, 2H), 1.70 - 1.41 (m, 4H), 1.22 (br s, 2H).

Step 2. A mixture of 1-(6-bicyclo[3.1.0]hexanyl)-3-(2-chloroethyl)urea(1.50 g, 7.40 mmol, 1.00 *eq*) in tetrahydrofuran (20.0 mL) was added sodium hydride (444 mg, 11.1 mmol, 60% purity, 1.50 *eq*) at 0 °C. The reaction mixture was stirred at 25 °C for 1 h. The reaction mixture was quenched in saturated ammonium chloride. The reaction mixture was diluted with water (30.0 mL) and extracted with ethyl acetate (3 × 50.0 mL). The organic phase was separated, washed with brine (3×10.0 mL), dried over sodium sulfate, filtered and concentrated under reduced pressure to give a residue. The residue was purified by column chromatography (SiO₂, ethyl acetate : methanol = 10/1 to 5/1) and concentrated to afford 1-(6-bicyclo[3.1.0]hexanyl)imidazolidin-2-one (280 mg, 1.68 mmol, 22% yield) as a light yellow solid. ¹H NMR (400 MHz, DMSO-*d6*) *δ* = 6.24 (br s, 1H), 3.68 (t, *J* = 6.3 Hz, 1H), 3.22 - 3.19 (m, 2H), 3.15 (s, 2H), 1.78 - 1.71 (m, 2H), 1.71 - 1.46 (m, 4H), 1.44 (br s, 2H). MS (ESI) m/z 167.2 [M+H]⁺

Step 3. To a solution of 1-(6-bicyclo[3.1.0]hexanyl)imidazolidin-2-one (10.0 mg, 60.1 umol, 1.00 *eq*) and 3-(5-bromo-1-oxo-isoindolin-2-yl)piperidine-2,6-dione (23.3 mg, 72.2 umol, 1.20 *eq*) in dioxane (1.00 mL) was added cesium carbonate (39.2 mg, 120 umol, 2.00 *eq), N,N*-dimethylethanne-1,2-diamine (1.06 mg, 12.03 umol, 1.30 uL, 0.200 *eq*) and copper iodide (1.15 mg, 6.02 umol, 0.100 *eq).* The reaction mixture was stirred at 100 °C for 12 h under nitrogen atmosphere. The reaction mixture was added dimethyl formamide (2.00 mL), the pH of the mixture was adjusted to 2~3 with formic acid and filtered. The filtrate was concentrated to give a residue. The residue was purified by *prep*-HPLC (column: Phenomenex luna C18 150*25mm* 10um; mobile phase: [water (formic acid)- acetonitrile]; B%: 26%-56%, 10min) and lyophilized to afford 3-(5-(3-(bicyclo[3.1.0]hexan-6-yl)-2-oxoimidazolidin-1-yl)-1-oxoisoindolin-2-yl)piperidine-2,6-dione (17.37 mg, 40.0 umol, 7% yield, 94% purity) as a yellow solid. ¹H NMR (400 MHz, DMSO-*d₆*) *δ* = 10.97 (s, 1H), 7.82 (s, 1H), 7.71 - 7.60 (m, 2H), 5.08 (dd, *J* = 5.0, 13.4 Hz, 1H), 4.47 - 4.36 (m, 1H), 4.32 - 4.22 (m, 1H), 3.85 - 3.75 (m, 2H), 3.44 - 3.39 (m, 2H), 2.95 - 2.83 (m, 1H), 2.59 (br dd, *J* = 1.6, 17.3 Hz, 1H), 2.39 - 2.33 (m, 1H), 2.26 (s, 1H), 1.99 (ddd, *J* = 2.3, 5.0, 10.0 Hz, 1H), 1.85 - 1.77 (m, 2H), 1.76 - 1.67 (m, 2H), 1.61 - 1.53 (m, 3H), 1.15 - 0.99 (m, 1H). MS (ESI) m/z 409.1 [M+H]⁺

### Example 65. Synthesis of Compound 125

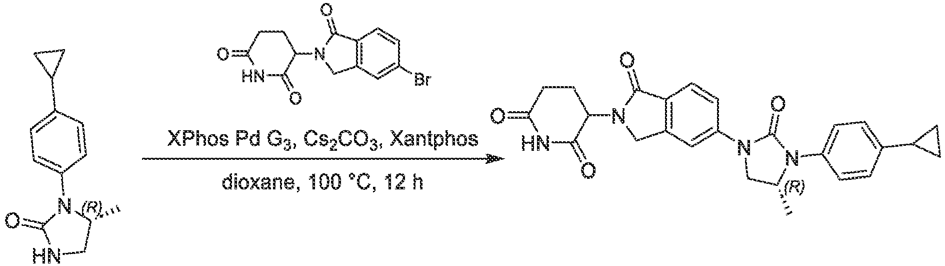

Step 1. To a solution of (*R*)-1 -(4-cyclopropylphenyl)-5-methylimidazolidin-2-one (50.0 mg, 231 umol, 1.00 *eq*), 3-(5-bromo-1-oxoisoindolin-2-yl)piperidine-2,6-dione (74.7 mg, 231 umol, 1.00 *eq*) in dioxane (2.00 mL) was added cesium carbonate (151 mg, 462 umol, 2.00 *eq*), methanesulfonato(2-dicyclohoxylphosphino-2,4,6-tri-i-propyl-1,1-biphenyl)(2-amino-1,1-biphenyl-2-yl)palladium(ii) (19.6 mg, 23.1 umol, 0.100 *eq*)*,* and 4,5-bis(diphenylphosphino)-9,9-dimethylxanthene (26.7 mg, 46.2 umol, 0.200 *eq).* The mixture was stirred at 100 °C for 12 h under nitrogen atmosphere. The reaction was filtered, and the filtrate was concentrated to afford a residue. The residue was purified by reversed-phase HPLC (C18, 80 g; condition: water/ acetonitrile = 100:0 to 0:100, 0.1% acetic acid) and lyophilized to afford 3-(5-((*R*)-3-(4-cyclpropylphenyl)-4-methyl-2-oxoimiazolidi-1yl)-1 oxoisoindolin-2-yl)piperidine-2,6-dione (240 mg, 508 µmol, 37% yield, 97% purity) as a yellow solid. ¹H NMR (400 MHz, DMSO-*d₆*) *δ* = 10.98 (s, 1H), 7.87 (br d, *J* = 4.6 Hz, 1H), 7.78 - 7.73 (m, 1H), 7.72 - 7.68 (m, 1H), 7.37 (d, *J* = 8.6 Hz, 2H), 7.11 (d, *J* = 8.6 Hz, 2H), 5.10 (dd, *J* = 5.2, 13.2 Hz, 1H), 4.61 - 4.52 (m, 1H), 4.47 - 4.41 (m, 1H), 4.35 - 4.27 (m, 1H), 4.22 (dt, *J* = 2.0, 9.2 Hz, 1H), 3.62 (ddd, *J* = 2.6, 6.2, 9.2 Hz, 1H), 2.99 - 2.84 (m, 1H), 2.63 - 2.57 (m, 1H), 2.40 (br dd, *J* = 4.4, 13.2 Hz, 1H), 2.04 - 1.96 (m, 1H), 1.95 - 1.88 (m, 1H), 1.24 (d, *J* = 6.0 Hz, 3H), 0.98 - 0.91 (m, 2H), 0.70 - 0.63 (m, 2H). MS (ESI) m/z 459.2 [M+H]⁺

### Example 66. Synthesis of Compound 137

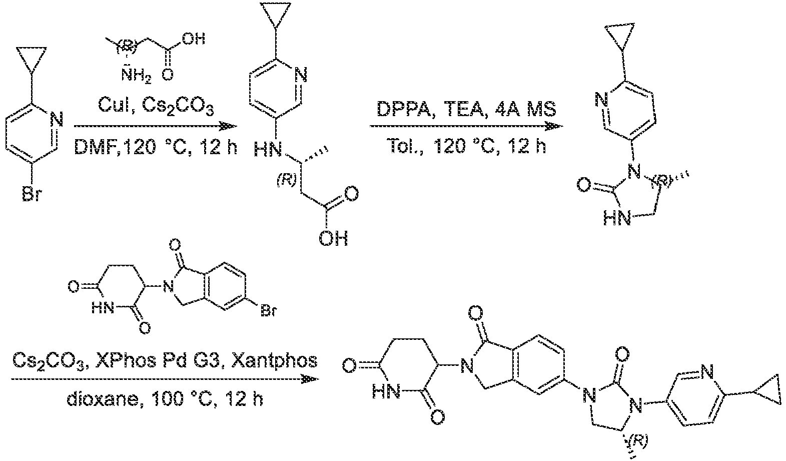

Step 1. To a mixture of 5-bromo-2-cyclopropylpyridine (1.00 g, 5.05 mmol, 1.00 *eq)* and (R)-3-aminobutanoic acid (1.04 g, 10.1 mmol, *2.00 eq)* in dimethyformamide (10.0 mL) was added cesium carbonate (3.29 g, 10.1 mmol, 2.00 *eq)* and cuprous iodide (192 mg, 1.01 mmol, 0.200 *eq).* The mixture was stirred at 120 °C for 12 h. The mixture was filterted. The filtrate was concentrated in vacuum. The residue was dissolved in water (20.0 mL). The crude product was purified by reversed-phase HPLC (0.1% ammonium hydroxide) and lyophilized to give (*R*)-3-((6-cyclopropylpyridin-3-yl)amino)butanoic acid (5.30 g, crude) as blue oil. MS (ESI) m/z 219.1 [M-H]⁺

Step 2. To a mixture of (*R*)-3-((6-cyclopropylpyridin-3-yl)amino)butanoic acid (5.00 g, 22.7 mmol, 1.00 *eq*) in toluene (25.0 mL) was added molecular sieves, 13x (2.00 g), diphenylphosphoryl azide (18.7 g, 68.1 mmol, 14.8 mL, 3.00 *eq*) and triethylamine (6.89 g, 68.1 mmol, 9.48 mL, 3.00 *eq*)*.* The mixture was stirred at 120 °C for 12 h under nitrogen. The mixture was filterted. The filtrate was diluted with water (100 mL) and extracted with ethyl acetate (3 × 100 mL). The combined organic layers were washed with brine (100 mL), dried over sodium sulfate, filtered and concentrated under reduced pressure to give a residue. The residue was purified by column chromatography (SiO2, Petroleum ether/Ethyl acetate = 10/1 to 0/1) and concentrated in vacuum to give (*R*)-1-(6-cyclopropylpyridin-3-yl)-5-methylimidazolidin-2-one (1.10 g, 5.06 mmol, 22% yield) as a yellow solid. ¹H NMR (400 MHz, DMSO-*d*₆) *δ* = 8.45 (d, *J* = 2.6 Hz, 1H), 7.72 (dd, *J* = 2.8, 8.5 Hz, 1H), 7.22 (d, *J* = 8.5 Hz, 1H), 6.92 (s, 1H), 4.50 - 4.37 (m, 1H), 3.64 - 3.50 (m, 1H), 3.03 - 2.94 (m, 1H), 2.08 - 1.99 (m, 1H), 1.16 (d, *J* = 6.0 Hz, 3H), 0.92 - 0.80 (m, 4H).

Step 3. To a mixture of 3-(5-bromo-1-oxoisoindolin-2-yl)piperidine-2,6-dione (50.0 mg, 230 umol, 1.00 *eq*) and 3-(5-bromo-1-oxoisoindolin-2-yljpiperidine-2,6-dione (89.2 mg, 276 umol, 1.20 *eq*) in dioxane (1.00 mL) was added 4,5-bis(diphenylphosphino)-9,9-dimethylxanthene (26.6mg, 46.0 umol, 0.200 eq), cesium carbonate (150 mg, 460 umol, 2.00 *eq*) and (2-dicyclohexylphosphino-2',4',6'-triisopropyl-1,1'-biphenyl)[2-(2'-amino-1,1'-biphenyl)]palladitim(II) methanesulfonate (19.5 mg, 23.0 umol, 0.100 *eq*). The mixture was stirred at 100 °C for 12 h. The mixture was concentrated in vacuum. The residue was dissolved in dimethyformamide (4.00 mL) and filterted. The filtrate was purified by reversed-phase (column: spherical C18, 20-45 um, 100Å, SW 120, mobile phase: [water(0.1%Formic Acid)- ACN) and lyophilized. The reverse phase collection was purified by *prep*-HPLC (column: Phenomenex luna C18 150*25mm* 10um;mobile phase: [water(FA)-ACN];B%: 6%-36%, 10min) and lyophilized to give 3-(5-((*R*)-3-(6-cyclopropylpyridin-3-yl)-4-methyl-2-oxoimidazolidin-1-yl)-1-oxcisoindolin-2-yl)piperidine-2,6-dione (21.32 mg, 43.6 umol, 3% yield, 94% purity) as a white solid. ¹H NMR (400 MHz, DMSO-*d*₆) *δ* = 10.97 (br s, 1H), 8.55 (d, *J* = 2.4 Hz, 1H), 7.87 (s, 1H), 7.80 - 7.75 (m, 2H), 7.73 - 7.69 (m, 1H), 7.33 (d, *J* = 8.4 Hz, 1H), 5.09 (dd, *J* = 5.1, 13.3 Hz, 1H), 4.61 (td, *J* = 0.0, 8.8 Hz, 1H), 4.48 - 4.41 (m, 1H), 4.35 - 4.28 (m, 1H), 4.28 - 4.21 (m, 1H), 3.70 - 3.62 (m, 1H), 2.95 - 2.86 (m, 1H), 2.62 (br d, *J* = 2.3 Hz, 1H), 2.41 - 2.33 (m, 1H), 2.13 - 2.08 (m, 1H), 2.04 - 1.95 (m, 1H), 1.26 (d, *J* = 6.1 Hz, 3H), 0.96 - 0.88 (m, 4H). MS (ESI) m/z 460.4 [M+H]⁺

### Example 67. Synthesis of Compound 155

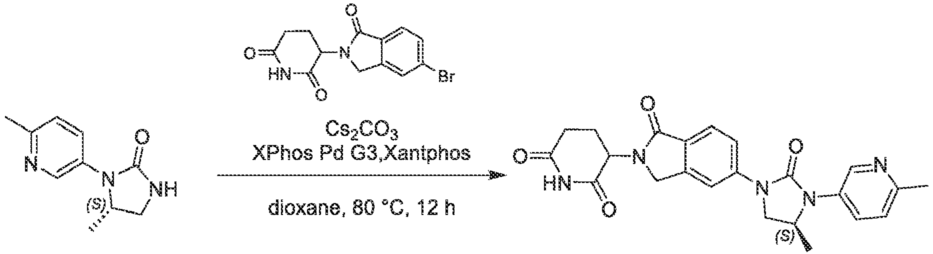

Step 1. To a solution of 3-(5-bromo-1-oxoisoindolin-2-yl)piperidine-2,6-dione (50.0 mg, 154 umol, 1.00 *eq*)*,* (S)-5-methyl-1-(6-methylpyridin-3-yl)imidazolidin-2-one (29.5 mg, 154 umol, 1.00 *eq*)*,* cesium carbonate (100 mg, 309 umol, 2.00 *eq)* in 1,4-dioxane (2.00 mL) was added methanesulfonato[9,9-dimethyl-4,5-Bis(diphenylphosphino)xanthene][2-amino-1,1-biphenyl]palladium(II)dichloromethaneadduct (13.1 mg, 15.4 umol, 0.100 *eq*)*,* 4,5-bis(diphenylphosphino)-9,9-dimethylxanthene (17.9 mg, 30.9 umol, 0.200 *eq*) under nitrogen. The mixture was stirred at 80 °C for 12 h. The solution was added formic acid to adjust pH =5 and was concentrated under reduced pressure to give a residue which was added dimethyl formamide (3.00 mL) and filtered to give a filter liquor which was purified by reversed-phase HPLC(column: spherical C18, 20-45 um, 100Å, SW 80, mobile phase: [water(0.1%Formic Acid)- acetonitrile) to give 3-(5-((*S*)-4-methyl-3-(6-methylpyridin-3-yl)-2-oxoimidazolidin-1-yl)-1-oxoisoindolin-2-yl)piperidine-2,6-dione (10.4 mg, 24.1 umol, 3% yield) as a white solid. ¹H NMR (400 MHz, DMSO-*d*₆) *δ* = 10.97 (br s, 1H), 8.62 (d, *J* = 2.5 Hz, 1H), 7.88 (br s, 1H), 7.82 (dd, *J* = 2.7, 8.4 Hz, 1H), 7.79 - 7.74 (m, 1H), 7.73 - 7.70 (m, 1H), 7.30 (d, *J* = 8.5 Hz, 1H), 510 (dd, *J* = 5.1, 13.2 Hz, 1H), 4.69 - 4.59 (m, 1H), 4.49 - 4.41 (m, 1H), 4.35 - 4.29 (m, 1H), 4.29 - 4.23 (m, 1H), 3.71 - 3.63 (m, 1H), 2.97 - 2.86 (m, 1H), 2.60 (br dd, *J* = 1.8, 15.4 Hz, 1H), 2.46 (s, 3H), 2.43 - 2.37 (m, 1H), 2.04 - 1.96 (m, 1H), 1.27 (d, *J* = 6.1 Hz, 3H). MS (ESI) m/z 434.1 [M+H]⁺

### Example 68. Synthesis of Compound 135

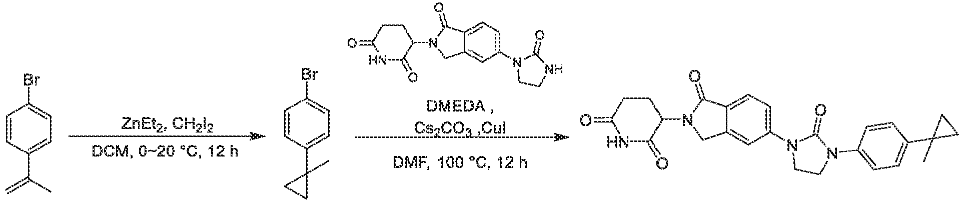

Step 1. To a solution of 1-bromo-4-isopropenyl-benzene (500 mg, 2.54 mmol, 1.00 *eq*) in dichloromethane (5.00 mL) was added diethylzinc (1 M in toluene, 12.6 mL, 5.00 *eq*) and diiodomethane (6.80 g, 25.3 mmol, 2.05 mL, 10.0 *eq*) dropwise at 0 °C under nitrogen atmosphere. Then the reaction mixture was stirred at 70 °C for 12 h. The reaction mixture was quenched by addition water (100 mL) at 25 °C, and extracted with ethyl acetate (3 × 50 mL). The combined organic layers were washed with brine (3 × 10 mL), dried over sodium sulfate, filtered and concentrated under reduced pressure to give a residue. The residue was purified by reversed phase column chromatography (C18, 80 g; condition: water/acetonitrile = 1/0 to 0/1, 0.1% formic acid) and concentrated in vacuum to get 1-bromo-4-(1-methylcyclopropyl)benzene (200 mg, 947 umol, 37% yield) as yellow oil. ¹H NMR (400 MHz, DMSO-*d*₆) *δ* = 7.49 - 7.38 (m, 2H), 7.21 - 7.14 (m, 2H), 1.36 (s, 3H), 0.85 - 0.81 (m, 2H), 0.78 -0.74 (m, 2H).

Step 2. To a solution of 1-bromo-4-(1-methylcyclopropyl)benzene (6.43 mg, 30.4 umol, 1.00 *eq*) and 3-(1-oxo-5-(2-oxoimidazolidin-1-yl)isoindolin-2-yl )piperidine-2,6-dione (10.0 mg, 30.4 umol, 1.00 *eq*) in dimethylformamide (0.500 mL) was added cesium carbonate (19.8 mg, 60.9 umol, 2.00 *eq*) , *N¹,N²*-dimethylethane-1,2-diamine (268 ug, 3.05 umol, 3.28^{e-1} ul, 0.100 *eq*) and copper iodide (1.16 mg, 6.09 umol, 0.200 *eq*)*.* Then the reaction mixture was stirred at 100 °C for 12 h under nitrogen atmosphere. The reaction mixture was adjusted pH to 5-6 by formic acid (0.5 mL) and filtered. The filtrate was purified by *prep*-HPLC(column: Phenomenex luna C18 150*25mm * 10um;mobile phase: [water(formic acid)- acetonitrile];B%: 41%-71%, 10 min) and lyophilized. The filter cake was triturated with formic acid (3 mL) and dimethylformamide (3 mL) at 25 °C for 15 min and filtered. The filter cake was triturated with water (15 mL) and filtered. The filter cake was lyophilized to afford 3-(5-(3-(4-(1-methylcyclopropyl)phenyl)-2-oxoimidazolidin-1-yl)-1-oxoisoindolin-2-yl)piperidine-2,6-dione (76.0 mg, 159 umol, 65%) yield, 96% purity) as a yellow solid. ¹H NMR (400 MHz, DMSO-*d₆*) *δ* = 10.99 (s, 1H), 7.91 (s, 1H), 7.81 - 7.75 (m, 1H), 7.74 - 7.68 (m, 1H), 7.54 (br d, *J* = 8.4 Hz, 2H), 7.23 (br d, *J* = 8.4 Hz, 2H), 5.10 (br dd, *J* = 4.8, 13.2 Hz, 1H), 4.49 - 4.41 (m, 1H), 4.36 - 4.28 (m, 1H), 4.07 - 3.95 (m, 4H), 2.97 - 2.87 (m, 1H), 2.63-2.56 (m, 1H), 2.40 (br dd, *J* = 3.6, 13.2 Hz, 1H), 2.05 - 1.96 (m, 1H), 1.38 (s, 3H), 0.84 - 0.78 (m, 2H), 0.78 - 0.71 (m, 2H). MS (ESI) m/z. 459.2 [M+H]⁺

### Example 69. Synthesis of Compound 133 & 181

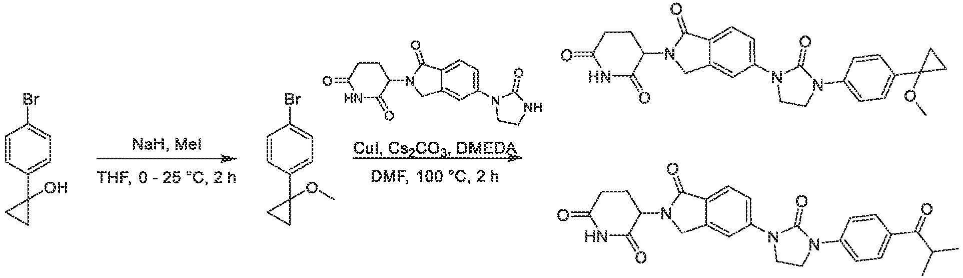

Step 1. To a mixture of 1-(4-bromophenyl)cyclopropanol (500 mg, 2.35 mmol, 1.00 *eq*) in tetrahydrofuran (5.00 mL) was added sodium hydride (140 mg, 3.52 mmol, 60% purity, 1.50 *eq*) and iodomethane (666 mg, 4.69 mmol, 292 uL, 2.00 *eq*) at 0 °C. The mixture was stirred at 25 °C for 1h. The reaction mixture was quenched by addition formic acid (1.00 mL) aquenous solution. The diluted reaction mixture was extracted with ethyl acetate (3 × 50 mL), washed with brine (50 mL), dried over sodium sulfate, filtered via vacuum filtration, and concentrated in vacuum. The residue was purified by column chromatography (SiO2, Petroleum ether/Ethyl acetate= 1/0 to 0/1) and concentrated in vacuum to give 1-bromo-4-(1-methoxycyclopropyl)benzene (250 mg, crude) as yellow oil. ¹H NMR (400 MHz, DMSO-*d*₆) *δ* = 7.93 - 7.83 (m, 2H), 7.73 (d, *J* = 8.6 Hz, 2H), 3.13 (s, 3H), 1.18 - 1.13 (m, 2H), 0.99 - 0.91 (m, 2H).

Step 2. To a mixture of 1-bromo-4-(1-methoxycyclopropyl)benzene (50.0 mg, 220 µmol, 1.00 *eq*) and 3-(1-oxo-5-(2-oxoimidazolidin-1-yl)isoindolin-2-yl)pipenidine-2,6-dione (43.3 mg, 132 µmol, 0.600 *eq*) in dimethylformamide (1.00 mL) was added cesium carbonate (215 mg, 660 µmol, *3.00 eq*)*, N,N'*-dimethylethylenediamine (9.70 mg, 110 µmol, 11.8 µL, 0.500 *eq*) and copper iodide (8.39 mg, 44.0 µmol, 0.200 *eq*). The mixture was stirred at 100 °C for 2 h under nitrogen atmosphere. The mixture was filtered under reduced pressure to obtain a filtrate. The filtrate was purified by *prep*-HPLC (column: Phenomenex luna C18 150*25mm* 10um;mobile phase: [water(formic acid)-ACN];B%: 30%-60%,10min) and lyophilized to give 3-(5-(3-(4-isobutyrylphenyl)-2-oxoimidazolidin-1-yl)-1-oxoisoindolin-2-yl)piperidine-2,6-dione (11.32 mg, 23.9 µmol, 10% yield) as a white solid and 3-(5-(3-(4-(1-methoxycyclopropyl)phenyl)-2-oxoimidazolidin-1-yl)-1-oxoisoindolin-2-yl)piperidine-2,6-dione (30 mg, crude), the crude product was purified by *Prep*-HPLC (column: Phenomenex luna C18 150*25mm^{™} 10um;mobile phase: [water(FA)-ACN];B%: 30%-60%,10min) to afford 3-(5-(3-(4-(1-methoxycyclopropyl)phenyl)-2-oxoimidazolidin-1-yl)-1-oxoisoindolin-2-yl)piperidine-2,6-dione (10.05 mg, 21.18 µmol, 10% yield) as a white solid.

¹H NMR (400 MHz, DMSO-*d*₆) *δ* = 10.99 (s, 1H), 7.92 (s, 1H), 7.82 - 7.77 (m, 1H), 7.75 - 7.70 (m, 1H), 7.63 (d, *J* = 8.8 Hz, 2H), 7.30 (d, *J* = 8.8 Hz, 2H), 5.11 (dd, *J* = 5.1, 13.3 Hz, 1H), 4.51 - 4.43 (m, 1H), 4.38 - 4.29 (m, 1H), 4.12 - 3.97 (m, 4H), 3.15 (s, 3H), 2.98 - 2.88 (m, 1H), 2.65 - 2.59 (m, 1H), 2.44 - 2.39 (m, 1H), 2.05 - 1.98 (m, 1H), 1.15 - 1.08 (m, 2H), 0.99 - 0.90 (m, 2H).

¹H NMR (400 MHz, DMSO-*d*₆) *δ* = 10.98 (br d, *J* = 0.8 Hz, 1H), 8.02 (d, *J* = 9.0 Hz, 2H), 7.94 (s, 1H), 7.84 - 7.78 (m, 3H), 7.76 - 7.72 (m, 1H), 5.11 (dd, *J* = 5.2, 13.1 Hz, 1H), 4.51 - 4.43 (m, 1H), 4.38 - 4.30 (m, 1H), 4.09 (s, 4H), 3.70 - 3.62 (m, 1H), 2.94 - 2.87 (m, 1H), 2.62 (br d, *J* = 2.4 Hz, 1H), 2.46 - 2.42 (m, 1H), 2.05 - 1.97 (m, 1H), 1.11 (d, *J* = 6.8 Hz, 6H). MS (ESI) m/z 475.0 [M+H]⁺

### Example 70. Synthesis of Compound 147

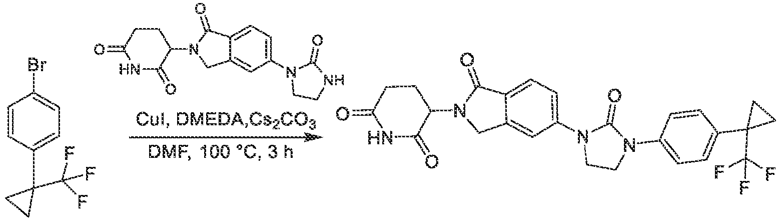

Step 1. To a mixture of 1-bromo-4-(1-(trifluoromethyl)cyclopropyl)benzene (50.0 mg, 188 umol, 1.00 *eq*) and 3-(1-oxo-5-(2-oxoimidazolidin-1-yl)isoindolin-2-yl)piperidine-2,6-dione (37.1 mg, 113 umol, 0.600 *eq*) in dimethyformamide (1.00 mL) was added cesium carbonate (122 mg, 377 umol, 2.00 *eq*), n,n'-dimethylethylenediamine (8.31 mg, 94.3 umol, 10.1 uL, 0.500 *eq*) and cuprous iodide (7.18 mg, 37.7 umol, 0.200 *eq*)*.* The mixture was stirred at 100 °C for 3 h under nitrogen. The reaction mixture was filterted. The crude product was purified by reversed phase (column: spherical C18, 20-45 um, 100Å, SW 120, mobile phase: [water(0.1%Formic Acid)-ACN) and lyophilized. The crude product was purified by *prep*-HPLC (column: Phenomenex luna C18 150*25mm* 10um;mobile phase: [water(FA)-ACN];B%. 38%-68%, 10min) and lyophilized to give 3-(1-oxo-5-(2-oxo-3-(4-(1-(trifluoromethyl)cyclopropyl)phenyl) imidazolidin-1-yl)isoindolin-2-yl)piperidine-2,6-dione (40.05 mg, 75.8 umol, 6% yield, 97% purity) as a white solid. ¹H NMR (400 MHz, DMSO-*d*₆) *δ* = 10.98 (br s, 1H), 7.91 (s, 1H), 7.77 (d, *J* = 1.9 Hz, 1H), 7.75 - 7.70 (m, 1H), 7.69 - 7.61 (m, 2H), 7.47 (d, *J* = 8.5 Hz, 2H), 5.10 (dd, *J =* 5.0, 13.3 Hz, 1H), 4.51 - 4.26 (m, 2H), 4.12 - 3.95 (m, 4H), 2.98 - 2.84 (m, 1H), 2.60 (br dd, *J* = 2.3, 15.3 Hz, 1H), 2.42 - 2.34 (m, 1H), 2.05 - 1.95 (m, 1H), 1.38 - 1.27 (m, 2H), 1.11 (br s, 2H). MS (ESI) m/z. 513.3 [M+H]⁺

### Example 71. Synthesis of Compound 128

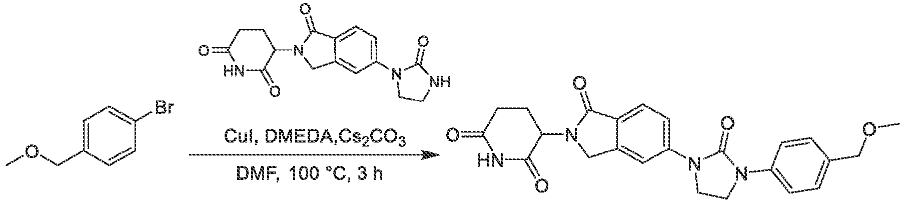

Step 1. To a solution of 1-bromo-4-(methoxymethyl)benzene (50.0 mg, 248 umol, 1.00 *eq*) and 3-(1-oxo-5-(2-oxoimidazolidin-1-yl)isoindolin-2-yl)piperidine-2,6-dione (48.9 mg, 149 umol, 0.600 *eq*) in dimethyformamide (1.00 mL) was added cesium carbonate (162 mg, 497 umol, 2.00 *eq*), n,n'-dimethylethylenediamine (10.9 mg, 124 umol, 13.4 uL, *0.500 eq*) and cuprous iodide (9.47 mg, 49.7 umol, 0.200 *eq*)*.* The mixture was stirred at 100 °C for 3 h under nitrogen. The reaction mixture was filterted. The filtrate was purified by reversed phase chromatography (column: spherical C18, 20-45 um, 100Å, SW 120, mobile phase: [water(0.1%Formic Acid)-ACN) and lyophilized to give 3-(5-(3-(4-(methoxymethyl)phenyl)-2-oxoimidazolidin-1-yl)-1-oxoisoindolin-2-yl)piperidine-2,6-dione (37.34 mg, 80.7 umol, 5% yield, 97% purity) as a off-white solid.

¹H NMR(400 MHz, DMSO-*d*₆) *δ* = 11.08 - 10.91 (m, 1H), 7.92 (s, 1H), 7.80 - 7.70 (m, 2H), 7.63 (br d, *J* = 8.4 Hz, 2H), 7.33 (br d, *J* = 8.4 Hz, 2H), 5.10 (br dd, *J* = 4.3, 12.9 Hz, 1H), 4.48 - 4.42 (m, 1H), 4.38 (s, 2H), 4.36 - 4.29 (m, 1H), 4.04 (br d, *J* = 4.5 Hz, 4H), 3.28 (s, 3H), 2.96 - 2.87 (m, 1H), 2.65 - 2.58 (m, 1H), 2.40 (br dd, *J* = 4.0, 13.4 Hz, 1H), 2.00 (br dd, *J* = 4.9, 6.2 Hz, 1H). MS (ESI) m/z. 449.1 [M+H]⁺

### Example 72. Synthesis of Compound 154

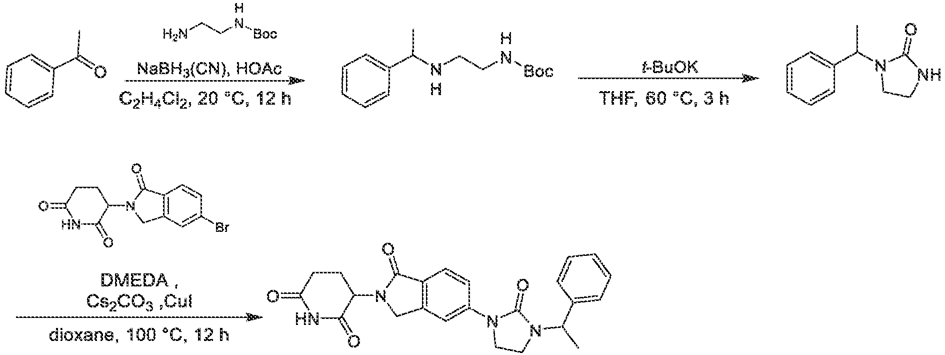

Step 1. To a solution of 1-phenylethanone (500 mg, 4.16 mmol, 486 µL, 1.00 *eq)* and *tert*-butyl *N*-(2-aminoethyl)carbamate (800 mg, 4.99 mmol, 787 µL, 1.20 *eq)* in dichlorodiethane (5.00 mL) was added sodium cyanoborohydride (784 mg, 12.4 mmol, 3.00 *eq*) and acetic acid (249 mg, 4.16 mmol, 238 µL, 1.00 *eq*)*.* The mixture was stirred at 20 °C for 12 hours. The reaction solution was filtered to get solids. The residue was purified by column chromatography (SiO₂, Petroleum ether/Ethyl acetate=100/1 to 1/1) to give *tert*-butyl (2-((1-phenylethyl)amino)ethyl)carbamate (425 mg, 1.61 mmol, 38% yield) as a white solid. ¹H NMR (400 MHz, DMSO-d6) δ = 8.00 - 7.82 (m, 4H), 7.37 (br s, 1H), 4.55 (s, 1H), 3.68 (s, 1H), 3.66 - 3.57 (m, 2H), 3.21 (br s, 1H), 3.12 - 3.00 (m, 2H), 1.91 - 1.85 (m, 9H), 1.72 - 1.68 (m, 3H)

Step 2. To a solution of *tert*-butyl (2-((1-phenylethyl)amino)ethyl)carbamate (420 mg, 1.59 mmol, 1.00 *eq*) in tetrahydrofuran (4.00 mL) was added potassium tert-butoxide (1 M, 4.77 mL, 3.00 *eq*). The mixture was stirred at 60 °C for 3 hours. The mixture was added water (5.00 mL) and extracted with ethyl acetate (3 × 5.00 mL). The combined organic layers were dried over anhydrous sodium sulfate and concentrated. The residue was purified by column chromatography (SiO₂, Petroleum ether/Ethyl acetate=50/1 to 1/1) to give 1-(1-phenylethyl)imidazolidin-2-one (186 mg, 977 µmol, 61% yield) as a white solid. ¹H NMR (400 MHz, DMSO-*d*₆) *δ* = 7.40 - 7.07 (m, 5H), 6.31 (br s, 1H), 5.00 (q, *J* = 7.2 Hz, 1H), 3.27 (s, 1H), 3.24 - 3.11 (m, 2H), 2.95 - 2.86 (m, 1H), 1.43 (d, *J* = 7.2 Hz, 3H).

Step 3. To a solution of 1-(1-phenylethyl)imidazolidin-2-one (20.0 mg, 105 µmol, 1.00 *eq*) and 3-(5-bromo-1-oxo-isoindolin-2-yl)piperidine-2,6-dione (40.7 mg, 126 µmol, 1.20 *eq*) in dioxane (1.00 mL) was added copper iodide (2.00 mg, 10.5 µmol, 0.100 *eq*)*, N¹,N²*-dimethylethane-1,2-diamine (1.85 mg, 21.0 µmol, 2.26 µL, 0.200 *eq*)*,* cesium carbonate (102 mg, 315 µmol, 3.00 *eq*)*.* Then the reaction mixture was stirred at 100 °C for 12 h under nitrogen atmosphere. The reaction mixture was adjusted pH to 5-6 by formic acid (0.2) mL and filtered. The filter cake was purified by reversed phase column chromatography (C18, 80 g; condition: water/acetonitrile = 1/0 to 0/1, 0.1% formic acid) and lyophilized to afford 3-(1-oxo-5-(2-oxo-3-(1-phenylethyl)imidazolidin-1-yl)isoindolin-2-yl)piperidine-2,6-dione (24.9 mg, 57.6 µmol, 18% yield, 99% purity) as a yellow solid. ¹H NMR (400 MHz, DMSO-*d*₆) *δ* = 10.97 (s, 1H), 7.85 (s, 1H), 7.75 - 7.69 (m, 1H), 7.69 - 7.64 (m, 1H), 7.43 - 7.34 (m, 4H), 7.34 - 7.27 (m, 1H), 5.19 (q, *J* = 7.2 Hz, 1H), 5.09 (dd, *J* = 5.2, 13.2 Hz, 1H), 4.48 - 4.38 (m, 1H), 4.35 - 4.25 (m, 1H), 3.96 - 3.81 (m, 2H), 3.61 - 3.52 (m, 1H), 3.19 - 3.12 (m, 1H), 2.98 - 2.84 (m, 1H), 2.60 (br d, *J* = 18.0 Hz, 1H), 2.43 - 2.37 (m, 1H), 2.03 - 1.95 (m, 1H), 1.55 (d, *J =* 7.2 Hz, 3H). MS (ESI) m/z. 433.3 [M+H]⁺

### Example 73. Synthesis of Compound 183

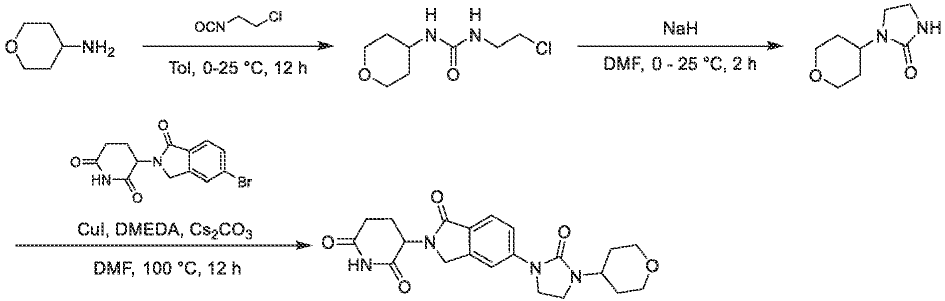

Step 1. To a solution of tetrahydropyran-4-amine (2.00 g, 19.8 mmol, 1.00 *eq*) in toluene (40.0 mL) was added 1-chloro-2-isocyanato-ethane (3.13 g, 29.6 mmol, 1.50 *eq*) at 0 °C. The mixture was stirred at 25 °C for 12 h. The mixture was filtered and the filter cake was collected. The crude product was triturated with toluene (5 mL) at 20 °C for 5 min to get 1-(2-chloroethyl)-3-tetrahydropyran-4-yl-urea (4.01 g, 19.4 mmol, 98% yield) as a white solid. ¹H NMR. (400 MHz, DMSO-*d*6) *δ* = 6.08 (br d, *J* = 7.6 Hz, 1H), 6.02 (br t, *J* = 5.6 Hz, 1H), 3.78 (td, *J* = 3.6, 11.2 Hz, 2H), 3.61 - 3.50 (m, 3H), 3.35 (d, *J* = 2.0 Hz, 1H), 3.32 (br s, 1H), 3.31 - 3.27 (m, 2H), 1.75 - 1.66 (m, 2H), 1.36 - 1.23 (m, 2H).

Step 2. To a solution of 1-(2-chloroethyl)-3-tetrahydropyran-4-yl-urea (2.00 g, 9.68 mmol, 1.00 *eq*) in tetrahydrofuran (20.0 mL) was added sodium hydride (580 mg, 14.5 mmol, 60% purity, 1.50 *eq*)*,* and the mixture was stirred at 0 °C for 2 hr. The reaction mixture was quenched by addition ammonium chloride (30 mL) at 25 °C, and then extracted with ethyl acetate (20 mL × 3). The combined organic layers were washed with brine (50 mL), dried over sodium sulfate, filtered and concentrated under reduced pressure to give a residue. The crude product was triturated with ethyl acetate (2 mL) at 20 °C for 5 min to get 1-tetrahydropyran-4-ylimidazolidin-2-one (464 mg, 2.73 mmol, 28% yield) as a white solid. ¹H NMR (400 MHz, D₂O) *δ* = 4.01 (br dd, *J* = 4.4, 11.6 Hz, 2H), 3.78 (tt, *J* = 4.0, 12.0 Hz, 1H), 3.56 - 3.45 (m, 4H), 3.42 - 3.35 (m, 2H), 1.81 - 1 68 (m, 2H), 1.66 - 1.59 (m, 2H).

Step 3. To a solution of 1-tetrahydropyran-4-ylimidazolidin-2-one (27.5 mg, 161 µmol, 1.04 *eq*) and 3-(5-bromo-1-oxo-isoindolin-2-yl)piperidine-2,6-dione (50.0 mg, 154 umol, 1.00 *eq*) in dimethylformamide (1.00 mL) was added cesium carbonate (151 mg, 464 µmol, 3.00 *eq*)*, N¹,N²-*dime*t*hylethane-1,2-diamine (1.36 mg, 15.4 µmol, 1.67 µL, 0.100 *eq*) and copper iodide (5.89 mg, 30.9 µmol, 0.200 *eq).* Then the reaction mixture was stirred at 100 °C for 12 h under nitrogen atmosphere. The reaction mixture was adjusted pH to 5-6 by formic acid (0.1 mL) and filtered. The filtrate was purified by *prep*-HPLC(column: Phenomenex luna C18 150*25mm* 10um;mobile phase: [water(formic acid)-acetonitrile];B%: 7%-37%,10min) and lyophilized to afford 3-(1-oxo-5-(2-oxo-3-(tetrahydro-2*H*-pyran-4-yl)imidazolidin-1-yl)isoindolin-2-yl)piperidine-2,6-dione (16.7 mg, 40.0 µmol, 9% yield, 99% purity) as a white solid.

¹H NMR (400 MHz, DMSO-*d*₆) *δ* = 10.97 (s, 1H), 7.81 (s, 1H), 7.76 - 7.71 (m, 1H), 7.69 - 7.64 (m, 1H), 5.09 (dd, *J =* 5.2, 13.2 Hz, 1H), 4.45 - 4.39 (m, 1H), 4.32 - 4.26 (m, 1H), 3.94 (br d, *J* = 4.4 Hz, 1H), 3.93 - 3.89 (m, 2H), 3.89 - 3.84 (m, 2H), 3.53 - 3.47 (m, 2H), 3.45 - 3.38 (m, 2H), 2.97 - 2.86 (m, 1H), 2.63 - 2.56 (m, 1H), 2.39 (br dd, *J* = 4.4, 12.8 Hz, 1H), 2.04 - 1.95 (m, 1H), 1.74 (dq, *J* = 4.0, 12.0 Hz, 2H), 1.60 (br dd, *J* = 2.0, 12.0 Hz, 2H). MS (ESI) m/z. 413.2 [M+H]⁺

### Example 74. Synthesis of Compound 164

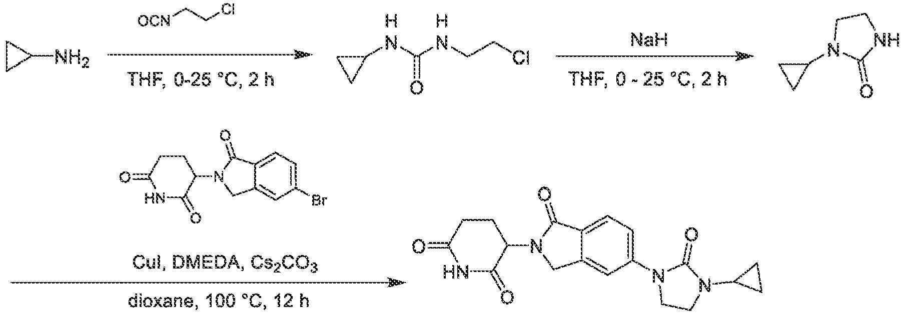

Step 1. To a solution of cyclopropanamine (1.00 g, 17.5 mmol, 1.21 mL, 1.00 *eq*) in tetrahydrofuran (10.0 mL) was added 1-chloro-2-isocyanato-ethane (1.85 g, 17.5 mmol, 1.00 *eq*). The mixture was stirred at 25 °C for 2 hours. The reaction solution was filtered to obtain solids to give 1-(2-chloroethyl)-3-cyclopropyl-urea (2.12 g, 12.5 mmol, 71% yield, 96% purity) as a white solid. MS (ESI) m/z 163.2 [M+H]⁺

Step 2. To a solution of 1-(2-chloroethyl)-3-cyclopropyl-urea (1.00 g, 6.15 mmol, 1.00 *eq*) in tetrahydrofuran (10.0 mL) was added sodium hydride (295 mg, 7.38 mmol, 60.0% purity, 1.20 *eq*) at 0 °C. After addition, the mixture was stirred at 25 °C for 2 hours. The reaction was quenched by adding ammonium chloride saturated solution (3.00 mL) and the products were extracted with dichloromethane (3 × 10.0 mL). The combined organic layers were dried over sodium sulfate, filtered and concentrated under vacuum. The residue was purified by column chromatography (silicon dioxide, Petroleum ether/Ethyl acetate=10/1 to 0/1) to give 1-cyclopropylimidazolidin-2-one (514 mg, 4.07 mmol, 66% yield) as a white solid. MS (ESI) m/z. 127.3 [M+H]⁺

Step 3. To a solution of 1-cyclopropylimidazolidin-2-one (30.0 mg, 2.38 mmol, 1.00 *eq*) and 3-(5-bromo-1-oxo-isoindolin-2-yl)piperidine-2,6-dione (76.8 mg, 2.38 mmol, 1.00 *eq*) in 1,4-dioxane (2.00 mL) was added cesium carbonate (155 mg, 4.76 mmol, 2.00 *eq*) , chloride (2 - dicyclohexyl phosphonic base - 2 minus 2-3 isopropyl - 1, 1 - al phenyl) [2 - (2 - amino - 1, 1 - diphenyl)] palladium (III) (20.1 mg, 237 µmol, 0.100 *eq*) and 4,5-bis(diphenylphosphino)-9,9-dimethylxanthene (27.5 mg, 475 µmol, 0.200 *eq*) under nitrogen atmosphere. The mixture was stirred at 100 °C for 12 hours. The solvent was added ethyl acetate (3.00 mL) and basified with acidize aqueous hydrochloric acid (1.00 mL). The aqueous phase was extracted with ethyl acetate (4 × 20.0 mL). The combined organic layers were dried over sodium sulfate and concentrated in vacuum. The crude product was purified by *Prep*-HPLC (column: Phenomenex luna C18 150×25mm× 10um;mobile phase: [water(formic acid)- acetonitrile];gradient:12%-42% B over 2 min) to give 3-[5-(3-cyclopropyl-2-oxo-imidazolidin-1-yl)-1-oxo-isoindolin-2-yl]piperidine-2,6-dione (15.0 mg, 39.5 µmol, 1.7% yield, 97% purity) as a white solid. ¹H NMR (400 MHz, DMSO-*d*₆) δ = 11.00 - 10.96 (m, 1H), 7.82 (s, 1H), 7.73 - 7.70 (m, 1H), 7.68 - 7.64 (m, 1H), 5.08 (dd, *J* = 5.2, 13.2 Hz, 1H), 4.-45 - 4.38 (m, 1H), 4.33 - -4.23 (m, 1H), 3.87 - 3.77 (m, 2H), 3.50 - 3.42 (m, 2H), 2.97 - 2.85 (m, 1H), 2.63 - 2.55 (m, 2H), 2.39 (br dd, *J* = 4.8, 12.8 Hz, 1H), 2.04 - 1.93 (m, 1H), 0.70 - 0.65 (m, 4H). MS (ESI) m/z 369.2 [M+H]⁺

### Example 75. Synthesis of Compound 162

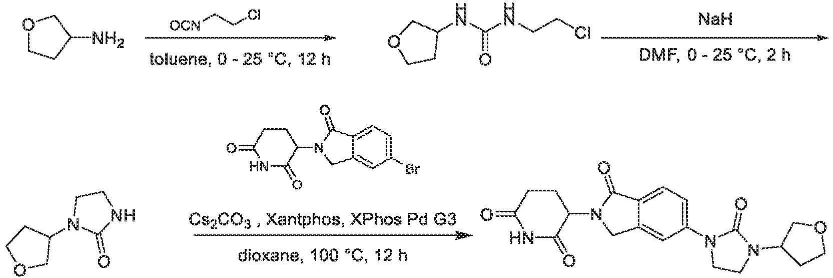

Step 1. To a solution of tetrahydrofuran-3-amine (1.00 g, 11.5 mmol, 1.00 *eq)* in toluene (30.0 mL) was added 1-chloro-2-isocyanatoethane (1.82 g, 17.2 mmol, 1.50 *eq)* at 0 °C. The mixture was stirred at 25 °C for 12 h. The reaction mixture was diluted with water (300 mL) and extracted with ethyl acetate (3 × 100 mL). The mixture was filtered. The filter cake was concentrated under reduced pressure to afford 1-(2-chloroethyl)-3-(tetrahydrofuran-3-yl)urea (1.30 g , crude) as white solid. ¹H NMR (400 MHz, DMSO-d6) δ = 6.32 (br d, *J =* 6.8 Hz, 1H), 6.02 (br t, *J* = 5.2 Hz, 1H), 4.14 - 4.06 (m, 1H), 3.80 - 3.65 (m, 3H), 3.56 (t, *J* = 6.2 Hz, 3H), 3.29 (s, 1H), 2.11 - 1.98 (m, 1H), 1.68 - 1.58 (m, 1H). MS (ESI) m/z 193.3 [M+H]⁺

Step 2. To a solution of 1-(2-chloroethyl)-3-(tetrahydrofuran-3-yl)urea (1.00 g, 5.20 mmol, 1.00 *eq*) in tetrahydrofuran (10.0 mL) was added sodium hydride (315 mg, 7.79 mmol, 60% purity, 1.50 *eq*) at 0 °C under nitrogen atmosphere. The mixture was stirred at 25 °C for 2 h. The mixture was quenched with saturated ammonium chloride aqueous solution (10.0 mL) at 0 °C, then extracted with ethyl acetate (3 × 100 mL) dried over sodium sulfate, filtered and concentrated under reduced pressure to give 1-(tetrahydrofuran-3-yl)imidazolidin-2-one (410 mg, 2.63 mmol, 50%) yield) as a white solid.

¹H NMR (400 MHz, DMSO-*d*₆) δ = 6.33 (br s, 1H), 4.39 - 4.31 (m, 1H), 3.82 (dt, *J* = 5.6, 8.4 Hz, 1H), 3.67 - 3.57 (m, 3H), 3.32 - 3.29 (m, 2H), 3.24 - 3.17 (m, 2H), 2.08 - 1.96 (m, 1H), 1.87 - 1.75 (m, 1H). MS (ESI) m/z 157.3 [M+H]⁺

Step 3. To a solution of 1-(tetrahydrofuran-3-yl)imidazolidin-2-one (200 mg, 128 µmol, 1.00 *eq*), 3-(5-bromo-1-oxoisoindolin-2-yl)piperidine-2,6-dione (410 mg, 128 µmol, 1.00 *eq*), cesium carbonate (831 mg, 256 µmol, 2.00 *eq*) in dioxane (5.00 mL) were added 4,5-bis(diphenylphosphino)-9,9-dimethylxanthene (14.8 mg, 25.6 µmol, 0.200 *eq*) and [2'-(Amino)[1,1'-biphenyl][bis(1,1-dimethyletheyl)[2',4,'6'-tris(1-methylethyl)[1,1'-biphenyl]-2-yl]phosphine](methanesfulfonato)palladium (101 mg, 12.8 µmol, 0.100 *eq).* The mixture was stirred at 100 °C for 12 h. The mixture was concentrated under reduced pressure. The residue was purified by *Prep*-HPLC (column: Phenomenex luna C18 150 * 25mm * 10um;mobile phase: [water(Formic acid)-acetonitrile];gradient:2%-32% B over 13 min) to give 3-(1-oxo-5-(2-oxo-3-(tetrahydrofuran-3-yl)imidazolidin-1-yl)isoindolin-2-yl)piperidine-2,6-dione (20.8 mg, 51.2 µmol, 2.1% yield) as a white solid. ¹H NMR (400 MHz, DMSO-*d*₆) δ = 11.03 - 10.89 (m, 1H), 7.81 (s, 1H), 7.75 - 7.69 (m, 1H), 7.68 - 7.61 (m, 1H), 5.12 - 4.99 (m, 1H), 4.59 - 4.50 (m, 1H), 4.46 - 4.36 (m, 1H), 4.33 - 4.23 (m, 1H), 3.94 - 3.79 (m, 3H), 3.78 - 3.73 (m, 1H), 3.72 - 3.60 (m, 2H), 3.56 - 3.47 (m, 2H), 2.97 - 2.83 (m, 1H), 2.64 - 2.54 (m, 1H), 2.40 - 2.31 (m, 1H), 2.20 - 2.06 (m, 1H), 2.03 - 1.89 (m, 2H). MS (ESI) m/z 399.0 [M+H]⁺

### Example 76. Synthesis of Compound 171

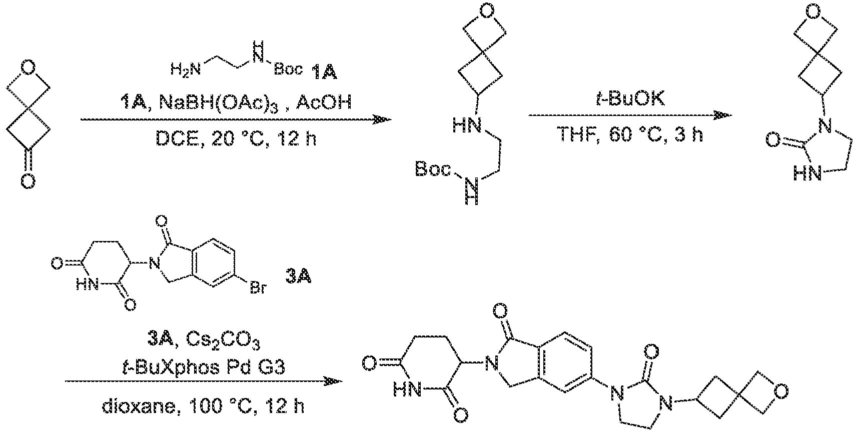

Step 1. To a solution of 2-oxaspiro[3.3]heptan-6-one (500 mg, 4.46 mmol, 1.00 *eq)* and *tert-butyl* (2-aminoethyl)carbamate (857 mg, 5.35 mmol, 844 µL, 1.20 *eq)* in 1,2-dichloroethane (10.0 mL), the mixture was stirred at 20 °C for 1 h, then sodium triacetoxyborohydride (2.84 g, 13.4 mmol, 3.00 *eq)* and acetic acid (2.68 g, 44.6 mmol, 2.55 mL, 10.0 *eq)* was added, the mixture was stirred at 20 °C for 11 h. The reaction mixture was concentrated to give a residue. The residue was purified by silica gel chromatography (Ethyl acetate/methanol = 5/1) to afford *tert-*butyl (2-((2-oxaspiro[3.3]heptan-6-yl)amino)ethyl)carbamate (1.50 g, crude) as yellow oil. ¹H NMR (400 MHz, DMSO-*d*₆) *δ* = 4.53 (s, 2H), 4.43 (s, 2H), 3.06 (br d, *J* = 6.0 Hz, 3H), 2.95 (br d, *J* = 7.0 Hz, 2H), 2.65 - 2.59 (m, 2H), 2.38 (br dd, *J* = 3.2, 7.2 Hz, 2H), 1.37 (br s, 9H).

Step 2. To a solution of *tert*-butyl (2-((2-oxaspiro[3.3]heptan-6-yl)amino)ethyl)carbamate (500 mg, 1.95 mmol, 1.00 *eq)* in tetrahydrofuran (5.00 mL) was added potassium *tert*-butoxide (657 mg, 5.85 mmol, 3.00 *eq),* the mixture was stirred at 60 °C for 3 h. The mixture was filtered to give filtrate, then was concentrated under reduced pressure to give a residue. The residue was purified by reversed phase-HPLC (FA condition) to afford 1-(2-oxaspiro[3.3]heptan-6-yl)imidazolidin-2-one (100 mg, 549 umol, 28% yield) as a white solid. ¹H NMR (400 MHz, DMSO-*d*₆) *δ* = 4.60 (s, 2H), 4.46 (s, 2H), 4.05 (d, *J* = 8.6 Hz, 1H), 3.30 - 3.26 (m, 2H), 3.20 (br d, *J* = 8.2 Hz, 2H), 2.29 (d, *J* = 8.6 Hz, 4H) (The spectra comes from pilot run.)

Step 3. To a solution of 1-(2-oxaspiro[3.3]heptan-6-yl)imidazolidin-2-one (40.0 mg, 220 µmol, 1.00 *eq*) and 3-(5-bromo-1-oxoisoindolin-2-yl)piperidine-2,6-dione (106 mg, 329 µmol, 1.50 *eq*) in dimethylformamide (1.00 mL) was added cesium carbonate (215 mg, 659 µmol, 3.00 *eq*) and methanesulfonato(2-di-t-butylphosphino-2,4,6-tri-i-propyl-1,1-biphenyl)(2-amino-1,1-biphenyl-2-yl)palladium(II) (17.4 mg, 22.0 µmol, 0.100 *eq*), the mixture was stirred at 100 °C for 12 h under nitrogen atmosphere. The reaction mixture was diluted with water (30.0 mL) and exacted with ethyl acetate (3 × 30.0 mL). The organic phase was separated, washed with brine (2 × 20.0 mL), dried over sodium sulfate, filtered and concentrated under reduced pressure to give a residue. The residue was purified by *Prep-*HPLC (column: Phenomenex luna C18 150*25mm* 10um;mobile phase: [water(FA)-ACN]-gradient:10%-40% B over 10 min) to afford 3-(1-oxo-5-(2-oxo-3-(2-oxaspiro[3.3]heptan-6-yl)imidazolidin-1-yl)isoindolin-2-yl)piperidine-2,6-dione (27.6 mg, 60.6 µmol, 9% yield, 93% purity) as a white solid. ¹H NMR (400 MHz, DMSO-d₆) δ = 10.97 (s, 1H), 7.79 (s, 1H), 7.73 - 7.69 (m, 1H), 7.67 - 7.64 (m, 1H), 5.09 (dd, *J* = 5.2, 13.3 Hz, 1H), 4.65 (s, 2H), 4.51 (s, 2H), 4.45 - 4.38 (m, 1H), 4.32 - 4.20 (m, 2H), 3.87 (dd, *J* = 6.6, 9.0 Hz, 2H), 3.57 - 3.48 (m, 2H), 2.99 - 2.85 (m, 1H), 2.62 (br d, *J* = 2.0 Hz, 1H), 2.42 (br d, *J* = 8.5 Hz, 4H), 2.38 (br s, 1H), 2.03 - 1.96 (m, 1H). MS (ESI) m/z 425.3 [M+H]⁺

### Example 77. Synthesis of Compound 153

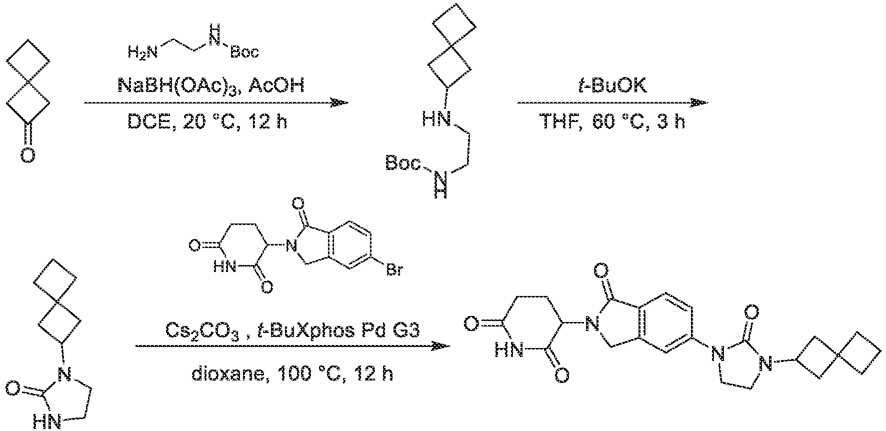

Step 1. To a solution of spiro[3.3]heptan-2-one (800 mg, 7.26 mmol, 1.00 *eq)* and *tert*-butyl (2-aminoethyl)carbamate (1.40 g, 8.72 mmol, 1.37 mL, 1.20 *eq)* in 1,2-dichloroethane (20.0 mL) was stirred at 20 °C for 1 h. Then sodium triacetoxyborohydride (4.62 g, 21.7 mmol, 3.00 *eq)* and acetic acid (4.36 g, 72.6 mmol, 4.16 mL, 10.0 *eq*) was added and the mixture was stirred at 20 °C for 11 h. The mixture was concentrated to give a residue. The residue was purified by silica gel chromatography (Petroleum ether/Ethyl acetate = 0/1) to afford *tert*-butyl (2-(spiro[3.3]heptan-2-ylamino)ethyl)carbamate (1.80 g, 7.08 mmol, 97% yield) as brown oil. ¹H NMR (400 MHz, CDCl₃) *δ* = 6.18 (br s, 1H), 3.38 (br s, 3H), 2.86 (br s, 2H), 2.32 (br s, 2H), 2.11 (br d, *J* = 9.4 Hz, 2H), 2.02 - 1.93 (m, 4H), 1.88 - 1.79 (m, 2H), 1.44 (s, 9H). MS (ESI) m/z 255.3 [M+H]⁺

Step 2. To a solution of *tert*-butyl (2-(spiro[3.3]heptan-2-ylamino)ethyl)carbamate (900 mg, 3.54 mmol, 1.00 *eq*) and potassium *tert*-butoxide (1.19 g, 10.6 mmol, 3.00 *eq*) in tetrahydrofuran (5.00 mL) was stirred at 60 °C for 3 h. The reaction mixture was poured into water (100 mL) and extracted with dichloromethane (3 × 50.0 mL). The combined organic phase was separated, washed with brine (100 mL), dried over anhydrous sodium sulfate, filtered and concentrated to afford 1-(spiro[3.3]heptan-2-yl)imidazolidin-2-one (500 mg, 2.77 mmol, 78% yield) as a yellow solid. ¹H NMR (400 MHz, DMSO-*d₆*) *δ* = 6.24 (br s, 1H), 4.14 - 4.03 (m, 1H), 3.34 - 3.31 (m, 2H), 3.23 - 3.15 (m, 2H), 2.07 - 1.98 (m, 6H), 1.91 - 1.85 (m, 2H), 1.82 - 1.75 (m, 2H). MS (ESI) m/z 181.2 [H+H]⁺

Step 3. To a solution of 3-(5-bromo-1-oxoisoindolin-2-yl)piperidine-2,6-dione (300 mg, 928 µmol, 1.50 *eq*)*,* 1-(spiro[3.3]heptan-2-yl)imidazolidin-2-one (111 mg, 618 umol, 1.00 *eq*), methanesulfonato(2-di-t-butylphosphino-2,4,6-tri-i-propyl-1,1-biphenyl)(2-amino-1,1-biphenyl-2-yl)palladium(II) (491 mg, 618 µmol, 1.00 *eq*) and cesium carbonate (604 mg, 1.86 mmol, 3.00 *eq*) in dimethyl formamide (10.0 mL) was stirred at 100 °C for 12 h. The reaction mixture was diluted with dioxane (50 mL), filtered and concentrated to give a residue. The residue was purified by reversed-phase HPLC (column: spherical C18, 20-45 um, 100A, SW 40, mobile phase: [water (0.1% Formic Acid) - acetonitrile) to afford 3-(1-oxo-5-(2-oxo-3-(spiro[3.3]heptan-2-yl)imidazolidin-1-yl)isoindolin-2-yl)piperidine-2,6-dione (61.54 mg, 114 umol, 23% yield, 99% purity) as an off-white solid. ¹H NMR (400 MHz, DMSO-*d*₆) *δ* = 10.96 (br s, 1H), 7.80 (s, 1H), 7.74 - 7.68 (m, 1H), 7.68 - 7.63 (m, 1H), 5.13 - 5.04 (m, 1H), 4.47 - 4.36 (m, 1H), 4.32 - 4.21 (m, 2H), 3.87 (br t, *J* = 7.6 Hz, 2H), 3.53 (br t, *J* = 8.0 Hz, 2H), 2.97 - 2.85 (m, 1H), 2.64 - 2.59 (m, 1H), 2.39 (br dd, *J =* 4.2, 13.2 Hz, 1H), 2.22 - 2.12 (m, 4H), 2.07 (br t, *J* = 7.2 Hz, 2H), 2.03 - 1.97 (m, 1H), 1.96 - 1.90 (m, 2H), 1.85 - 1.77 (m, 2H). MS (ESI) m/z 423.2 [M+H]⁺

### Example 78. Synthesis of Compound 159

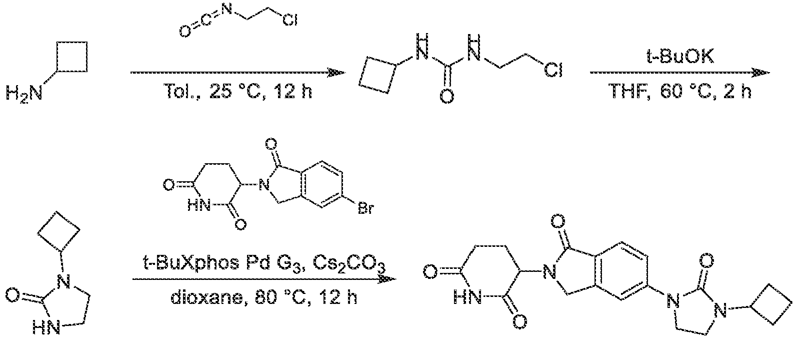

Step 1. To a solution of cyclobutanamine (1.00 g, 14.1 mmol, 1.20 mL, 1.00 *eq)* and 1-chloro-2-isocyanatoethane (2.23 g, 21.1 mmol, 1.80 mL, 1.50 *eq)* in toluene (10.0 mL) was stirred at 25 °C for 12 h. The reaction mixture was filtered to afford 1-(2-chloroethyl)-3-cyclobutylurea (1.00 g, 5.66 mmol, 40% yield) as a white solid. ¹H NMR (400 MHz, DMSO-*d*₆) *δ* = 6.34 (br d, *J* = 8.4 Hz, 1H), 6.03 (br t, *J* = 5.6 Hz, 1H), 4.08 - 3.95 (m, 1H), 3.54 (t, *J*= 6.4 Hz, 2H), 3.27 (q, *J*= 6.4 Hz, 2H), 2.12 (ttd, *J* = 2.4, 5.0, 13.6 Hz, 2H), 1.84 - 1.66 (m, 2H), 1.61 - 1.43 (m, 2H). MS (ESI) m/z 177.2 [M+H]⁺

Step 2. To a solution of 1-(2-chloroethyl)-3-cyclobutylurea (700 mg, 3.96 mmol, *1.00 eq)* and potassium *tert-*butoxide (1.33 g, 11.9 mmol, 3.00 *eq)* in tetrahydrofuran (20.0 mL) was stirred at 60 °C for 2 h. The reaction was filtered to give filtrate and concentrated under reduced pressure to give a residue. The residue was purified by column chromatography (silicon dioxide, Petroleum ether/Ethyl acetate = 1/1 to 0/1) to afford 1-cyclobutylimidazolidin-2-one (180 mg, 1.28 mmol, 32% yield) as a white solid. ¹H NMR (400 MHz, CDCl₃) *δ* = 4.43 (quin, *J* = 8.8 Hz, 1H), 3.53 - 3.47 (m, 2H), 3.43 - 3.37 (m, 2H), 2.17 - 2.05 (m, 4H), 1.70 - 1.538 (m, 2H). MS (ESI) m/z 141.2 [M+H]⁺.

Step 3. To a solution of 1-cyclobutylimidazolidin-2-one (60.0 mg, 428 µmol, 1.00 *eq*) and 3-(5-bromo-1-oxoisoindolin-2-yl)piperidine-2,6-dione (166 mg, 514 µmol, 1.20 *eq*) in dioxane (2.00 mL) was added cesium carbonate (418 mg, 1.28 mmol, 3.00 *eq*) and [2-(2-aminophenyl)phenyl]-methylsulfonyloxy-palladium;ditert-butyl-[2-(2,4,6-triisopropylphenyl)phenyl]phosphane (34.0 mg, 42.8 µmol, 0.100 *eq*)*.* The reaction was stirred at 80 °C for 12 h under nitrogen. The reaction was filtered to give filtrate and concentrated under reduced pressure to give a residue. The residue was purified by *Prep-*HPLC (column: Phenomenex luna C18 150*25mm* 10um;mobile phase: [water(FA)-ACN];gradient:20%-50% B over 10 min) to afford 3-(5-(3-cyclobutyl-2-oxoimidazolidin-1-yl)-1-oxoisoindolin-2-yl)piperidine-2,6-dione (16.25 mg, 41.6 µmol, 3% yield, 98% purity) was obtained as a white solid. ¹H NMR (400 MHz, DMSO-*d*₆) *δ* = 10.97 (br s, 1H), 7.80 (s, 1H), 7.74 - 7.68 (m, 1H), 7.68 - 7.62 (m, 1H), 5.08 (dd, *J* = 5.2, 13.2 Hz, 1H), 4.46 - 4.35 (m, 2H), 4.32 - 4.24 (m, 1H), 3.94 - 3.82 (m, 2H), 3.63 - 3.54 (m, 2H), 2.98 - 2.84 (m, 1H), 2.59 (br d, *J =* 16.8 Hz, 1H), 2.41 - 2.32 (m, 1H), 2.29 - 2.17 (m, 2*H*), 2.07 - 1.94 (m, 3H), 1.71 - 1.57 (m, 2H). MS (ESI) m/z 382.9 [M+H]⁺

### Example 79. Synthesis of Compound 179

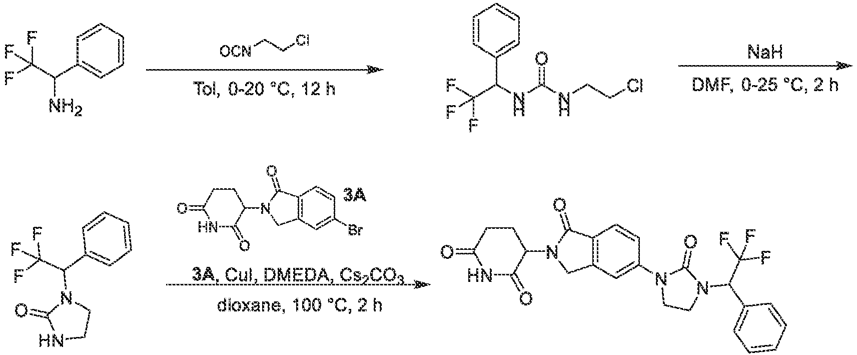

Step 1. To a solution of 2,2,2-trifluoro-1-phenylethan-1-amine (400 mg, 2.28 mmol, 1.00 *eq*) in toluene (1.00 mL) was added 1-chloro-2-isocyanatoethane (361 mg, 3.43 mmol, 292 µL, 1.50 *eq*) at 0 °C , the mixture was stirred at 20 °C for 12 h. The mixture was filtered to give a filter cake, then was concentrated under reduced pressure to afford 1-(2-chloroethyl)-3-(2,2,2-trifluoro-1-phenylethyl)urea (500 mg, 1.78 mmol, 78% yield) as a white solid. MS (ESI) m/z 281.1 [M+H]⁺

Step 2. To a solution of 1-(2-chloroethyl)-3-(2,2,2-trifluoro-1-phenylethyl)urea (500 mg, 1.78 mmol, 1.00 *eq*) in dimethylformamide (5.00 mL) was added sodium hydride (85.5 mg, 2.14 mmol, 60% purity, 1.20 *eq*) at 0 °C, the mixture was stirred at 25 °C for 2 h. The reaction mixture was quenched with water (2.00 mL) and the mixture was filtered to give filtrate. The filtrate was purified by reversed phase-HPLC (0.1% FA condition) to afford 1-(2,2,2-trifluoro-1-phenylethyl)imidazolidin-2-one (300 mg, 1.23 mmol, 69% yield) as a white solid. ¹H NMR (400 MHz, DMSO-*d*₆) *δ* = 7.50 - 7.44 (m, 3H), 7.43 - 7.38 (m, 2H), 6.91 (s, 1H), 5.68 (q, *J* = 9.6 Hz, 1H), 3.53 - 3.43 (m, 1H), 3.34 - 3.29 (m, 1H), 3.22 (dt, *J* = 4.6, 8.8 Hz, 1H), 3.06 (q, *J* = 8.6 Hz, 1H). MS (ESI) m/z 245.1 [M+H]⁺

Step 3. To a solution of 1-(2,2,2-trifluoro-1-phenylethyl)imidazolidin-2-one (50.0 mg, 205 µmol, 1.00 *eq*) and 3-(5-bromo-1-oxoisoindolin-2-yl)piperidine-2,6-dione (79.4 mg, 246 µmol, 1.20 *eq)* in dioxane (1.00 mL) was added potassium carbonate (84.9 mg, 614 µmol, 3.00 *eq*)*, N,N*-dimethylethylenediamine (18.1 mg, 205 µmol, 22.0 µL, 1.00 *e*q) and copper iodide (39.0 mg, 205 µmol, 1.00 *eq*), the mixture was stirred at 100 °C for 2 h under nitrogen atmosphere. The mixture was filtered to give filtrate, then purified by *Prep*-HPLC (column: Phenomenex Luna C18 150*25mm*10um;mobile phase: [water(FA)-ACN];gradient:35%-65% B over 10 min) to afford 3-(1-oxo-5-(2-oxo-3-(2,2,2-trifluoro-1-phenylethyl)imidazolidin-1-yl)isoindolin-2-yl)piperidine-2,6-dione (15.98 mg, 32.5 µmol, 8% yield, 99% purity) as a gray solid. ¹H NMR (400 MHz, DMSO-*d*₆) *δ* = 10.98 (s, 1H), 7.85 (br s, 1H), 7.76 - 7.69 (m, 2H), 7.53 - 7.49 (m, 5H), 5.97 - 5.85 (m, 1H), 5.10 (dd, *J* = 4.8, 13.2 Hz, 1H), 4.48 - 4.40 (m, 1H), 4.37 - 4.27 (m, 1H), 4.02 - 3.90 (m, 2H), 3.73 - 3.64 (m, 1H), 3.28 (br s, 1H), 2.97 - 2.85 (m, 1H), 2.62 (br d, *J* = 2.4 Hz, 1H), 2.40 (br dd, *J* = 4.2, 12.8 Hz, 1H), 2.07 - 1.95 (m, 1H). MS (ESI) m/z 487.2 [M+H]⁺

### Example 80. Synthesis of Compound 178

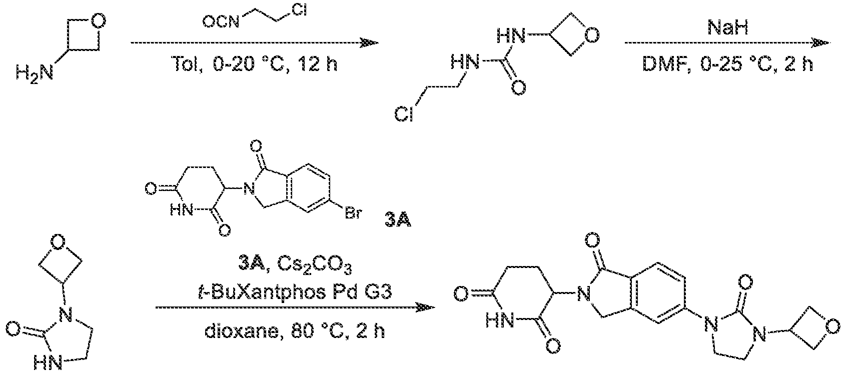

Step 1. To a solution of oxetan-3-amine (1.00 g, 13.7 mmol, 1.00 *eq)* in toluene (10.0 mL) was added 1-chloro-2-isocyanatoethane (2.17 g, 20.5 mmol, 1.75 mL, 1.50 *eq)* at 0 °C, the mixture was stirred at 20 °C for 12 h. The mixture was filtered to give a filter cake, then was concentrated under reduced pressure to afford 1-(2-chloroethyl)-3-('oxetan-3-yl)urea (2.00 g, 11.2 mmol, 82% yield) as a white solid. ¹H NMR (400 MHz, DMSO-*d*₆) *δ* = 6.85 (br d, *J* = 6.0 Hz, 1H), 6.21 (br t, *J* = 5.6 Hz, 1H), 4.73 - 4.64 (m, 3H), 4.38 - 4.32 (m, 2H), 3.57 (t, *J* = 6.2 Hz, 2H), 3.30 (d, *J* = 6.2 Hz, 2H).

Step 2. To a solution of 1-(2-chloroethyl)-3-(oxetan-3-yl)urea (2.00 g, 11.2 mmol, 1.00 *eq)* in dimethylformamide (20.0 mL) was added sodium hydride (537 mg, 13.4 mmol, 60% purity, 1.20 *eq*) *at* 0 °C, the mixture was stirred at 25°C for 2 h. The reaction mixture was quenched with methanol (10.0 mL), then filtered to give a filter cake, then was concentrated under reduced pressure to give a residue. The residue was purified by *Prep-*HPLC (column: Waters Atlantis T3 150*30mm*5mm;mobile phase: [water(FA)-ACN];B%: 1%-20%,10mm) to afford 1-(oxetan-3-yl)imidazolidin-2-one (200 mg, 1.41 mmol, 13% yield) as a white solid. ¹H NMR (400 MHz, DMSO-*d*₆) *δ* = 4.85 (quin, *J* = 7.2 Hz, 1H), 4.70 (t, *J* = 6.6 Hz, 2H), 4.61 (d, *J* = 7.2 Hz, 2H), 3.59 - 3.52 (m, 2H), 3.29 - 3.25 (m, 2H).

Step 3. To a solution of 1-(oxetan-3-yl)imidazolidin-2-one (20.0 mg, 141 µmol, *1.20 eq)* and 3-(5-bromo-1-oxoisoindolin-2-yl)piperidine-2,6-dione (37.9 mg, 117 µmol, 1.00 *eq)* in dimethylformamide (2.00 mL) was added cesium carbonate (115 mg, 352 µmol, 3.00 *eq*) and methanesulfonato(2-di-t-butylphosphino-2,4,6-tri-i-propyl-1,1-biphenyl)(2-amino-1,1-biphenyl-2-yl)palladium(II) (9.31 mg, 11.7 µmol, 0.100 *eq*), the mixture was stirred at 80 °C for 2 h under nitrogen atmosphere. The reaction mixture was diluted with water (30.0 mL) and exacted with ethyl acetate (3 × 30.0 mL). The organic phase was separated, washed with brine (2 × 20.0 mL), dried over sodium sulfate, filtered and concentrated under reduced pressure to give a residue. The residue was purified by *Prep*-HPLC (column: Phenomenex luna C18 150*25mm* 10um;mobile phase: [water(FA)-ACN];B%: 5%-35%,10min) to afford 3-(5-(3-(oxetan-3-yl)-2-oxoimidazolidin-1-yl)-1-oxoisoindolin-2-yl)piperidine-2,6-dione (20.62 mg, 53.6 µmol, 23 % yield) as a white solid. ¹H NMR (400 MHz, DMSO-d₆) δ *=* 10.98 (br s, 1H), 7.82 (s, 1H), 7.75 - 7.71 (m, 1H), 7.69 - 7.66 (m, 1H), 5.11 - 5.04 (m, 2H), 4.80 (t, *J* = 6.6 Hz, 2H), 4.73 - 4.67 (m, 2H), 4.47 - 4.39 (m, 1H), 4.33 - 4.26 (m, 1H), 3.98 - 3.93 (m, 2H), 3.81 - 3.76 (m, 2H), 2.94 - 2.87 (m, 1H), 2.62 (br d, *J* = 2.2 Hz, 1H), 2.39 (br dd, *J* = 4.8, 13.2 Hz, 1H), 2.03 - 1.96 (m, 1H). MS (ESI) m/z 385.1 [M+H]⁺

### Example 81. Synthesis of Compounds 158 and 173

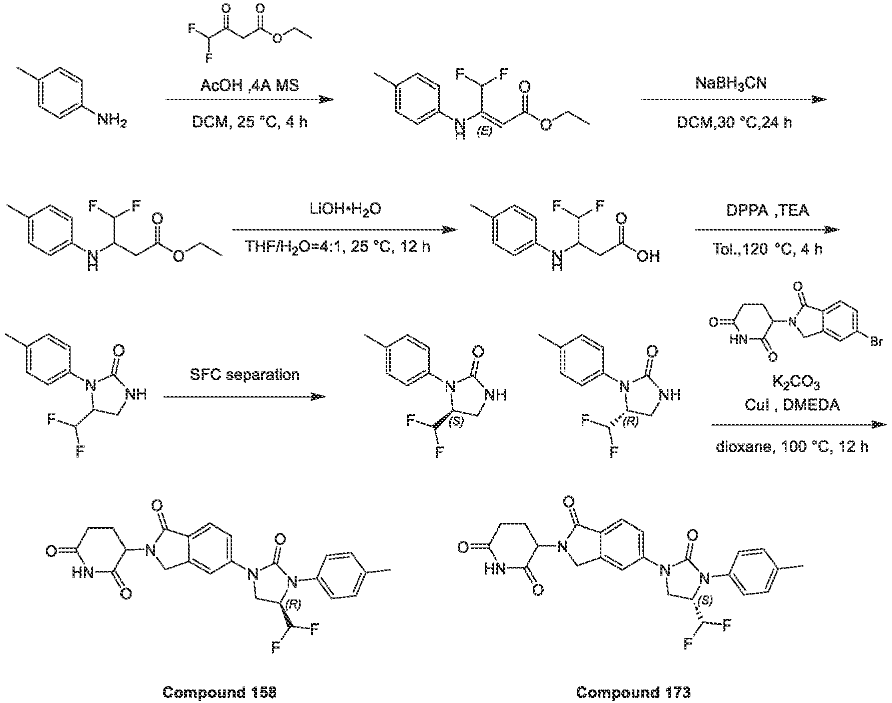

Step 1. To a solution of *p*-toluidine (10.0 g, 93.3 mmol, 10.2 mL, 1.00 *eq*) and ethyl 4,4-difluoro-3-oxobutanoate (15.5 g, 93.3 mmol, 1.00 *eq*) in dichloromethane (200 mL) was added acetic acid (560 mg, 9.33 mmol, 533 uL, 0.10 *eq*) and Molecular sieves 4A (10.0 g, 1.87 mmol). The mixture was stirred at 25 °C for 4 h under nitrogen atmosphere. The mixture was filtered to afford (*E*)-ethyl 4,4-difluoro-3-(p-tolylamino)but-2-enoate (23.0 g, crude) as brown liquid

Step 2. To a solution of ethyl (*E*)-ethyl 4,4-difluoro-3-(p-tolylamino)but-2-enoate (23.0 g, 90.1 mmol, 1.00 *eq*) in dichloromethane (100.0 mL) was added sodium cyanoborohydride (33.9 g, 540 mmol, 6.00 *eq*) at 0 °C. The mixture was stirred at 30 °C for 24 h. The reaction mixture was diluted with water (30.0 mL) and extracted with ethyl acetate (2 × 30 mL). The organic phase was separated, washed with brine (10.0 mL), dried over sodium sulfate, filtered and concentrated under reduced pressure to give a residue. The crude product was purified by reversed-phase HPLC (column: spherical C18, 20-45 um, 100Å, SW 40, mobile phase: [water-ACN];B%: 5%-45%, 30 min). The desired fraction was collected and lyophilized to afford ethyl 4,4-difluoro-3-(p-tolylamino)butanocate (8.40 g, 32.6 mmol, 18% yield) as brown oil. ¹H NMR (400 MHz, DMSO-*d₆*) *δ =* 6.89 (d, *J* = 8.3 Hz, 2H), 6.59 (d, *J =* 8.4 Hz, 2H), 6.02 (dt, *J* = 2.8, 56 Hz, 1H), 4.21 - 4.07 (m, 1H), 4.07 - 3.95 (m, 2H), 2.69 (dd, *J* = 4.5, 15.8 Hz, 1H), 2.47 (s, 1H), 2.14 (s, 3H), 1.12 (t, *J =* 7.1 Hz, 3H). MS (ESI) m/z 258.2 [M+H]⁺

Step 3. To a solution of ethyl 4,4-difluoro-3-(p-tolylamino)butanoate (6.50 g, 25.2 mmol, 1.00 *eq*) in tetrahydrofuran (20.0 mL) and water (2.00 mL) was added lithium hydroxide monohydrate (3.18 g, 75.7 mmol, 3.00 *eq*)*.* The mixture was stirred at 25 °C for 12 h. The mixture was concentrated under reduced pressure to afford 4,4-difluoro-3-(*p-*tolylamino)butanoic acid (12.0 g, crude) as a yellow solid. MS (ESI) m/z 230.2 [M+H]⁺

Step 4. To a solution of 4,4-difluoro-3-(*p*-tolylamino)butanoic acid (5.50 g, 23.9 mmol, 1.00 *eq*) in toluene (100.0 mL) was added Molecular sieves 4A (5.50 g). Then diphenylphosphoryl azide (13.2 g, 47.9 mmol, 10.4 mL, 2.00 *eq*), triethylamine (4.86 g, 47.9 mmol, 6.68 mL, 2.00 *eq*) was added into the mixture. Then the mixture was stirred at 120 °C for 4 h under nitrogen. The mixture was filtered to give the filtrate. The filtrate was purified by reversed-phase HPLC (column: spherical C18, 20-45 um, 100Å, SW 330, mobile phase: fwater(0.1%Formic Acid)-ACN];B%: 5%-80%, 30 min). The desired fraction was collected and lyophilized to afford 5-(difluoromethyl)-1-(*p*-tolyl)imidazolidin-2-one (4.00 g, 17.6 mmol, 36% yield) as a black solid. ¹H NMR (400 MHz, DMSO-d₆) δ = 7.38 (d, *J* = 8.5 Hz, 2H), 7.13 (d, *J =* 8.4 Hz, 2H), 7.02 (s, 1H), 6.13 (dt, *J* = 3.2, 54.8 Hz, 1H), 4.94 - 4.79 (m, 1H), 3.59 (t, *J =* 9.8 Hz, 1H), 3.35 (dd, *J* = 4.1, 9.9 Hz, 11-f), 2.26 (s, 3H). MS (ESI) m/z 227.4 [M+H]⁺

Step 5. The product 5-(difluoromethyl)-1-(p-tolyl)imidazolidin-2-one (1.00 g) was purified by SFC (column: DAICEL CHIRALPAK AS(250mm*30mm,10um);mobile phase: [0 1%NH₃H₂O MeOH];B%: 25%-25%,0min). The desired fraction was concentrated reduced pressure to afford (*S*)-5-(difluoromethyl)-1-(*p*-tolyl)imidazolidin-2-one (460 mg, 2.03 mmol, 46% yield) as a yellow solid and (*R*)-5-(difluoromethyl)-1-(p-tolyl)imidazolidin-2-one (430 mg, 1.90 mmol, 43% yield) was obtained as a yellow solid.

¹H NMR (400 MHz, DMSO-*d₆*) *δ* = 7.37 (d, *J* = 8.5 Hz, 2H), 7.13 (d, *J* = 8.3 Hz, 2H), 7.01 (s, 1H), 6.13 (dt, *J* = 2.8, 54.8 Hz, 1H), 4.95 - 4.79 (m, 1H), 3.59 (t, *J =* 9.8 Hz, 1H), 3.36 (br s, 1H), 2.26 (s, 3H). MS (ESI) m/z 227.1 [M+H]⁺

¹H NMR (400 MHz, DMSO-*d₆*) *δ* = 7.37 (d, *J* = 8.4 Hz, 2H), 7.13 (br d, *J* = 8.3 Hz, 2H), 7.01 (br s, 1H), 6.13 (dt, *J* = 2.8, 55.2 Hz, 1H), 4.94 - 4.79 (m, 1H), 3.59 (br t, *J* = 9.8 Hz, 1H), 3.36 (br s, 1H), 2.26 (s, 3H). MS (ESI) m/z 227.0 [M+H]⁺

Step 6. Compound 158. To a solution of (*R*)-5-(difluoromethyl)-1-(*p-*tolyl)imidazolidin-2-one (50.0 mg, 221 umol, 1.00 *eq*) and 3-(5-bromo-1-oxoisoindolin-2-yl)piperidine-2,6-dione (71.4 mg, 221 umol, 1.00 *eq*) in dioxane (1.00 mL) was added potassium carbonate (91.6 mg, 663) umol, 3.00 *eq*)*,* cuprous iodide (4.21 mg, 22.1 umol, 0*.*100 *eq*) and *N,N*'-dimethylethane-1,2-diamine (9.74 mg, 110 umol, 11.8 uL, 0.500 *eq*). The mixture was stirred at 100 °C for 12 h under nitrogen atmosphere. The mixture was filtered. The filtrate purified by reversed-phase HPLC (column: spherical C18, 20-45 um, 100Å, SW 40, mobile phase: [water-ACN];B%: 5%-45%,20 min). The desired fraction was collected and lyophilized to afford 3-(5-((*R*)-4-(difluoromethyl)-2-oxo-3-(p-tolyl)imidazolidin-1-yl)-1-oxoisoindolin-2-yl)piperidine-2,6-dione (69.7 mg, 144 umol, 32% yield, 97% purity) as a purple solid. ¹H NMR (400 MHz, DMSO-d*₆*) *δ =* 10.98 (br s, 1H), 7.90 (br d, *J =* 4.4 Hz, 1H), 7.84 - 7.78 (m, 1H), 7.71 (d, *J* = 8.5 Hz, 1H), 7.46 (d, *J* = 8.4 Hz, 2H), 7.22 (d, *J* = 8.3 Hz, 2H), 6.25 (dt, *J =* 2.8, 54.4 Hz, 1H), 5.13 - 4.98 (m, 2H), 4.49 - 4.40 (m, 1H), 4.37 - 4.23 (m, 2H), 3.98 (td, *J* = 3.6, 10.1 Hz, 1H), 2.97 - 2.86 (m, 1H), 2.62 - 2.57 (m, 1H), 2.40 (br dd, *J* = 4.6, 13.2 Hz, 1H), 2.31 (s, 3H), 2.01 (br dd, *J* = 5.0, 10.3 Hz, 1H), MS (ESI) m/z 469.1 [M+H]⁺

Step 7. Compound 173. To a solution of (*S*)-5-(difluoromethyl)-1-(*p-*tolylyimidazolidin-2-one (50.0 mg, 221 umol, 1.00 *eq*) and 3-(5-bromo-1-oxoisoindolin-2-yl)piperidine-2,6-dione (71.4 mg, 221 umol, 1.00 *eq*) in dioxane (1.00 mL) was added potassium carbonate (91.6 mg, 663 umol, 3.00 *eq*)*,* cuprous iodide (4.21 mg, 22.1 umol, 0.100 *eq*) and *N,N*'-dimethylethane-1,2-diamine (9.74 mg, 110 umol, 11.8 uL, 0.50 *eq*)*.* The mixture was stirred at 100 °C for 12 h under nitrogen atmosphere. The mixture was filtered. The filtrate was purified by reversed-phase HPLC (column: spherical C18, 20-45 um, 100Å, SW 40, mobile phase: [water-ACN];B%: 5%-45%,20 min). The desired fraction was collected and lyophilized to afford 3-(5-((*S*)-4-(difluoromethyl)-2-oxo-3-(p-tolyl)imidazolidin-1-yl)-1-oxoisoindolin-2-yl)piperidine-2,6-dione (26.4 mg, 54.7 umol, 12% yield, 97% purity) as a purple solid. ¹H NMR (400 MHz, DMSO-d₆) *δ =* 10.98 (br s, 1H), 7.94 - 7.88 (m, 1H), 7.84 - 7.78 (m, 1H), 7.71 (d, *J* = 8.5 Hz, 1H), 7.46 (d, *J =* 8.4 Hz, 2H), 7.22 (d, *J* = 8.4 Hz, 2H), 6.25 (dt, *J* = 2.8, 54.4 Hz, 1H), 5.13 - 4.98 (m, 2H), 4.50 - 4.40 (m, 1H), 4.37 - 4.23 (m, 2H), 3.98 (td, *J* = 3.5, 10.0 Hz, 1H), 2.97 - 2.86 (m, 1H), 2.63 - 2.56 (m, 1H), 2.40 (br dd, *J* = 4.3, 13.1 Hz, 1H), 2.31 (s, 3H), 2.04 - 1.95 (m, 1H), MS (ESI) m/z 469.1 [M+H]⁺

### Example 82. Synthesis of Compounds 114 and 115

Compounds 114 and 115 was prepared using a method analogous to the syntheses of other compounds disclosed herein.

### Example 83. CK1α NanoBit Assay

HEK293 clonal lines with CRISPR KI HiBiT tag on CSNK1A1 and stably expressing LgBiT protein were obtained from Promega (Madison, WI). Cells were plated at 5000 cells per well using Multiflo (BioTek) in 384-well white solid bottom plates (Corning, 3570BC) in 25 µl volume in DMEM media (DMEM, high glucose, HEPES, no phenol red (ThermoFisher Scientific, 21063029)) containing 10% FBS (Corning, 35-075-CV), 1% Peniciliin/Streptomycin ((ThermoFisher Scientific, 15140-122), and 0.2% Endurazine (Nano-Glo Endurazine Live Cell Substrate (Promega, N2571)). Cells were incubated for 16 hours at 37 °C, 5% CO₂. Depending on experiment 25 or 75 nL of a compound at 10 mM were added into the plate using an Echo^{®} 650 liquid handler (Labcyte) to achieve final concentration of 10 or 30 µM in wells. Cells were incubated at 37 °C, 5% CO₂ for 24 hours and then signal was read on a Pherastar FSX using "LUM plus" optic module.

Data analysis was performed in Scinamic (Scinamic, Cambridge, MA). Luminescence response (R) was calculated by the formula: response = 100 * (S - N) / (P-N) where S is the signal of the well, N and P the mean negative and positive control values respectively of the same plate. The luminescence response was then fitted in Scinamic using a 3-parameter agonist logistic fit (hillslope = 1, EC50 > 0, top/bottom unconstrained).

DC₅₀ data are reported in **Table 2** for compounds in **Table 1**. In **Table 2** below, According to the code, A represents a DC₅₀ value of ≤0. 1µM, B represents a DC₅₀ value >0.1 µM and ≤1 µM, C represents an DC₅₀ value >1 µM.

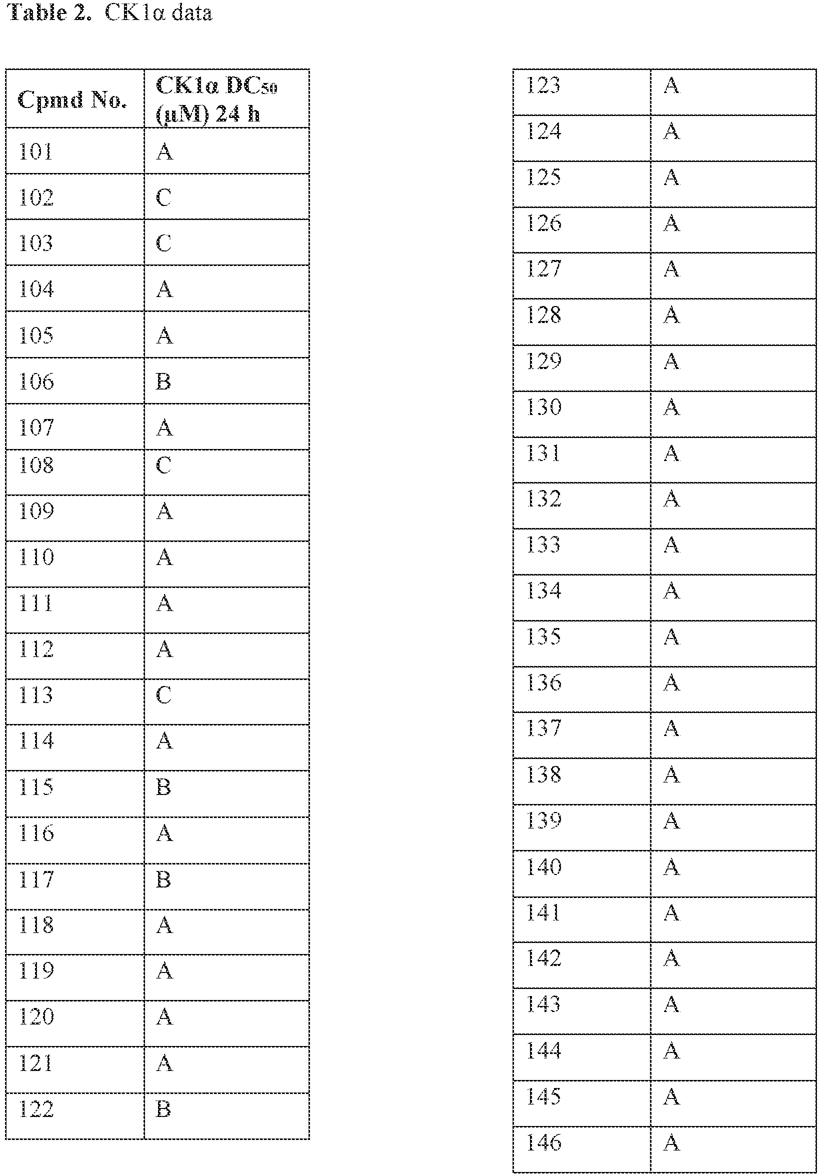

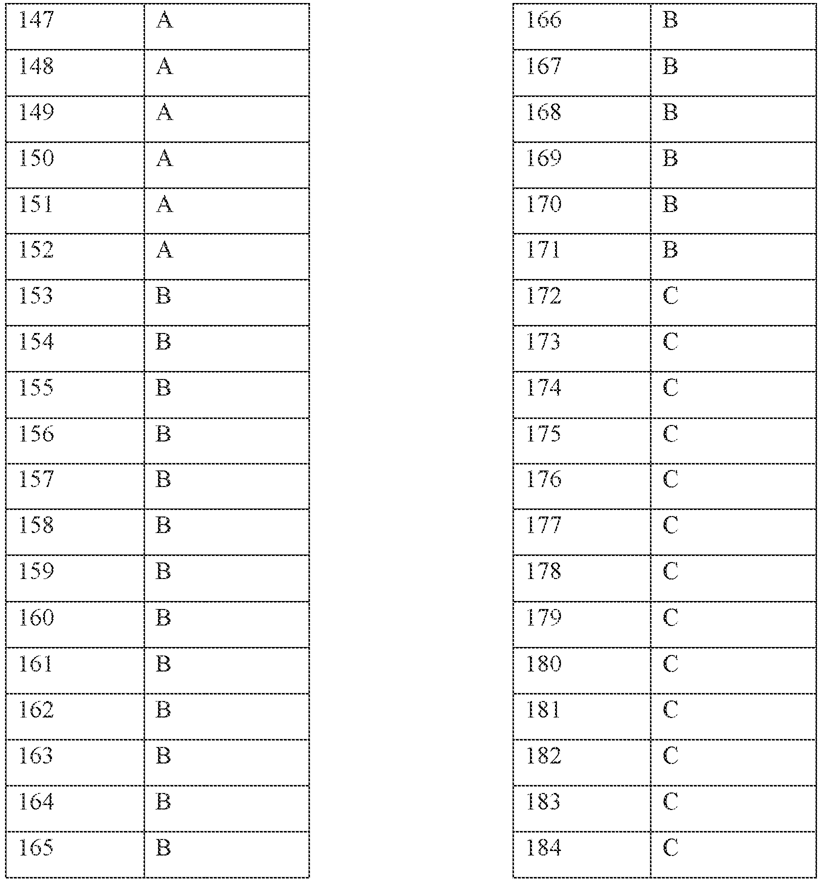

### Example 84. Western Blot Protocol

HEK293 cell line is purchased from ATCC (CRL-1573). HEK293 CRBN knock out (k/o) cell line (B2) is generated internally using CRISPR/Cas9 method and clonally expanded. Cells are plated at 2 X 10⁵ cells per well in 6-well tissue culture plates (VWR) in 2 ml of DMEM media (Gibco) containing 10% FBS (Gibco), and incubated for 16 hours at 37 °C, 5% CO₂. Compounds are added to final concentration of 0.1 µM, 1 µM, 10 µM (DMSO concentration is kept constant at 0.1%), following incubation at 37 °C, 5% CO₂ for additional 24 hours. Cell lysis is performed using RIPA buffer (Pierce) containing Halt^{™} Protease Inhibitor Cocktail (ThermoFisher Scientific). Lysates are boiled at 95 °C for 10 minutes and 12 µg of protein lysate per sample is resolved by SDS-PAGE using 12% gels (BioRad) and transferred to nitrocellulose membrane (BioRad). Membranes are blocked using LI-COR blocking buffer (LI-COR) at room temperature for 1hour, followed by overnight incubation with rabbit anti-CK1α (Abcam ab206652), rabbit anti-CRBN (Sigma HPA045910) and mouse anti-α-tubulin (Sigma T9026) antibodies at 4 °C. Secondary antibodies (LI-COR) are added for 1hour at room temperature, followed by imaging with LI-COR imaging system Odyssey^{®} Clix.

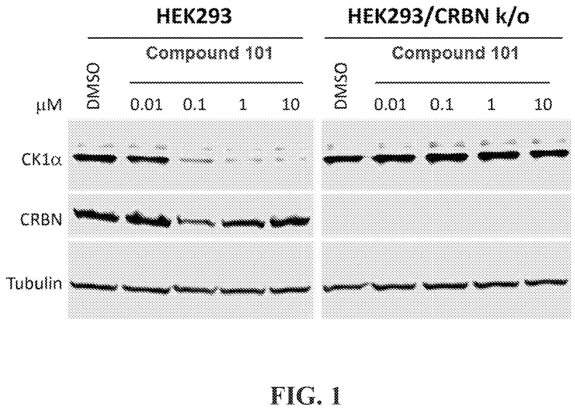

Exemplary Western Blots obtained from the protocol is shown in **FIG. 1****.**

### Example 85. HTRF CRBN & Ternary Complex Assay

### HTRF binding assay

Binding of test compounds to CRBN/DDB1 was monitored in an HTRF assay using 1-[5-({2-[2-(2-{[2-(2,6-dioxopiperidin-3-yl)-1,3-dioxo-2,3-dihydro-1H-isoindol-4-yl]oxy}acetamido)ethoxy]ethyl}carbamoyl)pentyl]-3,3-dimethyl-2-[(1R,3R)-5-[(2R)-1,3,3-trimethyl-5-sulfo-2,3-dihydro-1H-indol-2-ylidene]penta-1,3-dien-1-yl]-3H-indol-1-ium-5-sulfonate as a fluorescent probe. In this assay, compounds displace the fluorescently labeled thalidomide-based probe bound to CRBN and fluorescence is monitored with increasing compound concentration. Assays were conducted in Greiner white 384 well HiBase plates (Cat. No 78-4075-25) in 10 µL total volume. A one pot detection solution of CRBN/DDB1 (2.5 nM), anti-His Terbium Cryptate Gold (1X, PerkinElmer Cat.#: 61HI2TLB), and Cy5-Thalidomide (100 nM, Tenova Cat.: T52461) was prepared in 20 mM HEPES, 20 mM NaCl, 0.2 mM TCEP, 0.2 mM EDTA, and 0.005% Tween20 was dispensed to each assay plate. Test compounds were stored in dry, ambient temperatures at 10 mM. A 10-point, 1:3 dilution series was prepared from 10 mM stock concentrations in Echo-compatible LDV plates. 10 nL of each compound dilution series was dispensed into assays wells using an Echo 650 (Labcyte inc. USA). 10 nL of 10 mM lenalidomide (Selleckchem Cat no. S1029) was transferred into the active-control wells for the assay and 10 nL of DMSO was transferred into the negative control wells. The assay was then allowed to incubate for 30 min at ambient temperature after transferring the test compounds. Plate measurements were taken on a Pherastar FSX (BMG Labtech, Germany) using the HTRF Red filter (Ex. 337 nm, em1: 620 nm, em2: 665 nm) (Flashes: 50, Integration time: 60-400 µs, Z-height: 10 mm, Ratio-multiplier: 10,000). Analysis, HTRF ratio and IC₅₀ values were derived using KNIME analytics (KNIME Zurich) transformation and fitting within Collaborative Drug Discovery (Collaborative Drug Discovery USA) using a 4-parameter logistic fit. HTRF ratio calculation was performed using the following formula: $HTRF Ratio = \frac{{em}_{665 nm}}{{em}_{620 nm}} ∗ 10 , 000$ where *em₆₆₅ₙₘ* represents the measured emission at 665 nm upon excitation at 337 nm and *em₆₂₀ₙₘ* the measured emission at 620nm upon excitation at 337 nm. The 4 parameter logistic fitting model was performed using the following formula: $= {Y}_{Min} + \frac{\left({Y}_{Max}-{Y}_{Min}\right)}{\left(1+{\left(\frac{{IC}_{50}}{X}\right)}^{HillSlope}\right)}$ where *Y* represents the HTRF ratio response (as defined previously), X the compound concentration in µM, *Yₘᵢₙ* the minimum response plateau, *Yₘₐₓ* the maximum response plateau, *IC₅₀* the concentration of agonist that gives a response half way between *Yₘᵢₙ* and *Yₘₐₓ* and *HillSlope* the steepness of the family of curves. No fitting model constraints was applied on *Yₘᵢₙ,* or *Yₘₐₓ* whilst *IC₅₀ >* 0 and -0.5 *= HillSlope >* -3

### Cheng-Prusoff transformation

Ki values were derived from the geometric mean of the IC₅₀ values using the Cheng-Prusoff transformation: ${K}_{i} = \frac{{IC}_{50}}{1 + \left(\frac{\left[L\right]}{{K}_{d}}\right)} = \frac{{IC}_{50}}{1 + \left(\frac{0.1 uM}{0.1216 uM}\right)}$ where *[L]* represents the concentration of fluorescent probe in µM, *K_{d}* the affinity (binding constant) of the fluorescent probe in units of µM and *IC₅₀* the concentration of agonist that gives a response halfway between *Yₘᵢₙ* and *Yₘₐₓ* (as described in 1.2.1)

### Statistical Methods or Analyses

Analysis and IC₅₀ values were derived using KNIME analytics (KNIME, Zurich) transformation and fitting within Collaborative Drug Discovery (Collaborative Drug Discovery, USA) as described in 1.2.1. Ki values were derived from the geometric mean of the IC₅₀ values using the Cheng-Prusoff transformation as described in 2.2.2. Data was visualized in GraphPad Prism 8.1.2 (GraphPad, USA) and reported as mean and standard deviation. Microsoft Office Excel 2012 (Redmond, WA) was used for calculation of mean and standard deviation.

IC₅₀ data are reported in **Table 3** for compounds in **Table 1.** In **Table 3** below, According to the code, A represents a IC₅₀ value of ≤0.1 µM, B represents a IC₅₀ value >0.1 µM and ≤1 µM, C represents an IC₅₀ value >1 µM.

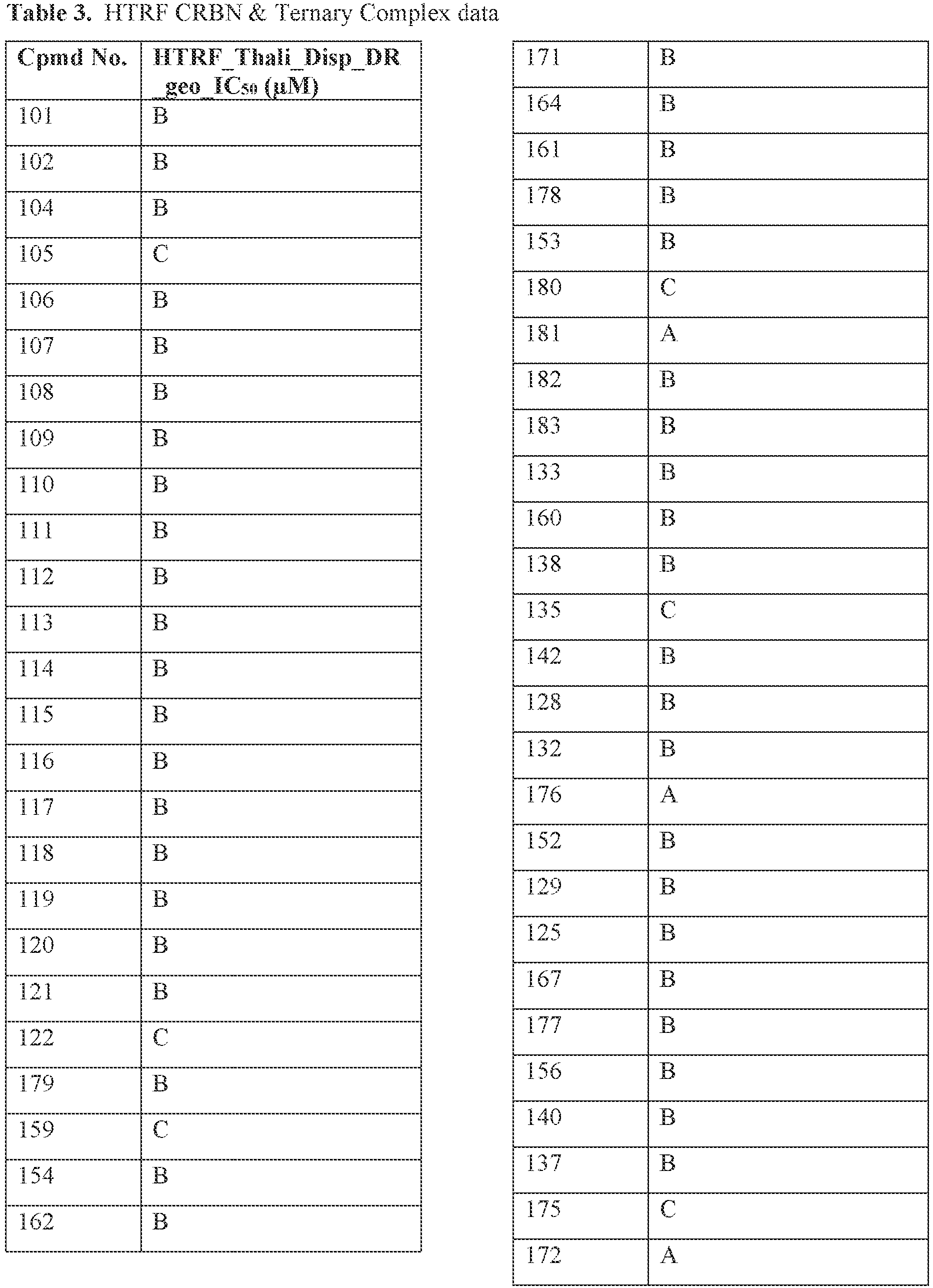

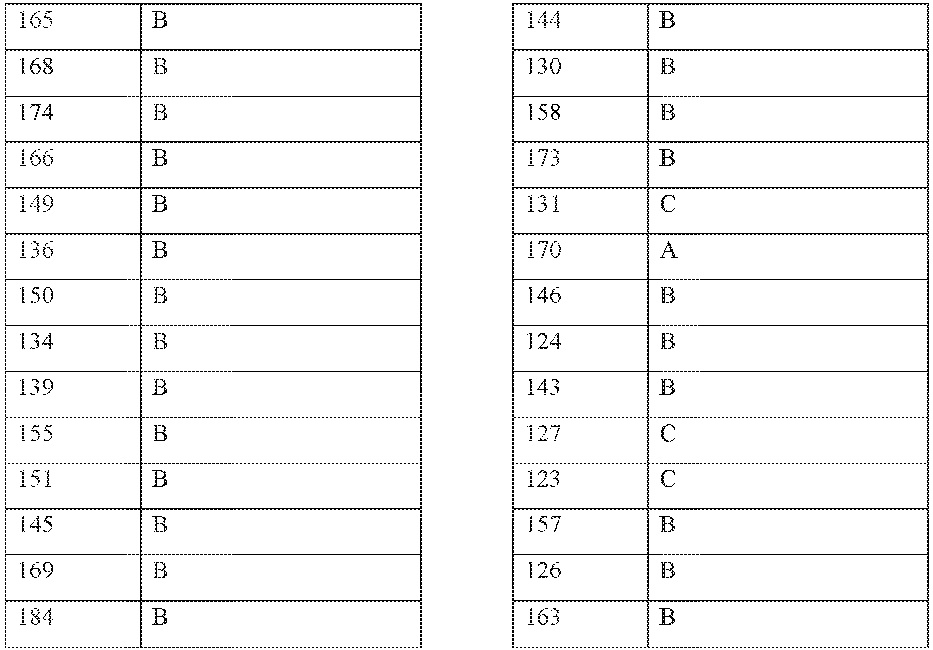

### Example 86. GI₅₀ data

SKCO1, LS180, and LS174T cell lines were purchased from ATCC. CW2 cell line was purchased from Riken. GP2D cell line was purchased from Sigma/ECACC. All cell lines were cultured in manufacturer's recommended media at 37⁰C, 5% CO₂. Briefly, cells were suspended in 200 µL of media and seeded at 300 to 1,800 cells per well in tissue culture treated 96-well plates with black walls and clear bottom. Plates were incubated overnight and initial (T0) read was performed the following day using CyQUANT Direct Cell Proliferation Assay Kit (Thermo Fisher Scientific) according to manufacturer's protocol. All compounds were solubilized in DMSO and prepared at 3-fold serial dilution, using 9 points starting at 30 µM as highest concentration, administered to cells and incubated for 120 to 168 hours, at which point CyQUANT assay measurement was performed using Acumen Cellista instrument (Ex/Em 480/520 nm). Media-only wells were used as blank, and 0.3% DMSO was used as DMSO-treated cell control. Growth inhibition (percent response) was calculated using the following formula in Excel: "=if ([Compound treated Day N] > [Untreated Day 0, AVG], ([Compound treated Day N] - [Untreated Day 0, AVG])/([Untreated Day N] - [Untreated Day 0, AVG])*100, ([Compound Treated Day N] - [Untreated Day 0, AVG])/([Untreated Day 0, AVG])*100)". GI₅₀ is the response corresponding to the 50% of untreated control.

GI₅₀ data are reported in **Tables 4a, 4b, 4c, 4d,** and **4e** for compounds in **Table 1.** In **Tables 4a, 4b, 4c, 4d,** and **4e** below, According to the code, A represents a GI₅₀ value of ≤0.1 µM, B represents a GI₅₀ value >0.1 µM and ≤1 µM, C represents an GI₅₀ value >1 µM.

**Table 4a. SKCO1 GI₅₀ data**

| **Cpmd No.** | **SKCO1** |
|---|---|
| | **GI₅₀ (µM)** |
| 101 | B |
| 104 | B |
| 107 | B |
| 111 | A |
| 124 | B |
| 126 | B |
| 137 | B |
| 123 | A |
| 125 | A |

**Table 4b. CW2 GI₅₀ data**

| **Cpmd No.** | **CW2** |
|---|---|
| | **GI₅₀ (µM)** |
| 101 | B |
| 104 | B |
| 107 | A |
| 111 | A |
| 124 | A |
| 126 | A |
| 137 | B |
| 123 | A |
| 125 | A |

**Table 4c. GP2D GI₅₀ data**

| **Cpmd No.** | **GP2D** |
|---|---|
| | **GI₅₀ (µM)** |
| 101 | C |
| 104 | B |
| 107 | B |
| 111 | B |
| 124 | B |
| 126 | B |
| 137 | C |
| 123 | B |
| 125 | B |

**Table 4d. LS180 GI₅₀ data**

| **Cpmd No.** | **LS180** |
|---|---|
| | **GI₅₀ (µM)** |
| 101 | C |
| 104 | B |
| 111 | B |
| 124 | B |
| 126 | B |
| 137 | C |
| 123 | B |
| 125 | B |

**Table 4e. LS174T GI₅₀ data**

| **Cpmd No.** | **LS174T** |
|---|---|
| | **GI₅₀ (µM)** |
| 101 | C |
| 104 | B |
| 107 | C |
| 111 | B |
| 124 | A |
| 126 | B |
| 137 | B |
| 123 | A |
| 125 | A |

### Example 87. Pharmacokinetics of Oral Absorption for compound 107

### Methods

Male BALB/c mice were fasted overnight and administered a discrete, oral (PO) dose of Compound 107 or Compound 125 at 10 mg/kg.

Blood samples were collected serially from three animals/time-point following PO administration of the test compounds to the mice and placed in tubes containing K2EDTA. The blood samples were centrifuged, and the aliquots of the resulting plasma stored frozen at -60 to -90°C.

The plasma samples were analyzed by LC-MS/MS to determine concentrations of the test compounds. The in-life portion of the study and the bioanalysis of plasma samples was conducted at Wuxi (China).

### Data Analysis

The pharmacokinetic parameters for the tested compounds were calculated by non-compartmental analysis using Phoenix WinNonlin software.

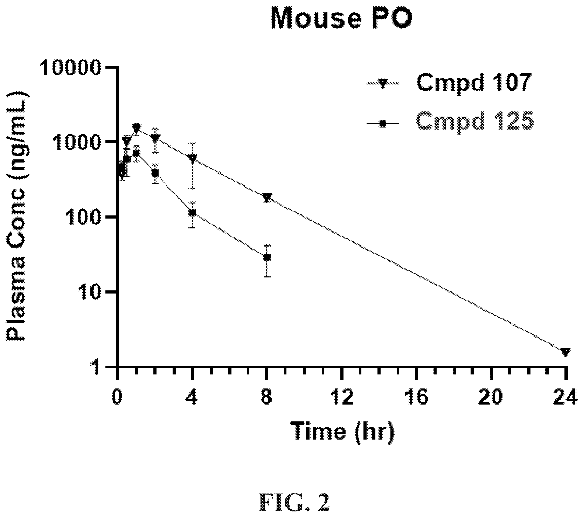

The pharmacokinetic parameters for mouse pharmacokinetic at 10 mg/kg for exemplary compounds are shown in **Table 5.** **FIG. 2** shows the plasma concentration at different timepoints for Compound 107 or Compound 125.

**Table 5. PK Parameters for mouse PK at 10 mg/kg for exemplary compounds**

| **PK Parameters** | **Mean±SD** | |
|---|---|---|
| | **Compound 107** | **Compound 125** |
| | 10 mg/kg | 10 mg/kg |
| T_{1/2} (hr) | 2.53±0.37 | N/A |
| Tmax (hr) | 1 | 1 |
| Cₘₐₓ (ng/mL) | 1516±262 | 717±167 |
| AUCₗₐₛₜ (hr*ng/mL) | 5587±1833 | 1793±504 |
| AUCₐₗₗ (hr*ng/mL) | 5883±1599 | 1865±495 |

### EQUIVALENTS AND SCOPE

While specific embodiments have been discussed, the above specification is illustrative and not restrictive. Many variations of the embodiments will become apparent to those skilled in the art upon review of this specification. The full scope of what is disclosed should be determined by reference to the claims.

## Claims

1. A compound of Formula I: 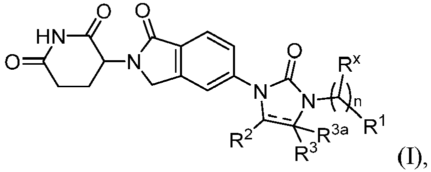 or a pharmaceutically acceptable salt thereof, wherein
- - - - - - denotes a single bond or a double bond; and
R¹ is selected from the group consisting of 5-6 membered monocyclic aryl, 5-6 membered heteroaryl, 8-10 bicyclic heteroaryl, 8-10 membered bicyclic heterocyclyl, 7-8 membered spirocyclic bicyclic heterocyclyl, C₃₋₁₀ cycloalkyl, 4-6 membered heterocyclyl, 6-8 membered bicyclic cycloalkyl, and 7-8 membered spirocyclic cycloalkyl, wherein the aryl, heteroaryl, heterocyclyl, and cycloalkyl is optionally substituted by one, two or three substituents each independently selected from halogen, cyano, hydroxyl, C₁₋₆ alkoxy, C₁₋₆ alkyl, C₁₋₆ haloalkyl, C₃₋₁₀ cycloalkyl, 4-6 membered heterocyclyl, and -C(O)-(C₁₋₆ alkyl), wherein the alkyl is optionally substituted by one or more C₁₋₆ alkoxy; and the cycloalkyl or heterocyclyl is optionally substituted by one, two or three substituents each independently selected from C₁₋₆ alkyl, C₁₋₆ haloalkyl, phenyl, and pyridinyl; and
each of R² and R³ is independently selected from the group consisting of H, -CN, C₁₋₆ alkoxy, C₁₋₆ alkyl, and C₃₋₁₀ cycloalkyl, wherein the alkoxy, or cycloalkyl is optionally substituted by one or more halogen, and alkyl is optionally substituted by one or more halogen or C₁₋₆ alkoxy;
or R² and R³ together with the atoms to which they are attached to form a C₃₋₁₀ cycloalkyl, wherein the cycloalkyl is optionally substituted by one or more halogen;
R^{3a} is hydrogen, or R³ and R^{3a}, together with the atoms to which they are attached to, form a C₃₋₁₀ cycloalkyl; and R^{3a} is absent when **- - - - - -** is a double bond;
R^{x} is hydrogen, C₁₋₆ alkyl, or C₁₋₆ haloalkyl; and
n is 0 or 1.

2. The compound of claim 1, wherein - - - - - - is a single bond.

3. The compound of claim 1 or 2, wherein:
(i) R², R³, and R^{3a} are hydrogen; or
(ii) R² and R^{3a} are hydrogen, and R³ is C₁₋₆ alkyl or C₁₋₆ haloalkyl, wherein the alkyl is optionally substituted with C₁₋₆ alkoxy; or
(iii) R² is C₁₋₆ alkyl, and R³ and R^{3a} are hydrogen; or
(iv) R² and R³ are C₁₋₆ alkyl, and R^{3a} is hydrogen.

4. The compound of claim 1 or 2, wherein the compound of Formula I is represented by Formula I-A: 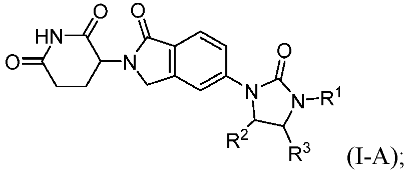 optionally wherein:
(i) R² is H and R³ is H; or
(ii) R² is H and R³ is C₁₋₆ alkyl or C₁₋₆ haloalkyl, wherein the alkyl is optionally substituted with C₁₋₆ alkoxy; or
(iii) R² is C₁₋₆ alkyl and R³ C₁₋₆ alkyl.

5. The compound of claim 1 or 2, wherein the compound of Formula I is represented by Formula I-AB: 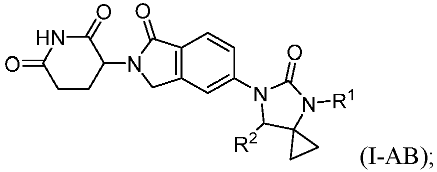 optionally wherein R² is H.

6. The compound of claim 1, wherein - - - - - - is a double bond.

7. The compound of claim 1, wherein the compound of Formula I is represented by Formula I-B: 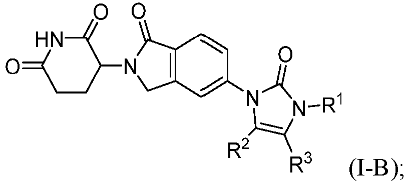 optionally wherein R² is H and R³ is H.

8. The compound of any one of claims 1-7, wherein:
(i) R¹ is unsubstituted or substituted 5-6 membered monocyclic aryl;
optionally wherein R¹ is 5-6 membered monocyclic aryl, wherein the aryl is optionally substituted by one, two or three substituents each independently selected from halogen, cyano, hydroxyl, C₁₋₆ alkoxy, C₁₋₆ alkyl, C₁₋₆ haloalkyl, C₃₋₁₀ cycloalkyl, 4-6 membered heterocyclyl, and -C(O)-(C₁₋₆ alkyl), wherein the alkyl is optionally substituted by one or more C₁₋₆ alkoxy; and the cycloalkyl or heterocyclyl is optionally substituted by one, two or three substituents each independently selected from C₁₋₆ alkyl, C₁₋₆ haloalkyl, phenyl, and pyridinyl;
optionally wherein R¹ is phenyl optionally substituted by one, two or three substituents each independently selected from -OCH₃, -CH₃, -CH(CH₃)₂, -CF₃, -Cl, -CN, -CHF₂, -CH₂OCH₃, -CH(CH₃)OCH₃, -C(O)CH(CH₃)₂, 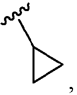 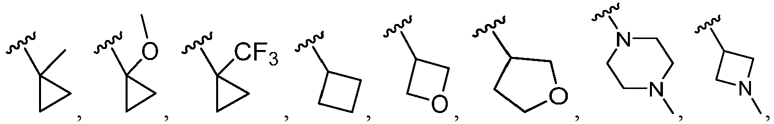 and 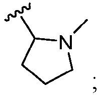 or
(ii) R¹ is unsubstituted or substituted 5-6 membered heteroaryl;
optionally wherein R¹ is 5-6 membered heteroaryl, wherein the heteroaryl is optionally substituted by one, two or three substituents each independently selected from halogen, cyano, hydroxyl, C₁₋₆ alkoxy, C₁₋₆ alkyl, C₁₋₆ haloalkyl, C₃₋₁₀ cycloalkyl, 4-6 membered heterocyclyl, and -C(O)-(C₁₋₆ alkyl), wherein the alkyl is optionally substituted by one or more C₁₋₆ alkoxy; and the cycloalkyl or heterocyclyl is optionally substituted by one, two or three substituents each independently selected from C₁₋₆ alkyl, C₁₋₆ haloalkyl, phenyl, and pyridinyl;
optionally wherein R¹ is optionally susbistuted pyridinyl or pyrimidinyl;
optionally wherein R¹ is pyridinyl optionally substituted by one, two or three substituents each independently selected from F, Cl, Br, -CN, - CH₃, -CH₂CH₃, -CF₃, -OCH₂CH₃, and 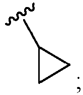 or
(iii) R¹ is unsubstituted or substituted 8-10 bicyclic heterocyclyl or 7-8 membered spirocyclic bicyclic heterocyclyl;
optionally wherein R¹ is dihydrobenzofuranyl, isoindolinyl, benzoimidazolyl, or 2-oxaspiroheptanyl; or
(iv) R¹ is unsubstituted or substituted 8-10 bicyclic heteroaryl; or
(v) R¹ is unsubstituted or substituted 4-6 membered heterocyclyl;
optionally wherein R¹ is oxetanyl, tetrahydrofuranyl, or tetrahydropyranyl; or
(vi) R¹ is unsubstituted or substituted 6-8 membered bicyclic cycloalkyl or 7-8 membered spirocyclic cycloalkyl; or
(vii) R¹ is

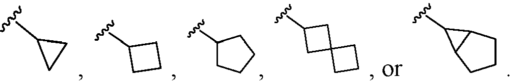

9. The compound of any one of claims 1-8, wherein n is 1 and R^{x} is C₁₋₆ alkyl or C₁₋₆ haloalkyl, wherein the alkyl is optionally substituted with one or more halogen; optionally wherein n is 1 and R^{x} is CF₃.

10. The compound of any one of claims 1-8, wherein:
(i) n is 1; or
(ii) n is 0.

11. The compound of any one of claims 1-10, wherein the compound is selected from the group consisting of: 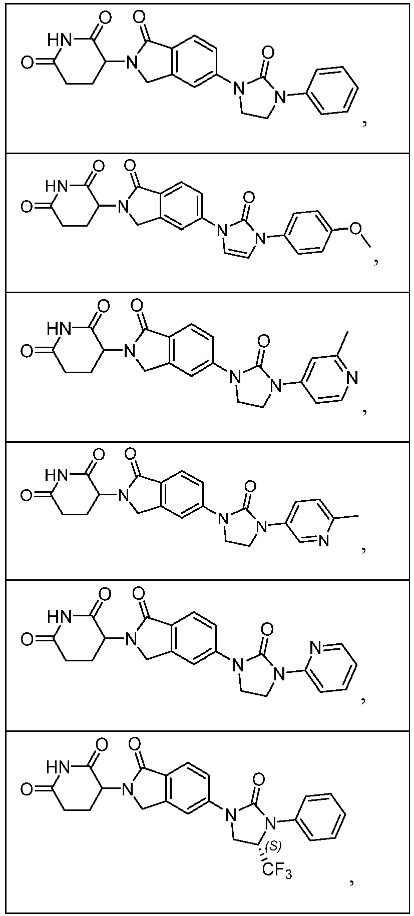 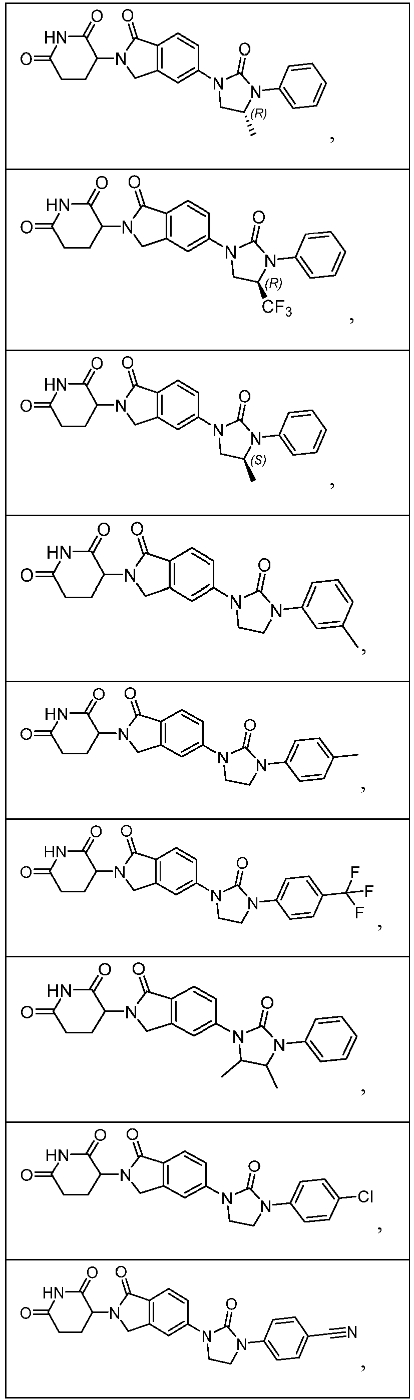 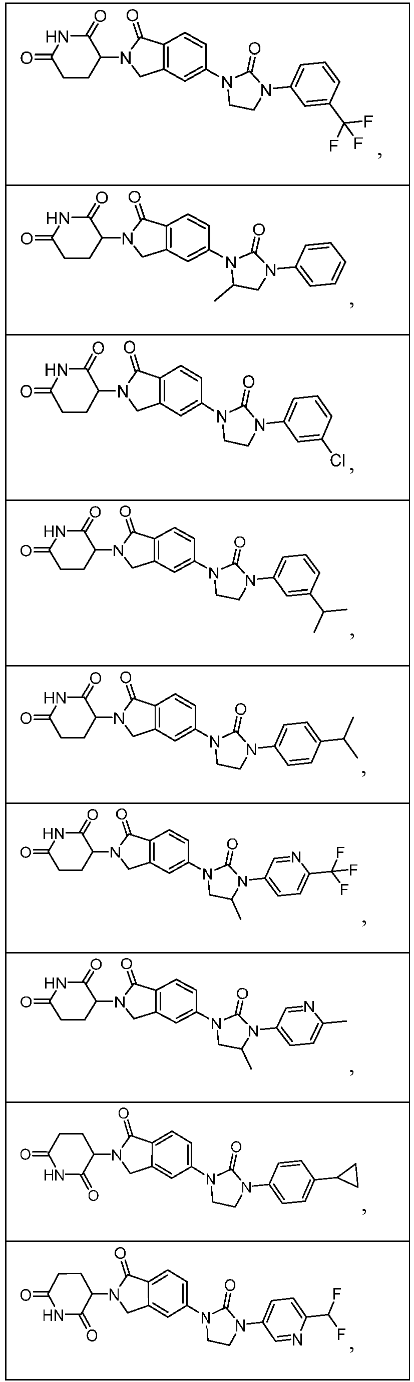 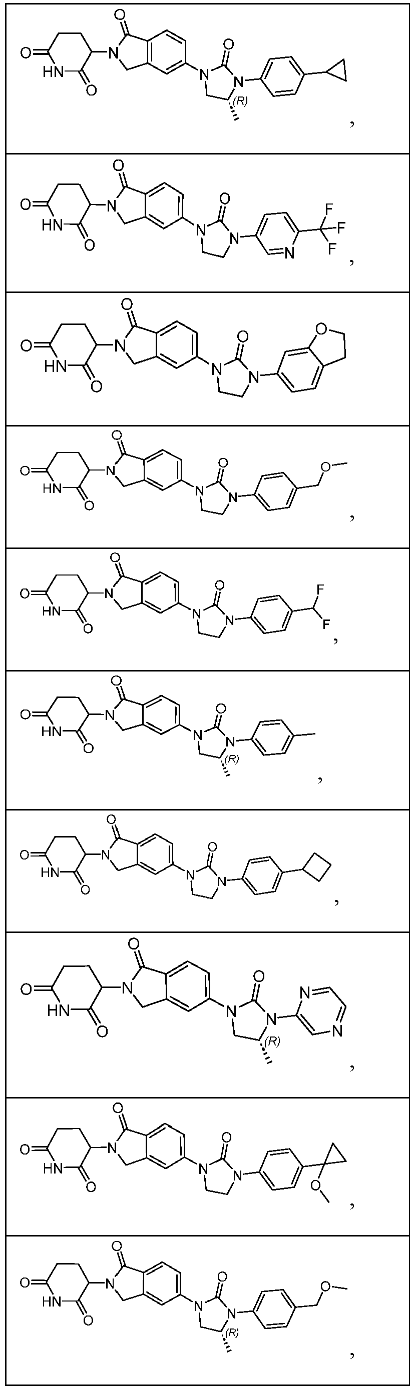 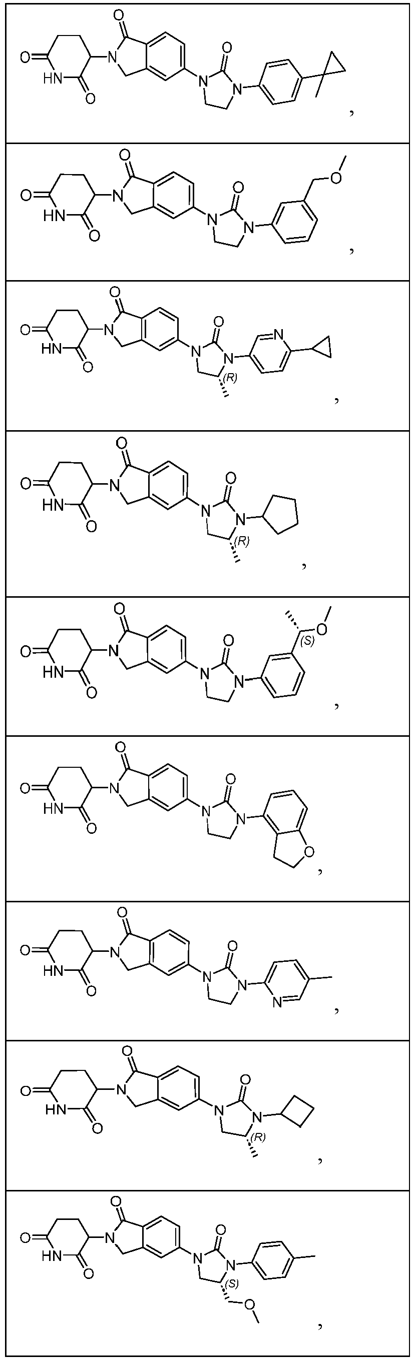 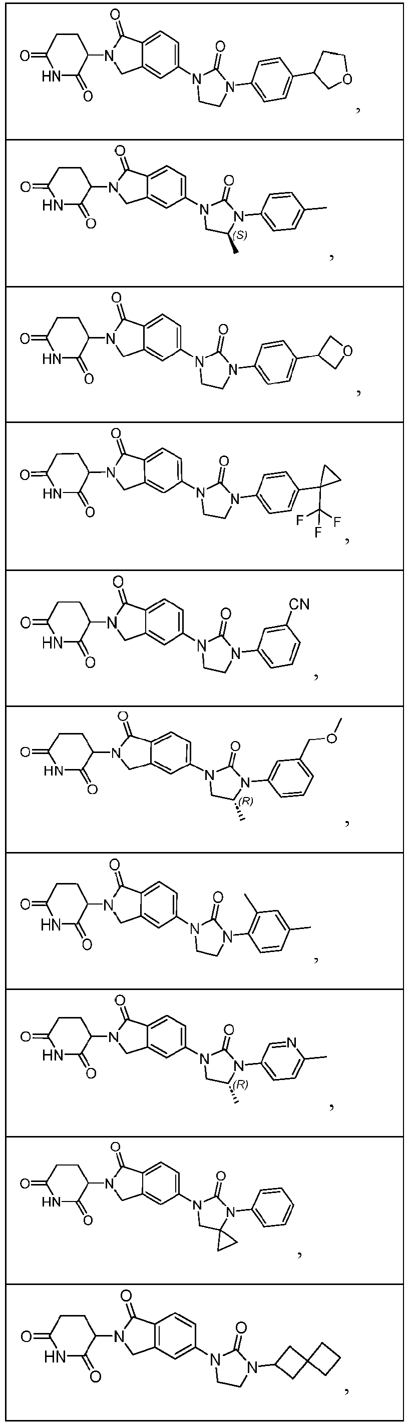 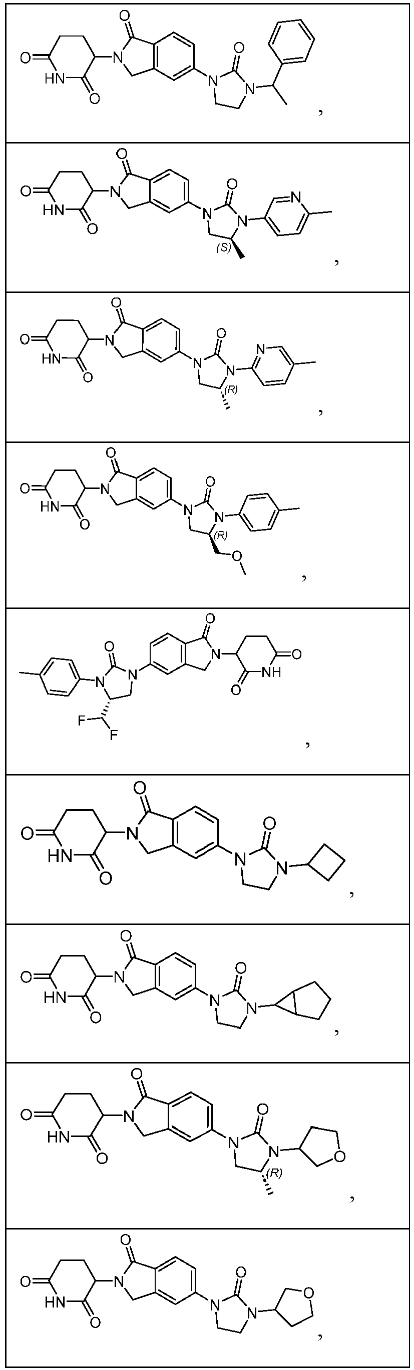 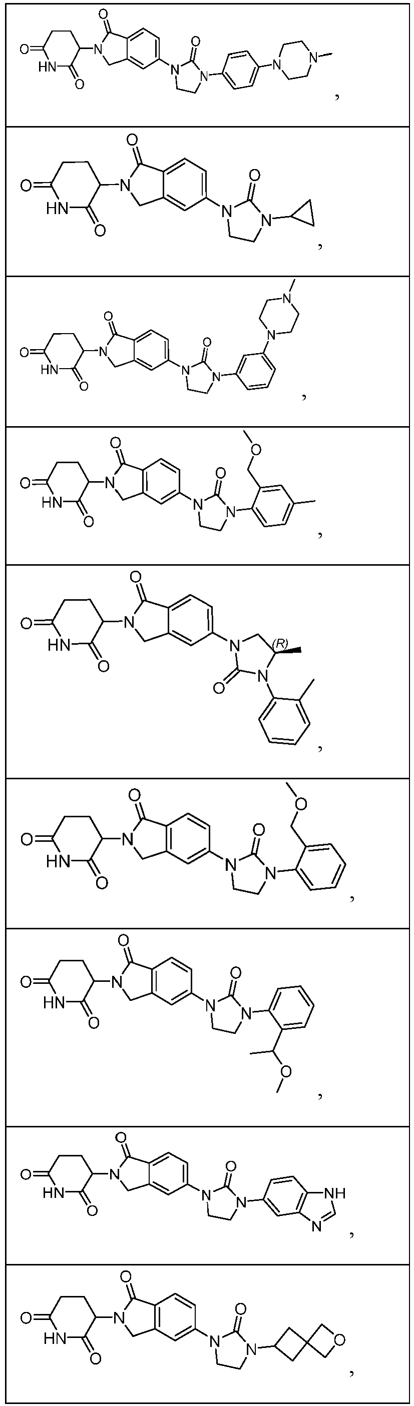 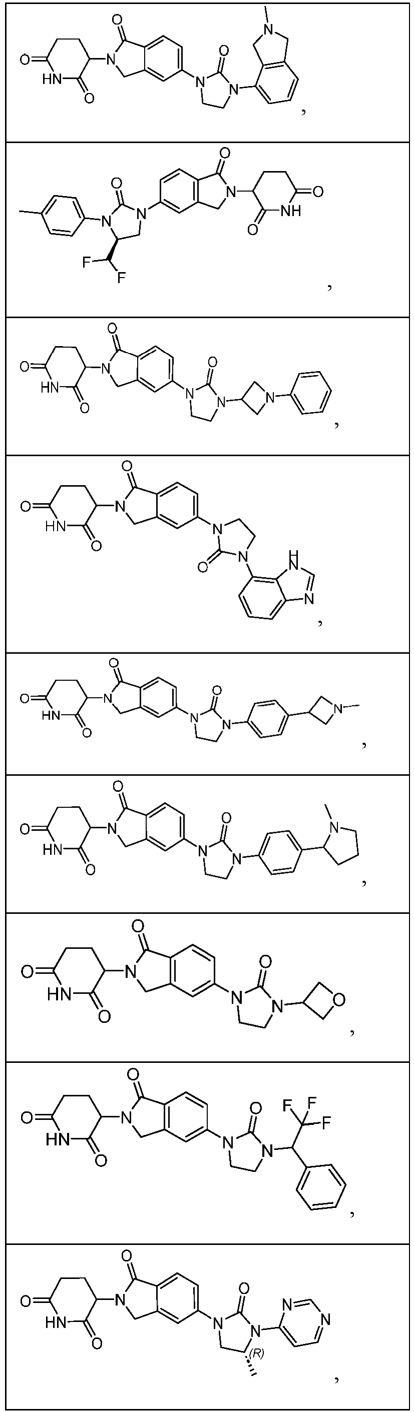 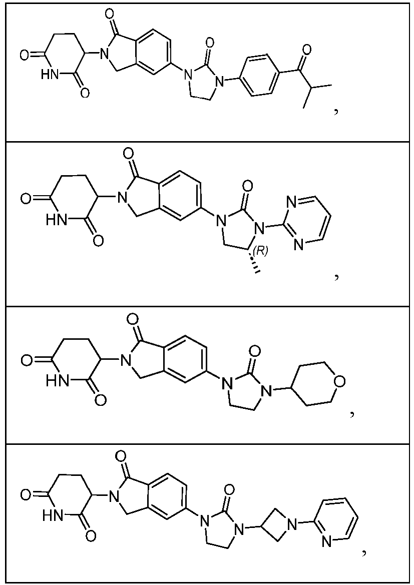 and a pharmaceutically acceptable salt thereof.

12. A pharmaceutical composition comprising the compound of any one of claims 1-11, or pharmaceutically acceptable salt thereof, and a pharmaceutically acceptable excipient.

13. The compound of any one of claims 1-11, or pharmaceutically acceptable salt thereof, or the pharmaceutical composition of claim 12, for use in a method of degrading CK1α in a subject suffering from cancer, said method comprising administering to the subject an effective amount of the compound of any one of claims 1-11, or pharmaceutically acceptable salt thereof, or the pharmaceutical composition of claim 12.

14. The compound of any one of claims 1-11, or pharmaceutically acceptable salt thereof, or the pharmaceutical composition of claim 12, for use in a method of treating cancer, a solid tumor, or a liquid tumor, in a subject in need thereof, said method comprising administering to the subject a therapeutically effective amount of the compound of any one of claims 1-11, or pharmaceutically acceptable salt thereof, or the pharmaceutical composition of claim 12.

15. The compound, pharmaceutically acceptable salt, or pharmaceutical composition for use according to claim 13 or 14, wherein the method further comprises administering to the subject an additional therapeutic agent.

## Patentansprüche

1. Verbindung der Formel I: 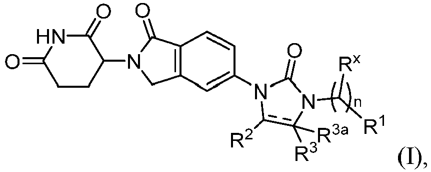
oder ein pharmazeutisch unbedenkliches Salz davon, wobei - - - - - - eine Einfachbindung oder eine Doppelbindung bedeutet; und
R¹ aus der Gruppe bestehend aus 5-6-gliedrigem monocyclischem Aryl, 5-6-gliedrigem Heteroaryl, 8-10-gliedrigem bicyclischem Heteroaryl, 8-10-gliedrigem bicyclischem Heterocyclyl, 7-8-gliedrigem spirocyclischem bicyclischen Heterocyclyl, C₃₋₁₀-Cycloalkyl, 4-6-gliedrigem Heterocyclyl, 6-8-gliedrigem bicyclischem Cycloalkyl und 7-8-gliedrigem spirocyclischem Cycloalkyl ausgewählt ist, wobei das Aryl, Heteroaryl, Heterocyclyl und Cycloalkyl gegebenenfalls durch einen, zwei oder drei Substituenten, die jeweils unabhängig aus Halogen, Cyano, Hydroxyl, C₁₋₆-Alkoxy, C₁₋₆-Alkyl, C₁₋₆-Halogenalkyl, C₃₋₁₀-Cycloalkyl, 4-6-gliedrigem Heterocyclyl und -C(O)-(C₁₋₆-Alkyl) ausgewählt sind, substituiert ist, wobei das Alkyl gegebenenfalls durch ein oder mehrere C₁₋₆-Alkoxy substituiert ist und das Cycloalkyl oder Heterocyclyl gegebenenfalls durch einen, zwei oder drei Substituenten, die jeweils unabhängig aus C₁₋₆-Alkyl, C₁₋₆-Halogenalkyl, Phenyl und Pyridinyl ausgewählt sind, substituiert ist; und
R² und R³ jeweils unabhängig aus der Gruppe bestehend aus H, -CN, C₁₋₆-Alkoxy, C₁₋₆-Alkyl und C₃₋₁₀-Cycloalkyl ausgewählt sind, wobei das Alkoxy oder Cycloalkyl gegebenenfalls durch ein oder mehrere Halogene substituiert ist und Alkyl gegebenenfalls durch ein oder mehrere Halogene oder C₁₋₆-Alkoxy substituiert ist;
oder R² und R³ zusammen mit den Atomen, an die sie gebunden sind, ein C₃₋₁₀-Cycloalkyl bilden, wobei das Cycloalkyl gegebenenfalls durch ein oder mehrere Halogene substituiert ist;
R^{3a} für Wasserstoff steht oder R³ und R^{3a} zusammen mit den Atomen, an die sie gebunden sind, ein C₃₋₁₀-Cycloalkyl bilden und R^{3a} fehlt, wenn - - - - - - eine Doppelbindung ist; R^{x} für Wasserstoff, C₁₋₆-Alkyl oder C₁₋₆-Halogenalkyl steht und
n für 0 oder 1 steht.

2. Verbindung nach Anspruch 1, wobei - - - - - - eine Einfachbindung ist.

3. Verbindung nach Anspruch 1 oder 2, wobei
(i) R², R³ und R^{3a} für Wasserstoff stehen; oder
(ii) R² und R^{3a} für Wasserstoff stehen und R³ für C₁₋₆-Alkyl oder C₁₋₆-Halogenalkyl steht, wobei das Alkyl gegebenenfalls durch C₁₋₆-Alkoxy substituiert ist; oder
(iii) R² für C₁₋₆-Alkyl steht und R³ und R^{3a} für Wasserstoff stehen; oder
(iv) R² und R³ für C₁₋₆-Alkyl stehen und R^{3a} für Wasserstoff steht.

4. Verbindung nach Anspruch 1 oder 2, wobei die Verbindung der Formel I durch Formel I-A wiedergegeben wird: 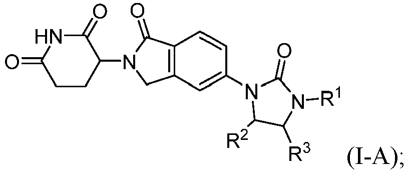 gegebenenfalls wobei:
(i) R² für H steht und R³ für H steht; oder
(ii) R² für H steht und R³ für C₁₋₆-Alkyl oder C₁₋₆-Halogenalkyl steht, wobei das Alkyl gegebenenfalls durch C₁₋₆-Alkoxy substituiert ist; oder
(iii) R² für C₁₋₆-Alkyl steht und R³ für C₁₋₆-Alkyl steht.

5. Verbindung nach Anspruch 1 oder 2, wobei die Verbindung der Formel I durch Formel I-AB wiedergegeben wird: 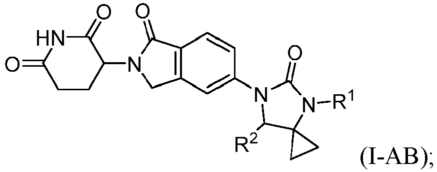 gegebenenfalls wobei R² für H steht.

6. Verbindung nach Anspruch 1, wobei - - - - - - eine Doppelbindung ist.

7. Verbindung nach Anspruch 1, wobei die Verbindung der Formel I durch Formel I-B wiedergegeben wird: 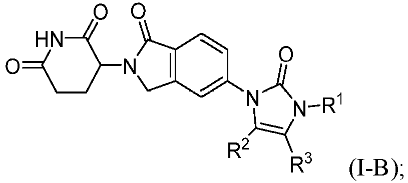 gegebenenfalls wobei R² für H steht und R³ für H steht.

8. Verbindung nach einem der Ansprüche 1-7, wobei:
(i) R¹ für unsubstituiertes oder substituiertes 5-6-gliedriges monocyclisches Aryl steht;
gegebenenfalls wobei R¹ für 5-6-gliedriges monocyclisches Aryl steht, wobei das Aryl gegebenenfalls durch einen, zwei oder drei Substituenten, die jeweils unabhängig aus Halogen, Cyano, Hydroxyl, C₁₋₆-Alkoxy, C₁₋₆-Alkyl, C₁₋₆-Halogenalkyl, C₃₋₁₀-Cycloalkyl, 4-6-gliedrigem Heterocyclyl und -C(O)-(C₁₋₆-Alkyl) ausgewählt sind, substituiert ist, wobei das Alkyl gegebenenfalls durch ein oder mehrere C₁₋₆-Alkoxy substituiert ist und das Cycloalkyl oder Heterocyclyl gegebenenfalls durch einen, zwei oder drei Substituenten, die jeweils unabhängig aus C₁₋₆-Alkyl, C₁₋₆-Halogenalkyl, Phenyl und Pyridinyl ausgewählt sind, substituiert ist;
gegebenenfalls wobei R¹ für Phenyl steht, das gegebenenfalls durch einen, zwei oder drei Substituenten, die jeweils unabhängig aus -OCH₃, -CH₃, -CH(CH₃)₂, -CF₃, - Cl, -CN, -CHF₂, -CH₂OCH₃, -CH(CH₃)OCH₃, -C(O)CH(CH₃)₂, 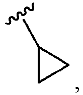 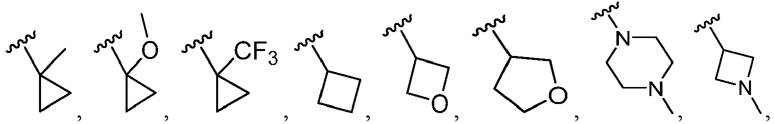 und 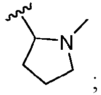 ausgewählt sind, substituiert ist, oder
(ii) R¹ für unsubstituiertes oder substituiertes 5-6-gliedriges Heteroaryl steht;
gegebenenfalls wobei R¹ für 5-6-gliedriges Heteroaryl steht, wobei das Heteroaryl gegebenenfalls durch einen, zwei oder drei Substituenten, die jeweils unabhängig aus Halogen, Cyano, Hydroxyl, C₁₋₆-Alkoxy, C₁₋₆-Alkyl, C₁₋₆-Halogenalkyl, C₃₋₁₀-Cycloalkyl, 4-6-gliedrigem Heterocyclyl und -C(O)-(C₁₋₆-Alkyl) ausgewählt sind, substituiert ist, wobei das Alkyl gegebenenfalls durch ein oder mehrere C₁₋₆-Alkoxy substituiert ist und das Cycloalkyl oder Heterocyclyl gegebenenfalls durch einen, zwei oder drei Substituenten, die jeweils unabhängig aus C₁₋₆-Alkyl, C₁₋₆-Halogenalkyl, Phenyl und Pyridinyl ausgewählt sind, substituiert ist;
gegebenenfalls wobei R¹ für gegebenenfalls substituiertes Pyridinyl oder Pyrimidinyl steht;
gegebenenfalls wobei R¹ für Pyridinyl steht, das gegebenenfalls durch einen, zwei oder drei Substituenten, die jeweils unabhängig aus F, Cl, Br, -CN, -CH₃, -CH₂CH₃, -CF₃, -OCH₂CH₃ und 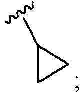 ausgewählt sind, substituiert ist, oder
(iii) R¹ für unsubstituiertes oder substituiertes 8-10 bicyclisches Heterocyclyl oder 7-8-gliedriges spirocyclisches bicyclisches Heterocyclyl steht; gegebenenfalls wobei R¹ für Dihydrobenzofuranyl, Isoindolinyl, Benzoimidazolyl oder 2-Oxaspiroheptanyl steht; oder
(iv) R¹ für unsubstituiertes oder substituiertes 8-10 bicyclisches Heteroaryl steht; oder
(v) R¹ für unsubstituiertes oder substituiertes 4-6-gliedriges Heterocyclyl steht;
gegebenenfalls wobei R¹ für Oxetanyl, Tetrahydrofuranyl oder Tetrahydropyranyl steht; oder
(vi) R¹ für unsubstituiertes oder substituiertes 6-8-gliedriges bicyclisches Cycloalkyl oder 7-8-gliedriges spirocyclisches Cycloalkyl steht; oder
(vii) R¹ für 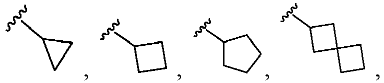 oder 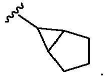 steht.

9. Verbindung nach einem der Ansprüche 1-8, wobei n für 1 steht und R^{x} für C₁₋₆-Alkyl oder C₁₋₆-Halogenalkyl steht, wobei das Alkyl gegebenenfalls durch ein oder mehrere Halogene substituiert ist;
gegebenenfalls wobei n für 1 steht und R^{x} für CF₃ steht.

10. Verbindung nach einem der Ansprüche 1-8, wobei:
(i) n für 1 steht; oder
(ii) n für 0 steht.

11. Verbindung nach einem der Ansprüche 1-10, wobei die Verbindung ausgewählt ist aus der Gruppe bestehend aus: 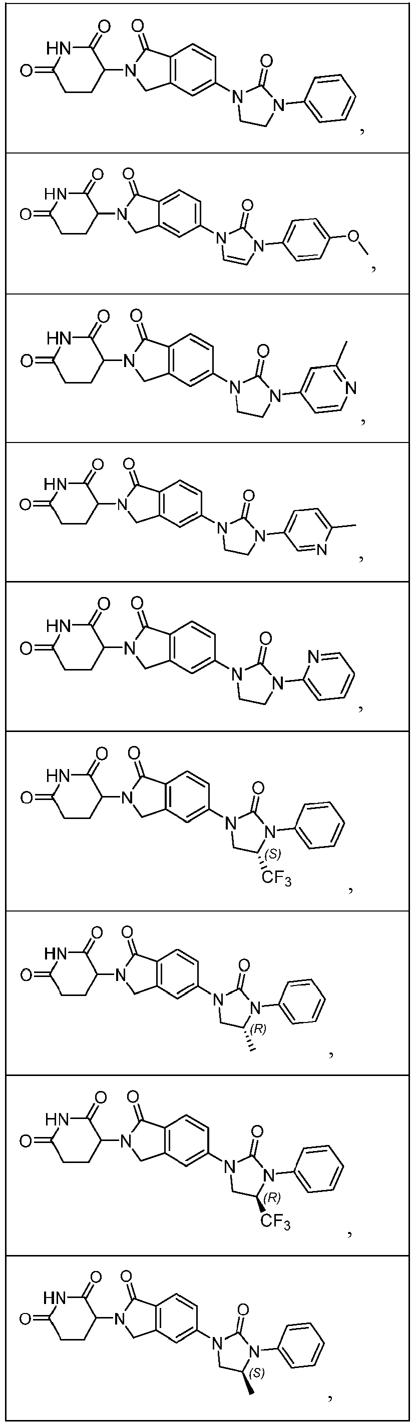 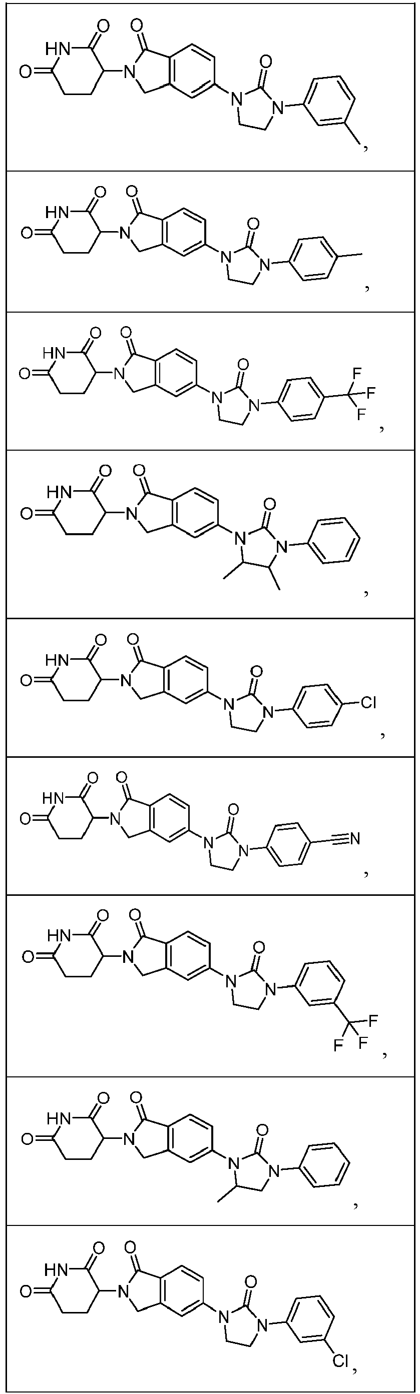 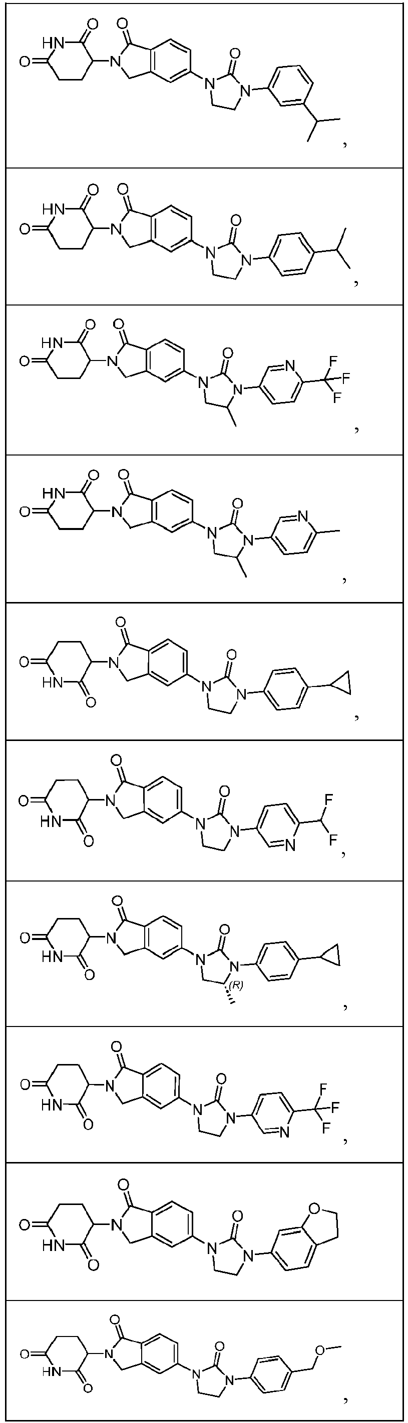 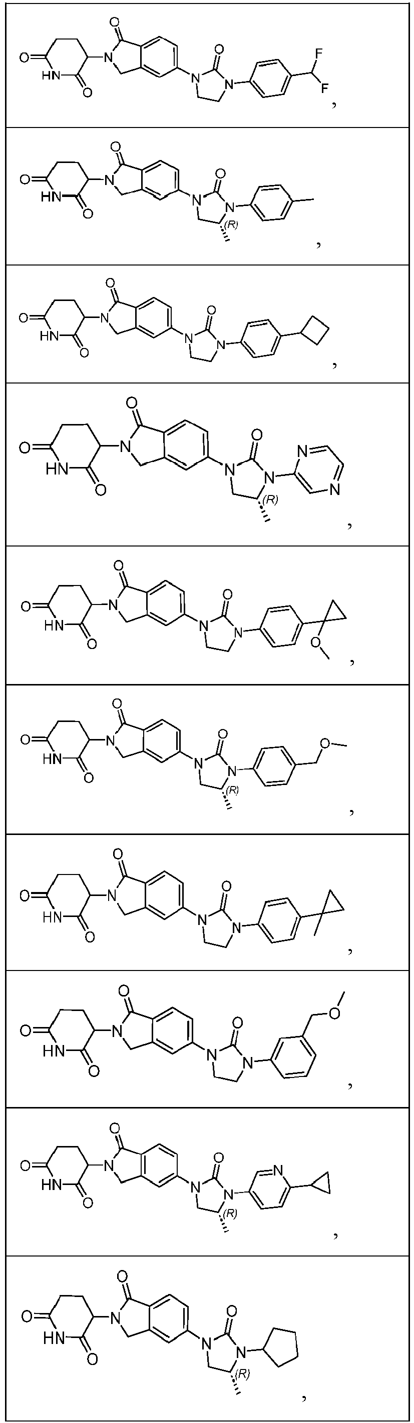 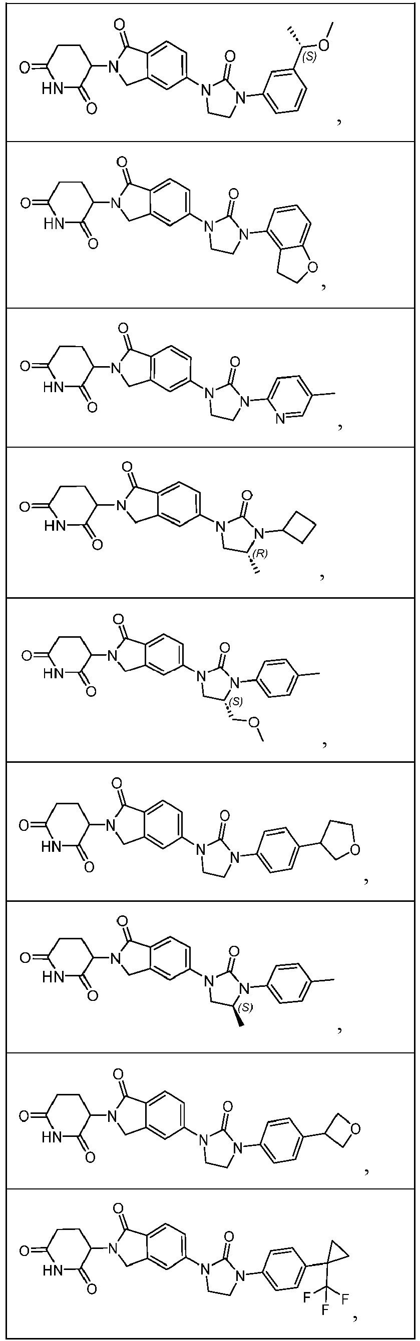 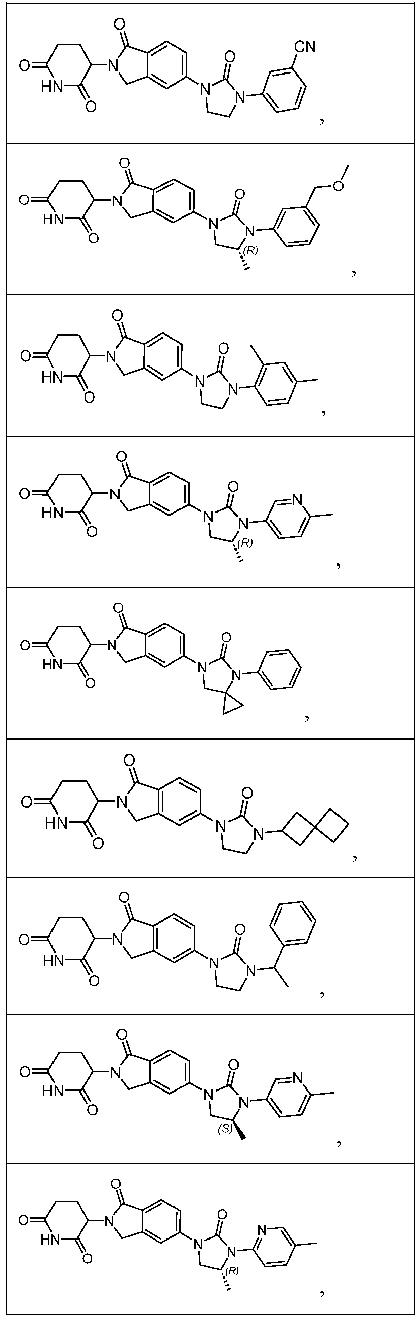 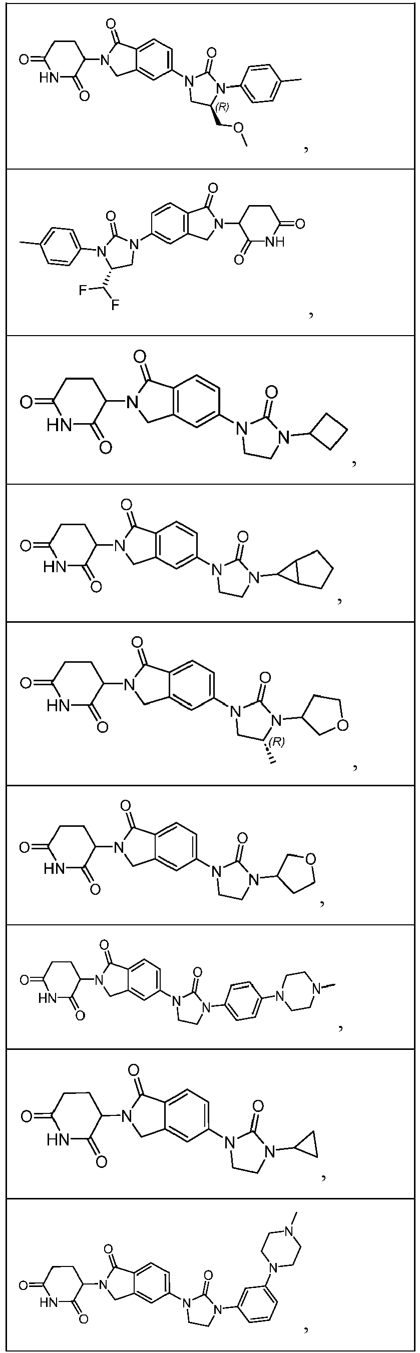 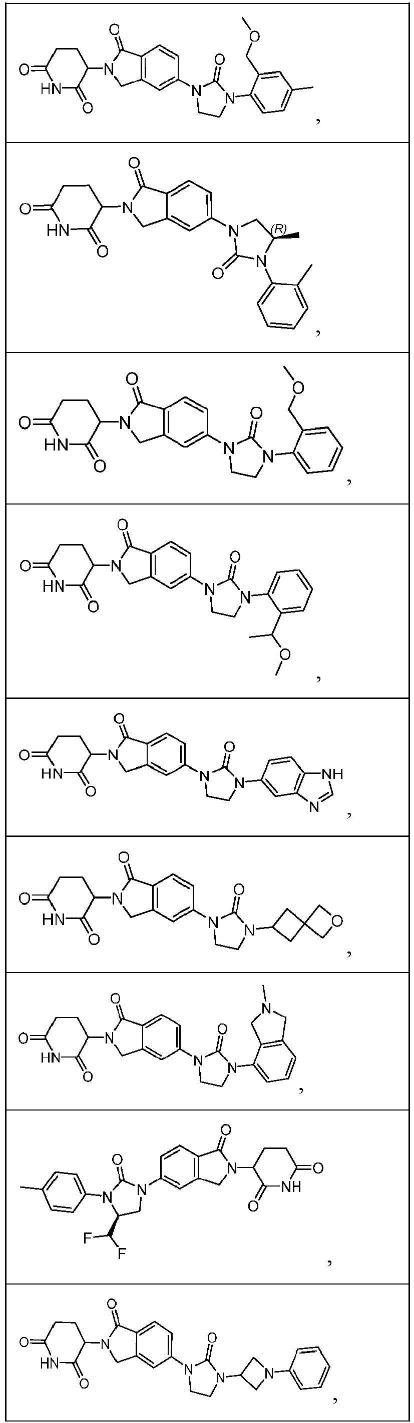 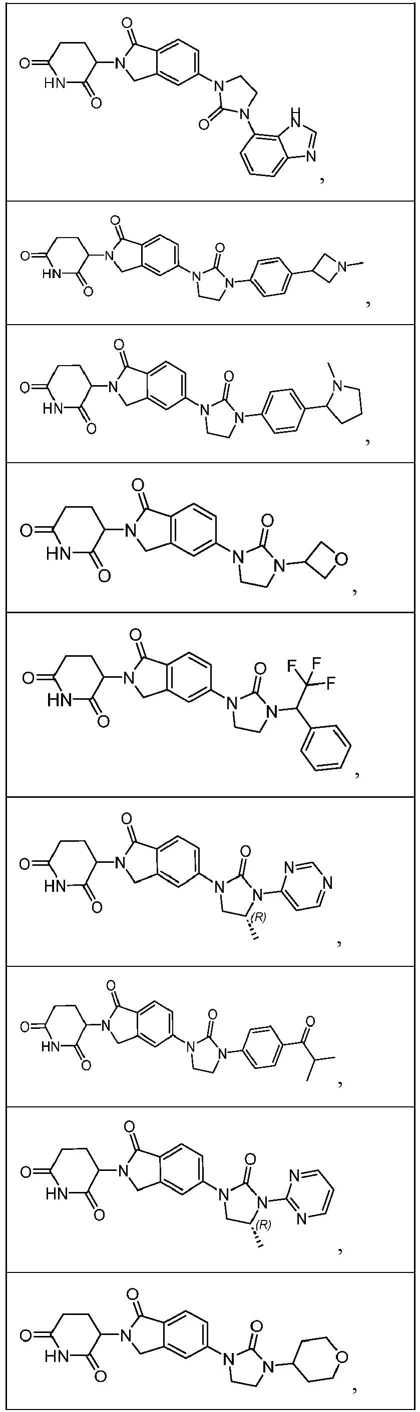 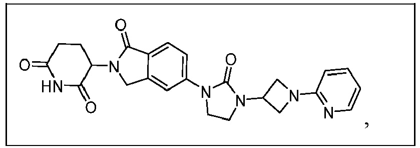 und einem pharmazeutisch unbedenklichen Salz davon.

12. Pharmazeutische Zusammensetzung, umfassend die Verbindung nach einem der Ansprüche 1-11 oder das pharmazeutisch unbedenkliche Salz davon und einen pharmazeutisch unbedenklichen Hilfsstoff.

13. Verbindung nach einem der Ansprüche 1-11 oder pharmazeutisch unbedenkliches Salz davon oder pharmazeutische Zusammensetzung nach Anspruch 12 zur Verwendung bei einem Verfahren zum Abbau von CK1α bei einem Individuum, das an Krebs leidet, wobei das Verfahren das Verabreichen einer wirksamen Menge der Verbindung nach einem der Ansprüche 1-11 oder des pharmazeutisch unbedenklichen Salzes davon oder der pharmazeutischen Zusammensetzung nach Anspruch 12 an das Individuum umfasst.

14. Verbindung nach einem der Ansprüche 1-11 oder pharmazeutisch unbedenkliches Salz davon oder pharmazeutische Zusammensetzung nach Anspruch 12 zur Verwendung bei einem Verfahren zur Behandlung von Krebs, einem soliden Tumor oder einem flüssigen Tumor bei einem Individuum, bei dem diesbezüglicher Bedarf besteht, wobei das Verfahren das Verabreichen einer wirksamen Menge der Verbindung nach einem der Ansprüche 1-11 oder des pharmazeutisch unbedenklichen Salzes davon oder der pharmazeutischen Zusammensetzung nach Anspruch 12 an das Individuum umfasst.

15. Verbindung, pharmazeutisch unbedenkliches Salz oder pharmazeutische Zusammensetzung zur Verwendung nach Anspruch 13 oder 14, wobei das Verfahren ferner das Verabreichen eines zusätzlichen therapeutischen Mittels an das Individuum umfasst.

## Revendications

1. Composé de Formule I : 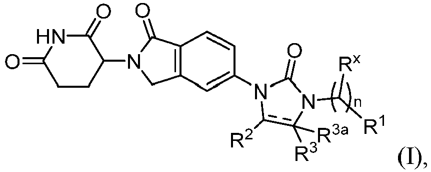
ou sel pharmaceutiquement acceptable correspondant,
- - - - - - désignant une simple liaison ou une double liaison ; et
R¹ étant choisi dans le groupe constitué par aryle monocyclique à 5 à 6 chaînons, hétéroaryle à 5 à 6 chaînons, hétéroaryle bicyclique à 8 à 10 chaînons, hétérocyclyle bicyclique à 8 à 10 chaînons, hétérocyclyle bicyclique spirocyclique à 7 à 8 chaînons, C₃₋₁₀ cycloalkyle, hétérocyclyle à 4 à 6 chaînons, cycloalkyle bicyclique à 6 à 8 chaînons, et cycloalkyle spirocyclique à 7 à 8 chaînons, l'aryle, hétéroaryle, hétérocyclyle, et cycloalkyle étant éventuellement substitué par un, deux ou trois substituants chacun indépendamment choisi parmi halogène, cyano, hydroxyle, C₁₋₆ alcoxy, C₁₋₆ alkyle, C₁₋₆ halogénoalkyle, C₃₋₁₀ cycloalkyle, hétérocyclyle à 4 à 6 chaînons, et -C(O)-(C₁₋₆ alkyle), l'alkyle étant éventuellement substitué par un ou plusieurs C₁₋₆ alcoxy ; et le cycloalkyle ou hétérocyclyle étant éventuellement substitué par un, deux ou trois substituants chacun indépendamment choisi parmi C₁₋₆ alkyle, C₁₋₆ halogénoalkyle, phényle, et pyridinyle ; et
chacun parmi R² et R³ étant indépendamment choisi dans le groupe constitué par H, -CN, C₁₋₆ alcoxy, C₁₋₆ alkyle, et C₃₋₁₀ cycloalkyle, l'alcoxy, ou cycloalkyle étant éventuellement substitué par un ou plusieurs halogènes, et alkyle étant éventuellement substitué par un ou plusieurs halogènes ou C₁₋₆ alcoxy ;
ou R² et R³ conjointement avec les atomes auxquels ils sont fixés formant un C₃₋₁₀ cycloalkyle, le cycloalkyle étant éventuellement substitué par un ou plusieurs halogènes ;
R^{3a} étant hydrogène, ou R³ et R^{3a}, conjointement avec les atomes auxquels ils sont fixés formant un C₃₋₁₀ cycloalkyle ; et R^{3a} étant absent lorsque - - - - - - est une double liaison ;
R^{x} étant hydrogène, C₁₋₆ alkyle, ou C₁₋₆ halogénoalkyle ; et
n étant 0 ou 1.

2. Composé selon la revendication 1, - - - - - - étant une simple liaison.

3. Composé selon la revendication 1 ou 2,
(i) R², R³, et R^{3a} are hydrogène ; ou
(ii) R² et R^{3a} étant hydrogène, et R³ étant C₁₋₆ alkyle ou C₁₋₆ halogénoalkyle, l'alkyle étant éventuellement substitué par C₁₋₆ alcoxy ; ou
(iii) R² étant C₁₋₆ alkyle, et R³ et R^{3a} étant hydrogène ; ou
(iv) R² et R³ étant C₁₋₆ alkyle, et R^{3a} étant hydrogène.

4. Composé selon la revendication 1 ou 2, le composé de formule I étant représenté par la formule I-A : 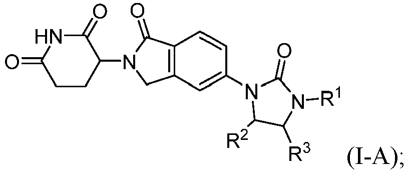 éventuellement,
(i) R² étant H et R³ étant H ; ou
(ii) R² étant H et R³ étant C₁₋₆ alkyle ou C₁₋₆ halogénoalkyle, l'alkyle étant éventuellement substitué par C₁₋₆ alcoxy ; ou
(iii) R² étant C₁₋₆ alkyle et R³ C₁₋₆ alkyle.

5. Composé selon la revendication 1 ou 2, le composé de formule I étant représenté par la formule I-AB : 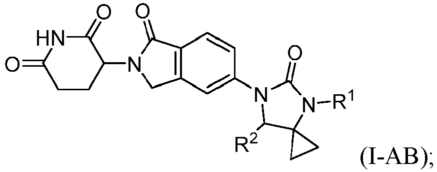 éventuellement, R² étant H.

6. Composé selon la revendication 1, - - - - - - étant une double liaison.

7. Composé selon la revendication 1, le composé de formule I étant représenté par la formule I-B : 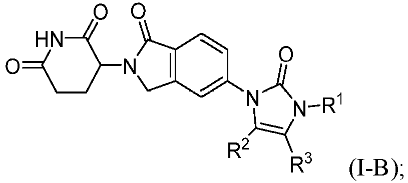 éventuellement, R² étant H et R³ étant H.

8. Composé selon l'une quelconque des revendications 1-7,
(i) R¹ étant aryle monocyclique à 5 à 6 chaînons non substitué ou substitué ;
éventuellement, R¹ étant aryle monocyclique à 5 à 6 chaînons, l'aryle étant éventuellement substitué par un, deux ou trois substituants chacun indépendamment choisi parmi halogène, cyano, hydroxyle, C₁₋₆ alcoxy, C₁₋₆ alkyle, C₁₋₆ halogénoalkyle, C₃₋₁₀ cycloalkyle, hétérocyclyle à 4 à 6 chaînons, et -C (O) - (C₁₋₆ alkyle), l'alkyle étant éventuellement substitué par un ou plusieurs C₁₋₆ alcoxy ; et le cycloalkyle ou hétérocyclyle étant éventuellement substitué par un, deux ou trois substituants chacun indépendamment choisi parmi C₁₋₆ alkyle, C₁₋₆ halogénoalkyle, phényle, et pyridinyle ;
éventuellement, R¹ étant phényle éventuellement substitué par un, deux ou trois substituants chacun indépendamment choisi parmi -OCH₃, -CH₃, -CH(CH₃)₂, -CF₃, -Cl, -CN, -CHF₂, -CH₂OCH₃, -CH(CH₃)OCH₃, -C(O)CH(CH₃)₂, 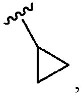 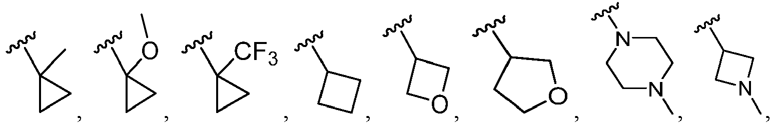 et 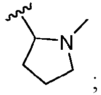 ou
(ii) R¹ étant hétéroaryle à 5 à 6 chaînons non substitué ou substitué ;
éventuellement, R¹ étant hétéroaryle à 5 à 6 chaînons, l'hétéroaryle étant éventuellement substitué par un, deux ou trois substituants chacun indépendamment choisi parmi halogène, cyano, hydroxyle, C₁₋₆ alcoxy, C₁₋₆ alkyle, C₁₋₆ halogénoalkyle, C₃₋₁₀ cycloalkyle, hétérocyclyle à 4 à 6 chaînons, et -C(O)-(C₁₋₆ alkyle), l'alkyle étant éventuellement substitué par un ou plusieurs C₁₋₆ alcoxy ; et le cycloalkyle ou hétérocyclyle étant éventuellement substitué par un, deux ou trois substituants chacun indépendamment choisi parmi C₁₋₆ alkyle, C₁₋₆ halogénoalkyle, phényle, et pyridinyle ;
éventuellement, R¹ étant pyridinyle ou pyrimidinyle éventuellement substitué ;
éventuellement, R¹ étant pyridinyle éventuellement substitué par un, deux ou trois substituants chacun indépendamment choisi parmi F, Cl, Br, -CN, -CH₃, -CH₂CH₃, -CF₃, -OCH₂CH₃, et 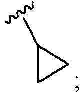 ou
(iii) R¹ étant hétérocyclyle bicyclique à 8 à 10 chaînons ou hétérocyclyle bicyclique spirocyclique à 7 à 8 chaînons non substitué ou substitué ;
éventuellement, R¹ étant dihydrobenzofuranyle, isoindolinyle, benzoimidazolyle, ou 2-oxaspiroheptanyle ; ou
(iv) R¹ étant hétéroaryle bicyclique à 8 à 10 chaînons non substitué ou substitué ; ou
(v) R¹ étant hétérocyclyle à 4 à 6 chaînons non substitué ou substitué ;
éventuellement, R¹ étant oxétanyle, tétrahydrofuranyle, ou tétrahydropyranyle ; ou
(vi) R¹ étant cycloalkyle bicyclique à 6 à 8 chaînons ou cycloalkyle spirocyclique à 7 à 8 chaînons non substitué ou substitué ; ou
(vii) R¹ étant

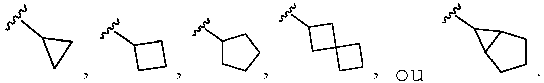

9. Composé selon l'une quelconque des revendications 1-8, n étant 1 et R^{x} étant C₁₋₆ alkyle ou C₁₋₆ halogénoalkyle, l'alkyle étant éventuellement substitué par un ou plusieurs halogènes ;
éventuellement, n étant 1 et R^{x} étant CF₃.

10. Composé selon l'une quelconque des revendications 1-8,
(i) n étant 1 ; ou
(ii) n étant 0.

11. Composé selon l'une quelconque des revendications 1 à 10, le composé étant choisi dans le groupe constitué par : 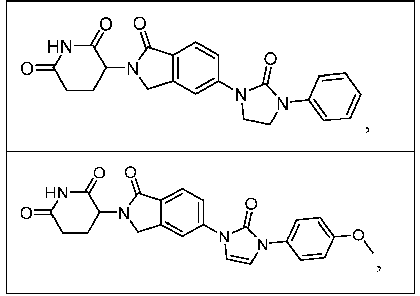 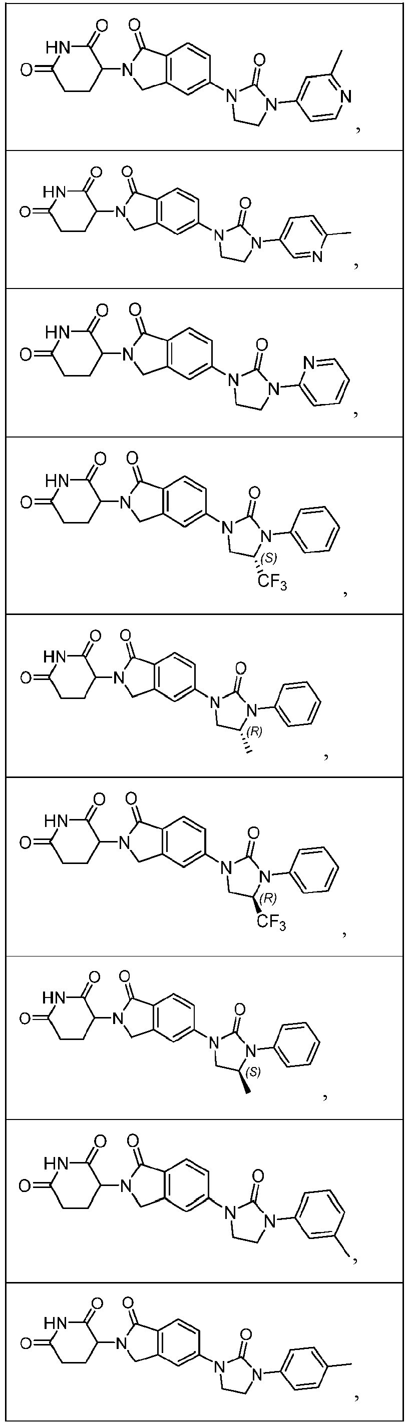 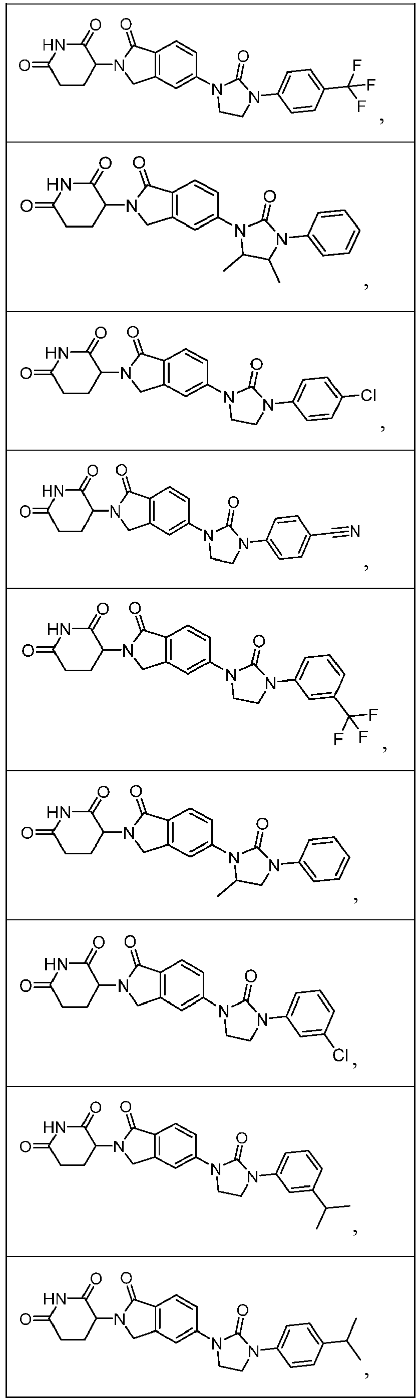 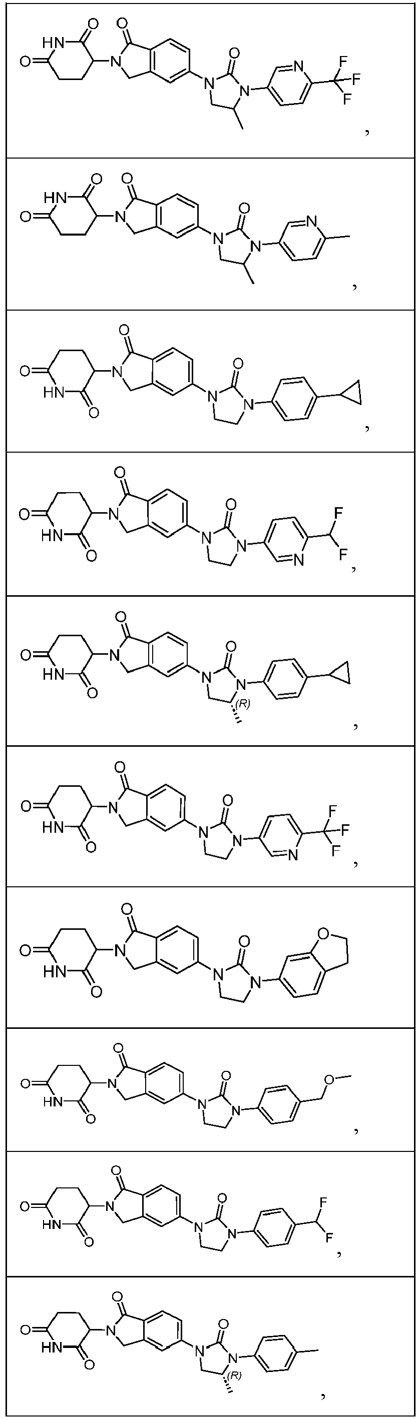 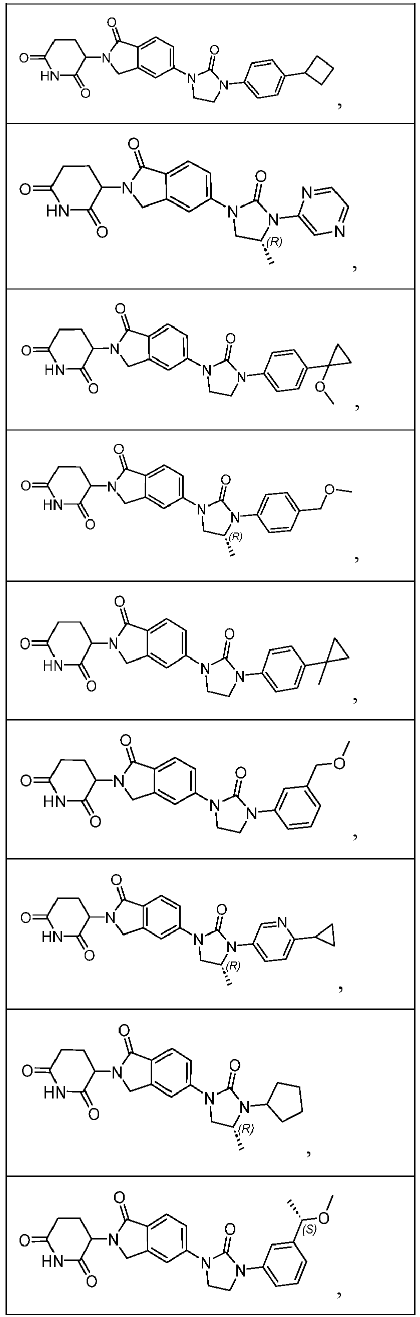 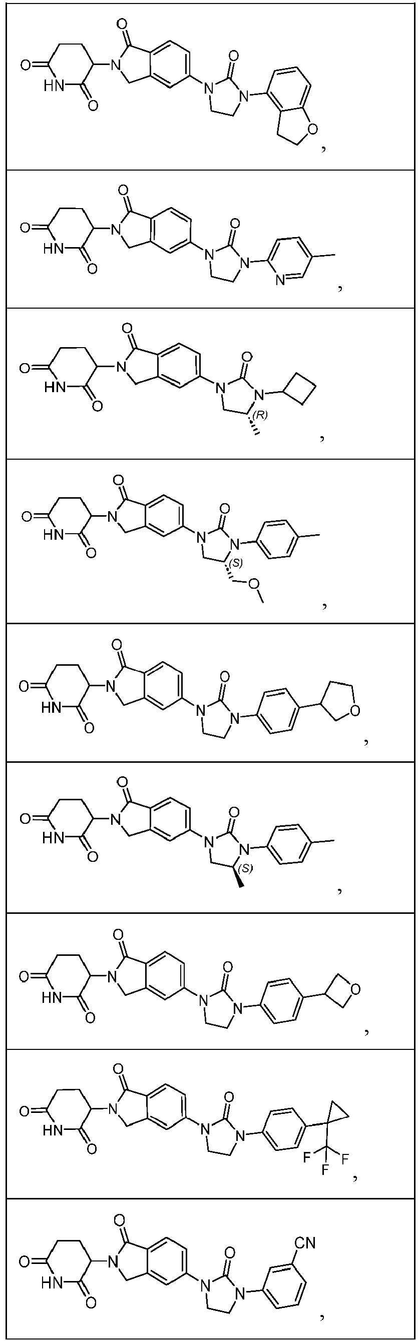 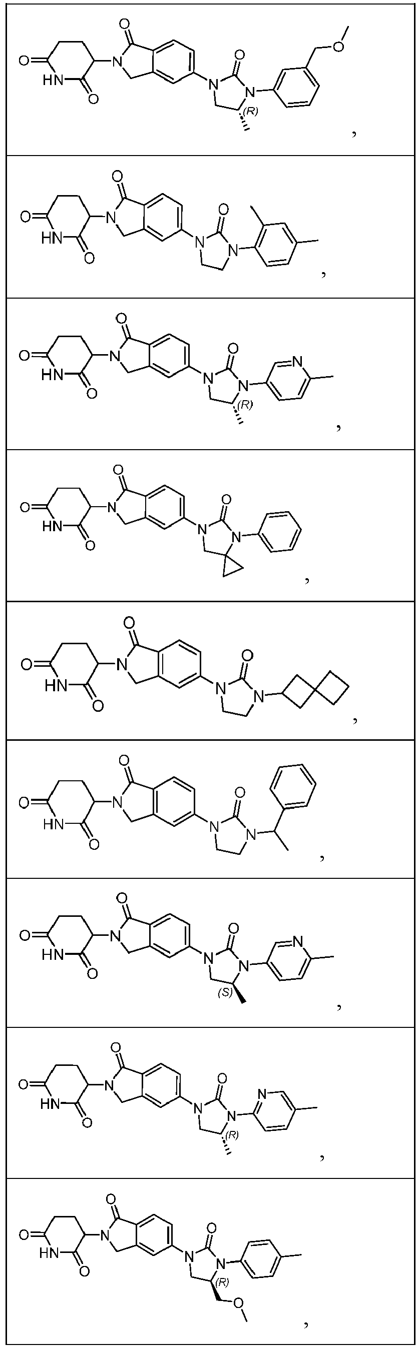 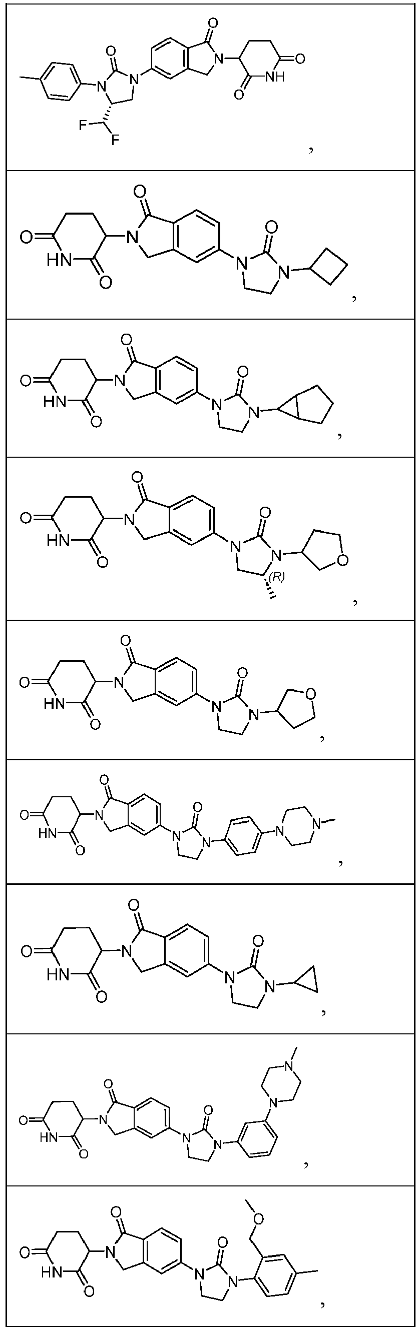 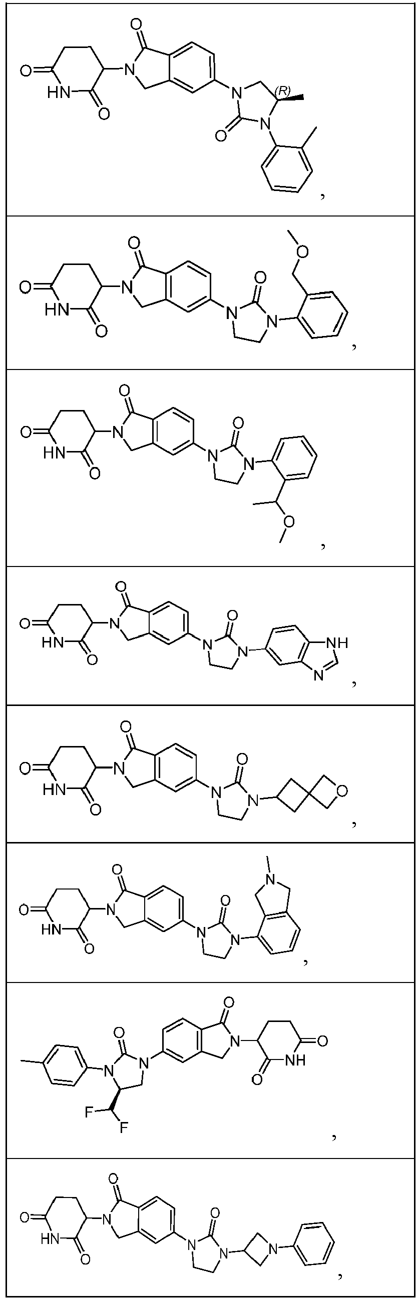 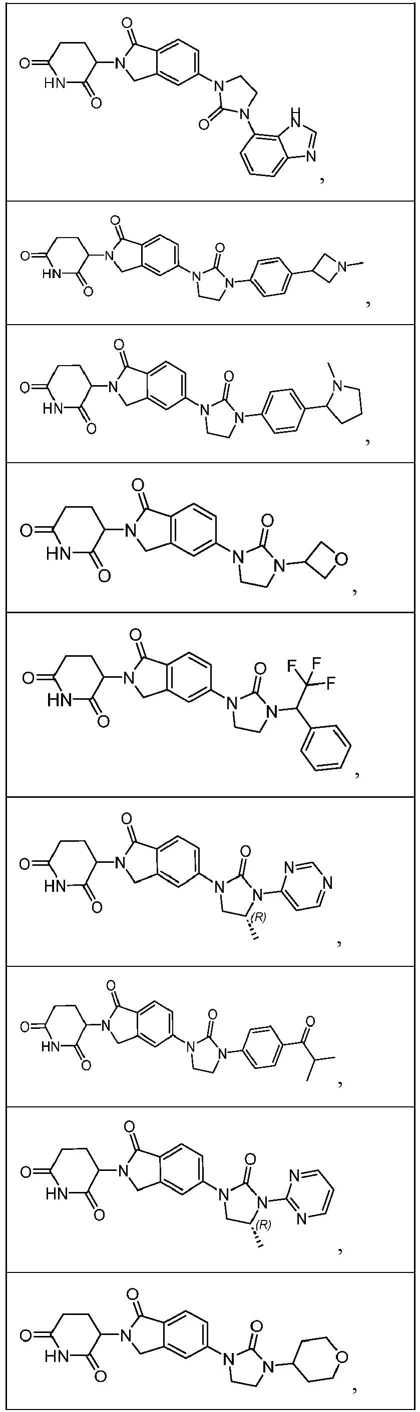 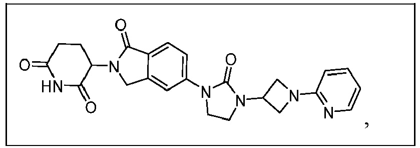 et un sel pharmaceutiquement acceptable de ceux-ci.

12. Composition pharmaceutique comprenant le composé selon l'une quelconque des revendications 1 à 11, ou un sel pharmaceutiquement acceptable correspondant, et un excipient pharmaceutiquement acceptable.

13. Composé selon l'une quelconque des revendications 1 à 11, ou sel pharmaceutiquement acceptable de celui-ci, ou composition pharmaceutique selon la revendication 12, pour utilisation dans un procédé de dégradation de CK1α chez un sujet souffrant d'un cancer, ledit procédé comprenant l'administration au sujet d'une quantité efficace du composé selon l'une quelconque des revendications 1 à 11, ou d'un sel pharmaceutiquement acceptable correspondant ou de la composition pharmaceutique selon la revendication 12.

14. Composé selon l'une quelconque des revendications 1 à 11, ou sel pharmaceutiquement acceptable correspondant, ou composition pharmaceutique selon la revendication 12 pour utilisation dans un procédé de traitement d'un cancer, d'une tumeur solide ou d'une tumeur liquide, chez un sujet en ayant besoin, ledit procédé comprenant l'administration au sujet d'une quantité thérapeutiquement efficace du composé selon l'une quelconque des revendications 1 à 11, ou d'un sel pharmaceutiquement acceptable correspondant ou de la composition pharmaceutique selon la revendication 12.

15. Composé, sel pharmaceutiquement acceptable ou composition pharmaceutique pour utilisation selon la revendication 13 ou 14, où le procédé comprend en outre l'administration au sujet d'un agent thérapeutique supplémentaire.
